# EUROPEAN PATENT APPLICATION

(11) **EP 4 818 155 A1**
(43) Date of publication of application: **07.10.2026**
(21) Application number: 24896639.2
(22) Date of filing: 28.11.2024
(51) Int. Cl.: C07D 207/452, A61K 47/50, C07K 16/28, A61P 35/00

(54) **AZOBENZENE-CONTAINING LINKER, PROTEIN-COUPLED DRUG CONTAINING LINKER, AND PHARMACEUTICAL USE THEREOF**

(30) Priority: 28.11.2023 CN 202311603188
(71) Applicant: ZHEJIANG YANGSHENGTANG INSTITUTE OF NATURAL MEDICATION CO., LTD., HANGZHOU, Zhejiang 310024 (CN)
(72) Inventor: XIAO, Dian, Hangzhou, Zhejiang 310024 (CN); ZHOU, Xinbo, Hangzhou, Zhejiang 310024 (CN); LI, Song, Hangzhou, Zhejiang 310024 (CN); ZHONG, Wu, Hangzhou, Zhejiang 310024 (CN); LIU, Lianqi, Hangzhou, Zhejiang 310024 (CN); XIE, Fei, Hangzhou, Zhejiang 310024 (CN); XIAO, Junhai, Hangzhou, Zhejiang 310024 (CN); ZHENG, Zhibing, Hangzhou, Zhejiang 310024 (CN); LI, Xingzhou, Hangzhou, Zhejiang 310024 (CN); FAN, Shiyong, Hangzhou, Zhejiang 310024 (CN); CAO, Ruiyuan, Hangzhou, Zhejiang 310024 (CN); LI, Wei, Hangzhou, Zhejiang 310024 (CN); LI, Pengyun, Hangzhou, Zhejiang 310024 (CN); WANG, Zihao, Hangzhou, Zhejiang 310024 (CN); YAN, Linjie, Hangzhou, Zhejiang 310024 (CN)
(74) Representative: PGA S.p.A., Milano, Succursale di Lugano
(86) International application number: PCT/CN2024/135215
(87) International publication number: WO 2025/113553

(57) **Abstract**

The present disclosure relates to an azobenzene-containing linker represented by formula I, a protein-coupled drug containing the linker, and a use of the linker, and also relates to a pharmaceutical composition containing the protein-coupled drug and further relates to a use of the protein-coupled drug for treating and/or preventing diseases.

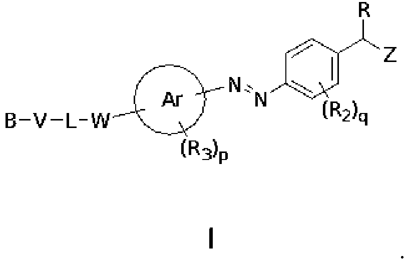

## Description

The present application is based on and claims the benefit of priority from Chinese Application No. 202311603188.4, filed on Nov. 28, 2023, the disclosures of which are incorporated herein by reference in its entirety.

### Technical Field

The present disclosure belongs to the field of medicinal chemistry, and specifically relates to an azobenzene-containing linker, a protein-drug conjugate containing the linker, also to a pharmaceutical composition comprising the protein-drug conjugate, and further to use of these protein-drug conjugates for treating and/or preventing diseases.

### Background Art

Antibody-drug conjugates (ADCs) are formed by covalently conjugating antibodies and effector molecules through linkers, and they integrate the advantages of strong targeting capability of antibodies and potent bioactivity of the effector molecules, and are known as "biological missiles" for precise delivery of effector molecules (Signal Transduction and Targeted Therapy, 2022, 7(1): 93).

Essentially, all commercially available ADCs are macromolecular prodrugs with targeting efficiency that is increased by 100 folds as compared to small molecular drugs (Clinical Cancer Research, 2015, 21(22): 5131-5138). A linker is a crucial factor that affects the therapeutic index of the ADCs. To date, two universally acknowledged principles for designing linkers, that is, high stability during blood circulation and efficient release of effector molecules in lesion sites, have achieved differential discrimination between blood and lesion sites to a certain extent (Nature Reviews Clinical Oncology, 2021, 18(6): 327-344; Chemical Society Reviews, 2019, 48(16): 4361-4374). Accordingly, highly efficient lytic enzymes widely distributed in human tissues, such as cathepsins and glucuronidases, are often taken into consideration for linker design of macromolecular conjugates (Cancer Res, 2017, 77(24): 7027-7037; Bioconjugate chemistry, 2006, 17(3): 831-840). However, the aforementioned lytic enzyme-responsive ADCs fail to achieve site-specific payload release at lesion sites, which readily leads to off-target toxicity and other associated drawbacks.

Specifically, ADCs have been widely used in the field of anti-tumor therapy. However, clinical research has demonstrated that less than 0.1% of ADCs injected into human bodies accumulate at lesion sites, with over 99% of the ADCs undergoing off-target distribution (Clinical cancer research, 2011, 17(20): 6389-6397; The AAPS journal, 2015, 17: 525-534). Therefore, ADCs distributed in normal tissues (such as the liver and the lung) inevitably undergo non-specific release of effector molecules (for example, MMAE) under the action of lytic enzymes, resulting in severe off-target toxicity (such as myelosuppression and hepatotoxicity), which further limit the escalation of clinical effective doses (Acta pharmaceutical Sinica B, 2020, 10(9): 1589-1600; Current opinion in immunology, 2016, 40: 14-23; Pharmacology & Therapeutics, 2019, 200: 110-125). Evidently, the existing linker technologies fail to really achieve the specific release of effector molecules at lesion sites and merely rely on the targeting capacity of antibodies, which results in unavoidable off-target of the effector molecules and limit the realization of the advantages of the ADCs.

On the other hand, antibiotic-resistant *Staphylococcus Aureus* remains a public health concern, and about 40% of bloodstream infections in the United States of America are caused by methicillin-resistant *Staphylococcus Aureus* (MRSA). There are few FDA-approved therapeutic options for MRSA bloodstream infections, and vancomycin remains the first-line antibiotic. Despite the adoption of appropriate antibiotic therapy, the mortality rate of *Staphylococcus Aureus* bloodstream infections remains about 18%, prompting research into combination therapies capable of improving therapeutic outcomes. The application of ADC technology in the antibacterial field is relatively limited. Reported antibacterial conjugates rely on host cells rather than microenvironment of lesion sites to release antibiotics to exert the antibacterial effects (Nature 527.7578(2015): 323-328), which limits the application of the ADC technology in the antibacterial field to a certain extent.

Accordingly, there is still a need to develop a new linker to address the aforementioned drawbacks.

### Summary of the Disclosure

The present disclosure provides an azobenzene-containing linker, and also provides a protein-drug conjugate containing the linker. Research has demonstrated that azoreductases are a class of reductive enzymes widely distributed in hypoxic tumors (Chemical Communications, 2019, 55(22): 3235-3238; Chemical Communications, 2019, 55(87): 13172-13175) and bacteria (Crit. Rev. Microbiol. 18: 175-190; Curr. Prot. Pept. Sci. 7: 101-111), while scarcely distributed in normal tissues. The protein-drug conjugate provided by the present disclosure can be specifically recognized by azoreductases, which reduce the azobenzene structure to trigger cleavage of the azo bond, thereby enabling site-specific drug release at lesion sites, to exert therapeutic effects against diseases.

### Linkers

The present disclosure provides a compound represented by Formula I, a geometric isomer, an optical isomer, a salt, a hydrate, a solvate, or a polymorph thereof, 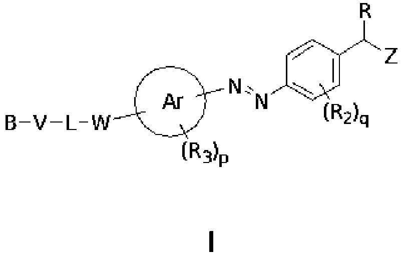
wherein 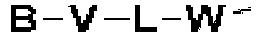 represents a linker, for conjugating an azobenzene group to a targeting compound,
Ar is a 5- or 6-membered aryl or heteroaryl;
R₂ and R₃ each independently are hydrogen, C₁₋₆ alkyl, fluoro, chloro, bromo, iodo, hydroxy, nitro, or cyano;
p is 0, 1, 2, or 3;
q is 0, 1, 2 or 3;
R is H, C₁₋₄ alkyl, 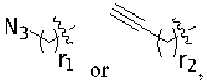 wherein r₁ and r₂ each independently are an integer from 1 to 12;
Z is hydroxy, fluoro, chloro, bromo, iodo or 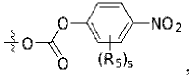 wherein s is 0, 1, 2, 3 or 4, and R₅ is hydrogen, fluoro, chloro, bromo, iodo, C₁₋₄ alkyl, nitro or C₁₋₄ alkoxy.

In some embodiments, in the compound represented by Formula I, B, V, L, and W in 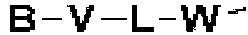 are defined as follows:
B is 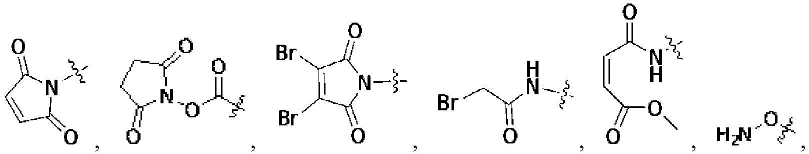 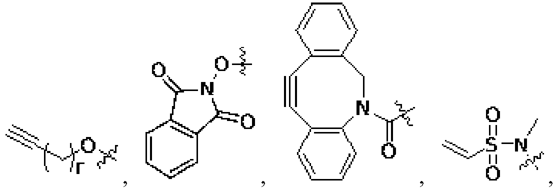 or H, wherein r is an integer from 1 to 4; or
B is selected from the group consisting of 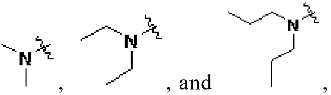 provided that V, L, W₁, W₂, and W₃ are simultaneously absent;
V is 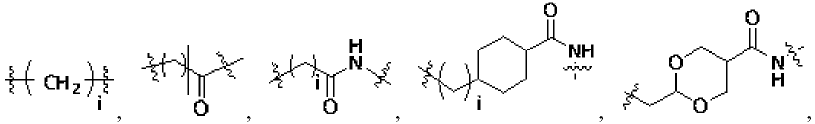 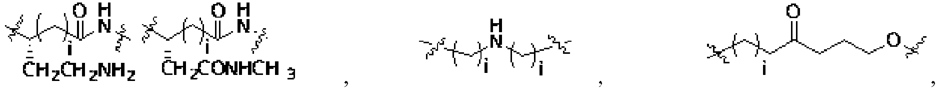 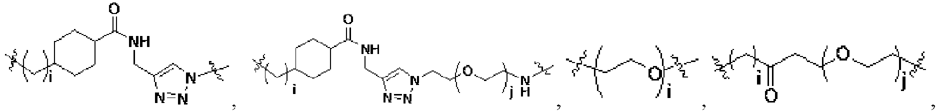 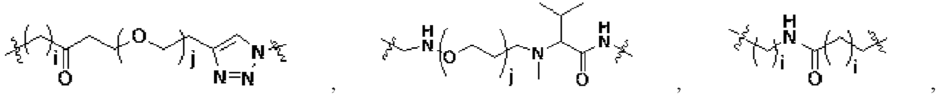 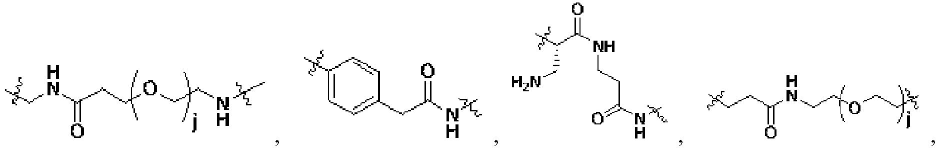 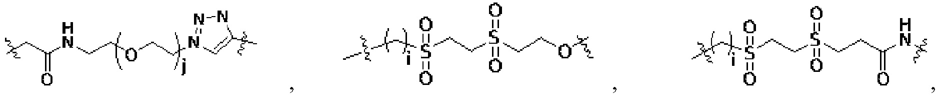 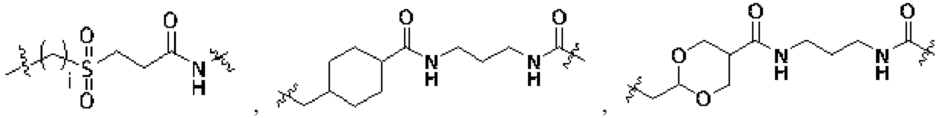 or absent, wherein each i independently is an integer from 0 to 6, and each j independently is an integer from 0 to 8;
L is -(CH₂CH₂O)ₖ-(CH₂)₁-, -CHR₁-, -(CH₂)ₘ -, 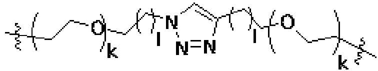 or absent, wherein R₁ is -(CH₂)ₙ-NHC(O)-(CH₂CH₂O)ₖ-CH₃, each k independently is an integer from 0 to 12, each 1 independently is an integer from 0 to 12, n is an integer from 0 to 12, and m is an integer from 0 to 30;
W is -W1-W2-W3-, wherein W1 is connected to L, W3 is connected to Ar, and W1 is selected from the group consisting of -O-, -S-, -NR₁₁-, -CONH-, -NHCO-, -S(O₂)NH-, and -NHS(O₂)-, or W1 is absent;W2 is selected from the group consisting of -(CH₂)ₒ-, and -[CH(R₁₁)CH(R₁₂)]ₒ-, or W2 is absent;W3 is selected from the group consisting of -O-, -S-, -NR₁₁-, -CONH-, -NHCO-, -S(O₂)NH-, and - NHS(O₂)-, or W3 is absent; wherein o is an integer from 0 to 6, each R₁₁ independently is selected from the group consisting of hydrogen and C₁₋₆ alkyl, and R₁₂ is selected from the group consisting of hydrogen and C₁₋₆ alkyl.

In some embodiments, in the compound represented by Formula I, W is selected from the group consisting of -NH-CH₂-C(O)-NH-, -NH-CH₂-CH₂-C(O)-NH-, -C(O)-NH-, -NH-C(O)-, -C(O)-NH-CH₂-C(O)-NH-, -C(O)-NH-CH₂-CH₂-C(O)-NH-, -C(O)-NH-, -NH-C(O)-, -NH-CH(R₁₀)-C(O)-NH-, - C(O)-NH-(CH₂)ₒ-NH-C(O)-, -C(O)-NH-[CH(R₁₁)CH(R₁₂)]ₒ-NH-C(O)-, -NH-(CH₂)ₒ-NH-C(O)-, and - NH-[CH(R₁₁)CH(R₁₂)]ₒ-NH-C(O)-, wherein R₁₀ is H, methyl or ethyl, and o, R₁₁ and R₁₂ are as defined in the present disclosure.

In some embodiments, in the compound represented by Formula I,
B is

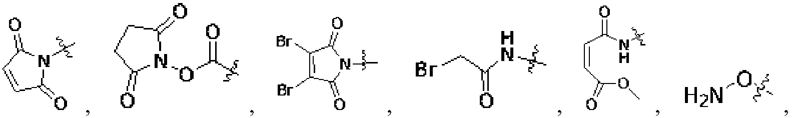

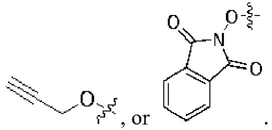

In some embodiments, in the compound represented by Formula I,
B is

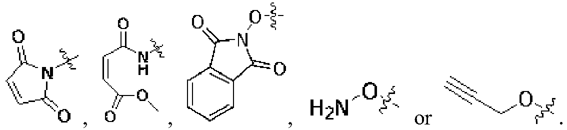

In some embodiments, in the compound represented by Formula I,
B is

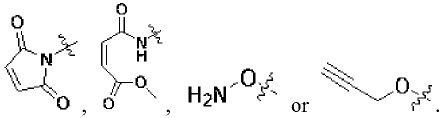

In some embodiments, in the compound represented by Formula I,
B is

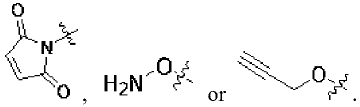

In some embodiments, in the compound represented by Formula I,
B is

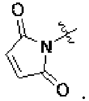

In some embodiments, in the compound represented by Formula I,
B is

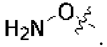

In some embodiments, in the compound represented by Formula I,
B is

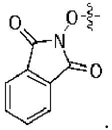

In some embodiments, in the compound represented by Formula I, V is 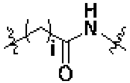 or absent, wherein i is as defined in the present disclosure. In some embodiments, i is 1, 2, 3, 4, 5 or 6. In some embodiments, i is 1. In some embodiments, i is 2. In some embodiments, i is 3. In some embodiments, i is 4. In some embodiments, i is 5. In some embodiments, i is 6.

In some embodiments, B is 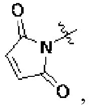 V is 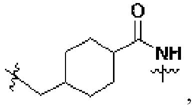 introducing a cyclohexyl group proximal to the maleimide can enhance the stability of maleimide-derived thioethers (Eur. J. Biochem. 101, 395-399 (1979), to reduce off-target effects caused by retro-Michael addition.

In some embodiments, B is 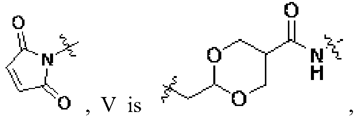 and on the above basis, introducing oxygen atoms into the cyclohexane group can further enhance the stability of maleimide-derived thioethers, and improve the water solubility of the linker (Sci Rep. 2016 Aug 9:6:30835.).

In some embodiments, B is 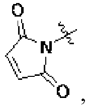 V is 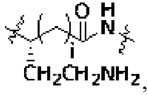 wherein i is as defined in the present disclosure, introducing an amino group proximal to the maleimide induces spontaneous hydrolytic ring-opening of the maleimide to form a stable ring-opened conjugated form (Bioconjugate Chem. 2015, 26, 1, 145-152).

In some embodiments, B is 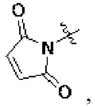 V is 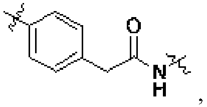 introducing an electron-withdrawing group benzene ring proximal to the maleimide induces spontaneous hydrolytic ring-opening of the maleimide to form a stable ring-opened conjugate form (Antibodies (Basel). 2017 Nov 28;6(4):20.).

In some embodiments, in the compound represented by Formula I, j is 1, 2, 3, 4, 5, 6, 7 or 8. In some embodiments, in the compound represented by Formula I, j is 2, 3, 4, 5, 6, 7 or 8. In some embodiments, in the compound represented by Formula I, j is 9 or 10.

In some embodiments, in the compound represented by Formula I, L is -(CH₂CH₂O)ₖ-(CH₂)ₗ-, - CHR₁- or -(CH₂)ₘ-, wherein k, l, R₁, and m are as defined in the present disclosure. In some embodiments, k is 1, 2, 3, 4, 5, 6, 7, 8, 9, 10, 11 or 12. In some embodiments, k is 1, 2, 3, 4, 5, 6, 7, 8, 9 or 10. In some embodiments, k is 1, 2, 3, 4, 5, 6, 7 or 8. In some embodiments, k is 2, 3, 4, 5, 6, 7 or 8. In some embodiments, l is 1, 2, 3, 4, 5, 6, 7, 8, 9 or 10. In some embodiments, l is 1, 2, 3, 4, 5, 6, 7 or 8. In some embodiments, l is 1, 2, 3, 4, 5 or 6. In some embodiments, l is 1, 2, 3 or 4. In some embodiments, l is 1. In some embodiments, l is 2. In some embodiments, l is 3. In some embodiments, l is 4. In some embodiments, l is 5. In some embodiments, l is 6. In some embodiments, n is 1, 2, 3, 4, 5, 6, 7, 8, 9 or 10. In some embodiments, n is 1, 2, 3, 4, 5, 6, 7 or 8. In some embodiments, n is 1, 2, 3, 4, 5 or 6. In some embodiments, n is 1, 2, 3 or 4. In some embodiments, n is 1. In some embodiments, n is 2. In some embodiments, n is 3. In some embodiments, n is 4. In some embodiments, n is 5. In some embodiments, n is 6. In some embodiments, m is an integer from 1 to 20. In some embodiments, m is an integer from 1 to 15. In some embodiments, m is an integer from 1 to 10. In some embodiments, m is an integer from 1 to 8. In some embodiments, m is an integer from 1 to 6. In some embodiments, m is an integer from 1 to 4.

In some embodiments, in the compound represented by Formula I, W is -C(O)-NH-(CH₂)ₒ-NH-C(O)- or -C(O)-NH-[CH(R₁₁)CH(R₁₂)]ₒ-NH-C(O)-, and o, R₁₁ and R₁₂ are as defined in the present disclosure.

In some embodiments, in the compound represented by Formula I,-V-L-W- is -(CH₂)ₘ-C(O)-NH-(CH₂)ₒ-NH-C(O)-, -(CH₂)ᵢ-C(O)-NH-(CH₂CH₂O)ₖ-(CH₂)ₗ-C(O)-NH-(CH₂)ₒ-NH-C(O)-, -(CH₂)ᵢ-C(O)-NH-CHR₁-C(O)-NH-(CH₂)ₒ-NH-C(O)-, or -(CH₂CH₂O)ₖ-(CH₂)ₗ-C(O)-NH-(CH₂)ₒ-NH-C(O)-, wherein R₁ is -(CH₂)ₙ-NHC(O)-(CH₂CH₂O)ₖ-CH₃, and m, o, i, k, l, and n are as defined in the present disclosure.

In some embodiments, in the compound represented by Formula I, each i independently is 1, 2, 3 or 4; each k independently is 1, 2, 3, 4, 5, 6, 7, 8, 9, 10, 11 or 12; each m independently is 1, 2, 3 or 4; each o independently is 1, 2, 3 or 4; each l independently is 1, 2, 3 or 4; n is 1, 2, 3, 4, 5, 6, 7 or 8.

In some embodiments, in the compound represented by Formula I, each i independently is 1, 2 or 3; each k independently is 1, 2, 3, 4, 5, 6, 7, 8, 9 or 10; each m independently is 1, 2 or 3; each o independently is 1, 2 or 3; each l independently is 1, 2 or 3; n is 1, 2, 3, 4 or 5.

In some embodiments, in the compound represented by Formula I, -V-L-W- is -(CH₂)₂-C(O)-NH-(CH₂)₂-NH-C(O)-, -(CH₂)₂-C(O)NH-(CH₂CH₂O)₂-CH₂-CH₂-C(O)-NH-(CH₂)₂-NH-C(O)-, -(CH₂)₂-C(O)NH-(CH₂CH₂O)₄-CH₂-CH₂-C(O)-NH-(CH₂)₂-NH-C(O)-, -(CH₂)₂-C(O)NH-(CH₂CH₂O)₆-CH₂-CH₂-C(O)-NH-(CH₂)₂-NH-C(O)-, -(CH₂)₂-C(O)NH-(CH₂CH₂O)₈-CH₂-CH₂-C(O)-NH-(CH₂)₂-NH-C(O)-, - (CH₂CH₂O)₃-(CH₂)₂-C(O)-NH-(CH₂)₂-NH-C(O)-, -(CH₂)₂-C(O)NH-CH[(CH₂)₄-NHC(O)-(CH₂CH₂O)₈-CH₃]-C(O)-NH-(CH₂)₂-NH-C(O)-, or -(CH₂CH₂O)₇-(CH₂)₂-C(O)-NH-(CH₂)₂-NH-C(O)-.

In some embodiments, in the compound represented by Formula I, Ar is phenyl, pyridyl, pyrimidinyl, furanyl, imidazolyl, pyrrolyl, thiazolyl, pyrazolyl or thienyl. In some embodiments, in the compound of formula I, Ar is phenyl, furanyl, imidazolyl or thienyl. In some embodiments, in the compound of formula I, Ar is phenyl.

In some embodiments, in the compound represented by Formula I, R is H or C₁₋₄ alkyl. In some embodiments, in the compound represented by Formula I, R is H, methyl, ethyl, n-propyl or n-butyl. In some embodiments, in the compound represented by Formula I, R is H, methyl, ethyl or n-propyl. In some embodiments, in the compound represented by Formula I, R is H, methyl or ethyl. In some embodiments, in the compound represented by Formula I, R is H. In some embodiments, in the compound represented by Formula I, R is methyl.

In some embodiments, in the compound represented by Formula I, R₂ and R₃ each independently are hydrogen, C₁₋₄ alkyl, fluoro, chloro, bromo, iodo, hydroxyl, nitro or cyano. In some embodiments, in the compound represented by Formula I, R₂ and R₃ each independently are hydrogen, methyl, ethyl, n-propyl, iso-propyl, n-butyl, iso-butyl, sec-butyl, or tert-butyl. In some embodiments, in the compound represented by Formula I, R₂ and R₃ each independently are fluoro, chloro, bromo, iodo, hydroxyl, nitro or cyano. In some embodiments, in the compound represented by Formula I, R₂ and R₃ each independently are hydrogen.

In some embodiments, in the compound represented by Formula I, q is 0, 1 or 2, preferably is 0 or 1.

In some embodiments, in the compound represented by Formula I, p is 0, 1 or 2, preferably is 0 or 1.

In some embodiments, in the compound represented by Formula I, Z is hydroxyl, fluoro, chloro, bromo, iodo or 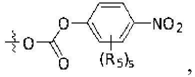 wherein s is 0, 1, 2, 3 or 4; R₅ is hydrogen, fluoro, chloro, bromo, iodo, methyl, ethyl, n-propyl, iso-propyl, n-butyl, iso-butyl, sec-butyl, tert-butyl, nitro, methoxy, ethoxy, n-propoxy, iso-propoxy, n-butoxy, iso-butoxy, sec-butoxy or tert-butoxy. In some embodiments, s is 0, 1 or 2, preferably is 0 or 1. In some embodiments, R₅ is hydrogen, fluoro, chloro, bromo, iodo, methyl, ethyl or n-propyl. In some embodiments, R₅ is nitro, methoxy, ethoxy, or n-propoxy.

In some embodiments, in the compound represented by Formula I, Z is chloro, OH or 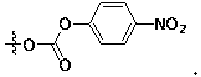 In some embodiments, in the compound represented by Formula I, Z is OH or 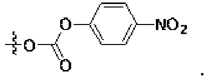 In some embodiments, in the compound represented by Formula I, Z is 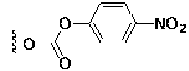 . In some embodiments, in the compound represented by Formula I, Z is chloro. In some embodiments, in the compound represented by Formula I, Z is OH.

In some embodiments, Z is 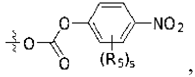 wherein R₅ is hydrogen, fluoro, chloro, bromo, iodo, C₁₋₄ alkyl, nitro or C₁₋₄ alkoxy, and s is 0, 1, 2, 3 or 4. In some embodiments, s is 0. In some embodiments, s is 1. In some embodiments, s is 2. In some embodiments, s is 3. In some embodiments, s is 4. In some embodiments, R₅ is hydrogen. In some embodiments, R₅ is fluoro. In some embodiments, R₅ is chloro. In some embodiments, R₅ is bromo. In some embodiments, R₅ is iodo. In some embodiments, R₅ is C₁₋₄ alkyl. In some embodiments, R₅ is nitro. In some embodiments, R₅ is C₁₋₄ alkoxy. In some embodiments, R₅ is methyl. In some embodiments, R₅ is ethyl. In some embodiments, R₅ is propyl. In some embodiments, R₅ is methoxy. In some embodiments, R₅ is ethoxy. In some embodiments, R₅ is propoxy.

In some embodiments, the compound represented by Formula I has the structure represented by Formula I-1, 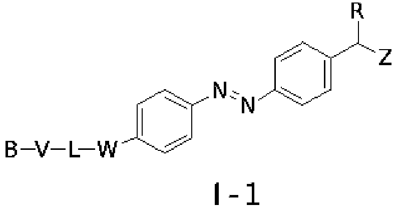 wherein B, V, L, W, R, Z are as defined in the present disclosure.

In some embodiments, in the compound represented by Formula I, B is selected from the group consisting of 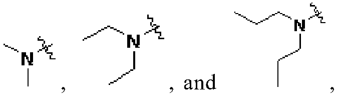 and V, L, W1, W2, and W3 each are absent; R is 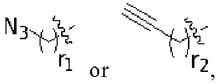 wherein r₁ and r₂ are as defined in the present disclosure. In some embodiments, r₁ and r₂ each independently are 1, 2, 3, 4, 5, 6, 7, 8, 9, 10, 11 or 12. In some embodiments, r₁ and r₂ each independently are 1, 2, 3, 4, 5, 6, 7, 8, 9 or 10. In some embodiments, r₁ and r₂ each independently are 1, 2, 3, 4, 5, 6, 7 or 8. In some embodiments, r₁ and r₂ each independently are 1, 2, 3, 4, 5 or 6. In some embodiments, r₁ and r₂ each independently are 1, 2, 3 or 4.

In some embodiments, the compound represented by Formula I is selected from the group consisting of: 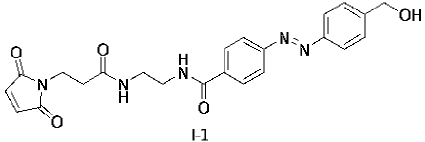 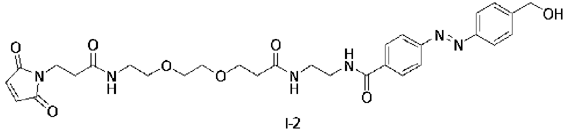 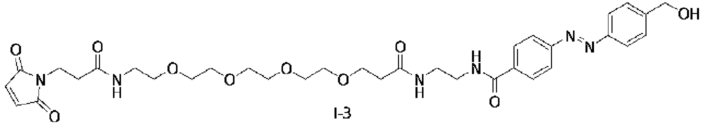 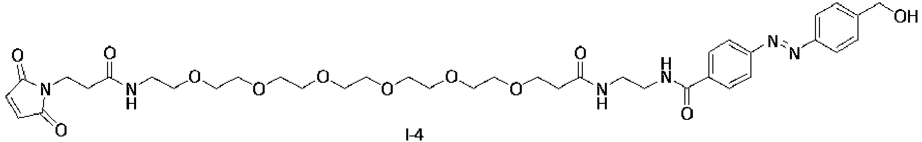 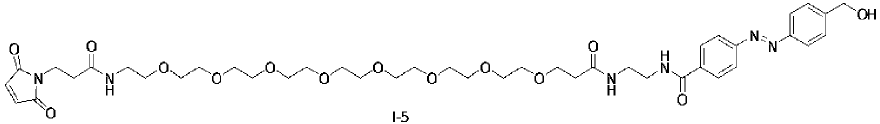 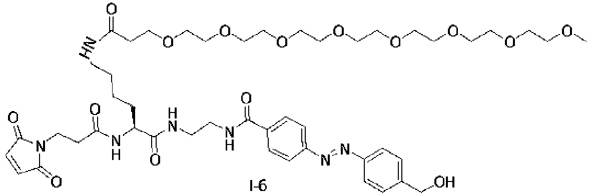 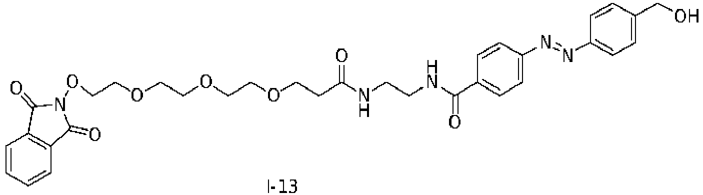 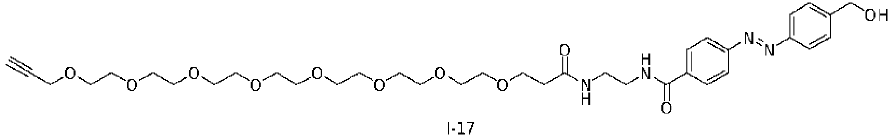 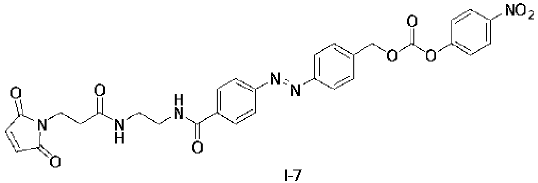 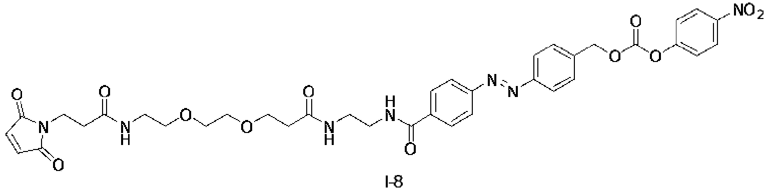 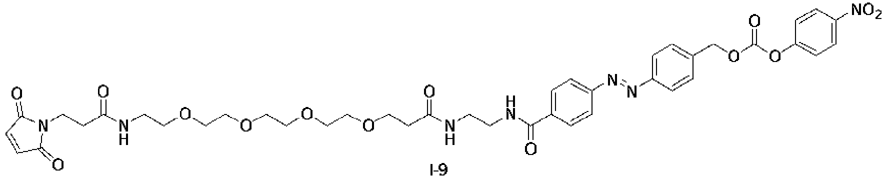 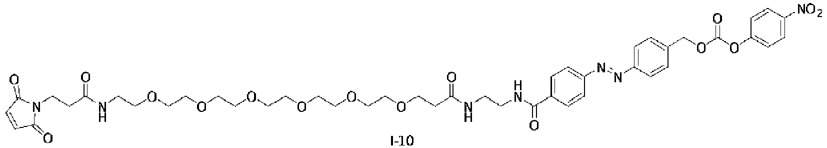 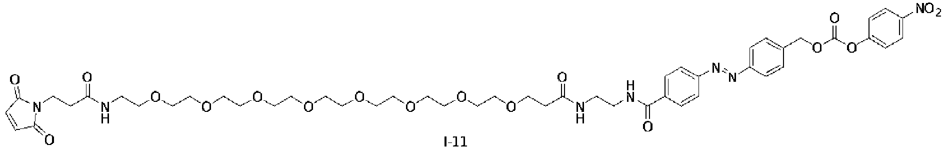 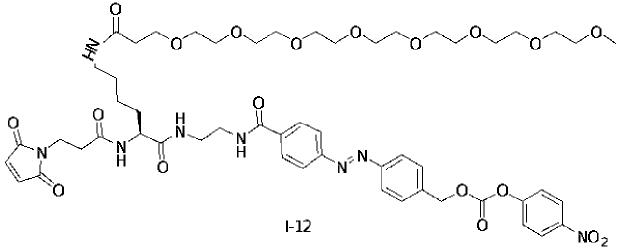 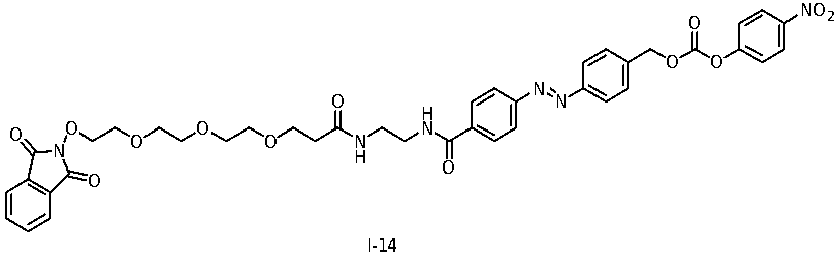 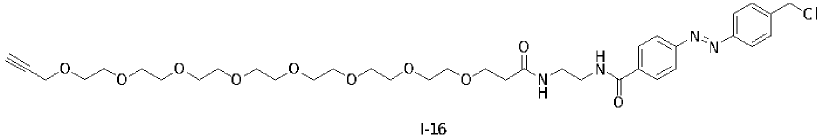 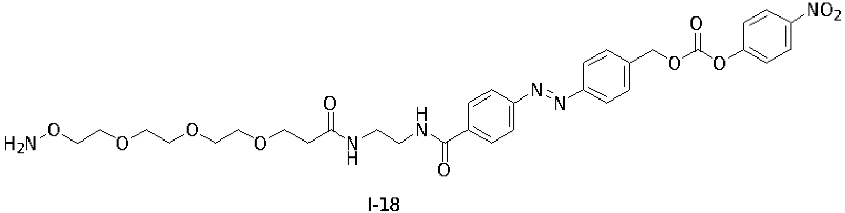 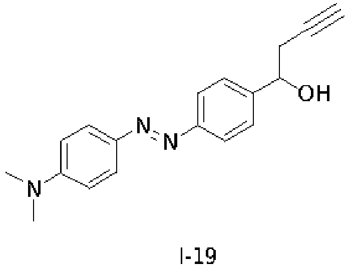 and

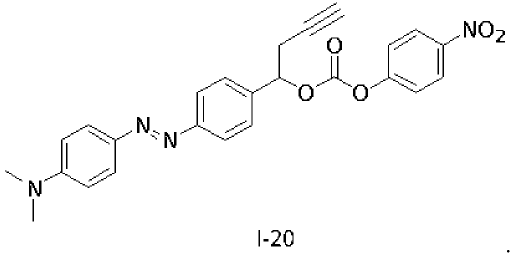

The linker provided by the present disclosure contains an azobenzene structure, which can be specifically recognized and reduced by azoreductases, resulting in cleavage of the azo bond.

The present disclosure also provides use of the compound represented by Formula I, a geometric isomer, an optical isomer, a salt, a hydrate, a solvate or a polymorph thereof in the manufacture of an antibody-drug conjugate.

### Linker-drug conjugates

The present disclosure also provides a compound represented by Formula II, a geometric isomer, an optical isomer, a salt, a hydrate, a solvate or a polymorph thereof, 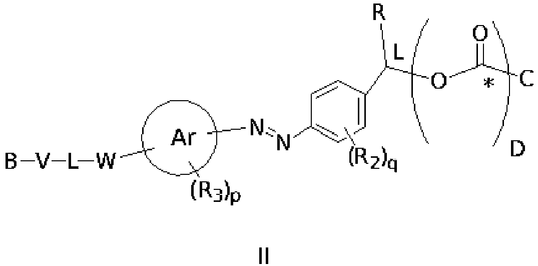
wherein: 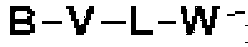 represents a linker, for conjugating an azobenzene group to a targeting compound,
Ar is a 5- or 6-membered aryl or heteroaryl;
R₂ and R₃ each independently are hydrogen, C₁₋₆ alkyl, fluoro, chloro, bromo, iodo, hydroxyl, nitro or cyano;
p is 0, 1, 2, or 3;
q is 0, 1, 2 or 3;
R is hydrogen, C₁₋₄ alkyl, 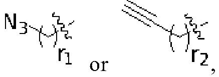 wherein r₁ and r₂ each independently are an integer from 1 to 12;
D is 0 or 1;
C is an active compound, selected from the group consisting of a drug, a cytotoxin, a detection reagent, a diagnostic reagent, and a targeting carrier; preferably, C is an antitumor drug, an antiviral drug, an antibiotic, an antibacterial drug or an immunomodulatory drug; further preferably, C is an antitumor drug, such as a small molecule, a peptide or a nucleotide including a cytotoxin, a tubulin inhibitor, a DNA alkylating agent, a DNA intercalating agent, an RNA polymerase inhibitor, a kinase inhibitor, a topoisomerase inhibitor, a kinesin spindle protein inhibitor, and an antimetabolite drug, an immunomodulatory drug such as a Toll-like receptor agonist (e.g., a TLR7 agonist) or a STING agonist, or an antibacterial drug
such as rifampicin and an analog thereof (e.g., rifalogue, KRM-1657);
C is conjugated to the carbonyl group (i.e. the position *) or the C atom at the position L via the N atom or the O atom in the active compound molecule.

In some embodiments, in the compound represented by Formula II, B, V, L, and W in 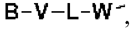 are as defined as follows:
B is 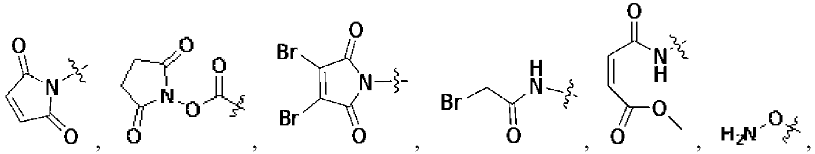 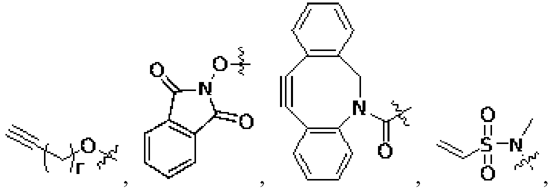 or H, wherein r is 1-4; or
B is selected from the group consisting of 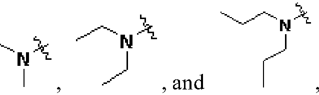 provided that V, L, W1, W2, and W3 are simultaneously absent;
V is 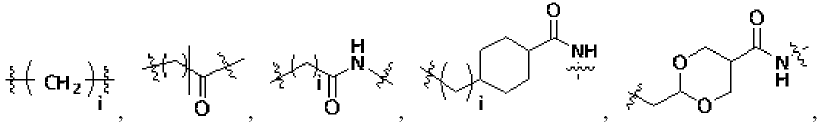 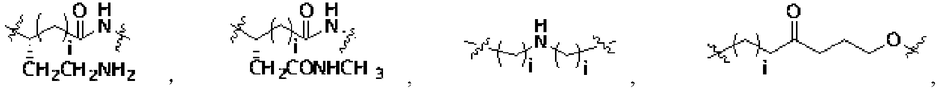 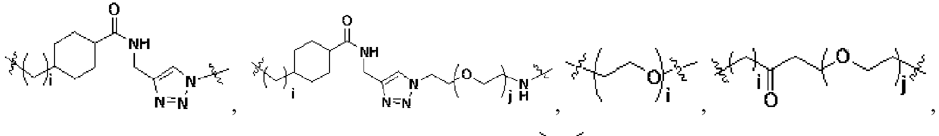 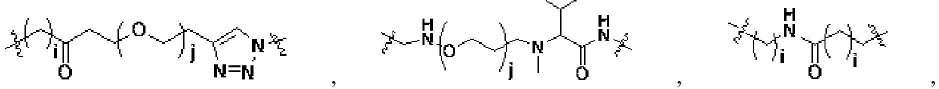 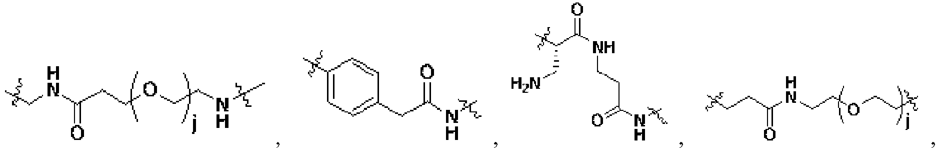 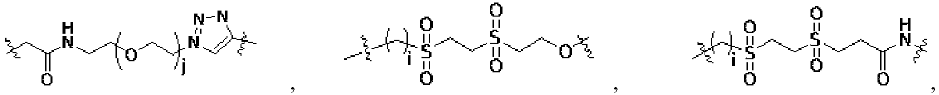 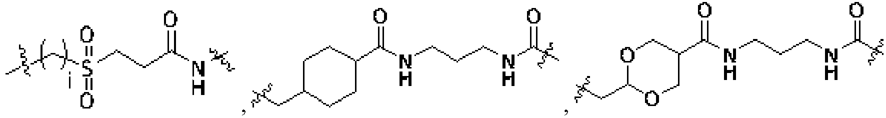 or absent, wherein each i independently is an integer from 0 to 6, and each j independently is an integer from 0 to 8;
L is -(CH₂CH₂O)ₖ-(CH₂)₁-, -CHR₁-, -(CH₂)ₘ -, 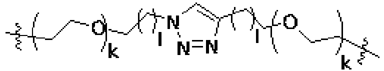 or absent, wherein R₁ is -(CH₂)ₙ-NHC(O)-(CH₂CH₂O)ₖ-CH₃, each k independently is an integer from 0 to 12, each l independently is an integer from 0 to 12, n is an integer from 0 to 12, and m is an integer from 0 to 30;
W is -W1-W2-W3-, wherein W1 is connected to L, W3 is connected to Ar, and W1 is selected from the group consisting of -O-, -S-, -NR₁₁-, -CONH-, -NHCO-, -S(O₂)NH-, and -NHS(O₂)-, or W1 is absent; W2 is selected from the group consisting of -(CH₂)ₒ-, and -[CH(R₁₁)CH(R₁₂)]ₒ-, or W2 is absent; W3 is selected from the group consisting of -O-, -S-, -NR₁₁-, -CONH-, -NHCO-, -S(O₂)NH-, and - NHS(O₂)-, or W3 is absent; wherein o is an integer from 0 to 6, each R₁₁ independently is selected from the group consisting of hydrogen and C₁₋₆ alkyl, and R₁₂ is selected from the group consisting of hydrogen and C₁₋₆ alkyl.

In some embodiments, in the compound represented by Formula II, W is selected from the group consisting of -NH-CH₂-C(O)-NH-, -NH-CH₂-CH₂-C(O)-NH-, -C(O)-NH-, -NH-C(O)-, -C(O)-NH-CH₂-C(O)-NH-, -C(O)-NH-CH₂-CH₂-C(O)-NH-, -C(O)-NH-, -NH-C(O)-, -NH-CH(R₁₀)-C(O)-NH-, - C(O)-NH-(CH₂)ₒ-NH-C(O)-, -C(O)-NH-[CH(R₁₁)CH(R₁₂)]ₒ-NH-C(O)-, -NH-(CH₂)ₒ-NH-C(O)-, and - NH-[CH(R₁₁)CH(R₁₂)]ₒ-NH-C(O)-, wherein R₁₀ is hydrogen, methyl or ethyl, and o, R₁₁ and R₁₂ are as defined in the present disclosure.

In some embodiments, in the compound represented by Formula II, B is

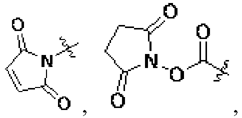

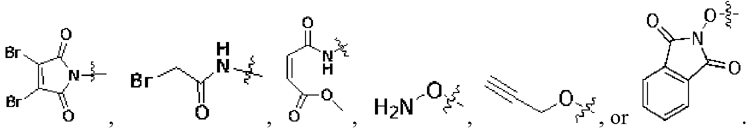

In some embodiments, in the compound represented by Formula II, B is

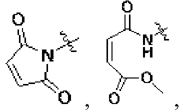

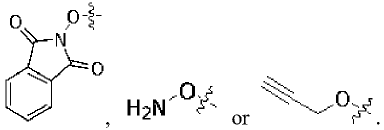

In some embodiments, in the compound represented by Formula II,
B is

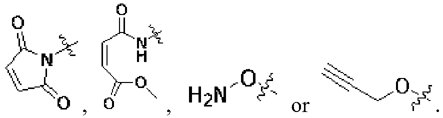

In some embodiments, in the compound represented by Formula II,
B is

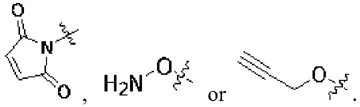

In some embodiments, in the compound represented by Formula II,
B is

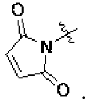

In some embodiments, in the compound represented by Formula II,
B is

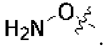

In some embodiments, in the compound represented by Formula II,
B is

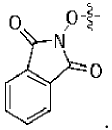

In some embodiments, in the compound represented by Formula II, V is 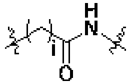 or absent, wherein i is as defined in the present disclosure. In some embodiments, i is 1, 2, 3, 4, 5 or 6. In some embodiments, i is 1. In some embodiments, i is 2. In some embodiments, i is 3. In some embodiments, i is 4. In some embodiments, i is 5. In some embodiments, i is 6.

In some embodiments, in the compound represented by Formula II, j is 1, 2, 3, 4, 5, 6, 7 or 8. In some embodiments, in the compound represented by Formula II, j is 2, 3, 4, 5, 6, 7 or 8. In some embodiments, in the compound represented by Formula II, j is 9 or 10.

In some embodiments, in the compound represented by Formula II, L is -(CH₂CH₂O)ₖ-(CH₂)ₗ-, - CHR₁- or -(CH₂)ₘ-, wherein k, l, R₁, and m are as defined in the present disclosure. In some embodiments, k is 1, 2, 3, 4, 5, 6, 7, 8, 9, 10, 11 or 12. In some embodiments, k is 1, 2, 3, 4, 5, 6, 7, 8, 9 or 10. In some embodiments, k is 1, 2, 3, 4, 5, 6, 7 or 8. In some embodiments, k is 2, 3, 4, 5, 6, 7 or 8. In some embodiments, l is 1, 2, 3, 4, 5, 6, 7, 8, 9 or 10. In some embodiments, l is 1, 2, 3, 4, 5, 6, 7 or 8. In some embodiments, l is 1, 2, 3, 4, 5 or 6. In some embodiments, l is 1, 2, 3 or 4. In some embodiments, l is 1. In some embodiments, l is 2. In some embodiments, l is 3. In some embodiments, l is 4. In some embodiments, l is 5. In some embodiments, l is 6. In some embodiments, n is 1, 2, 3, 4, 5, 6, 7, 8, 9 or 10. In some embodiments, n is 1, 2, 3, 4, 5, 6, 7 or 8. In some embodiments, n is 1, 2, 3, 4, 5 or 6. In some embodiments, n is 1, 2, 3 or 4. In some embodiments, n is 1. In some embodiments, n is 2. In some embodiments, n is 3. In some embodiments, n is 4. In some embodiments, n is 5. In some embodiments, n is 6. In some embodiments, m is an integer from 1 to 20. In some embodiments, m is an integer from 1 to 15. In some embodiments, m is an integer from 1 to 10. In some embodiments, m is an integer from 1 to 8. In some embodiments, m is an integer from 1 to 6. In some embodiments, m is an integer from 1 to 4.

In some embodiments, in the compound represented by Formula II, W is -C(O)-NH-(CH₂)ₒ-NH-C(O)- or -C(O)-NH-[CH(R₁₁)CH(R₁₂)]ₒ-NH-C(O)-, and o, R₁₁ and R₁₂ are as defined in the present disclosure. In some embodiments, o is an integer from 0 to 4. In some embodiments, o is 1, 2, 3 or 4. In some embodiments, o is 1. In some embodiments, o is 2. In some embodiments, o is 3. In some embodiments, o is 4. In some embodiments, R₁₁ and R₁₂ each independently are hydrogen. In some embodiments, R₁₁ and R₁₂ each independently are C₁₋₆ alkyl. In some embodiments, R₁₁ and R₁₂ each independently are C₁₋₄ alkyl. In some embodiments, R₁₁ and R₁₂ each independently are methyl, ethyl, n-propyl, iso-propyl, n-butyl, iso-butyl, sec-butyl or tert-butyl. In some embodiments, R₁₁ and R₁₂ each independently are methyl, ethyl or n-propyl.

In some embodiments, in the compound represented by Formula II, -V-L-W- is -(CH₂)ₘ-C(O)-NH-(CH₂)ₒ-NH-C(O)-, -(CH₂)ᵢ-C(O)-NH-(CH₂CH₂O)ₖ-(CH₂)ₗ-C(O)-NH-(CH₂)ₒ-NH-C(O)-, -(CH₂)ᵢ-C(O)-NH-CHR₁-C(O)-NH-(CH₂)ₒ-NH-C(O)-, or -(CH₂CH₂O)ₖ-(CH₂)ₗ-C(O)-NH-(CH₂)ₒ-NH-C(O)-, wherein R₁ is -(CH₂)ₙ-NHC(O)-(CH₂CH₂O)ₖ-CH₃, and m, o, i, k, l, and n are as defined in the present disclosure.

In some embodiments, in the compound represented by Formula II, each i independently is 1, 2, 3 or 4; each k independently is 1, 2, 3, 4, 5, 6, 7, 8, 9, 10, 11 or 12; each m independently is 1, 2, 3 or 4; each o independently is 1, 2, 3 or 4; each l independently is 1, 2, 3 or 4; n is 1, 2, 3, 4, 5, 6, 7 or 8.

In some embodiments, in the compound represented by Formula II, each i independently is 1, 2 or 3; each k independently is 1, 2, 3, 4, 5, 6, 7, 8, 9 or 10; each m independently is 1, 2 or 3; each o independently is 1, 2 or 3; each l independently is 1, 2 or 3; n is 1, 2, 3, 4 or 5.

In some embodiments, in the compound represented by Formula II, -V-L-W- is -(CH₂)₂-C(O)-NH-(CH₂)₂-NH-C(O)-, -(CH₂)₂-C(O)NH-(CH₂CH₂O)₂-CH₂-CH₂-C(O)-NH-(CH₂)₂-NH-C(O)-, -(CH₂)₂-C(O)NH-(CH₂CH₂O)₄-CH₂-CH₂-C(O)-NH-(CH₂)₂-NH-C(O)-, -(CH₂)₂-C(O)NH-(CH₂CH₂O)₆-CH₂-CH₂-C(O)-NH-(CH₂)₂-NH-C(O)-, -(CH₂)₂-C(O)NH-(CH₂CH₂O)₈-CH₂-CH₂-C(O)-NH-(CH₂)₂-NH-C(O)-, - (CH₂CH₂O)₃-(CH₂)₂-C(O)-NH-(CH₂)₂-NH-C(O)-, -(CH₂)₂-C(O)NH-CH[(CH₂)₄-NHC(O)-(CH₂CH₂O)₈-CH₃]-C(O)-NH-(CH₂)₂-NH-C(O)-, or -(CH₂CH₂O)₇-(CH₂)₂-C(O)-NH-(CH₂)₂-NH-C(O)-.

In some embodiments, in the compound represented by Formula II, Ar is phenyl, pyridyl, pyrimidinyl, furanyl, imidazolyl, pyrrolyl, thiazolyl, pyrazolyl or thienyl. In some embodiments, in the compound represented by Formula II, Ar is phenyl, furanyl, imidazolyl or thienyl. In some embodiments, in the compound represented by Formula II, Ar is phenyl.

In some embodiments, in the compound represented by Formula II, R is H or C₁₋₄ alkyl. In some embodiments, in the compound represented by Formula II, R is H, methyl, ethyl, n-propyl or n-butyl. In some embodiments, in the compound represented by Formula II, R is H, methyl, ethyl or n-propyl. In some embodiments, in the compound represented by Formula II, R is H, methyl or ethyl. In some embodiments, in the compound represented by Formula II, R is H. In some embodiments, in the compound represented by Formula II, R is methyl.

In some embodiments, in the compound represented by Formula II, R₂ and R₃ each independently are hydrogen, C₁₋₄ alkyl, fluoro, chloro, bromo, iodo, hydroxyl, nitro or cyano. In some embodiments, in the compound represented by Formula II, R₂ and R₃ each independently are hydrogen, methyl, ethyl, n-propyl, iso-propyl, n-butyl, iso-butyl, sec-butyl, or tert-butyl. In some embodiments, in the compound represented by Formula II, R₂ and R₃ each independently are fluoro, chloro, bromo, iodo, hydroxyl, nitro or cyano. In some embodiments, in the compound represented by Formula II, R₂ and R₃ each independently are hydrogen.

In some embodiments, in the compound represented by Formula II, q is 0, 1 or 2, preferably is 0 or 1.

In some embodiments, in the compound represented by Formula II, p is 0, 1 or 2, preferably is 0 or 1.

In some embodiments, in the compound represented by Formula II, C is selected from the group consisting of: auristatin, monomethyl auristatin E (MMAE), monomethyl auristatin F (MMAF) and a derivative thereof, maytansine and a derivative thereof (such as maytansinoids, DM1, DM3, DM4), paclitaxel, calicheamicin, duocarmycin, doxorubicin, camptothecin, PBD (pyrrolobenzodiazepines) cytotoxin and a derivative thereof, SN-38, exatecan, doxorubicin, a TLR7 agonist with CAS No. 1821304-87-3, lapatinib, rifampicin and an analog thereof (for example, rifalogue, KRM-1657).

In some embodiments, in the compound represented by Formula II, C is monomethyl auristatin E (MMAE), rifampicin or rifalogue.

In some embodiments, in the compound represented by Formula II, C is monomethyl auristatin E (MMAE) or rifalogue.

In some embodiments, in the compound represented by Formula II, C is SN-38, exatecan, doxorubicin, a TLR7 agonist with CAS No. 1821304-87-3, lapatinib, MMAE, an MMAF derivative or rifalogue.

In some embodiments, in the compound represented by Formula II, C is an MMAF derivative, having a structure:

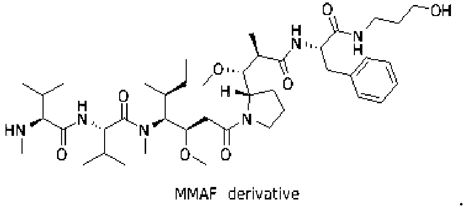

In some embodiments, the compound represented by Formula II has a structure represented by Formula II-1, 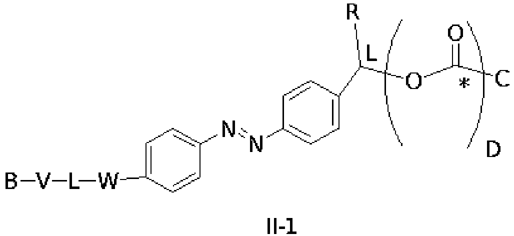 wherein B, V, L, W, R, C, and D are as defined in the present disclosure.

In some embodiments, in the compound represented by Formula II, B is selected from the group consisting of 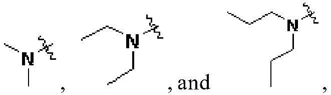 and V, L, W1, W2, and W3 each are absent; R is 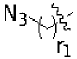 or 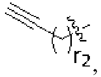 wherein r₁ and r₂ are as defined in the present disclosure. In some embodiments, r₁ and r₂ each independently are 1, 2, 3, 4, 5, 6, 7, 8, 9, 10, 11 or 12. In some embodiments, r₁ and r₂ each independently are 1, 2, 3, 4, 5, 6, 7, 8, 9 or 10. In some embodiments, r₁ and r₂ each independently are 1, 2, 3, 4, 5, 6, 7 or 8. In some embodiments, r₁ and r₂ each independently are 1, 2, 3, 4, 5 or 6. In some embodiments, r₁ and r₂ each independently are 1, 2, 3 or 4.

In some embodiments, the compound represented by Formula II is selected from the group consisting of: 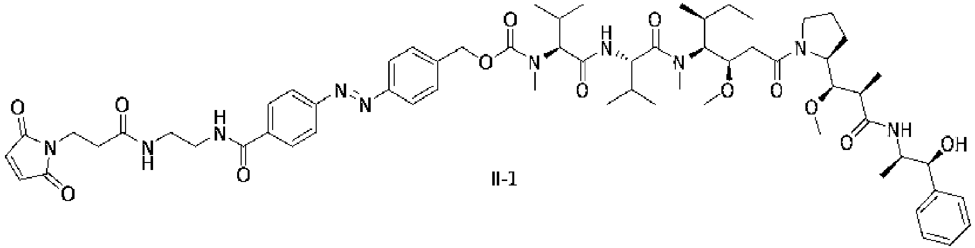 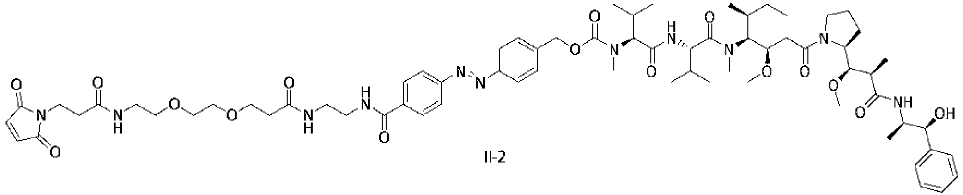 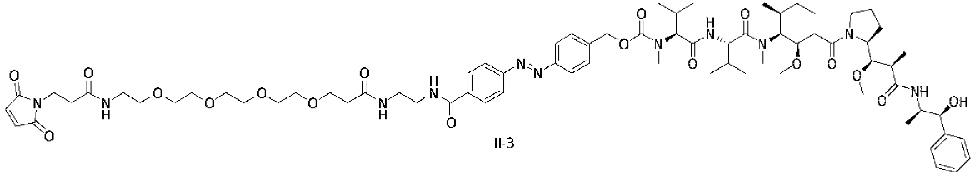 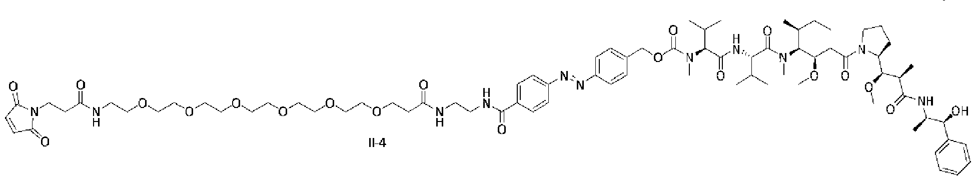 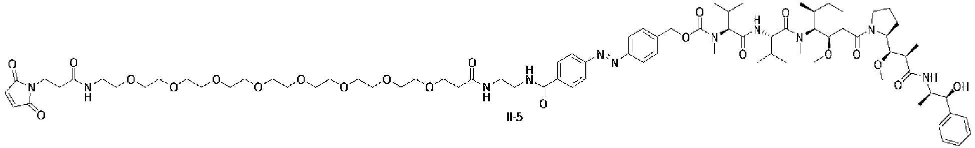 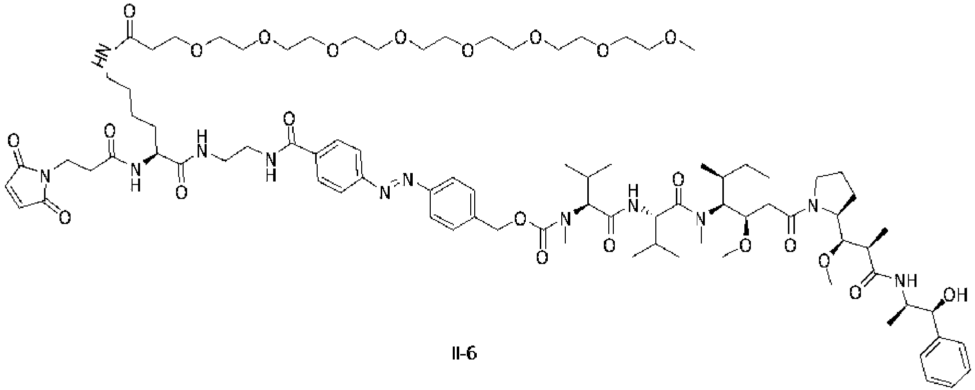 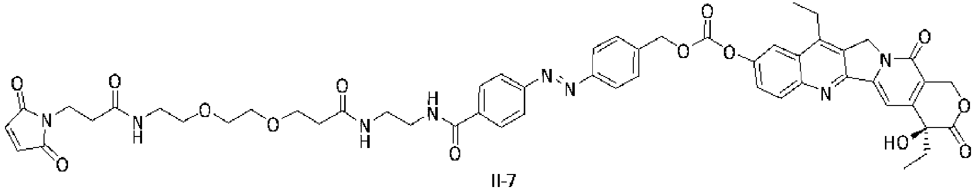 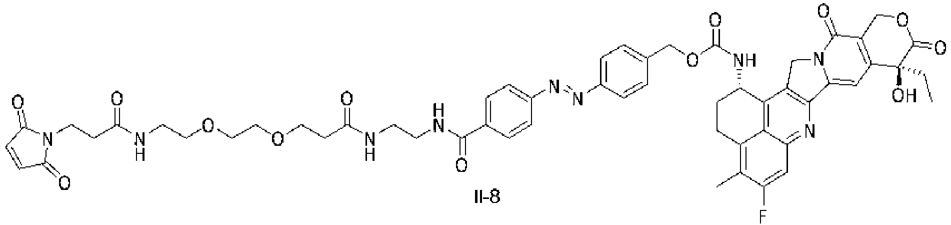 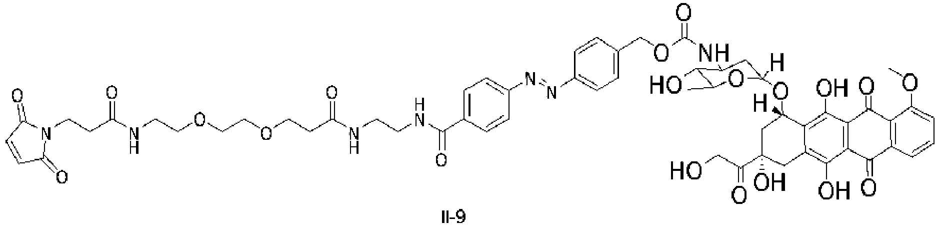 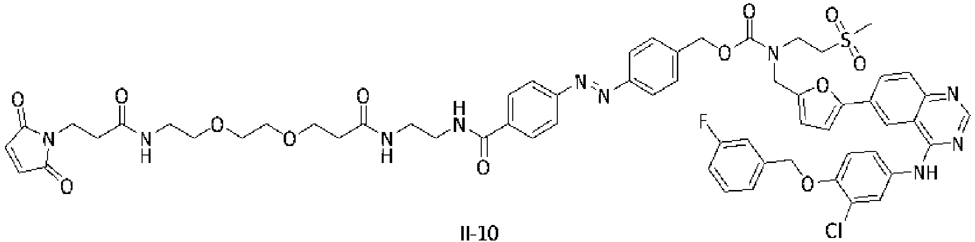 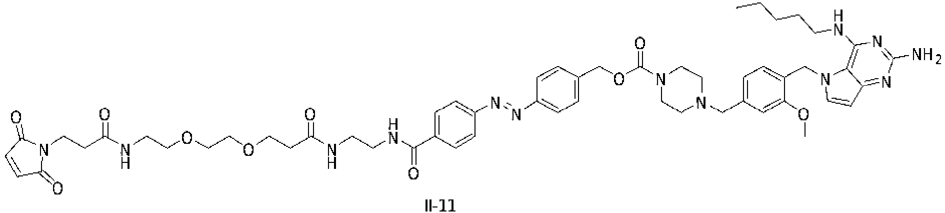 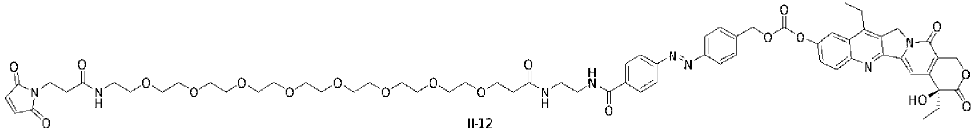 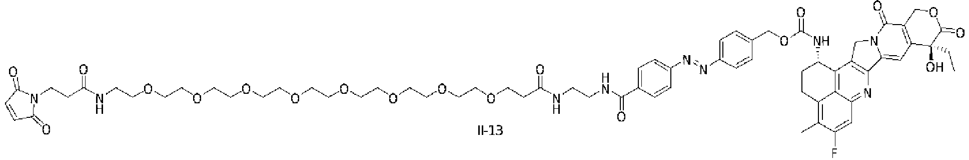 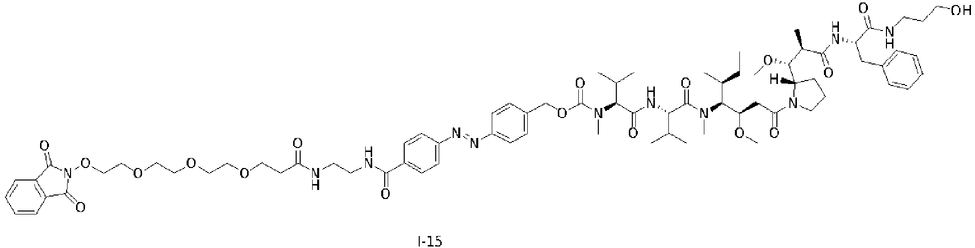 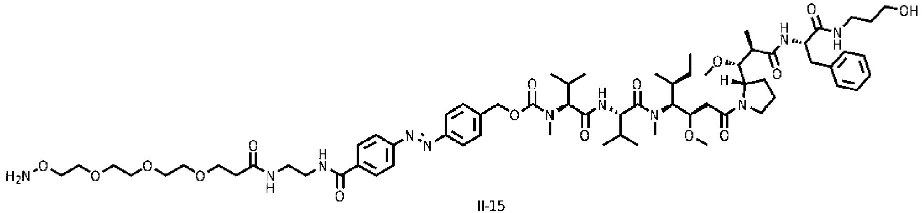 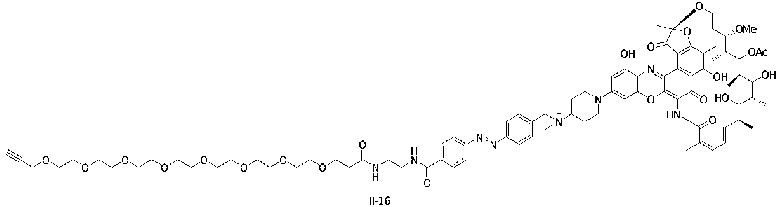 and

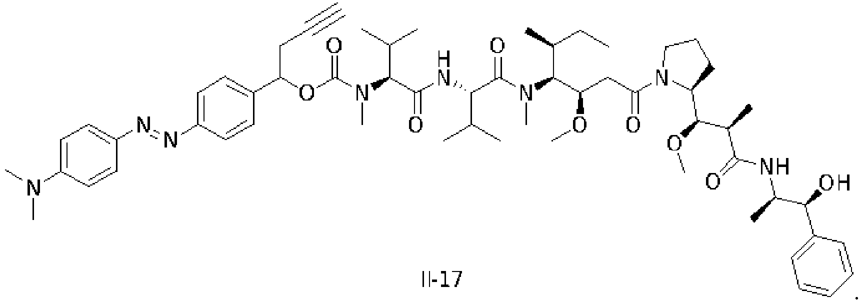

The present disclosure also provides use of the compound represented by Formula II, a geometric isomer, an optical isomer, a salt, a hydrate, a solvate or a polymorph thereof in the manufacture of an antibody-drug conjugate.

### Protein-drug conjugates

The present disclosure also provides a compound represented by Formula III or Formula IV, a geometric isomer, an optical isomer, a pharmaceutically acceptable salt, a hydrate, a solvate, or a polymorph thereof, 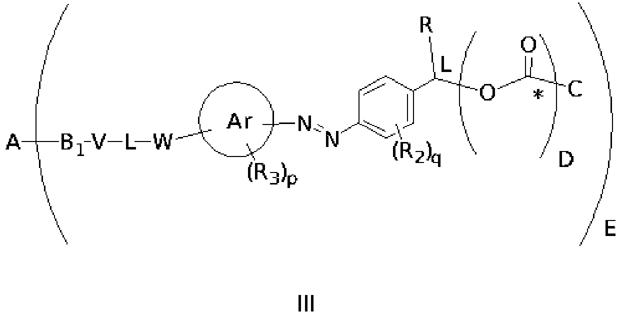 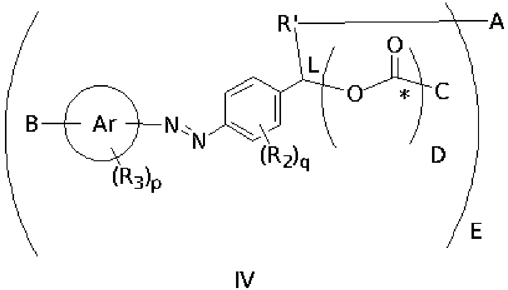
wherein, A represents a targeting compound; 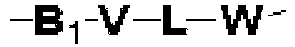
represents a linker, for conjugating an azobenzene group to a targeting compound,
Ar is a 5- or 6-membered aryl or heteroaryl;
R₂ and R₃ each independently are hydrogen, C₁₋₆ alkyl, fluoro, chloro, bromo, iodo, hydroxyl, nitro or cyano;
p is 0, 1, 2, or 3;
q is 0, 1, 2 or 3;
R is H, C₁₋₄ alkyl, 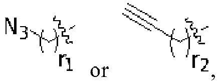 wherein r₁ and r₂ each independently are an integer from 1 to 12;
R' is 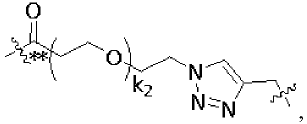 wherein k₂ is 1, 2, 3, 4, 5, 6, 7, 8, 9, 10, 11 or 12;
B is selected from the group consisting of 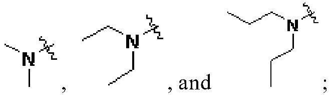
D is 0 or 1;
C is an active compound, selected from the group consisting of a drug, a cytotoxin, a detection reagent, a diagnostic reagent, and a targeting carrier; preferably, C is an antitumor drug, an antiviral drug, an antibiotic, an antibacterial drug or an immunomodulatory drug; further preferably, C is an antitumor drug, such as a small molecule, a peptide or a nucleotide including a cytotoxin, a tubulin inhibitor, a DNA alkylating agent, a DNA intercalating agent, an RNA polymerase inhibitor, a kinase inhibitor, a topoisomerase inhibitor, a kinesin spindle protein inhibitor, and an antimetabolite drug, an immunomodulatory drug such as a Toll-like receptor agonist (e.g., a TLR7 agonist) or a STING agonist, or an antibacterial drug such as rifampicin and an analog thereof (e.g., rifalogue, KRM-1657); C is conjugated to the carbonyl group (i.e. the position *) or the C atom at the position L via the N atom or the O atom in the active compound molecule;
E is an integer from 1 to 20.

In some embodiments, in the compound represented by Formula III, A represents a targeting compound; 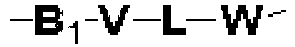
represents a linker, for conjugated an azobenzene group to a targeting compound,
Ar is a 5- or 6-membered aryl or heteroaryl;
R₂ are R₃ each independently are hydrogen, C₁₋₆ alkyl, fluoro, chloro, bromo, iodo, hydroxyl, nitro or cyano;
p is 0, 1, 2, or 3;
q is 0, 1, 2 or 3;
R is H, C₁₋₄ alkyl, 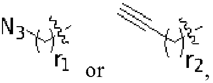 wherein r₁ and r₂ each independently are an integer from 1 to 12;
D is 0 or 1;
C is an active compound, selected from the group consisting of a drug, a cytotoxin, a detection reagent, a diagnostic reagent, and a targeting carrier; preferably, C is an antitumor drug, an antiviral drug, an antibiotic, an antibacterial drug or an immunomodulatory drug; further preferably, C is an antitumor drug, such as a small molecule, a peptide or a nucleotide including a cytotoxin, a tubulin inhibitor, a DNA alkylating agent, a DNA intercalating agent, an RNA polymerase inhibitor, a kinase inhibitor, a topoisomerase inhibitor, a kinesin spindle protein inhibitor, and an antimetabolite drug, an immunomodulatory drug such as a Toll-like receptor agonist (e.g., a TLR7 agonist) or a STING agonist, or an antibacterial drug such as rifampicin and an analog thereof (e.g., rifalogue, KRM-1657); C is conjugated to the carbonyl group(i.e. the position *) or the C atom at the position L via the N atom or the O atom in the active compound molecule.
E is an integer from 1 to 20.

In some embodiments, in the compound represented by Formula III, B₁, V, L, and W in 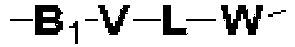 are as defined as follows:
B₁ is 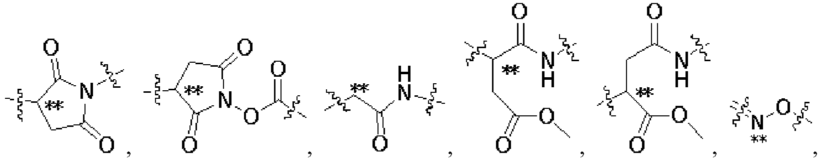 or 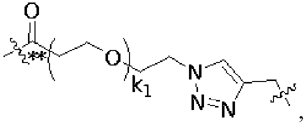 wherein k₁ is 1, 2, 3, 4, 5, 6, 7, 8, 9, 10, 11 or 12;
V is 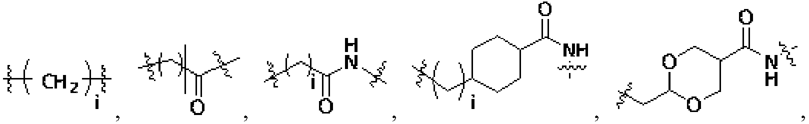 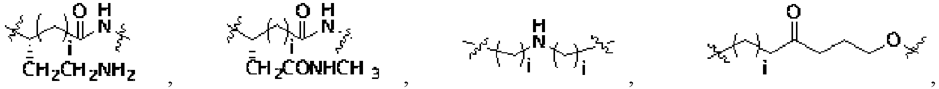 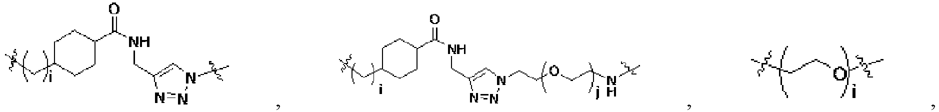 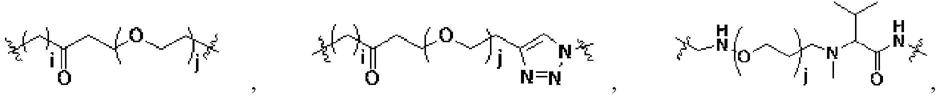 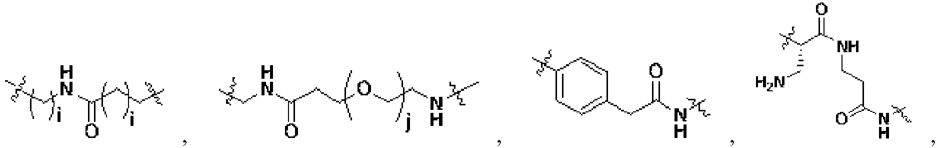 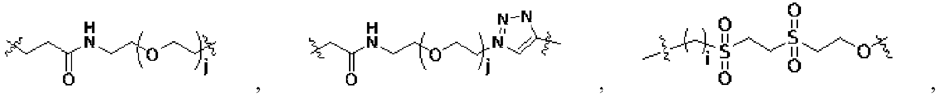 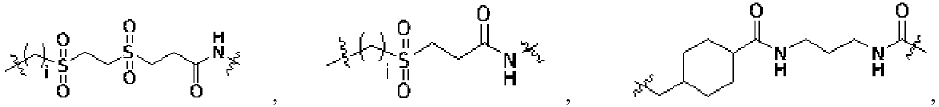 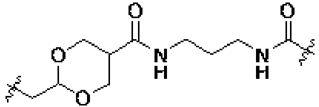 or absent, wherein each i independently is an integer from 0 to 6, and each j independently is an integer from 0 to 8;
L is -(CH₂CH₂O)ₖ-(CH₂)₁-, -CHR₁-, -(CH₂)ₘ -, 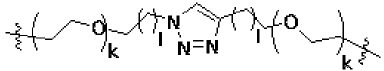 or absent, wherein R₁ is -(CH₂)ₙ-NHC(O)-(CH₂CH₂O)ₖ-CH₃, each k independently is an integer from 0 to 12, each l independently is an integer from 0 to 12, n is an integer from 0 to 12, and m is an integer from 0 to 30;
W is -W1-W2-W3-, wherein W1 is connected to L, W3 is connected to Ar, and W1 is selected from the group consisting of -O-, -S-, -NR₁₁-, -CONH-, -NHCO-, -S(O₂)NH-, and -NHS(O₂)-, or W1 is absent; W2 is selected from the group consisting of -(CH₂)ₒ-, and -[CH(R₁₁)CH(R₁₂)]ₒ- or W2 is absent; W3 is selected from the group consisting of -O-, -S-, -NR₁₁-, -CONH-, -NHCO-, -S(O₂)NH-, and - NHS(O₂)- or W3 is absent; wherein o is an integer from 0 to 6, each R₁₁ independently is selected from the group consisting of hydrogen and C₁₋₆ alkyl, and R₁₂ is selected from the group consisting of hydrogen and C₁₋₆ alkyl.

In some embodiments, in the compound represented by Formula III, A is a targeting compound, selected from the group consisting of proteins, antibodies, polypeptides, enzymes and small molecules.

In some embodiments, in the compound represented by Formula III, A is conjugated to the position ** of the group B₁ via the S atom or the N atom in the targeting compound molecule, or A is conjugated to the position ** of the group B₁ via oxime bond which formed by reacting the carbonyl group in the targeting compound molecule with the hydroxylamino group in the group B₁.

In some embodiments, in the compound represented by Formula III according to the present disclosure, W is selected from the group consisting of -NH-CH₂-C(O)-NH-, -NH-CH₂-CH₂-C(O)-NH-, -C(O)-NH-, -NH-C(O)-, -C(O)-NH-CH₂-C(O)-NH-, -C(O)-NH-CH₂-CH₂-C(O)-NH-, -C(O)-NH-, -NH-C(O)-, -NH-CH(R₁₀)-C(O)-NH-, -C(O)-NH-(CH₂)ₒ-NH-C(O)-, -C(O)-NH-[CH(R₁₁)CH(R₁₂)]ₒ-NH-C(O)-, -NH-(CH₂)ₒ-NH-C(O)-, and -NH-[CH(R₁₁)CH(R₁₂)]ₒ-NH-C(O)-, wherein R₁₀ is H, methyl or ethyl, and o, R₁₁ and R₁₂ are as defined in the present disclosure.

In some embodiments, in the compound represented by Formula III, B₁ is 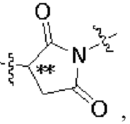 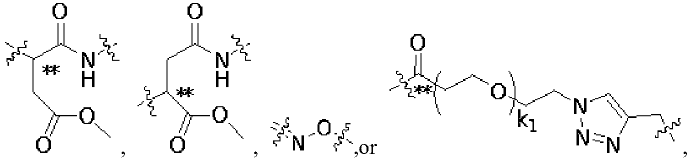 wherein k₁ is as defined in the present disclosure.

In some embodiments, in the compound represented by Formula III, B₁ is 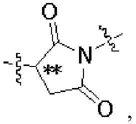 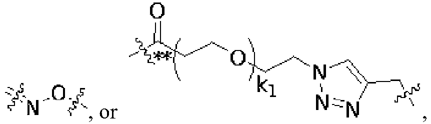 wherein k₁ is as defined in the present disclosure.

In some embodiments, in the compound represented by Formula III, k₁ is 1, 2, 3, 4, 5, 6, 7, 8, 9 or 10. In some embodiments, in the compound represented by Formula III, k₁ is 1, 2, 3, 4, 5, 6, 7 or 8. In some embodiments, in the compound represented by Formula III, k₁ is 2, 3, 4, 5, 6, 7 or 8. In some embodiments, in the compound represented by Formula III, k₁ is 4, 5, 6, 7 or 8. In some embodiments, in the compound represented by Formula III, k₁ is 9 or 10.

In some embodiments, in the compound represented by Formula III, V is 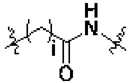 or absent, wherein i is as defined in the present disclosure. In some embodiments, i is 1, 2, 3, 4, 5 or 6. In some embodiments, i is 1. In some embodiments, i is 2. In some embodiments, i is 3. In some embodiments, i is 4. In some embodiments, i is 5. In some embodiments, i is 6.

In some embodiments, in the compound represented by Formula III, j is 1, 2, 3, 4, 5, 6, 7 or 8. In some embodiments, in the compound represented by Formula III, j is 2, 3, 4, 5, 6, 7 or 8. In some embodiments, in the compound represented by Formula III, j is 9 or 10.

In some embodiments, in the compound represented by Formula III, L is -(CH₂CH₂O)ₖ-(CH₂)ₗ-, -CHR₁- or -(CH₂)ₘ-, wherein k, l, R₁, and m are as defined in the present disclosure. In some embodiments, k is 1, 2, 3, 4, 5, 6, 7, 8, 9, 10, 11 or 12. In some embodiments, k is 1, 2, 3, 4, 5, 6, 7, 8, 9 or 10. In some embodiments, k is 1, 2, 3, 4, 5, 6, 7 or 8. In some embodiments, k is 2, 3, 4, 5, 6, 7 or 8. In some embodiments, l is 1, 2, 3, 4, 5, 6, 7, 8, 9 or 10. In some embodiments, l is 1, 2, 3, 4, 5, 6, 7 or 8. In some embodiments, l is 1, 2, 3, 4, 5 or 6. In some embodiments, l is 1, 2, 3 or 4. In some embodiments, l is 1. In some embodiments, l is 2. In some embodiments, l is 3. In some embodiments, l is 4. In some embodiments, l is 5. In some embodiments, l is 6. In some embodiments, n is 1, 2, 3, 4, 5, 6, 7, 8, 9 or 10. In some embodiments, n is 1, 2, 3, 4, 5, 6, 7 or 8. In some embodiments, n is 1, 2, 3, 4, 5 or 6. In some embodiments, n is 1, 2, 3 or 4. In some embodiments, n is 1. In some embodiments, n is 2. In some embodiments, n is 3. In some embodiments, n is 4. In some embodiments, n is 5. In some embodiments, n is 6. In some embodiments, m is an integer from 1 to 20. In some embodiments, m is an integer from 1 to 15. In some embodiments, m is an integer from 1 to 10. In some embodiments, m is an integer from 1 to 8. In some embodiments, m is an integer from 1 to 6. In some embodiments, m is an integer from 1 to 4.

In some embodiments, in the compound represented by Formula III, W is -C(O)-NH-(CH₂)ₒ-NH-C(O)- or -C(O)-NH-[CH(R₁₁)CH(R₁₂)]ₒ-NH-C(O)-, and o, R₁₁ and R₁₂ are as defined in the present disclosure. In some embodiments, o is an integer from 0 to 4. In some embodiments, o is 1, 2, 3 or 4. In some embodiments, o is 1. In some embodiments, o is 2. In some embodiments, o is 3. In some embodiments, o is 4. In some embodiments, R₁₁ and R₁₂ each independently are hydrogen. In some embodiments, R₁₁ and R₁₂ each independently are C₁₋₆ alkyl. In some embodiments, R₁₁ and R₁₂ each independently are C₁₋₄ alkyl. In some embodiments, R₁₁ and R₁₂ each independently are methyl, ethyl, n-propyl, iso-propyl, n-butyl, iso-butyl, sec-butyl or tert-butyl. In some embodiments, R₁₁ and R₁₂ each independently are methyl, ethyl or n-propyl.

In some embodiments, in the compound represented by Formula III, -V-L-W- is -(CH₂)ₘ-C(O)-NH-(CH₂)ₒ-NH-C(O)-, -(CH₂)ᵢ-C(O)-NH-(CH₂CH₂O)ₖ-(CH₂)ₗ-C(O)-NH-(CH₂)ₒ-NH-C(O)-, -(CH₂)ᵢ-C(O)-NH-CHR₁-C(O)-NH-(CH₂)ₒ-NH-C(O)-, or -(CH₂CH₂O)ₖ-(CH₂)ₗ-C(O)-NH-(CH₂)ₒ-NH-C(O)-, wherein R₁ is -(CH₂)ₙ-NHC(O)-(CH₂CH₂O)ₖ-CH₃, and m, o, i, k, l, and n are as defined in the present disclosure.

In some embodiments, in the compound represented by Formula III, each i independently is 1, 2, 3 or 4; each k independently is 1, 2, 3, 4, 5, 6, 7, 8, 9, 10, 11 or 12; each m independently is 1, 2, 3 or 4; each o independently is 1, 2, 3 or 4; each l independently is 1, 2, 3 or 4; n is 1, 2, 3, 4, 5, 6, 7 or 8.

In some embodiments, in the compound represented by Formula III, each i independently is 1, 2 or 3; each k independently is 1, 2, 3, 4, 5, 6, 7, 8, 9 or 10; each m independently is 1, 2 or 3; each o independently is 1, 2 or 3; each l independently is 1, 2 or 3; n is 1, 2, 3, 4 or 5.

In some embodiments, in the compound represented by Formula III according to the present disclosure, -V-L-W- is -(CH₂)₂-C(O)-NH-(CH₂)₂-NH-C(O)-, -(CH₂)₂-C(O)NH-(CH₂CH₂O)₂-CH₂-CH₂-C(O)-NH-(CH₂)₂-NH-C(O)-, -(CH₂)₂-C(O)NH-(CH₂CH₂O)₄-CH₂-CH₂-C(O)-NH-(CH₂)₂-NH-C(O)-, - (CH₂)₂-C(O)NH-(CH₂CH₂O)₆-CH₂-CH₂-C(O)-NH-(CH₂)₂-NH-C(O)-, -(CH₂)₂-C(O)NH-(CH₂CH₂O)₈-CH₂-CH₂-C(O)-NH-(CH₂)₂-NH-C(O)-, -(CH₂CH₂O)₃-(CH₂)₂-C(O)-NH-(CH₂)₂-NH-C(O)-, -(CH₂)₂-C(O)NH-CH[(CH₂)₄-NHC(O)-(CH₂CH₂O)₈-CH₃]-C(O)-NH-(CH₂)₂-NH-C(O)-, -(CH₂CH₂O)₇-(CH₂)₂-C(O)-NH-(CH₂)₂-NH-C(O)-.

In some embodiments, in the compound represented by Formula III, Ar is phenyl, pyridyl, pyrimidinyl, furanyl, imidazolyl, pyrrolyl, thiazolyl, pyrazolyl or thienyl. In some embodiments, in the compound represented by Formula III, Ar is phenyl, furanyl, imidazolyl or thienyl. In some embodiments, in the compound represented by Formula III, Ar is phenyl.

In some embodiments, in the compound represented by Formula III, R is H or C₁₋₄ alkyl. In some embodiments, in the compound represented by Formula III, R is H, methyl, ethyl, n-propyl or n-butyl. In some embodiments, in the compound represented by Formula III, R is H, methyl, ethyl or n-propyl. In some embodiments, in the compound represented by Formula III, R is H, methyl or ethyl. In some embodiments, in the compound represented by Formula III, R is H. In some embodiments, in the compound represented by Formula III, R is methyl.

In some embodiments, in the compound represented by Formula III, R₂ and R₃ each independently are hydrogen, C₁₋₄ alkyl, fluoro, chloro, bromo, iodo, hydroxyl, nitro or cyano. In some embodiments, in the compound represented by Formula III, R₂ and R₃ each independently are hydrogen, methyl, ethyl, n-propyl, iso-propyl, n-butyl, iso-butyl, sec-butyl, or tert-butyl. In some embodiments, in the compound represented by Formula III, R₂ and R₃ each independently are fluoro, chloro, bromo, iodo, hydroxyl, nitro or cyano. In some embodiments, in the compound represented by Formula III, R₂ and R₃ each independently are hydrogen.

In some embodiments, in the compound represented by Formula III, q is 0, 1 or 2, preferably is 0 or 1.

In some embodiments, in the compound represented by Formula III, p is 0, 1 or 2, preferably is 0 or 1.

In some embodiments, in the compound represented by Formula III, C is selected from the group consisting of auristatin, monomethyl auristatin E (MMAE), monomethyl auristatin F (MMAF) and a derivative thereof, maytansine and a derivative thereof (such as maytansinoids, DM1, DM3, DM4), paclitaxel, calicheamicin, duocarmycin, doxorubicin, camptothecin, PBD (pyrrolobenzodiazepines) cytotoxin and a derivative thereof, SN-38, exatecan, doxorubicin, a TLR7 agonist with CAS No. 1821304-87-3, lapatinib, rifampicin and an analog thereof (for example, rifalogue, KRM-1657).

In some embodiments, in the compound represented by Formula III, C is monomethyl auristatin E (MMAE), rifampicin or rifalogue.

In some embodiments, in the compound represented by Formula III, C is monomethyl auristatin E (MMAE) or rifalogue.

In some embodiments, in the compound represented by Formula III, C is SN-38, exatecan, doxorubicin, a TLR7 agonist with CAS No. 1821304-87-3, lapatinib, MMAE, an MMAF derivative or rifalogue.

In some embodiments, in the compound represented by Formula IV, C is rifampicin or rifalogue.

In some embodiments, in the compound represented by Formula IV, C is rifalogue.

In some embodiments, in the compound represented by Formula III, C is an MMAF derivative, having a structure:

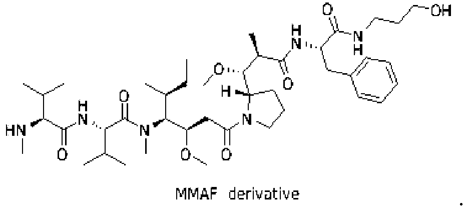

In some embodiments, in the compound represented by Formula III, Ais an antibody, preferably a monoclonal antibody (MAB), a bispecific antibody or a multispecific antibody.

In some embodiments, in the compound represented by Formula III, A comprises an antibody fragment or a substitute thereof or a variant thereof, a protein ligand or a protein scaffold.

In some embodiments, in the compound represented by Formula III, A is a monoclonal antibody, bispecific antibody or multispecific antibody with a thiol or amino group as a conjugating site, or a site-directed mutated or modified monoclonal antibody, bispecific antibody or multispecific antibody with a thiol or amino group as a conjugating site, or a site-directed mutated monoclonal antibody, bispecific antibody or multispecific antibody with a carbonyl group in *p*-acetylphenylalanine (pAF) as a conjugating site.

In some embodiments, in the compound represented by Formula III, A is selected from the group consisting of humanized ant-HER2 monoclonal antibody mil40, trastuzumab (HERCEPTIN), pertuzumab (PERJETA), cetuximab (ERBITUX), panitumumab (VECTIBIX), rituximab (RITUXAN), alemtuzumab (CAMPATH), ibritumomab tiuxetan (ZEVALIN), tositumomab (BEXXAR), ofatumumab (ARZERRA), bevacizumab (AVASTIN), ipilimumab (YERVOY), denosumab (XGEVA), pembrolizumab (KEYTRUDA), nivolumab (Opdivo), avelumab (Bavencio), atezolizumab (Tecentriq), durvalumab (Imfinzi), sacituzumab, rovalpituzumab, and biological analogs thereof, and antibacterial antibody.

In some embodiments, in the compound represented by Formula III, A is trastuzumab.

In some embodiments, in the compound represented by Formula III, A is albumin. In some embodiments, in the compound represented by Formula III, A is human serum albumin.

In some embodiments, in the compound represented by Formula III, A is an antibacterial antibody.

In some embodiments, in the compound represented by Formula III, A is an antibody site-specifically mutated with an unnatural amino acid, comprising:
(a) the following three heavy chain variable region (VH) complementarity determining regions (CDRs):
   (i) VH CDR1, having a CDR1 sequence contained in the heavy chain as set forth in SEQ ID NO:1, or having a sequence with substitution, deletion or addition of one or several amino acids (for example, substitution, deletion or addition of one or two amino acids) as compared with the CDR1 sequence contained in the VH;
   (ii) VH CDR2, having a CDR2 sequence contained in the heavy chain as set forth in SEQ ID NO:1, or having a sequence with substitution, deletion or addition of one or more amino acids (for example, substitution, deletion or addition of one or two amino acids) as compared with the CDR2 sequence contained in the VH; and
   (iii) VH CDR3, having a CDR3 sequence contained in the heavy chain as set forth in SEQ ID NO:1, or having a sequence with substitution, deletion or addition of one or more amino acids (for example, substitution, deletion or addition of one or two amino acids) as compared with the CDR3 sequence contained in the VH;
      and/or
(b) the following three light chain variable region (VL) CDRs:
   (iv) VL CDR1, having a CDR1 sequence contained in the light chain as set forth in SEQ ID NO:2, or having a sequence with substitution, deletion or addition of one or more amino acids (for example, substitution, deletion or addition of one or two amino acids) as compared with the CDR1 sequence contained in the VL;
   (v) VL CDR2, having a CDR2 sequence contained in the light chain as set forth in SEQ ID NO:2, or having a sequence with substitution, deletion or addition of one or several amino acids (for example, substitution, deletion or addition of one or two amino acids) as compared with the CDR2 sequence contained in the VL; and
   (vi) VL CDR3, having a CDR3 sequence contained in the light chain as set forth in SEQ ID NO:2, or having a sequence with substitution, deletion or addition of one or several amino acids (for example, substitution, deletion or addition of one or two amino acids) as compared with the CDR3 sequence contained in the VL.

In some embodiments, the substitution described in any one of (i) to (vi) is a conservative substitution. In some embodiments, the CDR1, CDR2 and CDR3 contained in the heavy chain variable region (VH), and/or the CDR1, CDR2 and CDR3 contained in the light chain variable region (VL) are defined by the Kabat, Chothia or IMGT numbering system. In some embodiments, the amino acid sequence of the heavy chain of the antibody is as set forth in SEQ ID NO:1, and the amino acid sequence of the light chain of the antibody is as set forth in SEQ ID NO:2.

In some embodiments, in the compound represented by Formula III, A is an antibacterial antibody, comprising:
(a) the following three heavy chain variable region (VH) complementarity determining regions (CDRs):
   (i) VH CDR1, having a CDR1 sequence contained in the heavy chain as set forth in SEQ ID NO:3, or having a sequence with substitution, deletion or addition of one or several amino acids (for example, substitution, deletion or addition of one or two amino acids) as compared with the CDR1 sequence contained in the VH;
   (ii) VH CDR2, having a CDR2 sequence contained in the heavy chain as set forth in SEQ ID NO:3, or having a sequence with substitution, deletion or addition of one or more amino acids (for example, substitution, deletion or addition of one or two amino acids) as compared with the CDR2 sequence contained in the VH; and
   (iii) VH CDR3, having a CDR3 sequence contained in the heavy chain as set forth in SEQ ID NO:3, or having a sequence with substitution, deletion or addition of one or more amino acids (for example, substitution, deletion or addition of one or two amino acids) as compared with the CDR3 sequence contained in the VH;
   and/or
(b) the following three light chain variable region (VL) CDRs:
   (iv) VL CDR1, having a CDR1 sequence contained in the light chain as set forth in SEQ ID NO:4, or having a sequence with substitution, deletion or addition of one or several amino acids (for example, substitution, deletion or addition of one or two amino acids) as compared with the CDR1 sequence contained in the VL;
   (v) VL CDR2, having a CDR2 sequence contained in the light chain as set forth in SEQ ID NO:4, or having a sequence with substitution, deletion or addition of one or several amino acids (for example, substitution, deletion or addition of one or two amino acids) as compared with the CDR2 sequence contained in the VL; and
   (vi) VL CDR3, having a CDR3 sequence contained in the light chain as set forth in SEQ ID NO:4 or having a sequence with substitution, deletion or addition of one or several amino acids (for example, substitution, deletion or addition of one or two amino acids) as compared with the CDR3 sequence contained in the VL.

In some embodiments, the substitution described in any one of (i) to (vi) is a conservative substitution. In some embodiments, the CDR1, CDR2 and CDR3 contained in the heavy chain variable region (VH), and/or the CDR1, CDR2 and CDR3 contained in the light chain variable region (VL) are defined by the Kabat, Chothia or IMGT numbering system. In some embodiments, the amino acid sequence of the heavy chain of the antibody is as set forth in SEQ ID NO:3, and the amino acid sequence of the light chain of the antibody is as set forth in SEQ ID NO:4.

In some embodiments, in the compound represented by Formula III, E is a number from 1 to 15. In some embodiments, in the compound represented by Formula III, E is a number from 1 to 10. In some embodiments, in the compound represented by Formula III, E is a number from 1 to 8, such as about 1, about 2, about 3, about 4, about 5, about 6, about 7, and about 8.

In some embodiments, the compound represented by Formula III has a structure represented by FormulaIII-1, Formula III-2 or Formula III-3, 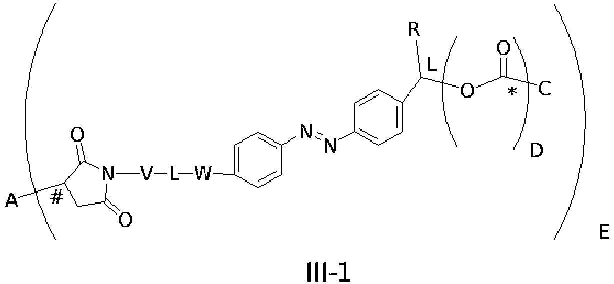 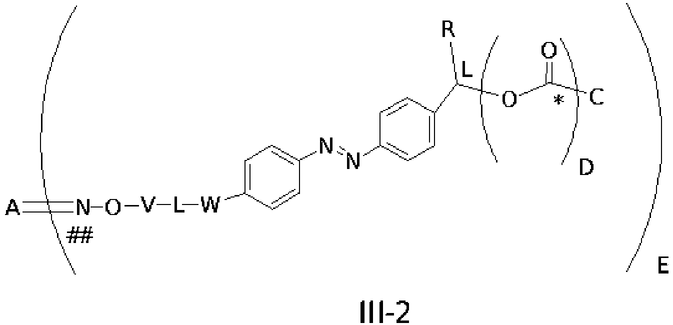 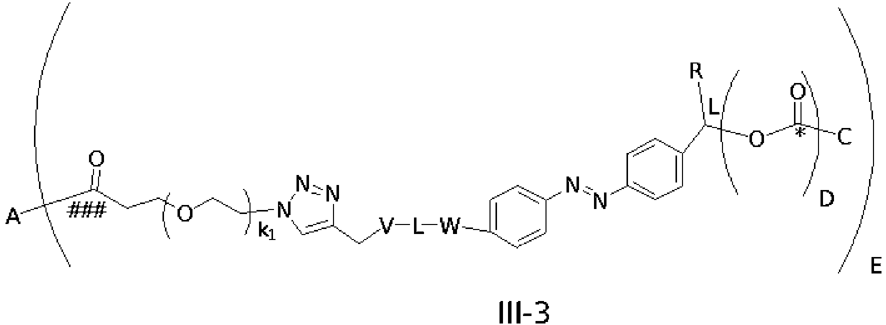 wherein A, k₁, V, L, W, R, C, D, and E are as defined in the present disclosure.

In some embodiments, in the compound of Formula III-1, A is conjugated to the position # via the S atom in the targeting compound molecule. In some embodiments, in the compound of Formula III-2, A is conjugated to the position ## via the N atom in the targeting compound molecule. In some embodiments, in the compound of Formula III-3, A is conjugated to the position ### via the carbonyl group in the targeting compound molecule.

In some embodiments, the compound represented by Formula III is selected from the group consisting of: 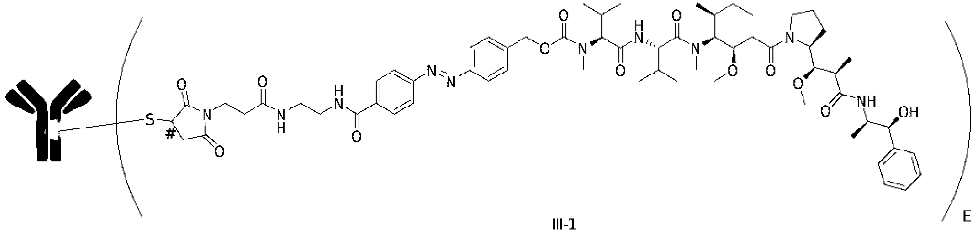 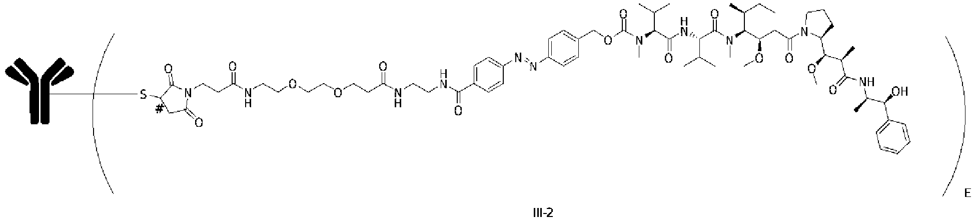 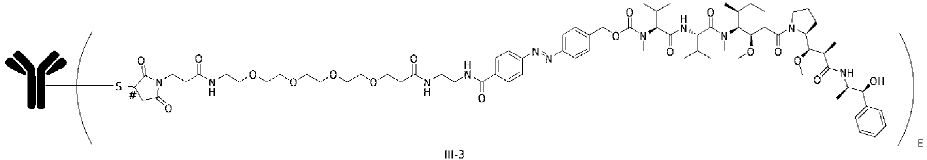 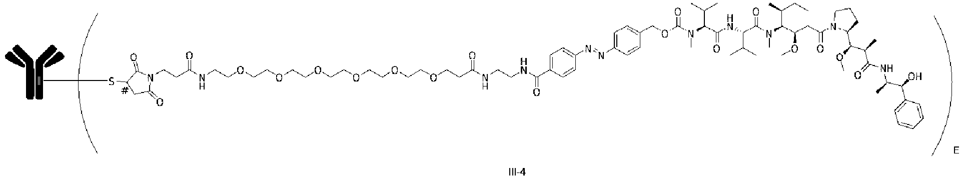 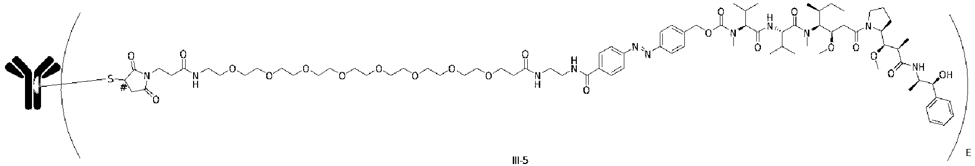 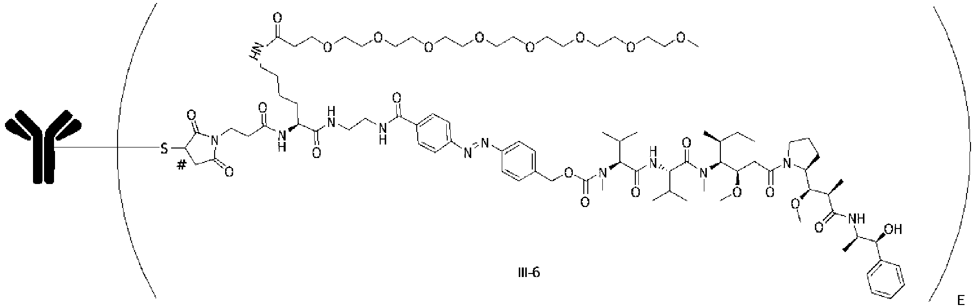 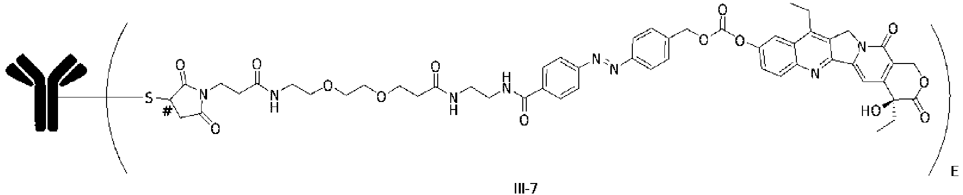 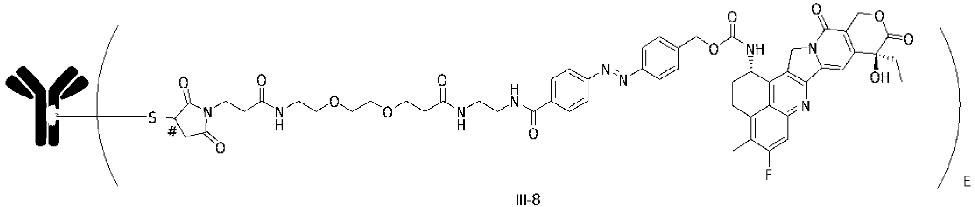 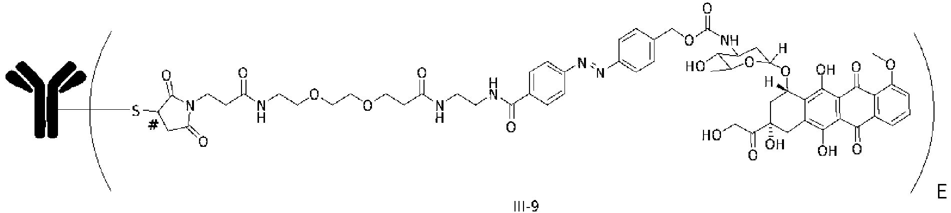 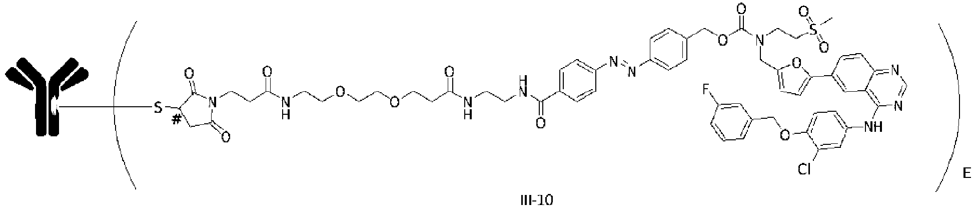 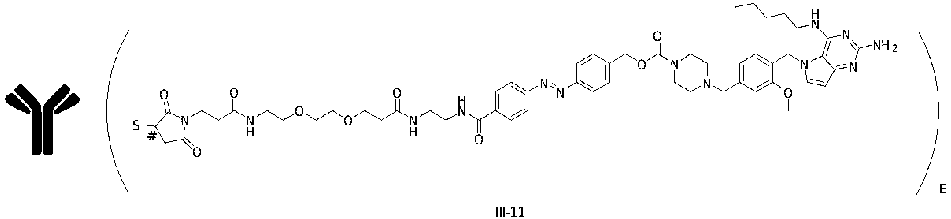 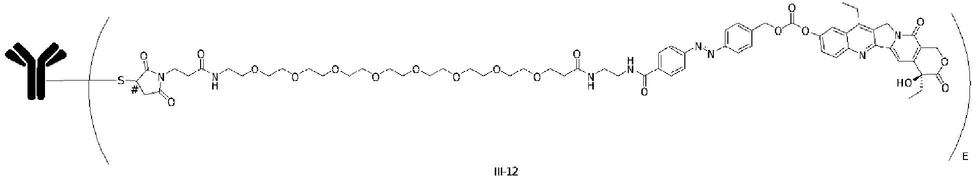 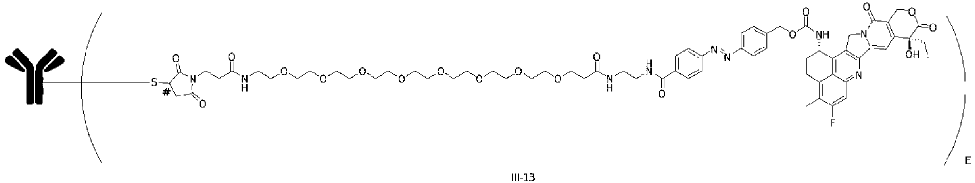 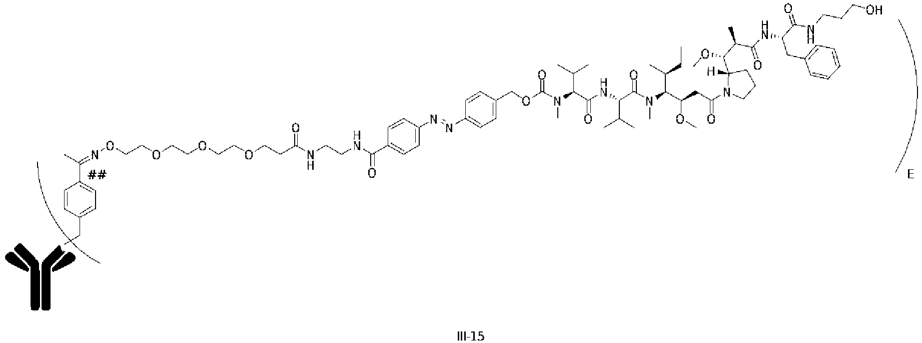 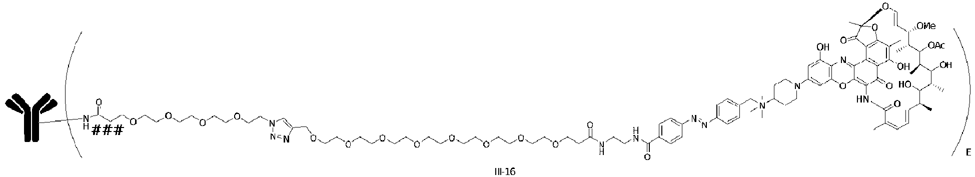 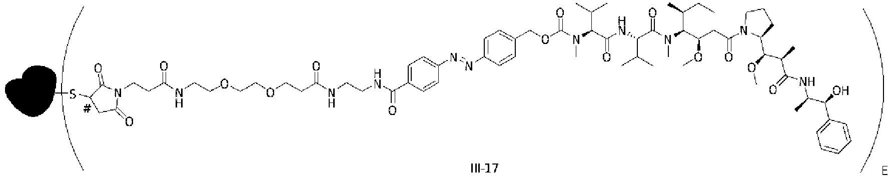 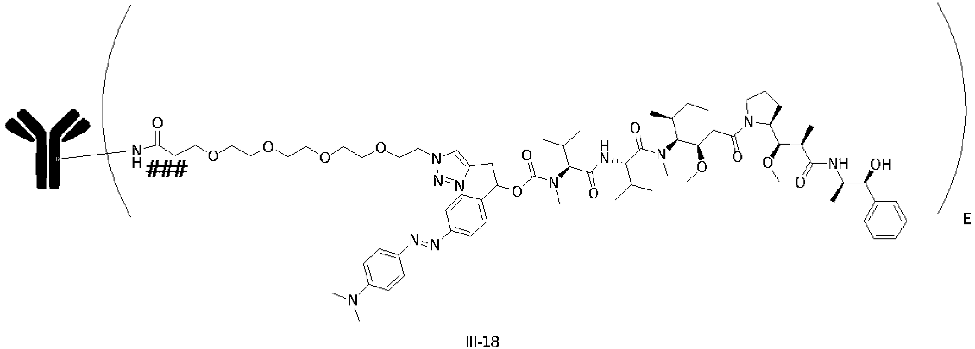 wherein, 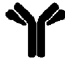 represents A, A and E are as defined in the present disclosure, and 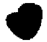 is a protein, preferably human serum albumin, the albumin being connected to the position # via the S atom in the albumin molecule. In some embodiments, in the above compound represented by Formula III, A is an antibody, preferably a monoclonal antibody (MAB), a bispecific antibody or a multispecific antibody, and further preferably trastuzumab, an antibody site-specifically mutated with an unnatural amino acid or an antibacterial antibody, and preferably, A is an antibody, which is connected to the position # via the S atom in the antibody molecule or connected to the position ## via the carbonyl group in the antibody molecule or connected to the position ### via the N atom in the antibody molecule. In some embodiments, in the above compound represented by Formula III, E is about 4.

In some embodiments, in the compound represented by Formula IV,
A represents a targeting compound;
Ar is a 5- or 6-membered aryl or heteroaryl;
R₂ and R₃ each independently are hydrogen, C₁₋₆ alkyl, fluoro, chloro, bromo, iodo, hydroxyl, nitro or cyano;
p is 0, 1, 2, or 3;
q is 0, 1, 2 or 3;
R' is 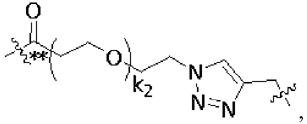 wherein k₂ is 1, 2, 3, 4, 5, 6, 7, 8, 9, 10, 11 or 12;
B is selected from the group consisting of 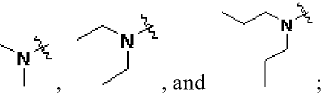
D is 0 or 1;
C is an active compound, selected from the group consisting of a drug, a cytotoxin, a detection reagent, a diagnostic reagent, and a targeting carrier; preferably, C is an antitumor drug, an antiviral drug, an antibiotic, an antibacterial drug or an immunomodulatory drug; further preferably, C is an antitumor drug, such as a small molecule, peptide or nucleotide including a cytotoxin, a tubulin inhibitor, a DNA alkylating agent, a DNA intercalating agent, an RNA polymerase inhibitor, a kinase inhibitor, a topoisomerase inhibitor, a kinesin spindle protein inhibitor, and an antimetabolite drug, an immunomodulatory drug such as a Toll-like receptor agonist (e.g., a TLR7 agonist) or a STING agonist, or an antibacterial drug such as rifampicin and an analog thereof (e.g., rifalogue, KRM-1657); C is conjugated to the carbonyl group (i.e. the position *) or the C atom at the position L via the N atom or the O atom in the active compound molecule;
E is a number from 1 to 20.

In some embodiments, in the compound represented by Formula IV, A is a targeting compound, selected from the group consisting of proteins, antibodies, polypeptides, enzymes and small molecules.

In some embodiments, in the compound represented by Formula IV, A is conjugated to the position ** of the group R' via the N atom in the targeting compound molecule.

In some embodiments, in the compound represented by Formula IV, Ar is phenyl, pyridyl, pyrimidinyl, furanyl, imidazolyl, pyrrolyl, thiazolyl, pyrazolyl or thienyl. In some embodiments, in the compound represented by Formula IV, Ar is phenyl, furanyl, imidazolyl or thienyl. In some embodiments, in the compound represented by Formula IV, Ar is phenyl.

In some embodiments, in the compound represented by Formula IV, R₂ and R₃ each independently are hydrogen, C₁₋₄ alkyl, fluoro, chloro, bromo, iodo, hydroxyl, nitro or cyano. In some embodiments, in the compound represented by Formula IV, R₂ and R₃ each independently are hydrogen, methyl, ethyl, n-propyl, iso-propyl, n-butyl, iso-butyl, sec-butyl, or tert-butyl. In some embodiments, in the compound represented by Formula IV, R₂ and R₃ each independently are fluoro, chloro, bromo, iodo, hydroxyl, nitro or cyano. In some embodiments, in the compound represented by Formula IV, R₂ and R₃ each independently are hydrogen.

In some embodiments, in the compound represented by Formula IV, q is 0, 1 or 2, preferably is 0 or 1.

In some embodiments, in the compound represented by Formula IV, p is 0, 1 or 2, preferably is 0 or 1.

In some embodiments, in the compound represented by Formula IV, C is selected from the group consisting of: auristatin, monomethyl auristatin E (MMAE), monomethyl auristatin F (MMAF) and a derivative thereof, maytansine and a derivative thereof (such as maytansinoids, DM1, DM3, DM4), paclitaxel, calicheamicin, duocarmycin, doxorubicin, camptothecin, PBD (pyrrolobenzodiazepines) cytotoxin and a derivative thereof, SN-38, exatecan, doxorubicin, a TLR7 agonist with CAS No. 1821304-87-3, lapatinib, rifampicin and an analog thereof (for example, rifalogue, KRM-1657).

In some embodiments, in the compound represented by Formula IV, C is monomethyl auristatin E (MMAE), rifampicin or rifalogue.

In some embodiments, in the compound represented by Formula IV, C is monomethyl auristatin E (MMAE) or rifalogue.

In some embodiments, in the compound represented by Formula IV, C is rifampicin or rifalogue.

In some embodiments, in the compound represented by Formula IV, C is rifalogue.

In some embodiments, in the compound represented by Formula IV, C is SN-38, exatecan, doxorubicin, a TLR7 agonist with CAS No. 1821304-87-3, lapatinib, MMAE, an MMAF derivative or rifalogue.

In some embodiments, in the compound represented by Formula IV, C is an MMAF derivative, having a structure

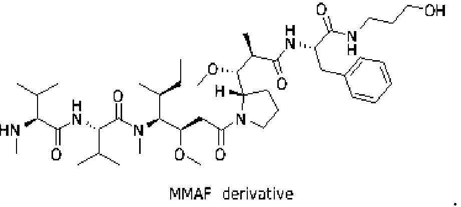

In some embodiments, in the compound represented by Formula IV, Ais an antibody, preferably a monoclonal antibody (MAB), a bispecific antibody or a multispecific antibody.

In some embodiments, in the compound represented by Formula IV, A comprises an antibody fragment or a substitute thereof or a variant thereof, a protein ligand or a protein scaffold.

In some embodiments, in the compound represented by Formula IV, A is a monoclonal antibody, bispecific antibody or multispecific antibody with an amino or thiol group as a conjugating site, or a site-directed mutated or modified monoclonal antibody, bispecific antibody or multispecific antibody with an amino or thiol group as a conjugating site.

In some embodiments, in the compound represented by Formula IV, A is selected from the group consisting of humanized anti-HER2 monoclonal antibody mil40, trastuzumab (HERCEPTIN), pertuzumab (PERJETA), cetuximab (ERBITUX), panitumumab (VECTIBIX), rituximab (RITUXAN), alemtuzumab (CAMPATH), ibritumomab tiuxetan (ZEVALIN), tositumomab (BEXXAR), ofatumumab (ARZERRA), bevacizumab (AVASTIN), ipilimumab (YERVOY), denosumab (XGEVA), pembrolizumab (KEYTRUDA), nivolumab (Opdivo), avelumab (Bavencio), atezolizumab (Tecentriq), durvalumab (Imfinzi), sacituzumab, rovalpituzumab, and biological analogs thereof, and antibacterial antibody.

In some embodiments, in the compound represented by Formula IV, A is an antibacterial antibody.

In some embodiments, in the compound represented by Formula IV, Ais trastuzumab.

In some embodiments, in the compound represented by Formula IV, A is albumin. In some embodiments, in the compound represented by Formula III, A is human serum albumin.

In some embodiments, in the compound represented by Formula IV, A is antibody site-specifically mutated an unnatural amino acid, comprising:
(a) the following three heavy chain variable region (VH) complementarity determining regions (CDRs):
   (i) VH CDR1, having a CDR1 sequence contained in the heavy chain as set forth in SEQ ID NO:1, or having a sequence with substitution, deletion or addition of one or several amino acids (for example, substitution, deletion or addition of one or two amino acids) as compared with the CDR1 sequence contained in the VH;
   (ii) VH CDR2, having a CDR2 sequence contained in the heavy chain as set forth in SEQ ID NO:1, or having a sequence with substitution, deletion or addition of one or more amino acids (for example, substitution, deletion or addition of one or two amino acids) as compared with the CDR2 sequence contained in the VH; and
   (iii) VH CDR3, having a CDR3 sequence contained in the heavy chain as set forth in SEQ ID NO:1, or having a sequence with substitution, deletion or addition of one or more amino acids (for example, substitution, deletion or addition of one or two amino acids) as compared with the CDR3 sequence contained in the VH;
      and/or
(b) the following three light chain variable region (VL)CDRs:
   (iv) VL CDR1, having a CDR1 sequence contained in the light chain as set forth in SEQ ID NO:2, or having a sequence with substitution, deletion or addition of one or more amino acids (for example, substitution, deletion or addition of one or two amino acids) as compared with the CDR1 sequence contained in the VL;
   (v) VL CDR2, having a CDR2 sequence contained in the light chain as set forth in SEQ ID NO:2, or having a sequence with substitution, deletion or addition of one or several amino acids (for example, substitution, deletion or addition of one or two amino acids) as compared with the CDR2 sequence contained in the VL; and
   (vi) VL CDR3, having a CDR3 sequence contained in the light chain as set forth in SEQ ID NO:2, or having a sequence with substitution, deletion or addition of one or several amino acids (for example, substitution, deletion or addition of one or two amino acids) as compared with the CDR3 sequence contained in the VL.

In some embodiments, the substitution described in any one of (i) to (vi) is a conservative substitution. In some embodiments, the CDR1, CDR2 and CDR3 contained in the heavy chain variable region (VH), and/or the CDR1, CDR2 and CDR3 contained in the light chain variable region (VL) are defined by the Kabat, Chothia or IMGT numbering system. In some embodiments, the amino acid sequence of the heavy chain of the antibody is as set forth in SEQ ID NO:1, and the amino acid sequence of the light chain of the antibody is as set forth in SEQ ID NO:2.

In some embodiments, in the compound represented by Formula IV, A is an antibacterial antibody, comprising:
(a) the following three heavy chain variable region (VH) complementarity determining regions (CDRs):
   (i) VH CDR1, having a CDR1 sequence contained in the heavy chain as set forth in SEQ ID NO:3, or having a sequence with substitution, deletion or addition of one or several amino acids (for example, substitution, deletion or addition of one or two amino acids) as compared with the CDR1 sequence contained in the VH;
   (ii) VH CDR2, having a CDR2 sequence contained in the heavy chain as set forth in SEQ ID NO:3, or having a sequence with substitution, deletion or addition of one or more amino acids (for example, substitution, deletion or addition of one or two amino acids) as compared with the CDR2 sequence contained in the VH; and
   (iii) VH CDR3, having a CDR3 sequence contained in the heavy chain as set forth in SEQ ID NO:3, or having a sequence with substitution, deletion or addition of one or more amino acids (for example, substitution, deletion or addition of one or two amino acids) as compared with the CDR3 sequence contained in the VH;
      and/or
(b) the following three light chain variable region (VL)CDRs:
   (iv) VL CDR1, having a CDR1 sequence contained in the light chain as set forth in SEQ ID NO:4, or having a sequence with substitution, deletion or addition of one or more amino acids (for example, substitution, deletion or addition of one or two amino acids) as compared with the CDR1 sequence contained in the VL;
   (v) VL CDR2, having a CDR2 sequence contained in the light chain as set forth in SEQ ID NO:4, or having a sequence with substitution, deletion or addition of one or several amino acids (for example, substitution, deletion or addition of one or two amino acids) as compared with the CDR2 sequence contained in the VL; and
   (vi) VL CDR3, having a CDR3 sequence contained in the light chain as set forth in SEQ ID NO:4 or having a sequence with substitution, deletion or addition of one or several amino acids (for example, substitution, deletion or addition of one or two amino acids) as compared with the CDR3 sequence contained in the VL.

In some embodiments, the substitution described in any one of (i) to (vi) is a conservative substitution. In some embodiments, the CDR1, CDR2 and CDR3 contained in the heavy chain variable region (VH), and/or the CDR1, CDR2 and CDR3 contained in the light chain variable region (VL) are defined by the Kabat, Chothia or IMGT numbering system. In some embodiments, the amino acid sequence of the heavy chain of the antibody is as set forth in SEQ ID NO:3, and the amino acid sequence of the light chain of the antibody is as set forth in SEQ ID NO:4.

In some embodiments, in the compound represented by Formula IV, E is a number from 1 to 15. In some embodiments, in the compound represented by Formula IV, E is a number from 1 to 10. In some embodiments, in the compound represented by Formula IV, E is a number from 1 to 8, such as about 1, about 2, about 3, about 4, about 5, about 6, about 7, and about 8.

In some embodiments, the compound represented by Formula IV has a structure represented by FormulaIV-1, 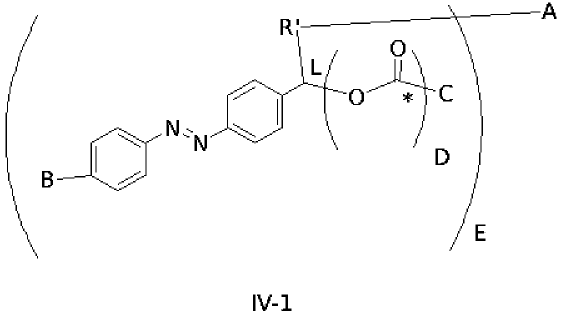 wherein B, R', C, D, E, and A are as defined in the present disclosure.

In some embodiments, in the compound represented by Formula IV, k₂ is 1, 2, 3, 4, 5, 6, 7, 8, 9 or 10. In some embodiments, in the compound represented by Formula IV, k₂ is 1, 2, 3, 4, 5, 6, 7 or 8.

In some embodiments, the compound represented by Formula IV is: 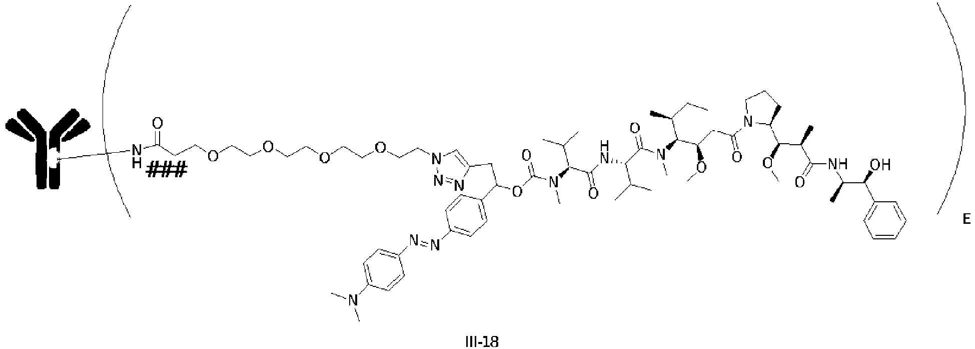 wherein, 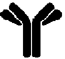 represents A, and A and E are as defined in the present disclosure. In some embodiments, A is trastuzumab, and E is about 3.

In some embodiments, in the above compound, A is an antibody, preferably a monoclonal antibody (MAB), a bispecific antibody or a multispecific antibody, further preferably trastuzumab, an antibody site-specifically mutated with an unnatural amino acid or an antibacterial antibody, and preferably, A is an antibody, which is connected to the position ### via the N atom in the antibody molecule.

The present disclosure also provides a pharmaceutical composition comprising a compound represented by Formula III or Formula IV, a geometric isomer, an optical isomer, a pharmaceutically acceptable salt, a hydrate, a solvate, or a polymorph thereof, and optionally one or more pharmaceutically acceptable carriers or excipients.

In some embodiments, the compound represented by Formula III or Formula IV, a geometric isomer, an optical isomer, a pharmaceutically acceptable salt, a hydrate, a solvate, or a polymorph thereof is present in the pharmaceutical composition in an effective amount.

In some embodiments, the pharmaceutical composition further comprises an additional drug. The additional drug comprises one or more other antibiotics (for example, antibiotics useful for MRSA infections) such as vancomycin, co-trimoxazole, tetracycline, doxycycline/minocycline, clindamycin, cephalosporins (for example, cephalexin), naficillin, fidaxomicin, linezolid, and/or any other suitable antibiotic.

The present disclosure also provides use of the compound represented by Formula III or Formula IV, a geometric isomer, an optical isomer, a pharmaceutically acceptable salt, a hydrate, a solvate, or a polymorph thereof in the manufacture of a medicament for treating a disease or condition or alleviating the severity of the disease or condition, wherein the disease or condition is selected from the group consisting of a tumor, an infectious disease, a hematological disease, a metabolic disease, and an inflammation.

The present disclosure also provides the compound of Formula III or Formula IV, a geometric isomer, an optical isomer, a pharmaceutically acceptable salt, a hydrate, a solvate, or a polymorph thereof for use in treating a disease or condition or alleviating the severity of the disease or condition, wherein the disease or condition is selected from the group consisting of a tumor, an infectious disease, a hematological disease, a metabolic disease, and inflammation.

The present disclosure also provides a method for treating a disease or condition or alleviating the severity of the disease or condition, comprising administering an effective amount of the compound represented by Formula III or Formula IV, a geometric isomer, an optical isomer, a pharmaceutically acceptable salt, a hydrate, a solvate, or a polymorph thereof to a subject in need thereof, wherein the disease or condition is selected from the group consisting of a tumor, an infectious disease, a hematological disease, a metabolic disease, and inflammation.

The present disclosure also provides a pharmaceutical composition for treating a disease or condition or alleviating the severity of the disease or condition, comprising the compound represented by Formula III or Formula IV, a geometric isomer, an optical isomer, a pharmaceutically acceptable salt, a hydrate, a solvate, or a polymorph thereof, and optionally one or more pharmaceutically acceptable carriers or excipients.

In some embodiments, the tumor is selected from the group consisting of cancer, lymphoma, lymphoid neoplasm, blastoma, sarcoma, and leukemia.

In some embodiments, the cancer is selected from the group consisting of: breast cancer (e.g., HER2 positive breast cancer); squamous cell carcinoma (e.g., epithelial squamous cell carcinoma); lung cancer, including small cell lung cancer, non-small cell lung cancer, lung adenocarcinoma and lung squamous cell carcinoma; peritoneal cancer; liver cancer; gastric cancer; gastrointestinal cancer; pancreatic adenocarcinoma; glioblastoma; cervical cancer; ovarian cancer; bladder cancer; urethral carcinoma; hepatocellular carcinoma; intestinal cancer; colon cancer; colorectal cancer; endometrial cancer; uterine cancer; salivary gland cancer; renal cancer; prostate cancer; vulvar cancer; thyroid cancer; anal cancer; penile cancer; melanoma; multiple myeloma and B-cell lymphoma; brain cancer; gallbladder cancer; esophageal cancer; cholangiocarcinoma; head and neck cancer and associated metastatic tumors.

In some embodiments, the infectious disease includes bacterial infections such as infections caused by *Staphylococcus aureus, Mycobacterium tuberculosis, Enterococcus faecium, Acinetobacter baumannii, Clostridium difficile, Streptococcus pneumoniae, and Pseudomonas aeruginosa,* infection-caused sepsis, tuberculosis, bacterial ocular infections, infections of heart, brain or skin, gastrointestinal tract infections, bacterial meningitis, and abscesses in any organ (such as muscle, liver, meninges, or lung).

In some embodiments, the infectious disease is cellulitis, bacteremia, skin necrosis, eyelid infection, ocular infection, neonatal conjunctivitis, osteomyelitis, impetigo, ecthyma, scalded skin syndrome, food poisoning, pneumonia, surgical infection, urinary tract infection, burn infection, meningitis, endocarditis, sepsis, toxic shock syndrome, septic arthritis, tuberculosis, prosthetic joint-associated infection, catheter-associated infection, or implant-associated infection.

In some embodiments, the infectious disease is periprosthetic joint infection caused by *Staphylococcus aureus.*

In some embodiments, the infectious disease is *Staphylococcus aureus* infection of catheter and/or tissue surrounding the catheter.

In some embodiments, the infectious disease is *Staphylococcus aureus* infection of foreign body and/or tissue surrounding the foreign body.

In some embodiments, the infectious disease is tuberculosis.

In the present disclosure, the rifampicin analog is rifalogue, having a structural formula:

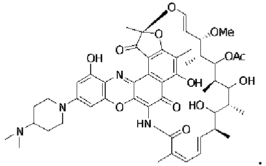

Rifampicin antibiotics (i.e., rifamycin antibiotics) inhibit bacterial RNA polymerases (RNAP) and exhibit potent activity against *Staphylococcus aureus.* However, monotherapy with such antibiotics may select for resistant bacterial populations during treatment. Accordingly, rifamycin antibiotics can be administered in combination with first-line antibiotics to improve clinical outcomes, and are commonly used for infections associated with prostheses or implanted foreign bodies.

The ADCs comprising a rifamycin analog (for example, a compound represented by Formula III or Formula IV, a geometric isomer, an optical isomer, a pharmaceutically acceptable salt, a hydrate, a solvate, or a polymorph thereof, wherein C is rifamycin or an analog thereof, and the other substituents are as described in any embodiment of the present disclosure, for example, a compound represented by Formula III-16) according to the present disclosure may be used for preventing or treating bacterial growth and/or bacterial infection in a subject.

In some embodiments, the bacterium is a Gram-positive bacterium (the Gram-positive bacterium is responsible for bacterial infection).

In some embodiments, the bacterium is a penicillin-resistant bacterium (the penicillin-resistant bacterium is responsible for bacterial infection).

In some embodiments, the bacterium is a *Staphylococcus aureus*, or a methicillin-resistant *Staphylococcus aureus* (MRSA) bacterium (the MRSA bacterium is responsible for bacterial infection).

In some embodiments, the bacterium is a methicillin-sensitive *Staphylococcus aureus* (MSSA) bacterium (the MSSA bacterium is responsible for bacterial infection).

In some embodiments, the bacterium is a vancomycin-resistant *Staphylococcus aureus* (VRSA) bacterium (the VRSA bacterium is responsible for bacterial infection). In some embodiments, the bacterium is a multidrug-resistant *Mycobacterium tuberculosis* (the multidrug-resistant *Mycobacterium tuberculosis* is responsible for bacterial infection).

In some embodiments, the bacterium is Chlamydia trachomatis resistant to antibiotics such as azithromycin (the Chlamydia trachomatis resistant to antibiotics such as azithromycin is responsible for bacterial infection).

In some embodiments, the bacterium is Clostridium difficile resistant to antibiotics such as metronidazole, vancomycin, and/or fidaxomicin (the Clostridium difficile resistant to antibiotics such as metronidazole, vancomycin, and/or fidaxomicin is responsible for bacterial infection).

The present disclosure provides a method of preventing or treating cellulitis, bacteremia, skin necrosis, eyelid infection, ocular infection, neonatal conjunctivitis, osteomyelitis, impetigo, ecthyma, scalded skin syndrome, food poisoning, pneumonia, surgical infection, urinary tract infection, burn infection, meningitis, endocarditis, sepsis, toxic shock syndrome, septic arthritis, mastitis, prosthetic joint-associated infection, catheter-associated infection, or implant-associated infection in a subject, comprising administering to the subject a therapeutically effective amount of an antibody-drug conjugate comprising a rifampicin analog (for example, the compound represented by Formula III or IV, a geometric isomer, an optical isomer a pharmaceutically acceptable salt, a hydrate, a solvate, or a polymorph thereof, wherein C is rifampicin or an analog thereof, and the other substituents are as described in any embodiment of the present disclosure, for example, a compound represented by Formula III-16).

The present disclosure also provides a method of preventing or treating intracellular bacterial infection in a subject, comprising administering to the subject a therapeutically effective amount of an antibody-drug conjugate comprising a rifampicin analog (for example, the compound represented by Formula III or IV, a geometric isomer, an optical isomer, a pharmaceutically acceptable salt, a hydrate, a solvate, or a polymorph thereof, wherein C is rifampicin or an analog thereof, and the other substituents are as described in any embodiment of the present disclosure, for example, a compound represented by Formula III-16).

In some embodiments, the present disclosure provides a treating method, comprising administering to a subject in need thereof an ADC comprising a Rifampicin analog (for example, the compound represented by Formula III or IV, a geometric isomer, an optical isomer, a pharmaceutically acceptable salt, a hydrate, a solvate, or a polymorph thereof, wherein C is rifampicin or an analog thereof, and the other substituents are as described in any embodiment of the present disclosure, for example, a compound represented by Formula III-16) for treating and/or preventing bacterial infection, and/or a disease or disorder or condition associated with *Staphylococcus* infection (for example, *Staphylococcus aureus* infection), and/or alleviating at least one symptom associated with this disease or disorder or condition in the subject. This disease, disorder or condition may be cellulitis, bacteremia, skin necrosis, eyelid infection, ocular infection, neonatal conjunctivitis, osteomyelitis, impetigo, ecthyma, scalded skin syndrome, food poisoning, pneumonia, surgical infection, urinary tract infection, burn infection, meningitis, endocarditis, sepsis, toxic shock syndrome, and septic arthritis. In some embodiments, the subject has a prosthetic joint, and the antibody-drug conjugate disclosed in the present disclosure may be useful for treating and/or preventing periprosthetic joint infection caused by *Staphylococcus aureus*.

In some embodiments, the subject has a catheter, and the antibody-drug conjugate disclosed in the present disclosure may be useful for treating and/or preventing *Staphylococcus aureus* infection of the catheter and/or tissue surrounding the catheter.

In some embodiments, the subject has an implanted foreign body, and the antibody-drug conjugate disclosed in the present disclosure may be useful for treating and/or preventing *Staphylococcus aureus* infection of the foreign body and/or tissue surrounding the foreign body.

In some embodiments, the subject has tuberculosis, and the antibody-drug conjugate disclosed in the present disclosure may be useful for treating tuberculosis.

In some embodiments, the ADCs comprising a rifampicin analog may be administered in combination with one or more other antibiotics (e.g., antibiotics useful for MRSA infections) such as vancomycin, co-trimoxazole, tetracycline, doxycycline/minocycline, clindamycin, cephalosporins (e.g., cephalexin), nafcillin, fidaxomicin, linezolid, and/or any other suitable antibiotics. In some embodiments, the ADCs comprising a rifamycin analog according to the present disclosure may be administered in combination with vancomycin and may be used for preventing or treating bacterial infection in a subject.

### Definitions

As used herein, the term "antibody" is a common immunoglobulin, being a Y-shaped protein used by the immune system to recognize and neutralize foreign bodies (such as bacteria and viruses). An antibody is capable of specifically recognizing a unique part of a foreign target (called as "antigen") because each tip of the Y-shaped protein antibody contains a site capable of specifically recognizing the antigen. Following binding of an antibody to its specific antigen, a variety of related biological effects can be mediated. An antibody consists of two identical heavy chains and two identical light chains, with the chains interconnected via disulfide bonds formed by thiol groups of cysteine residues. A "monoclonal antibody" is an antibody with a single antigenic specificity, in which all the antibody molecules consist of identical immune cell clones derived from a single parent cell, so all the antibody molecules are identical.

As used herein, the term "cytotoxin" refers to those molecules that exert toxic effects on cancer cells upon release in such cells. Cytotoxins of particular interest in the present disclosure include monomethyl auristatin E (MMAE), auristatin, maytansine or a derivative thereof (such as maytansinoids, DM1, DM3, DM4), calicheamicin, duocarmycin, doxorubicin, camptothecin, or PBD cytotoxins.

As used herein, the term "linker" is a molecule having two reactive ends, one end of which can be conjugated to an antibody and the other end of which is used to conjugate an active compound, such as a cytotoxin. The antibody conjugating end of the linker is generally a site capable of conjugation with the thiol group of a cysteine residues or the amino group of lysine residues on the antibody, and the cytotoxin conjugating end of the linker is generally a reactive site capable of conjugation with thiol, amino, carboxyl, hydroxyl or other functional groups on a cytotoxin molecule. When the term "linker" is used to describe a linker in conjugated form, since the linker has reacted with one or both of the antibody and the cytotoxin to form a covalent bond, it may no longer comprise one or both reactive sites at reactive ends (such as a leaving group of a thiol reactive group, and leaving group of an amine reactive group).

As used herein, the term "antibody conjugated drug" or "ADC" or "antibody-drug conjugate" has the identical meanings and refer to a product formed by conjugating multiple (typically 1 to 8) cytotoxin molecules to an antibody molecule via linkers respectively. An antibody is conjugated to one or more cytotoxins. The antibody is generally a monoclonal antibody having a selectivity for the specific antigen of a cancer.

As used herein, the term "about" can be understood as a value which is within +/-20%, +/-18%, +/-15%, +/-12%, +/-10%, +/-9%, +/-8%, +/-7%, +/-6%, +/-5%, +/-4%, +/-3%, +/-2%, +/-1%, +/-0.5%, +/-0.4%, +/-0.3%, +/-0.2%, +/-0.1% of the mentioned value. Unless obvious from the context, all numerical values provided herein are modified by the term "about".

Exemplary tumor diseases targeted by the antibody-drug conjugate described herein include, but are not limited to, cancers, breast cancer, lymphoma, lymphoid neoplasm, blastoma, sarcoma, and leukemia. More specific examples of such cancers include squamous cell carcinoma (e.g., epithelial squamous cell carcinoma); lung cancer, including small cell lung cancer, non-small cell lung cancer, lung adenocarcinoma, and lung squamous cell carcinoma; peritoneal cancer; liver cancer; gastric cancer including gastrointestinal cancer; pancreatic adenocarcinoma; glioblastoma; cervical cancer; ovarian cancer; liver cancer; bladder cancer; urethral carcinoma; hepatocellular carcinoma; breast cancers including HER2 positive breast cancers; colon cancer; rectal cancer; colorectal cancer; endometrial carcinoma; uterine cancer; salivary gland cancer; kidney cancer; prostate cancer; vulvar cancer; thyroid cancer; anal cancer; penile cancer; melanoma; myeloma and B cell lymphoma; brain cancer; head and neck cancer and associated metastatic tumors.

Exemplary infectious diseases targeted by the antibody-drug conjugate described herein include, but are not limited to, bacterial infections such as those caused by *Staphylococcus aureus*, *Enterococcus faecium*, *Acinetobacter baumannii*, *Clostridioides difficile*, *Streptococcus pneumoniae*, and *Pseudomonas aeruginosa*, infection-caused sepsis, tuberculosis, bacterial ocular infections, infections of heart, brain or skin, gastrointestinal tract infections, bacterial meningitis, and abscesses in any organ (such as muscle, liver, meninges, or lung).

As used herein, the term "salt" refers to salts that retain the biological effectiveness and properties of a compound and are not biologically or otherwise undesirable for pharmaceutical use. In many cases, the compounds disclosed herein are capable of forming acidic and/or basic salts via the presence of amino and/or carboxyl groups or similar groups. Pharmaceutically acceptable acidic addition salts may be formed with inorganic and organic acids. Inorganic acids from which salts can be derived include, for example, hydrochloric acid, hydrobromic acid, sulfuric acid, nitric acid, and phosphoric acid. Organic acids from which salts can be derived include, for example acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, and salicylic acid. Pharmaceutically acceptable basic addition salts may be formed with inorganic and organic bases. Inorganic bases from which salts can be derived include, for example, hydroxides of sodium, potassium, lithium, ammonium, calcium, magnesium, iron, zinc, copper, manganese, and aluminum; particularly preferred are salts of ammonium, potassium, sodium, calcium and magnesium. Organic bases from which salts can be derived include, for example, primary, secondary and tertiary amines, substituted amines, including naturally occurring substituted amines, cyclic amines, and basic ion exchange resin, specifically include, for example, isopropylamine, trimethylamine, diethylamine, triethylamine, tripropylamine, and ethanolamine. Many of such salts are known in the art, as described by WO87/05297, Johnston et al, published in 1987, 9, 11 (which is incorporated herein by reference in its entirety).

As used herein, the term "aryl" refers to an unsaturated aromatic carbocyclic group containing 5 to 14 carbon atoms with a single ring or two or more fused rings that possess a conjugated π-electron system. The "aryl" preferably contains 5 to 10, 5 to 8 or 5 to 6 carbon atoms. Representative examples of the "aryl" include, but are not limited to, phenyl, naphthyl, anthryl, and the like.

As used herein, the term "heteroaryl" refers to an aryl group as defined herein wherein at least one ring member is a heteroatom selected from the group consisting of nitrogen, oxygen and sulfur. The "heteroaryl" preferably has 5 to 10, 5 to 8 or 5 to 6 ring members. Representative examples of the "heteroaryl" include, but are not limited to, furyl, imidazolyl, thienyl, triazolyl, indolyl, tetrazolyl, pyridyl, pteridinyl, pyrimidinyl, triazolyl, quinolinyl, isoquinolinyl, quinazolinyl, quinoxalinyl, and the like.

As used herein, the term "aryl-fused heterocyclyl" refers to a cyclic group having two or more fused rings in which two or more carbon atoms are shared by two adjacent rings, at least one ring is the aryl as defined herein and at least one ring is a heterocyclyl.

As used herein, the term "heteroaryl-fused heterocyclyl" refers to a cyclic group with two or more fused rings in which two or more carbons are shared by two adjacent rings, at least one ring is the heteroaryl as defined herein and at least one ring is a heterocyclyl.

As used herein, the term "heterocyclyl" refers to a saturated or partially unsaturated cyclic hydrocarbyl group having a single ring or two or more fused rings (including fused, bridged, and spiro rings) containing 3 to 12 ring members, and at least one ring member is a heteroatom selected from the group consisting of nitrogen, oxygen, and sulfur. The "heterocyclyl" preferably has 3 to 10, 3 to 8, 5 to 8, 3 to 6 or 5 to 6 ring members. Representative examples of the "heterocyclyl" include, but are not limited to, tetrahydrofuranyl, tetrahydrothienyl, pyrrolidinyl, piperazinyl, thiazinyl, piperidinyl, and morpholinyl, and the like.

### Abbreviations

ADC: Antibody-drug conjugate;
AcOH: Acetic acid;
Boc: *t*-Butyloxy carbonyl;
Cys: *L*-Cysteine;
DAR: Drug to antibody ratio;
DCM: Dichloromethane;
DIPEA: *N,N*-Diisopropylethylamine;
DMAC: *N,N*-Dimethylacetamide;
DMF: *N,N*-Dimethylformamide;
DMSO: Dimethyl Sulphoxide;
Diox: 1,4-Dioxane;
DIAD: Diisopropylazodicarboxylate;
DIC: Diisopropylcarbodiimide;
EA: Ethyl acetate;
EtOH: Ethanol;
EDTA: Ethylenediamine tetraacetic acid;
EDCI: 1-(3-Dimethylaminopropyl)-3-ethylcarbodiimide Hydrochloride;
FITC: Fluorescein isothiocyanate;
HOBT: 1-Hydroxybenzotriazole;
HCl: Hydrogen chloride; HER2: Human epidermal growth factor receptor 2;
MAB: Monoclonal Antibody;
MMAE: Monomethyl auristatin E;
NAC: *N*-Acetyl-*L*-cysteine;
NADPH: Nicotinamide adenine dinucleotide phosphate;
NH₂NH₂: Hydrazine;
NHS: *N*-Hydroxy succinimide;
PPh₃: Triphenylphosphine;
TCEP: Tris(2-carboxyethyl)phosphine;
THF: Tetrahydrofuran;
TFA: Trifluoroacetic acid;
PE: Petroleum ether;
VC-ADC (Herceptin-VC-MMAE): a cathepsin-cleavable antibody-drug conjugate with MMAE as an effector molecule, which can be obtained by conjugating Herceptin with VC-MMAE (commercially available) according to methods reported in the art, wherein DAR is about 4.

When the names of the compounds used herein are inconsistent with the chemical structural formulas, the chemical structural formulas shall prevail.

The pharmaceutical composition as described herein comprises the compound represented by Formula III or Formula IV, a salt, or a solvate thereof according to the present disclosure, and a conventional pharmaceutical carrier or excipient. The pharmaceutical composition can be administered by routes such as oral or parenteral administration, for example, intravenous injection, intraperitoneal injection, intramuscular injection, and subcutaneous injection.

As used herein, the term "effective amount" refers to an amount sufficient to achieve a desired therapeutic effect, for example, an amount that achieves the alleviation of symptoms associated with a disease to be treated.

In addition, it should be pointed out that the dosage and administration regimen of the compounds according to the present disclosure depend upon a variety of factors including the patient's age, weight, gender, natural health status, nutritional status, activity intensity of a compound, administration time, metabolic rate, severity of disease, and subjective judgment of physician. The preferred dosage range is between 0.01 mg/kg body weight/day and 100 mg/kg body weight/day.

The linker provided in the present disclosure contains an azobenzene group and exhibits good enzymatic cleavage performance. The antibody-drug conjugate (ADC) containing the linker provided by the present disclosure has good structural stability, ideal enzymolytic drug release performance, hypoxia dependency, as well as desirable *in-vivo* and *in-vitro* efficacy and safety.

In some specific embodiments, a synthetic route of the ADC according to the present disclosure is as follows: 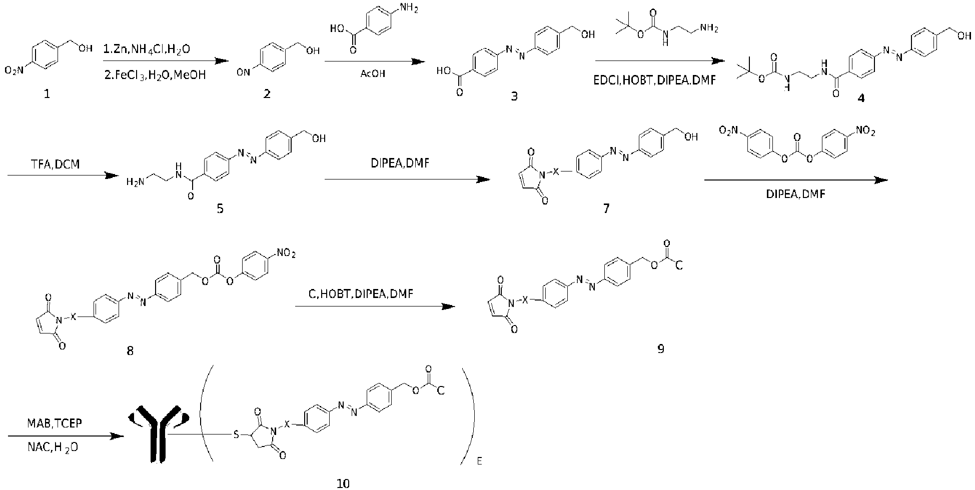 wherein: C, E, 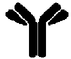 are as defined in the present disclosure, X represents -V-L-W-, and V, L, and W are as defined in the present disclosure.

p-Nitrobenzyl alcohol (1) is used as a starting material, and reduced with zinc powders to obtain an unstable intermediate p-aminobenzyl alcohol, which is further oxidized by ferric trichloride to obtain an intermediate (2). Intermediate (2) reacts with 4-aminobenzoic acid under acetic acid to prepare a compound (3). In the presence of EDCI, HOBT and DIPEA, amide condensation is subjected to prepare a compound (4). Deprotection of the Boc protecting group with trifluoroacetic acid gives a compound (5), which undergoes a subsequent substitution reaction to prepare a compound (7). The compound (7) reacts with bis(p-nitrophenyl) carbonate to obtain a compound (8), followed by reaction with the active compound C to prepare a corresponding ADC load (9), which is further conjugated to an antibody to obtain a final ADC product (10).

In some specific embodiments, another synthetic route of the ADC according to the present disclosure is as follows: 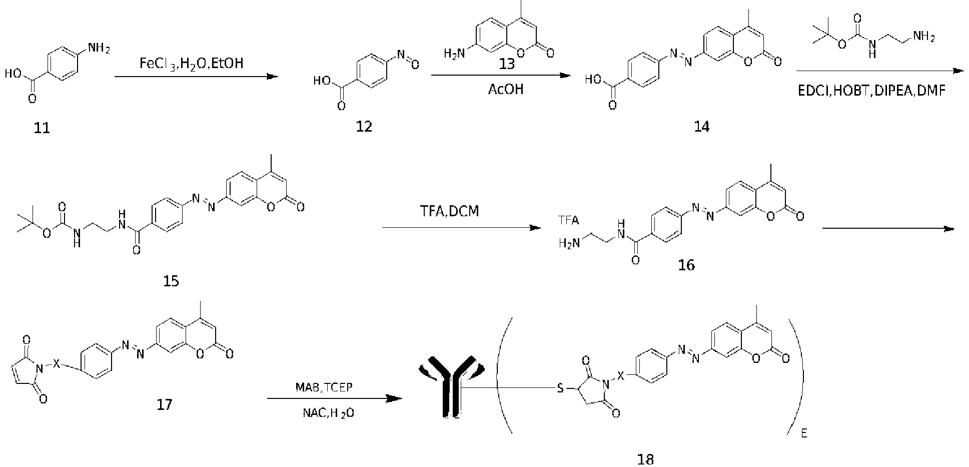 wherein: E, 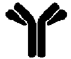 are as defined in the present disclosure, X represents -V-L-W-, and V, L, and W are as defined in the present disclosure.

p-Aminobenzoic acid (11) is used as a starting material, and oxidized with ferric trichloride to obtain a compound (12), which reacts with a compound (13) under acetic acid to prepare a compound (14). In the presence of EDCI, HOBT and DIPEA, amide condensation is subjected to prepare and obtain a compound (15). Deprotection of the Boc protecting group Boc protective group with trifluoroacetic acid gives a compound (16), which undergoes a subsequent substitution reaction to prepare a compound(17). The compound (17) is conjugated to an antibody to obtain a final ADC product (18).

In some specific embodiments, another synthetic route of the ADC according to the present disclosure is as follows: 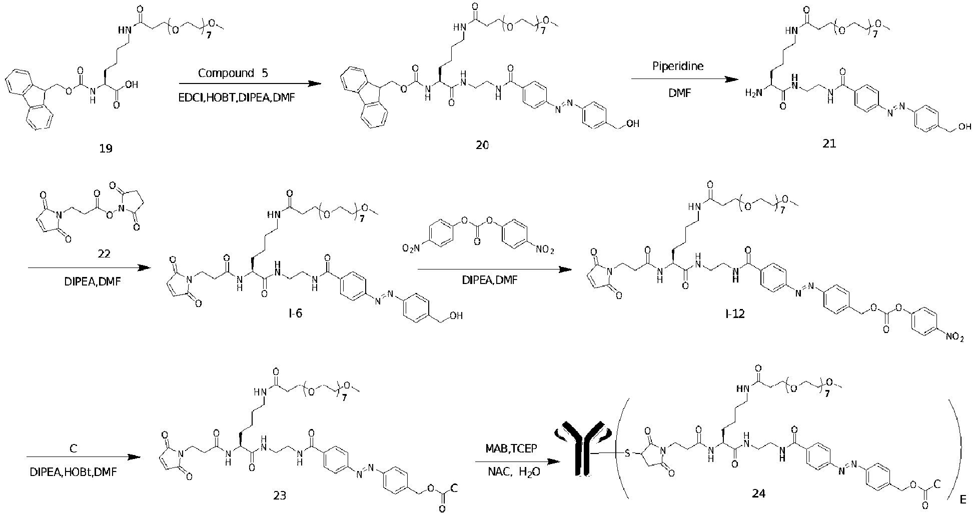 wherein: E, 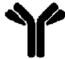 are as defined in the present disclosure,

Compound (19) is used as a starting material, and undergoes amide condensation in the presence of EDCI, HOBT and DIPEA to obtain compound (20), the Fmoc protecting group of which is removed with piperidine to obtain compound (21), which reacts with compound (22) to obtain compound I-6. The compound I-6 which reacts with bis(p-nitrophenyl) carbonate to obtain compound I-12, followed by reaction with the active compound C to obtain a corresponding ADC load (23), which is further conjugated with an antibody to obtain a final ADC (24).

In some specific embodiments, another synthetic route of the ADC according to the present disclosure is as follows: 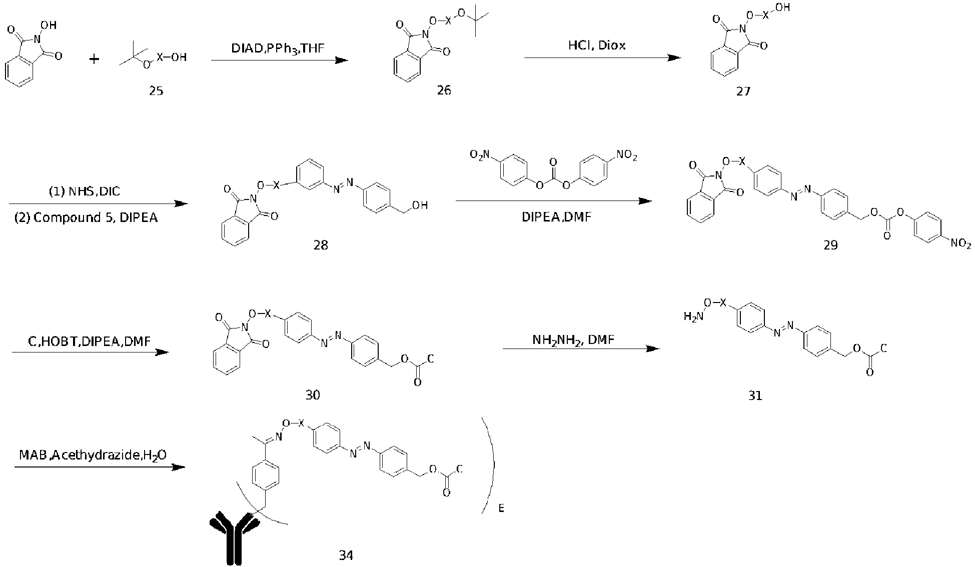 wherein: C, E, 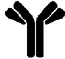 are as defined in the present disclosure, X represents -V-L-W-, and V, L, and W are as defined in the present disclosure.

2-Hydroxyisoindoline-1,3-dione and compound (25) are used as starting materials, compound (26) is prepared in the presence of DIAD and PPh₃, the tert-butoxy protecting group of which is removed with hydrochloric acid to prepare compound (27). Compound (27) undergoes amide condensation with compound (5) under the action of DIC to prepare compound (28). The compound (28) reacts with bis(p-nitrophenyl) carbonate to obtain compound (29), which further reacts with the active compound C to prepare compound (30), benzomaleimide group of which is removed with NH₂NH₂ to obtain compound (31), which is further conjugated with an antibody to obtain a final ADC product (32).

In some specific embodiments, another synthetic route of the ADC according to the present disclosure is as follows: 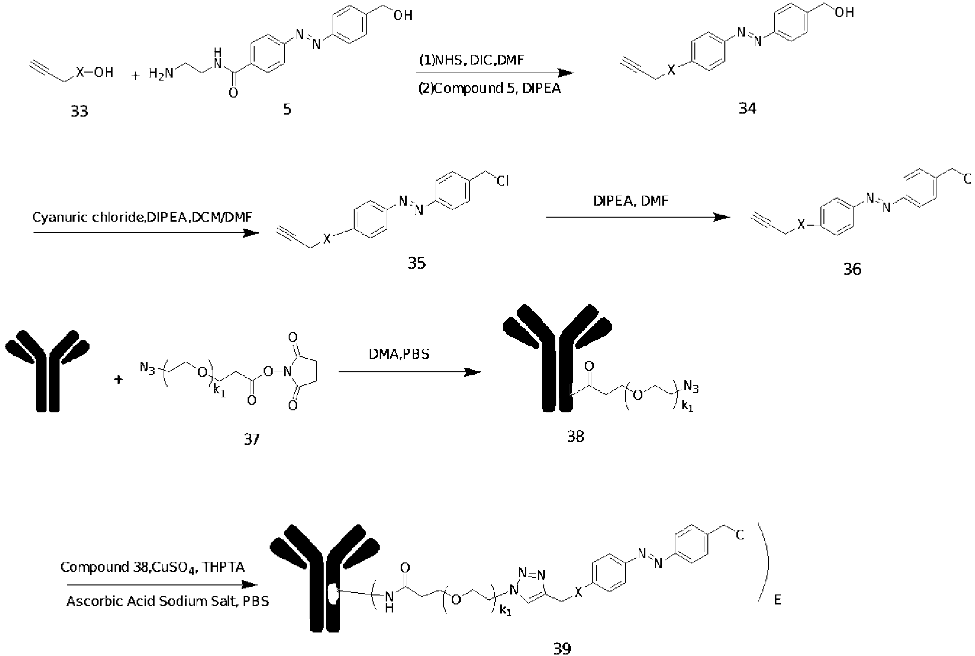 wherein: C, E, k₁ are as defined in the present disclosure, X represents -V-L-W-, and V, L, and W are as defined in the present disclosure.

Compound (33) and compound (5) are used as starting materials to prepare compound (34) in the presence of DIC, compound (34) undergoes chlorination with cyanuric chloride to prepare compound (35), which is then reacted with the active compound C to obtain compound (36). An antibody is conjugated to compound (37) to prepare a conjugate (38), and further, compound (38) is conjugated with compound (36) to obtain a final ADC product (39) via click reaction.

In some specific embodiments, the synthetic route of the albumin conjugate according to the present disclosure is as follows: 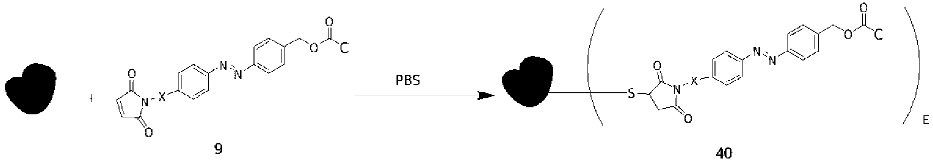 wherein: X, C, E are as defined in the present disclosure,

Compound (9) is conjugated with albumin to prepare conjugate (40).

### Brief Description of the Drawings

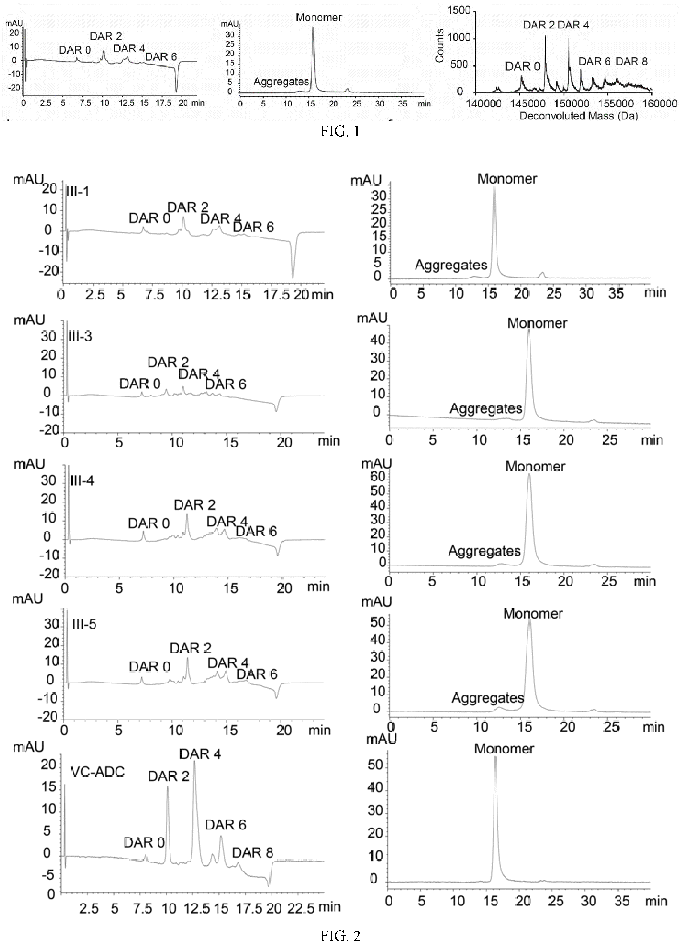

FIG. 1 shows the test results of DAR and aggregation degree of compound III-2.
FIG. 2 shows the test results of DAR and aggregation degree of compounds III-1, III-3, III-4, III-5 and VC-ADC.

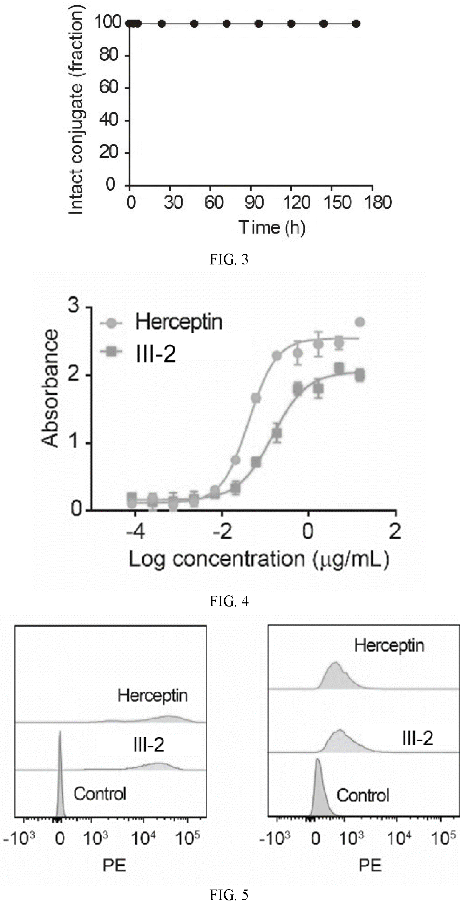

FIG. 3 shows the evaluation results of stability of compound III-2 in plasma.
FIG. 4 shows the study results of the binding affinity of compound III-2 to HER2 antigen. The results show that compound III-2 exhibits a binding affinity to HER2 antigen comparable to that of Herceptin, with EC₅₀ values of 0.14 µg/mL and 0.042 µg/mL, respectively (FIG. 4).
FIG. 5 shows the study results of the binding affinity of compound III-2 on cell lines. The results show that both Herceptin and compound III-2 exhibits strong fluorescence signals in HER2-positive SKOV3 cells (the right panel in FIG. 5), while weak fluorescence is observed in HER2-negative MCF-7 cells (the left panel in FIG. 5), indicating that compound III-2 specifically targets the HER2 antigens on tumor cells.

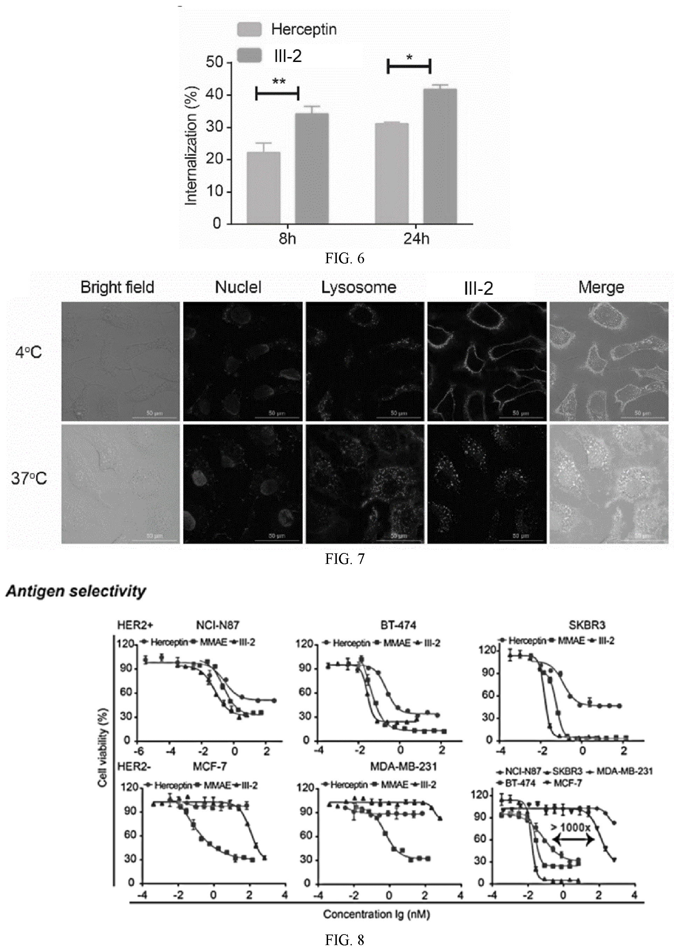

FIG. 6 shows the cellular internalization study results of compound III-2 on cell lines. The results show that compound III-2 exhibits a slightly higher internalization rate than Herceptin, with internalization efficiencies of 34.1% versus 22.1% at 8 h, and 41.7% versus 31.1% at 24 h.
FIG. 7 shows the cellular binding and internalization imaging results of Compound III-2 on cell lines. The results show that cells were incubated with Herceptin and compound III-2 at 4 °C, under such conditions, the antibody binds to the antigen on the cell membrane surface without intracellular internalization. After 24-hour incubation at 37 °C, intracellular protein fluorescence signals and lysosomal fluorescence signals are co-localized (pearson correlation coefficient = 0.87), indicating that compound III-2 is internalized and transported to lysosomes.
FIG. 8 shows the cell proliferation inhibitory activity study results of compound III-2 on cell lines with positive and negative HER2 antigen expressions. The results show that under hypoxic conditions, compound III-2 shows potent cell proliferation inhibitory activity on NCI-N87, BT-474 and SKBR3 cell lines, with EC₅₀ values of 0.07 nM, 0.026 nM and 0.015 nM, respectively, but shows weak activity on HER2-negative cell lines (MCF-7, MDA-MB-231) (with EC₅₀ >100 nM).

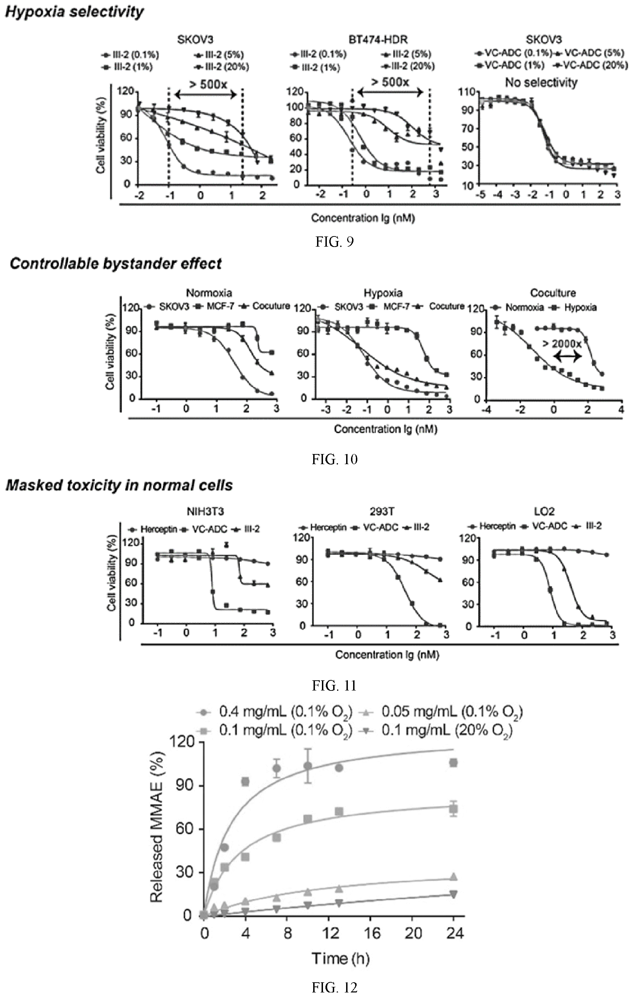

FIG. 9 shows in vitro hypoxia-dependent cytotoxicity study results of compound III-2. The results show that compound III-2 exhibits oxygen concentration-dependent cell proliferation inhibitory activity, and the cell proliferation inhibitory activity increases as the oxygen concentration decreases. Under the condition of the oxygen concentrations at 20%, 5%, 1% and 0.1%, the EC₅₀ values of compound III-2 against SKOV3 cells are 66.8 nM, 3.74 nM, 0.079 nM, and 0.063 nM, respectively, while the EC₅₀ values against BT474-HDR cells are 109.1 nM, 9.12 nM, 0.57 nM, and 0.19 nM, respectively.
FIG. 10 shows the bystander effect study results of compound III-2. Under the normoxic conditions, the cytotoxic activity of compound III-2 in a co-culture model decreases to the micromolar level (EC₅₀ ≈ about 0.1 µM), and the result shows that compound III-2 has high safety in normal tissues. Under hypoxic conditions, compound III-2 maintaines high activity in the co-culture model, with an EC₅₀ of 0.053 nM. The above results show that compound III-2 has bystander effect in tumor tissues and has killing effect on tumor cells with low HER2 expression.
FIG. 11 shows the proliferation inhibitory activity study results of compound III-2 on normal cell lines. At the maximum tested concentration (666 nM), compound III-2 shows weak inhibitory activity against normal NIH3T3 and 293T cells, with a maximum inhibition rate of <50%. However, conventional VC-ADCs still show high off-target cytotoxicity (with EC₅₀ <10 nM, and a maximum inhibition rate >90%).
FIG. 12 shows the drug release study results of compound III-2 under the action of azoreductase. The results show that the release of MMAE exhibits dual dependency on azoreductase concentration and oxygen concentration. Under hypoxic conditions (0.1% O₂), the release of MMAE increases as the concentration of the azoreductase increases, and after 24-hour incubation of the substrate with azoreductase (at 0.4 mg/mL, 0.1 mg/mL and 0.05 mg/mL), the release rates of MMAE are 100%, 74.3% and 27.49%, respectively. Under normoxic conditions (20% O₂), after 24-hour incubation of the substrate with 0.1 mg/mL of azoreductase, only 14.99% of MMAE is released, and the results indicate that the azoreductase-cleavable linker provided by the present disclosure can be cleaved to release cytotoxins under the combined action of hypoxia and azoreductase, whereas under the action of azoreductase alone, the release amount of the cytotoxins is very low.

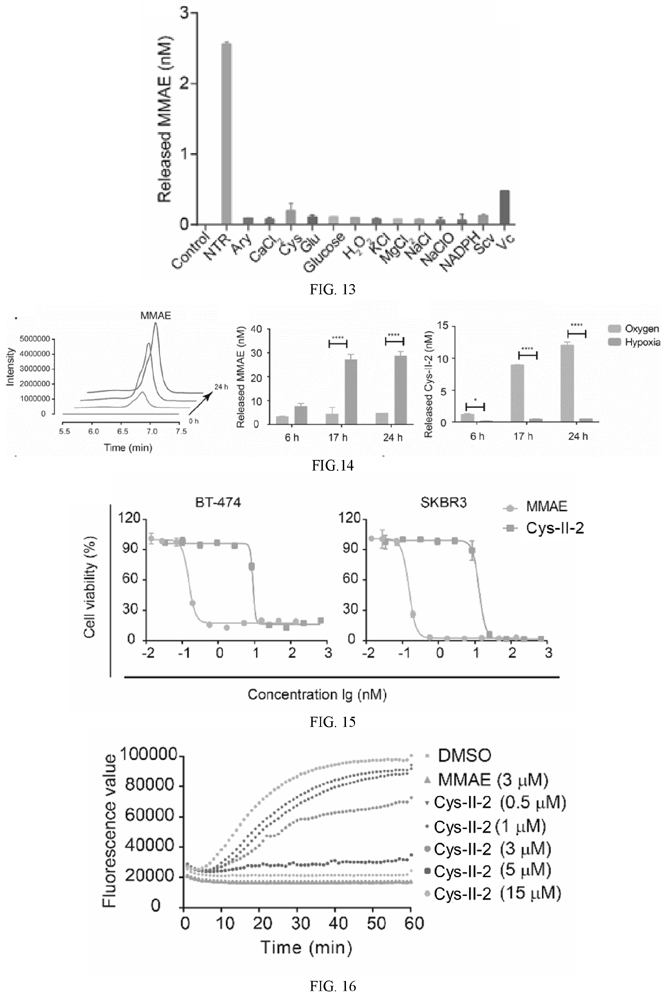

FIG. 13 shows study results of the effects of oxidants, reductants and salts on the stability of compound II-2. The results show that NAC-II-2 can release MMAE only under hypoxic conditions via azoreductase, and exhibits excellent stability when co-incubating with other substances related to redox, such as Cys, NADPH, and NaClO.
FIG. 14 shows the intracellular metabolite study results of compound III-2. The results show that SKOV3 cells were co-cultured with compound III-2 under hypoxic conditions, MMAE was detected in the SKOV3 cells, and the release of MMAE is time-dependent, directly indicating that compound III-2 can be cleaved to release the MMAE in tumor cells under hypoxic conditions. More importantly, when SKOV3 cells was co-cultured with compound III-2 under normoxic conditions, Cys-II-2 is the major metabolite and its release amount is time-dependent, while under hypoxic conditions, the amount of the Cys-II-2 in the cells is very low. The results show that the Cys-II-2 released after antibody degradation of compound III-2 under normoxic conditions is not further cleaved by azoreductase.
FIG. 15 shows the cytotoxicity study results of Cys-II-2. The results show that the cytotoxic activity of Cys-II-2 is more than ten folds to several hundred folds lower than that of MMAE.
FIG. 16 shows the tubulin inhibition study results of Cys-II-2. The results show that the tubulin inhibitory activity of the Cys-II-2 is reduced as compared to MMAE in a concentration-dependent manner.

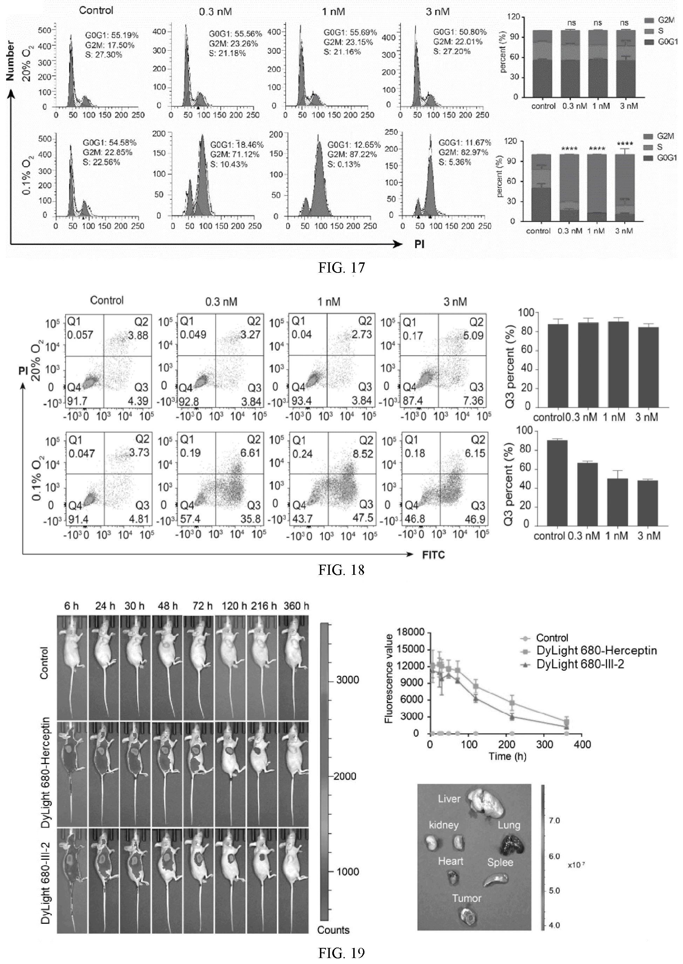

FIG. 17 shows the cell cycle arrest study results of compound III-2. The results show that compound III-2 induces G2/M phase arrest in tumor cells with hypoxia selectivity. Under normoxic conditions, after treatment with compound III-2 at the concentrations of 0.3 nM, 1 nM and 3 nM, the proportion of the cells in the G2/M phase is similar to that of the untreated control group (17.50%). In contrast, under hypoxic conditions, after treatment with compound III-2 under the same conditions, the proportion of the cells in the G2/M phase ranges from 71.12% to 87.22%.
FIG. 18 shows study results of compound III-2 for inducing cell apoptosis. The results show that compound III-2 almost has no effect on cell apoptosis under normoxic conditions (20% O₂), and in contrast, compound III-2 induces apoptosis in more than half of the cells under hypoxic conditions (0.1% O₂). The cell apoptosis results show that compound III-2 has good hypoxic selectivity.
FIG. 19 shows the *in vivo* distribution of Compound III-2 labeled with DyLight-680 dye in mice. The results shows that DyLight 680-labeled DyLight 680-Herceptin (25 mg/kg) and DyLight 680-III-2 (25 mg/kg) were administered to the mice via tail vein injection, strong fluorescence signals is clearly observed at a tumor site 6 hour after the administration, and the fluorescence signals at the tumor site are clearly maintained for about 9 days, with the fluorescence signal completely disappearing on the 15^{th} day after the administration. From the variation curve of the fluorescence signal intensity, it is found that the curve of the DyLight 680-III-2 is almost consistent with that of the DyLight 680-Herceptin, indicating that compound III-2 has metabolic properties similar to Herceptin. In addition, the fluorescence intensity at the tumor site is significantly higher than that in other normal tissues. The results show that compound III-2 has excellent tumor targeting property and a longer half-life in vivo.

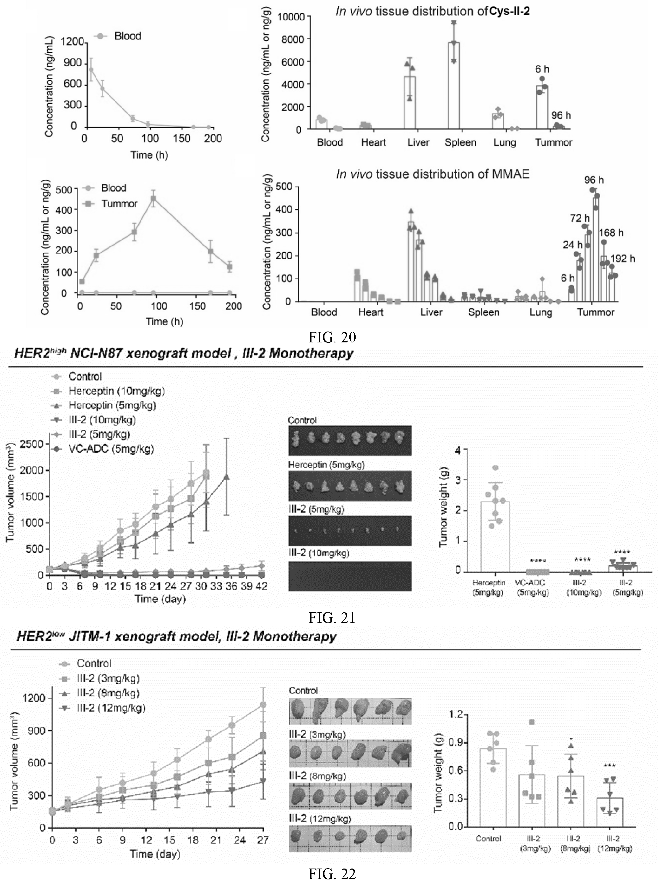

FIG. 20 shows drug distribution and metabolism of compound III-2. The results show that high concentrations of a prodrug Cys-II-2 are detected in blood and peripheral tissues 6 h after the administration of compound III-2. In addition, the results show that Cys-II-2 may be rapidly cleared in normal tissues, with a blood half-life of only 26 hours, which further reduce its toxic effects and side effects. At 96 hours after the administration, Cys-II-2 is hardly detectable in heart, liver, spleen, and lung. The peak concentration and area under the drug-time curve of the cytotoxin MMAE in tumors are 293 times and 941 times those in peripheral blood, respectively, further proving the excellent targeting capability of compound III-2. The area under the drug-time curve of MMAE in heart, liver, spleen and lung are 11%, 47%, 5% and 7% of area under the drug-time curve in tumors, respectively. The results show that Cys-II-2 in peripheral tissues cannot be metabolized to generate MMAE except in the liver, while Cys-II-2 in tumors can effectively release MMAE.
FIG. 21 shows antitumor efficacy of compound III-2 in NCI-N87 tumor-bearing mice. The results show that in the NCI-N87 tumor xenograft model, as compared with the control group, compound III-2 significantly inhibited tumor growth at the administration dose of 5 mg/kg, with a tumor inhibition rate of 90.97%. The results show that compound III-2 exhibits anti-tumor efficacy nearly equivalent to that of conventional VC-ADC. More importantly, complete tumor regression is observed in all mice in the administration group at the dose of 10 mg/kg, indicating the excellent antitumor therapeutic potential of compound III-2.
FIG. 22 shows antitumor efficacy of compound III-2 in JIMT-1 tumor-bearing mice. The results show that compound III-2 still exhibits antitumor effects at a dose of 12 mg/kg in the JIMT-1 xenograft model, and although complete tumor regression was not achieved, the tumor growth inhibition rate reaches 62.36%.

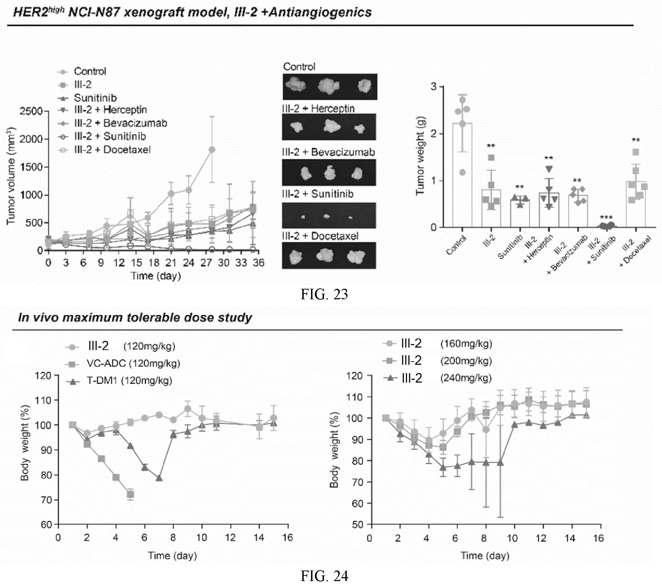

FIG. 23 shows antitumor efficacy of the combination administration of compound III-2 with Herceptin, bevacizumab, sunitinib or docetaxel in NCI-N87 tumor-bearing mice. The results show that the combination group of compound III-2 and sunitinib exhibits superior antitumor efficacy (p <0.01), which is better than compound III-2 monotherapy and other combination groups. In the combination administration group, treatment with only 3 mg/kg of compound III-2 can achieve nearly complete tumor regression, with a tumor growth inhibition rate of 98.93%. The above results show that the excellent tumor growth inhibitory effect of the combination therapy can be achieved due to the angiogenesis inhibitor that can expand the hypoxic regions of tumor tissues and aggravate the hypoxic degree of the tumor tissues, thereby resulting in the further improvement of the therapeutic efficacy of compound III-2.
FIG.24 shows the maximum tolerated dose (MTD) research results of compound III-2. The results show that the MTD of compound III-2 is higher than 200 mg/kg, while the mice in the VC-ADC group and the T-DM1 group experience continuous weight loss (>20%) and severe adverse reactions after administration, and for example, all mice in the VC-ADC group die after receipt of treatment at a dose of 120 mg/kg.

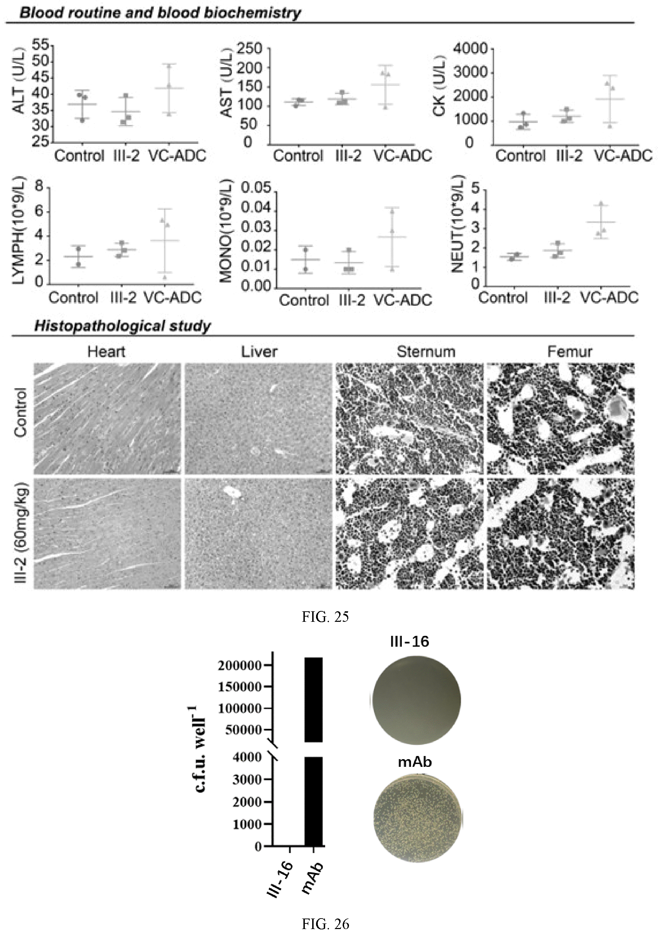

FIG.25 shows hematological and histopathological studies results of compound III-2 in BALB/C mice. The results show that there is no significant differences in biochemical indicators, routine blood parameters and histopathological blood markers between the compound III-2 treatment group and the control group, while the changes in serum biochemical indicators in the VC-ADC treatment group reflect liver side effects induced by VC-ADC, manifested as increased levels of serum alanine aminotransferase (ALT), aspartate aminotransferase (AST) and creatine kinase (CK). The side effects of VC-ADC are also reflected in the hematological changes, including increased counts of lymphocytes (LYMPH), monocytes(MONO), and neutrophils (NEUT).
FIG. 26 shows that compound III-16 has excellent anti-*Staphylococcus aureus* efficacy.

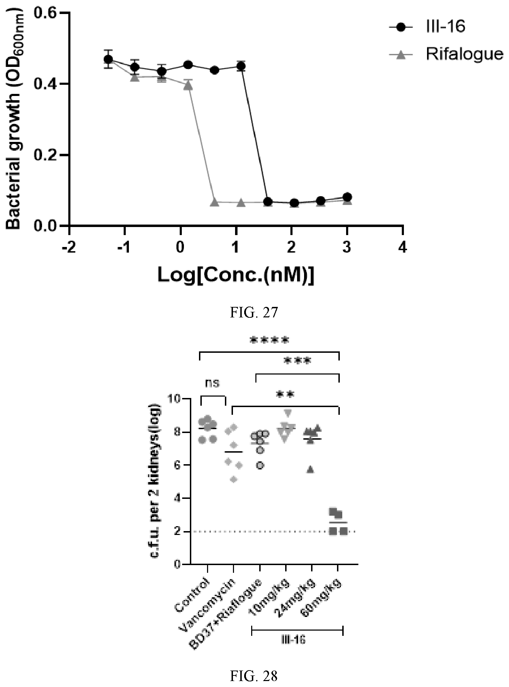

FIG. 27 shows that compound III-16 has antibacterial activity comparable to that of free rifalogue.
FIG. 28 shows that compound III-16 has significant therapeutic effect in a bacterial infection mouse model.

### Detailed Description of the Preferred Embodiments

The embodiments of the present disclosure will be described in detail below by combining the examples. However, it should be understood for a person skilled in the art that the following examples are only for illustrating the present disclosure but not construed as limiting the scope of the present disclosure. The examples, of which specific conditions are not specified, are carried out in accordance with the conventional conditions or the conditions recommended by manufacturers. All the used reagents or apparatus, of which manufacturers are not specified, are conventional commercially available products.

### Example 1: Preparation of III-1

1) Preparation of (4-nitrosophenyl) methanol

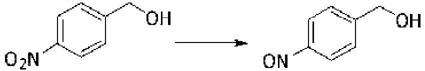

*p*-Nitrobenzyl alcohol (6.0 g, 39.18 mmol) was dissolved in 60 mL of methanol, after the solid was completely dissolved, zinc powders (7.69 g, 117.54 mmol) were added under stirring, and then a NH₄Cl (3.14 g, 58.77 mmol) aqueous solution (15 mL) was slowly dropwise added. Upon completion of the addition, the reaction was stirred at room temperature for 90 minutes, and the *p*-nitrobenzyl alcohol raw material was basically reacted completely. After the insoluble solids were removed by filtration, the filtrate was cooled at -5 °C. Thereafter, a FeCl₃ (58.77 mmol) solution (30 mL of methanol and 15 mL of water) was dropwise added slowly under stirring. After the dropwise addition was completed, the reaction was continuously stirred at low temperature for 1 hour. After the reaction was completed, the insoluble substance in the reaction solution was removed by filtration. An equal volume of saturated brine solution was added to the filtrate, followed by extraction with DCM. The organic phases were combined and further washed with the saturated saline solution three times. The organic phase was dried over anhydrous Na₂SO₄ and was concentrated under reduced pressure to obtain a crude product as a yellow residue. The residue was purified by silica gel column chromatography to obtain the target product as a yellow-green solid powder (3.0 g, 55.8% yield).¹H-NMR (400 MHz, DMSO-*d*6): δ 7.92 (d, *J* = 8.12 Hz, 2H), 7.66 (d, *J* = 8.12 Hz, 2H), 5.56 (t, *J* = 5.64 Hz, 1H), 4.63 (d, *J* = 5.64 Hz, 2H). MS (ESI) m/z: 138.06 [M+H] ⁺.

### 2) Preparation of (E)-4-((4-(hydroxymethyl)phenyl)diazenyl)benzoic acid

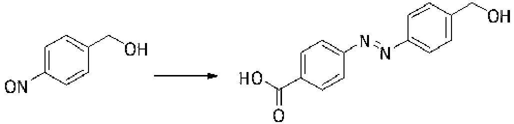

(4-nitrosophenyl) methanol (2.7 g, 19.69 mmol) was dissolved in 50 mL of AcOH, and after the solid was completely dissolved, *p*-aminobenzoic acid (2.7 g, 19.69 mmol) was added. The reaction was carried out at room temperature for 36 hours with stirring, and the insoluble substance was slowly precipitated during the reaction. After the reaction was completed, the insoluble solids precipitated during the reaction were separated by suction filtration, and the filter cake was washed with DCM to obtain the target product as an orange-yellow solid powder (3.3 g, 65.4% yield).¹H-NMR (400 MHz, DMSO-*d*6): δ 13.19 (br, 1H), 8.15 (dt, 2H), 7.97 (dt, 2H), 7.92 (dt, 2H), 7.56 (d, 2H), 5.46 (br, 1H), 4.63 (s, 2H). MS (ESI) m/z: 257.09 [M+H] ⁺.

### 3) Preparation of tert-butyl (E)-(2-(4-((4-(hydroxymethyl)phenyl)diazenyl)benzamido)ethyl)carbamate

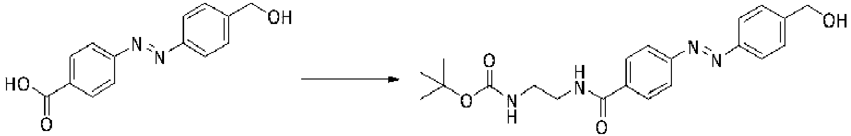

*(E)*-4-((4-(hydroxymethyl)phenyl)diazenyl)benzoic acid (2.5 g, 9.76 mmol) was dissolved in 40 mL of DMF, and after the solid was completely dissolved, mono Boc-ethylenediamine (2.03 g, 12.68 mmol), EDCI (2.43 g, 12.68 mmol) and HOBT (1.72 g, 12.68 mmol) were added in order, and the resulting mixture was reacted at room temperature for 5 h. After the reaction was completed, the solvent was removed by concentration under reduced pressure; the obtained residue was dispersed in DCM, the insoluble substance was separated by filtration, and the filter cake was washed with a mixture solution of PE and EA (1:1 v/v) to obtain the target product as a brown yellow solid (3.15 g, 81.0% yield).¹H-NMR (400 MHz, DMSO-d6): δ 8.66 (t, 1H), 8.04 (d, 2H), 7.95-7.90 (m, 4H), 7.55 (d, 2H), 6.97 (t, 1H), 5.42 (t, 1H), 4.62 (d, 2H), 3.32 (q, 2H), 3.12 (q, 2H), 1.38 (s, 9H). MS (ESI) m/z: 399.19 [M+H] ⁺; 421.18 [M+Na] ⁺.

### 4) Preparation of (E)-N-(2-aminoethyl)-4-((4-(hydroxymethyl)phenyl)diazenyl)benzamide

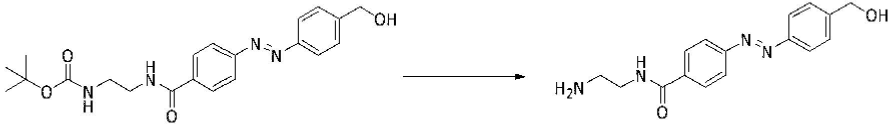

T-butyl *(E)*-((2-(4-(4-(hydroxymethyl)phenyl)diazenyl)benzamido)ethyl)carbamate (0.95 g, 2.38 mmol) was dissolved in 10 mL of DCM, and TFA (2 mL) was added dropwise under stirring. After the addition was completed, the mixture solution became clear, and the mixture was reacted at room temperature for 5 h. After the reaction was completed, the solvent was removed under reduced pressure; the obtained oily matter was re-dissolved with EA, and then PE was slowly dropwise added, to precipitate insoluble substance. The mixture was ultrasonicated and then filtered, and the filter cake was washed with diethyl ether to obtain the target product as an orange-yellow solid powder (0.9 g, 95.4% yield).¹H-NMR (400 MHz, DMSO-*d*6): δ 8.83 (t, 1H), 8.09 (d, 2H), 7.98 (m, 2H), 7.92 (m, 2H), 7.56 (d, 2H), 5.55 (s, 1H), 4.62 (d, 2H), 3.54 (q, 2H), 3.45 (m, 2H), 3.03 (q, 2H). MS (ESI) m/z: 299.15 [M+H] ⁺; 321.13 [M+Na] ⁺.

### 5) Preparation of (E)-N-(2-(3-(2,5-dioxo-2,5-dihydro-1H-pyrrol-1-yl)propanamido)ethyl)-4-((4-(hydroxymethyl)phenyl)diazenyl)benzamide (I-1)

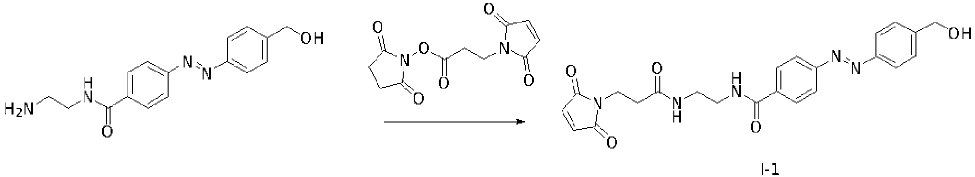

(*E*)-*N*-(2-aminoethyl)-4-((4-(hydroxymethyl)phenyl)diazenyl)benzamide (237.82 mg, 0.6 mmol) was dissolved in 10 mL of DMF, and 2,5-dioxopyrrolidin-1-yl 3-(2,5-dioxo-2,5-dihydro-1*H-*pyrrol-1-yl)propanoate (239.6 mg, 0.9 mmol) and DIPEA (155 mg, 1.2 mmol) were added under stirring. The mixture was stirred and reacted at room temperature for 24 h. After the reaction was completed, the solvent was removed by concentration under reduced pressure to obtain oily matter. Diethyl ether was added to the oily matter, and the mixture was ultrasonicated to precipitate insoluble solids. The precipitate was collected by filtration, and the filter cake was further purified by silica gel column chromatography to obtain the target product as an orange yellow solid powder (216 mg, 80% yield).¹H-NMR (400 MHz, DMSO-*d*6): δ 8.65 (t, 1H), 8.11 (t, 1H), 8.04 (m, 6H), 7.55 (d, 2H), 7.00 (s, 2H), 5.38 (t, 1H), 4.62 (d, 2H), 3.62 (t, 2H), 3.30 (s, 2H), 3.21 (m, 2H), 2.35 (m, 2H). MS (ESI) m/z :450.15 [M+H] ⁺; 472.15 [M+Na] ⁺.

### 6) Preparation of (E)-4-((4-((2-(3-(2,5-dioxo-2,5-dihydro-1H-pyrrol-1-yl)propanamido)ethyl)carbamoyl)phenyl)diazenyl)benzyl (4-nitrophenyl)carbonate (I-7)

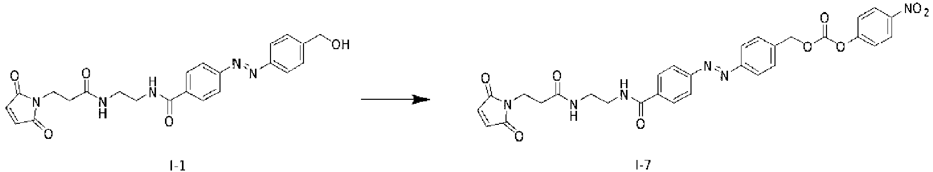

(*E*)-*N*-(2-(3-(2,5-dioxo-2,5-dihydro-1*H*-pyrrol-1-yl)propanamido))ethyl)-4-((4-(hydroxymethyl)phenyl)diazenyl)benzamide (I-1) (45 mg, 0.1 mmol) was dissolved in 15 mL of DMF, and *p*-nitrophenol carbonate (60.91 mg, 0.2 mmol) and DIPEA (19.4 mg, 0.15 mmol) were added in order under stirring. The mixture was stirred and reacted at room temperature overnight, and after the reaction was completed, the solvent was removed by concentration under reduced pressure; the obtained residue was dispersed in diethyl ether, and the mixture was ultrasonicated to precipitate insoluble solid. The precipitated insoluble solid was separated by filtration, the filter cake was washed with diethyl ether several times to obtain the target product as an orange yellow solid powder (54 mg, 88% yield).¹H-NMR (400 MHz, DMSO-*d*6): δ 8.69 (t, 1H), 8.39 (m, 0.5H), 8.33 (dt, 2H), 8.14 (t, 1H), 8.05 (m, 2H), 7.98 (m, 4H), 7.75 (m, 0.5H), 7.72 (d, 2H), 7.61 (dt, 2H), 7.01 (s, 2H), 5.44 (s, 2H), 3.62 (t, 2H), 3.30 (q, 2H), 3.20 (q, 2H), 2.35 (t, 2H). MS (ESI) m/z: 615.18 [M+H] ⁺; 637.17 [M+Na] ⁺.

### 7) Preparation of compound II-1

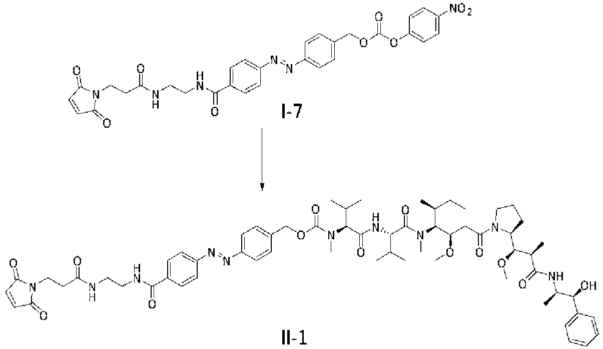

(*E*)-4-((4-((2-(3-(2,5-dioxo-2,5-dihydro-1*H*-pyrrol-1-yl)propanamido)ethyl)carbamoyl)phenyl)diazenyl)benzyl(4-nitrogenphenyl)carbonate (I-7) (43.0 mg, 0.07 mmol), monomethyl auristatin E (MMAE) (50.0 mg, 0.07 mmol), and HOBT (9.46 mg, 0.07 mmol) were added to a 10 mL eggplant-shaped flask, then 5 mL of anhydrous DMF were added, and the resulting mixture was stirred at room temperature until completely dissolved. After the solids were completely dissolved, DIPEA (18.1 µL, 0.14 mmol) was dropwise added, and the mixture was reacted at room temperature for 24 h. After the reaction was completed, the solvent was removed by concentration under reduced pressure, to obtain a crude product, which was purified by column chromatography to obtain the product as a white powdery solid (63 mg, 75.4% yield). HR-MS (ESI) m/z: 1193.6604 [M+H] ⁺; 1215.6453 [M+Na]⁺.

### 8) Synthesis of compound III-1

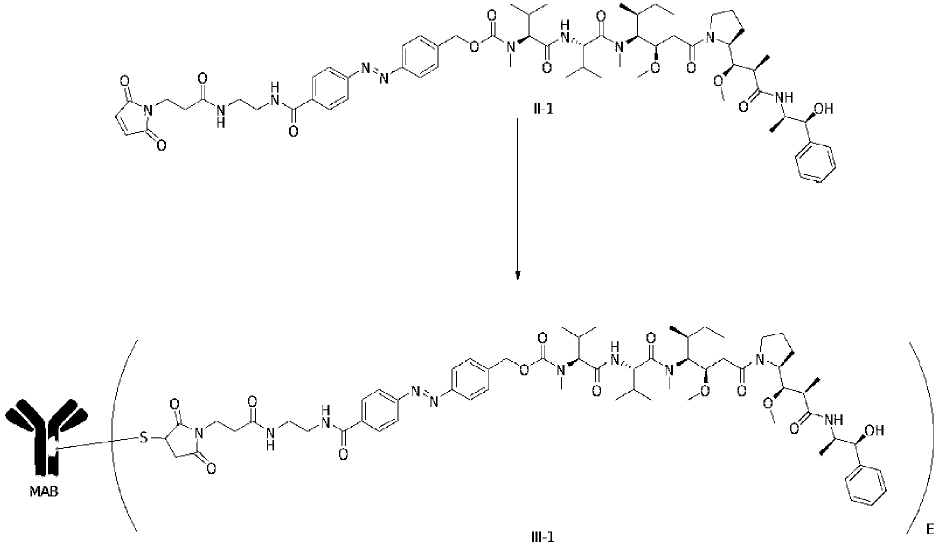

According to the method as described in the literature (Int J Mol Sci. 2017, 18(9): e1860.), compound II-1 was conjugated to HER2 antibody trastuzumab (purchased from Roche Pharmaceuticals) to obtain the target antibody-drug conjugate III-1, wherein, 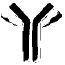 (MAB) represented HER2 antibody trastuzumab, and the drug-antibody ratio (DAR) E was about 4.

### Example 2: Preparation of compounds III-2, III-3, III-4, and III-5

1) With reference to the preparation method of Compound I-1 in Example 1, 2,5-dioxopyrrolidin-1-yl 3-(2,5-dioxo-2,5-dihydro-1H-pyrrol-1-yl)propanoate was substituted with the following compounds, 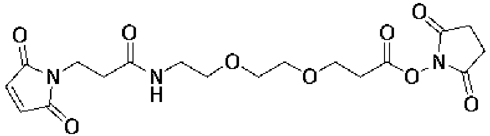 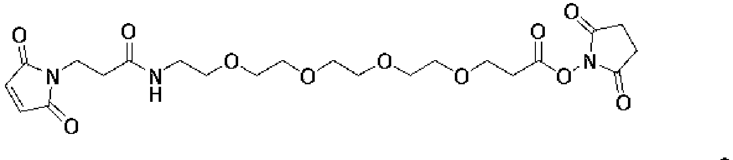 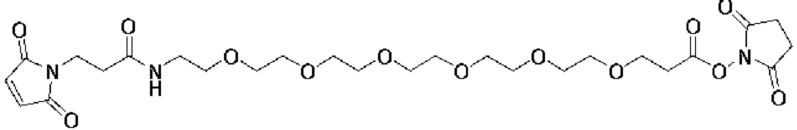 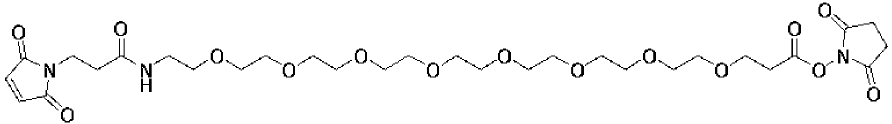
   to prepare compounds I-2, I-3, I-4, and I-5. 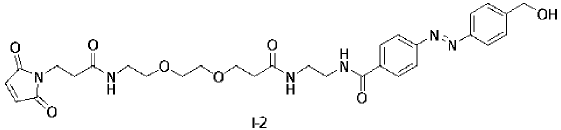
   I-2: ¹H-NMR (400 MHz, DMSO-d6): δ 8.68 (t, 1H), 8.04 (m, 4H), 7.93 (m, 4H), 7.56 (d, 2H), 7.00 (s, 2H), 5.42 (t, *J* = 5.8 Hz, 1H), 4.62 (d, *J* = 5.8 Hz, 2H), 3.60 (t, *J* = 6.7 Hz, 4H), 3.45 (m, 4H), 3.37 (m, 2H), 3.31 (m, 2H), 3.29 (m, 2H), 3.13 (q, *J* = 6.0 Hz, 2H), 2.32 (m, 4H). ESI-MS m/z (M+H)⁺ calculated for C₃₀H₃₇N₆O₃ 609.27, found 609.24; ESI-MS m/z (M+Na)⁺ calculated for C₃₀H₃₆N₆NaO₈ 631.25, found 631.23. 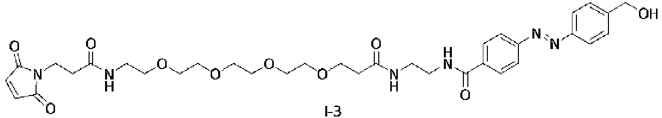
   I-3: ¹H-NMR (400 MHz, DMSO-d6): δ 8.69 *(t, J=* 5.5 Hz, 1H), 8.05 (m, 4H), 7.92 (m, 4H),
   7.55 (d, *J* = 8.7 Hz, 2H), 7.00 (s, 2H), 5.42 (t, *J = 5.7* Hz, 1H), 4.62 (d, *J* = 5.7 Hz, 2H), 3.61 (m, 4H), 3.46 (m, 20H), 3.39 (m, 2H), 3.31 (m, 2H), 3.22 (m, 4H), 2.32 (m, 4H). ESI-MS m/z (M+H)⁺ calculated for C₃₈H₅₃N₆O₁₂ 785.37, found 785.36; ESI-MS m/z (M+Na)⁺ calculated for C₃₈H₅₂N₆NaO₁₂ 807.35, found 807.35. 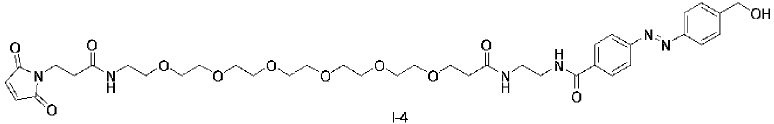
   I-4: ¹H-NMR (400 MHz, DMSO-d6): δ 8.69 (t, 1H), 8.05 (m, 4H), 7.92 (m, 4H), 7.55 (d, 2H), 7.00 (s, 2H), 5.42 (t, 1H), 4.62 (d, 2H), 3.61 (m, 4H), 3.46 (m, 20H), 3.39 (m, 2H), 3.31 (m, 2H), 3.22 (m, 4H), 2.32 (m, 4H). MS (ESI) m/z: 785.36 [M+H] ⁺; 807.35 [M+Na] ⁺. 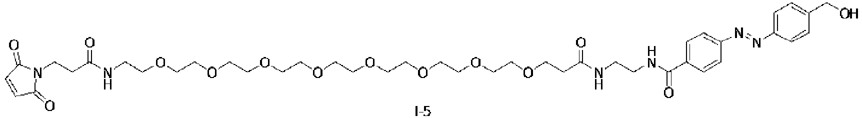
   I-5: ¹H-NMR (400 MHz, DMSO-d6): δ 8.68 (t, 1H), 8.04 (m, 4H), 7.92 (m, 4H), 7.55 (d, 2H), 7.00 (s, 2H), 5.42 (t, 1H), 4.62 (d, 2H), 3.61 (m, 4H), 3.46 (m, 28H), 3.36 (m, 2H), 3.26 (m, 2H), 3.14 (m, 4H), 2.32 (m, 4H). MS (ESI) m/z: 873.42 [M+H] ⁺; 895.40 [M+Na] ⁺.
2) With reference to the preparation method of compound I-7 in Example 1, compound I-1 was substituted with compounds I-2, I-3, I-4, and I-5 respectively, to prepare compounds I-8, I-9, I-10, and I-11. 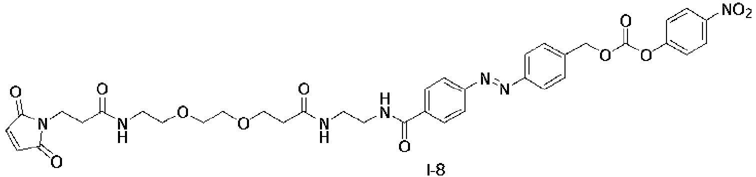
   I-8: ¹H-NMR (400 MHz, DMSO-d6): δ 8.70 (t, 1H), 8.33 (dt, 2H), 8.05 (m, 4H), 7.95 (m, 4H), 7.72 (d, 2H), 7.61 (d, 2H), 7.00 (s, 2H), 5.44 (s, 2H), 3.59 (q, 4H), 3.50 (m, 4H), 3.35 (m, 2H), 3.32 (m, 2H), 3.26 (t, 2H), 3.13 (q, 2H), 2.32 (m, 4H). MS (ESI) m/z: 774.27 [M+H] ⁺; 796.26 [M+Na]⁺. 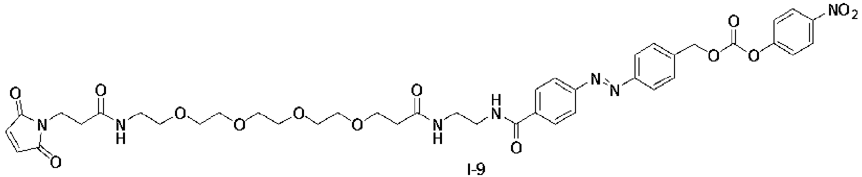
   I-9: ¹H-NMR (400 MHz, DMSO-d6): δ 8.70 (t, 1H), 8.41-8.33 (m, 3H), 8.04 (m, 4H), 7.96 (m, 4H), 7.74 (m, 3H), 7.61 (dt, 2H), 7.00 (s, 2H), 5.44 (s, 2H), 3.59 (q, 4H), 3.47 (m, 14H), 3.38 (m, 2H), 3.26 (t, 2H), 3.13 (q, 2H), 2.33 (m, 4H). MS (ESI) m/z: 862.31 [M+H] ⁺; 884.29 [M+Na]⁺. 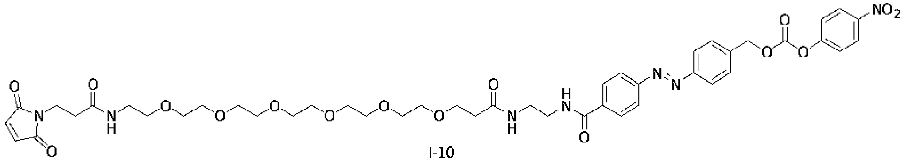
   I-10: ¹H-NMR (400 MHz, DMSO-d6): δ 8.70 (t, 1H), 8.34 (dt, 2H), 8.05 (m, 4H), 7.96 (m, 4H), 7.72 (d, 2H), 7.61 (dt, 2H), 7.00 (s, 2H), 5.44 (s, 2H), 3.59 (q, 4H), 3.47 (m, 22H), 3.36 (m, 2H), 3.26 (t, 2H), 3.14 (q, 2H), 2.33 (m, 4H). MS (ESI) m/z: 950.38 [M+H] ⁺; 972.36 [M+Na] ⁺. 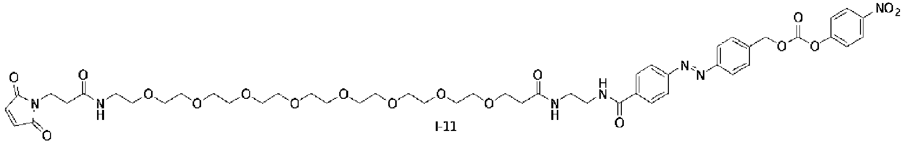
   I-11: ¹H-NMR (400 MHz, DMSO-d6): δ 8.70 (t, 1H), 8.34 (dt, 2H), 8.07-7.73 (m, 8H), 7.72 (d, 2H), 7.61 (dt, 2H), 7.00 (s, 2H), 5.44 (s, 2H), 3.59 (q, 4H), 3.49 (m, 30H), 3.36 (m, 2H), 3.26 (t, 2H), 3.14 (q, 2H), 2.33 (m, 4H). MS (ESI) m/z: 1038.43 [M+H] ⁺; 1060.41 [M+Na]⁺.
3) With reference to the preparation method of compound II-1 in Example 1, compound I-7 was substituted with compounds I-8, I-9, I-10, and I-11 respectively, to prepare compounds II-2, II-3, II-4, and II-5. 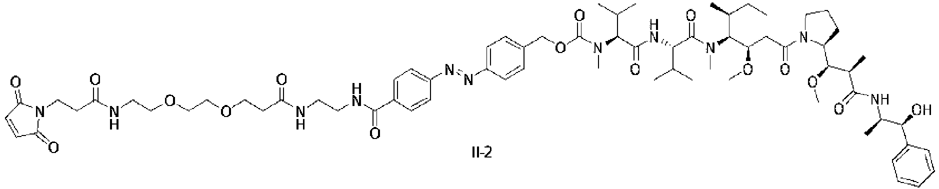
   II-2: ¹H NMR (600 MHz, CDCl₃-*d6*) 7.98-7.91 (m, 6H), 7.59-7.50 (m, 3H), 7.38-7.31 (m, 4H), 7.24 (t, *J* = 6.0 Hz, 1H), 7.10 (s, 1H), 6.83 (br, 1H), 6.66 (s, 1H), 6.54 (d, *J* = 12.0 Hz, 1H), 5.31 (d, *J* = 12.0 Hz, 1H), 5.18 (d, *J* = 12.0 Hz, 1H), 4.71-4.67 (m, 2H), 4.25-4.05 (m, 5H), 3.83 (t, *J* = 12.0 Hz, 3H), 3.71 (t, *J* = 6.0 Hz, 2H), 3.61-3.50 (m, 12H), 3.41-3.30 (m, 10H), 2.99-2.94 (m, 5H), 2.58-2.55 (m, 2H), 2.49 (t, *J* = 6.0 Hz, 2H), 2.05-1.94 (m, 4H), 1.28-1.21 (m, 8H), 1.03-0.99 (m, 4H), 0.96 (d, *J* = 6.0 Hz, 4H), 0.88 (t, *J* = 12.0 Hz, 10H), 0.82 (t, *J* = 6.0 Hz, 3H), 0.76-0.69 (m, 3H). ESI-HRMS m/z (M+H)⁺ calculated for C₇₀H₁₀₂N₁₁O₁₆1352.7506, found 1352.7502; ESI-HRMS m/z (M+Na)⁺ calculated for C₇₀H₁₀₁N₁₁NaO₁₆1374.7325, found 1374.7327. 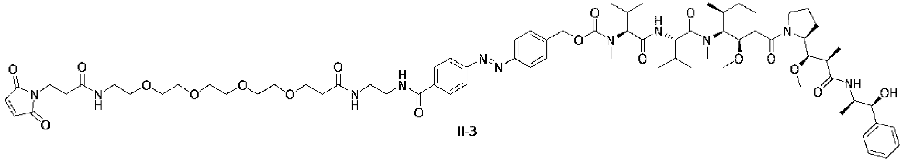
   II-3:¹H NMR (600 MHz, CDCl₃-*d6*) 11.01 (br, 1H), 8.04 (br, 3H), 7.91 (d, *J* = 6.0 Hz, 5H), 7.56-7.49 (m, 3H), 7.31 (br, 4H), 6.64 (s, 1H), 5.31 (d, *J* = 12.0 Hz, 1H), 5.18 (d, *J* = 12.0 Hz, 1H), 4.92-4.69 (m, 4H), 3.78-3.49 (m, 32H), 3.40-3.30 (m, 11H), 3.12-2.94 (m, 9H), 1.50-1.47 (m, 14H), 1.41 (d, *J* = 6.0 Hz, 11H), 0.99-0.76 (m, 25H). ESI-HRMS m/z (M+H)⁺ calculated for C₇₄H₁₁₀N₁₁O₁₈1440.8030, found 1440.8015; ESI-HRMS m/z (M+Na)⁺ calculated for C₇₄H₁₀₉N₁₁NaO₁₈1462.7836, found 1462.7836. 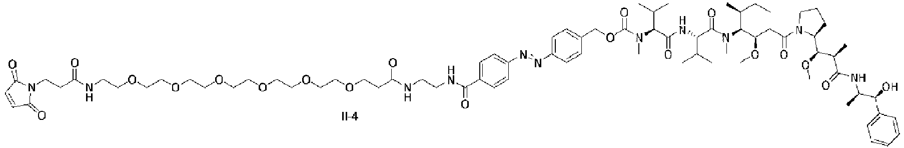
   II-4:¹H NMR (600 MHz, CDCl₃-*d6*) 8.04-7.91 (m, 6H), 7.56-7.50 (m, 3H), 7.35-7.32 (m, 5H), 7.26-7.24 (m, 1H), 6.67 (s, 2H), 5.30 (d, *J* = 12.0 Hz, 1H), 5.19 (d, *J* = 12.0 Hz, 1H), 4.93 (br, 1H), 3.82 (t, *J* = 6.0 Hz, 2H), 3.72-3.50 (m, 33H), 3.39-3.31 (m, 10H), 3.12-3.08 (m, 3H), 3.01 (br, 2H), 2.95 (d, *J* = 6.0 Hz, 2H), 2.52-2.51 (m, 4H), 1.48-1.40 (m, 15H), 1.28-1.23 (m, 8H), 1.00-0.77 (m, 25H). ESI-HRMS m/z (M+H)⁺ calculated for C₇₈H₁₁₈N₁₁O₂₀1528.8555, found 1528.8539; ESI-HRMS m/z (M+Na)⁺ calculated for C₇₈H₁₁₇N₁₁NaO₂₀1550.8374, found 1550.8363. 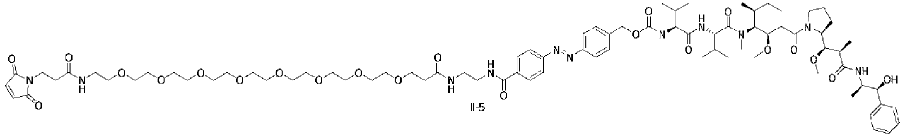
   II-5:¹H NMR (600 MHz, CDCl₃-*d6*) 8.05-7.91 (m, 7H), 7.56-7.50 (m, 3H), 7.35-7.32 (m, 4H), 7.26-7.24 (m, 1H), 6.68 (s, 2H), 5.31-5.27 (m, 1H), 5.19 (d, *J* = 12.0 Hz, 1H), 4.93 (br, 1H), 4.80-4.68 (m, 2H), 3.82 (t, *J* = 12.0 Hz, 2H), 3.63-3.51 (m, 40H), 3.40-3.31 (m, 11H), 3.12-3.08 (m, 8H), 1.48-1.39 (m, 14H), 1.29-1.24 (m, 9H), 1.00-0.78 (m, 26H). ESI-HRMS m/z (M+H)⁺ calculated for C₈₂H₁₂₆N₁₁O₂₂1616.9079, found 1616.9067; ESI-HRMS m/z (M+Na)⁺ calculated for C₈₂H₁₂₅N₁₁NaO₂₂1638.8898, found 1638.8886.
4) With reference to the preparation method of compound III-1 in Example 1, compound II-1 was substituted with compounds II-2, II-3, II-4, and II-5 respectively, to prepare compounds III-2, III-3, III-4, and III-5.

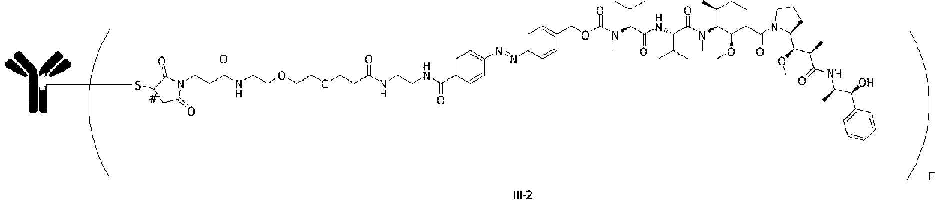

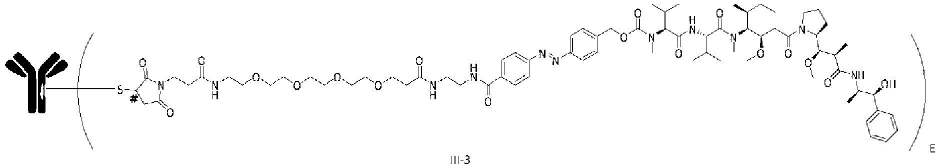

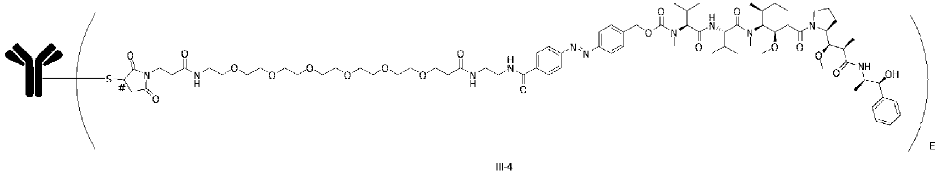

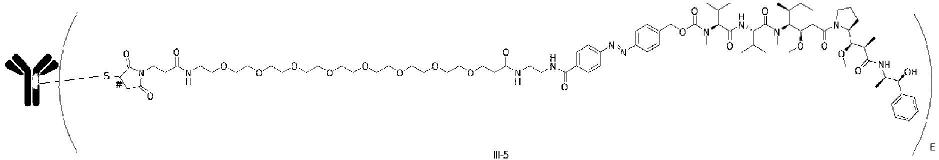

In compounds III-2, III-3, III-4, and III-5, the DAR values E each were about 4, and 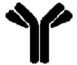 represents anti-HER2 antibody trastuzumab.

### Example 3: Preparation of compound III-6

1) Preparation of (9*H*-fluoren-9-yl)methyl (*S*,*E*)-(38-(4-((4-(hydroxymethyl)phenyl)diazenyl) phenyl)-26,33,38-trioxo-2,5,8,11,14,17,20,23-octaoxa-27,34,37-triazaoctatriacontan-32-yl)carbamate

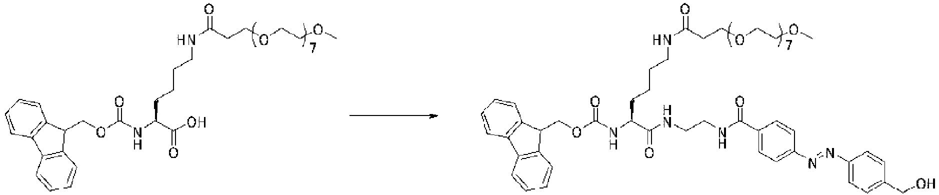

(*S*)-32-((((9*H*-fluoren-9-yl)methoxy)carbonyl)amino)-26-oxo-2,5,8,11,14,17,20,23-octaoxa-27-azatritriacontan-33-oic acid (810 mg, 1.06 mmol), and (*E*)-*N*-(2-aminoethyl)-4-((4-(hydroxymethyl)phenyl)diazenyl)benzamide (350 mg, 0.88 mmol) were dissolved in 20 mL of DMF, and then EDCI (254 mg, 1.325 mmol), HOBT (119.31 mg, 0.88 mmol) and DIPEA (171.2 mg, 1.325 mmol) were added. The mixture was reacted at room temperature overnight. After the reaction was completed, the solvent was removed by concentration under reduced pressure, and the obtain residue was purified by column chromatography to obtain the target product as an orange yellow solid powder (730 mg, 79.25% yield).¹H-NMR (400 MHz, DMSO-*d6*): δ 8.65 (t, 1H), 8.12 (t, 1H), 8.04 (d, 2H), 7.88 (m, 5H), 7.73 (m, 3H), 7.56 (d, 2H), 7.49 (t, 2H), 7.40 (m, 3H), 7.31 (t, 2H), 5.43 (t, 1H), 4.62 (d, 2H), 4.20 (m, 3H), 3.91 (q, 1H), 3.60 (m, 6H), 3.47 (m, 24H), 3.40 (m, 2H), 3.24 (m, 3H), 3.12 (q, 2H), 3.00 (t, 2H), 2.28 (t, 2H), 1.63 (m, 1H), 1.52 (m, 1H), 1.35 (m, 4H). MS (ESI) m/z: 1043.51 [M+H] ⁺; 1065.49 [M+Na] ⁺.

### 2) Preparation of (S,E)-N-(32-amino-26,33-dioxo-2,5,8,11,14,17,20,23-octaoxa-27,34-diazahexatriacontan-36-yl)-4-((4-(hydroxymethyl)phenyl)diazenyl)benzamide

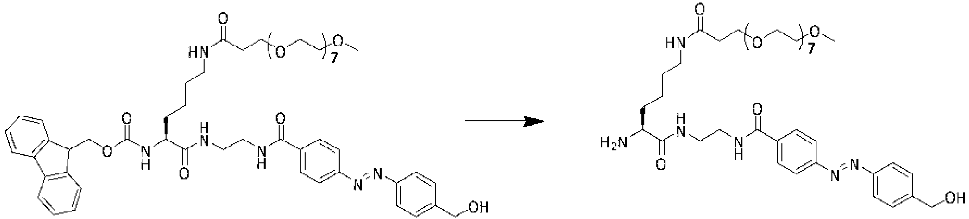

(9*H*-fluoren-9-yl)methyl (*S*,*E*)-(38-(4-((4-(hydroxymethyl)phenyl)diazenyl) phenyl)-26,33,38-trioxo-2,5,8,11,14,17,20,23-octaoxa-27,34,37-triazaoctatriacontan-32-yl)carbamate (450 mg, 0.43 mmol) was dissolved in 20 mL of DCM, and piperidine (0.8 mL) was added, the mixture was reacted at room temperature for 1 h. After the reaction was completed, the solvent was removed by concentration under reduced pressure, and the obtained residue was purified by silica gel column chromatography to obtain the target product as an orange yellow viscous oily matter (328 mg, 92.8% yield).¹H-NMR (400 MHz, DMSO-*d6*): δ 8.77 (t, 1H), 8.29 (t, 1H), 8.05 (m, 2H), 7.92 (m, 4H), 7.84 (t, 1H), 7.55 (d, 2H), 4.62 (d, 2H), 3.58-3.29 (m, 40H), 2.28 (t, 2H), 1.54 (m, 6H). MS (ESI) m/z: 821.45 [M+H]⁺; 843.43 [M+Na]⁺.

### 3) Preparation of compound I-6

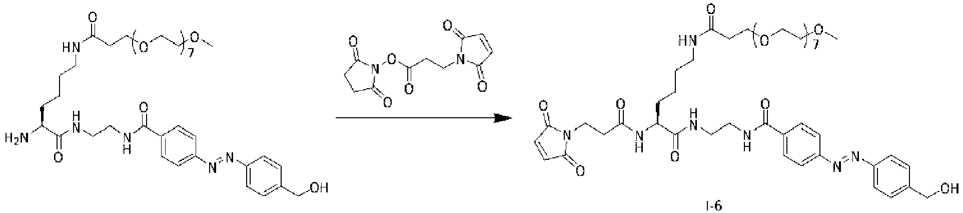

(*S*,*E*)-*N*-(32-amino-26,33-dioxo-2,5,8,11,14,17,20,23-octaoxa-27,34-diazahexatriacontan-36-yl)-4-((4-(hydroxymethyl)phenyl)diazenyl)benzamide (400 mg, 0.49 mmol) was dissolved in 20 mL of DMF, and 2,5-dioxopyrrolidin-1-yl 3-(2,5-dioxo-2,5-dihydro-1*H*-pyrrol-1-yl)propanoate (190 mg, 0.73 mmol) was added under stirring. The mixture was stirred and reacted at room temperature for 24 h. After the reaction was completed, the solvent was removed by concentration under reduced pressure, to obtain an orange yellow solid. The residue of the crude product was further purified by silica gel column chromatography to obtain the target product as an orange yellow oily matter (330 mg, 69.3% yield).¹H-NMR (400 MHz, DMSO-*d6*): δ 8.67 (t, 1H), 8.09 (m, 4H), 7.92 (m, 4H), 7.82 (t, 1H), 7.54 (d, 2H), 7.00 (s, 2H), 5.43 (t, 1H), 4.62 (d, 2H), 3.59 (m, 8H), 3.47 (m, 26H), 3.23 (d, 3H), 3.12 (m, 3H), 2.99 (br, 4H), 2.41 (t, 2H), 2.28 (t, 2H), 1.16 (t, 6H). MS (ESI) m/z: 972.48 [M+H]⁺; 994.46 [M+Na]⁺.

### 4) Preparation of compound I-12

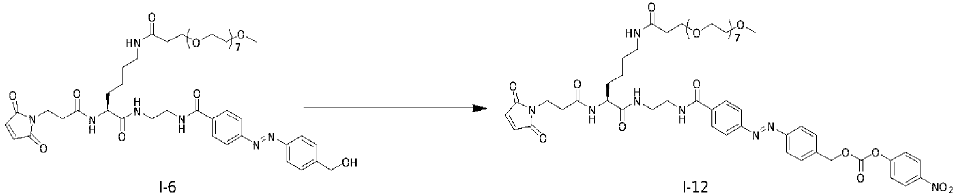

Compound I-6 (300 mg, 0.31 mmol) was dissolved in 15 mL of DMF, and bis(*p-*nitrophenyl)carbonate (187.76 mg, 0.62 mmol) and DIPEA (59.8 mg, 0.46 mmol) were added in order under stirring. The mixture was stirred and reacted at room temperature for 36 h. After the reaction was completed, the solvent was removed by concentration under reduced pressure. The obtained orange yellow oily residue was purified by silica gel column chromatography to obtain the target product as an orange yellow oily matter (200 mg, 56.7% yield).¹H-NMR (400 MHz, DMSO-*d6*): δ 8.71 (t, 1H), 8.34 (d, 2H), 8.15 (d, 1H), 8.08 (m, 2H), 7.94 (t, 4H), 7.72 (d, 2H), 7.61 (d, 2H), 7.00 (s, 2H), 5.44 (s, 2H), 3.62-3.46 (m, 42H), 2.98 (q, 2H), 2.41 (t, 2H), 2.28 (t, 2H), 1.59-1.41 (m, 4H), 1.12 (m, 2H). MS (ESI) m/z: 1137.48 [M+H]⁺; 1159.46 [M+Na]⁺.

### 5) Preparation of compound II-6

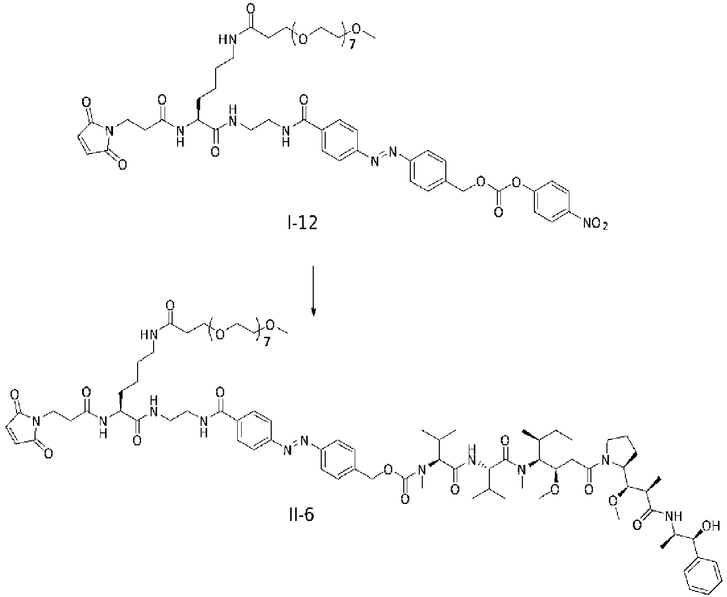

Compound I-12 (79.6 mg, 0.07 mmol), MMAE (50.0 mg, 0.07 mmol), and HOBT (9.46 mg, 0.07 mmol) were added to a 50 mL eggplant-shaped flask, then 10 mL anhydrous DMF were added thereto, and the resulting mixture was stirred at room temperature until completely dissolved. After the solids were completely dissolved, DIPEA (18.1 µL, 0.14 mmol) was dropwise added, and the mixture was reacted at room temperature for 24 h. After the reaction was completed, the solvent was removed by concentration under reduced pressure, to obtain a crude product, which is purified by column chromatography to obtain the target product as a white powdery solid (92 mg, 76.6% yield). HR-MS (ESI) m/z: 1715.9727 [M+H]⁺; 1738.9583 [M+Na]⁺.

### 6) Synthesis of compound III-6

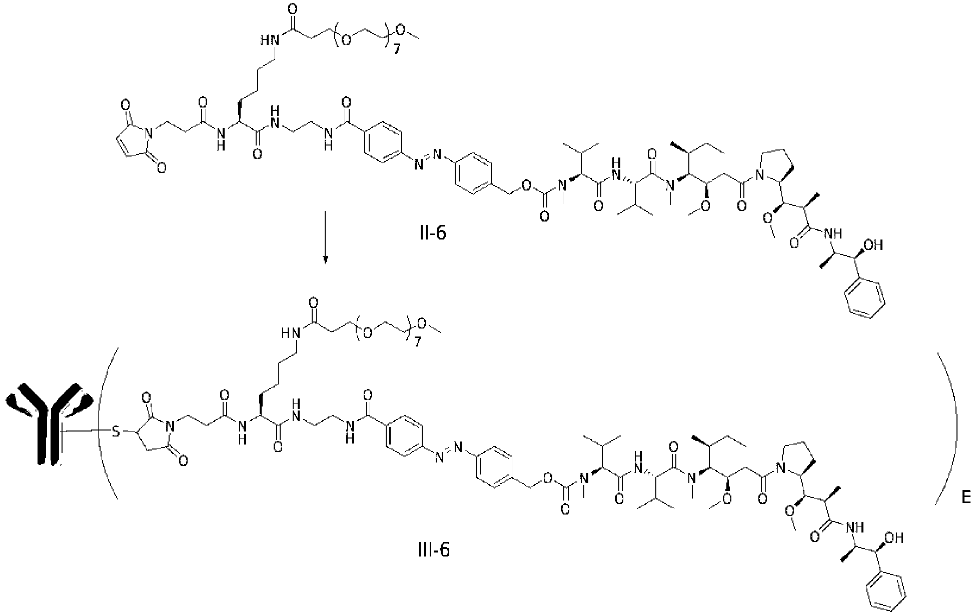

According to the method as described in the literature (Int J Mol Sci. 2017, 18(9): e1860.), compound II-6 was conjugated with anti-HER2 antibody trastuzumab to obtain the target antibody-drug conjugate III-6, wherein, 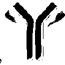 (MAB) represented anti-HER2 antibody trastuzumab, and the drug-antibody ratio (DAR) E was about 4.

### Example 4: Preparation of compounds III-7, III-8, III-9, III-10, III-11, III-12, and III-13

### 1) Preparation of compounds II-7, II-8, II-9, II-10, II-11, II-12, and II-13

With reference to the preparation method of compound II-1 in Example 1, compound I-7 was substituted with compound I-8, and MMAE was substituted with SN-38, exatecan, doxorubicin, lapatinib or a TLR7 agonist with CAS No. 1821304-87-3, to prepare compounds II-7, II-8, II-9, II-10, and II-11.

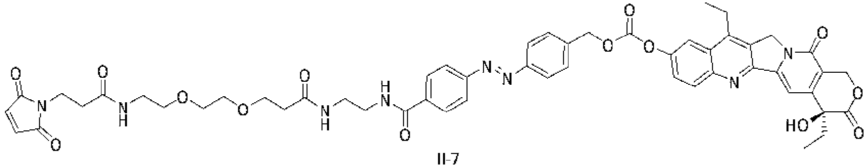

II-7:¹H NMR (600 MHz, DMSO-d6): δ 7.996 (d, *J* = 8.4 Hz, 2H), 7.925-7.828 (m, 6H), 7.610 (s, 1H), 7.530 (d, *J* = 8.4 Hz, 3H), 7.400 (br, 1H), 6.683-6.671 (m, 2H), 6.434-6.317 (m, 2H), 5.672 (d, *J* = 16.2 Hz, 1H), 5.352-5.143 (m, 6H), 3.796 (t, *J =* 7.2 Hz, 2H), 3.721 (t, *J =* 5.4 Hz, 3H), 3.616-3.485 (m, 42H), 3.378-3.370 (m, 2H), 3.126-3.095 (m, 2H), 2.508-2.476 (m, 5H), 2.346 (s, 3H), 1.924 (d, *J* = 6.6 Hz, 2H), 1.333-1.255 (m, 4H), 1.062 (t, *J* = 7.2 Hz, 3H). ESI-HRMS m/z (M+H)⁺ calculated for C₆₅H₇₉FN₈O₂₀ 1291.53, found 1291.54.

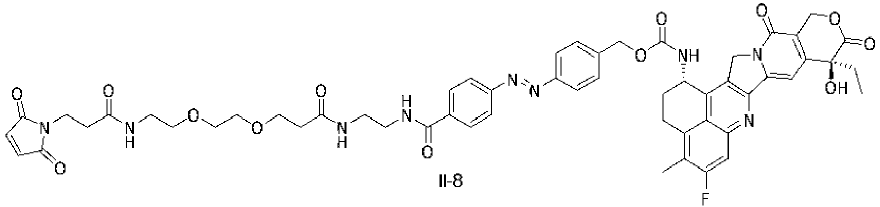

II-8: ¹H NMR (600 MHz, DMSO-d6): δ 8.706 (br, 1H), 8.211 (d, *J* = 8.4 Hz, 1H), 8.064-8.037 (m, 4H), 7.944 (d, *J =* 7.2 Hz, 4H), 7.753 (d, *J* = 10.8 Hz, 1H), 7.658 (d, *J* = 7.8 Hz, 2H), 7.305 (s, 1H), 6.992 (s, 2H), 5.435 (dd, *J* = 22.2 Hz, 16.8 Hz, 2H), 5.31-5.221 (m, 5H), 3.620-3.574 (m, 5H), 3.463 (d, *J* = 2.4 Hz, 4H), 3.355-3.336 (m, 4H), 3.258 (dd, *J* = 11.4 Hz, 5.4 Hz, 3H), 3.151-3.117 (m, 4H), 2.361-2.317 (m, 7H), 1.895-1.810 (m, 2H), 1.301-1.261 (m, 5H), 0.865 (t, *J =* 7.2 Hz, 3H). ESI-HRMS m/z (M+H)⁺ calculated for C₅₅H₅₇FN₉O₁₃ 1070.3982, found 1070.4094.

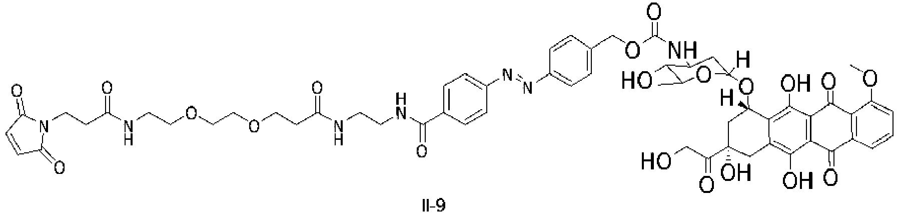

II-9: ¹H NMR (600 MHz, DMSO-d6): δ 13.991 (s, 1H), 13.231 (s, 1H), 8.674 (s, 1H), 8.027 (d, *J* = 7.8 Hz, 4H), 7.905 (d, *J* = 7.8 Hz, 2H), 7.857 (t, *J* = 7.2 Hz, 4H), 7.571-7.526 (m, 3H), 7.040 (d, *J* = 7.8 Hz, 1H), 6.991 (s, 2H), 5.442 (s, 1H), 5.229 (s, 1H), 5.078 (dd, *J* = 28.2 Hz, 13.2 Hz, 2H), 4.891 (d, *J* = 28.2 Hz, 2H), 4.758 (d, *J =* 5.4 Hz, 1H), 4.590 (d, *J =* 4.8 Hz, 2H), 4.185 (d, *J =* 6.0 Hz, 1H), 3.945 (s, 3H), 3.760 (br, 1H), 3.618-3.574 (m, 5H), 3.462 (s, 6H), 3.256 (d, *J =* 5.4 Hz, 2H), 3.138 (d, *J =* 5.4 Hz, 3H), 2.942 (dd, *J=* 51.0 Hz, 18.0 Hz, 2H), 2.337-2.317 (m, 4H), 2.214 (d, *J* = 13.2 Hz, 1H), 2.101 (d, *J= 9.6* Hz, 1H), 1.890 (t, *J =* 10.8 Hz, 1H), 1.511 (d, *J =* 10.2 Hz, 1H), 1.288-1.26 (m, 4H), 1.145 (d, *J =* 5.4 Hz, 3H), 1.091 (t, *J* = 7.2 Hz, 2H). ESI-HRMS m/z (M+H)⁺ calculated for C₅₈H₆₄N₇O₂₀ 1178.4128, found 1178.4195.

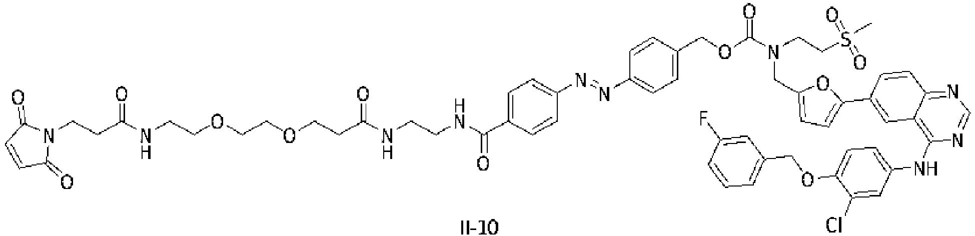

II-10: ¹H NMR (600 MHz, DMSO-d6): δ 9.930-9.863 (m, 1H), 8.738-8.563 (m, 3H), 8.039-7.811(m, 11H), 7.731-7.586 (m, 3H), 7.494-7.458 (m, 1H), 7.342-7.270 (m, 3H), 7.203-7.171 (m, 1H), 7.092 (d, *J =* 3.0 Hz, 1H), 6.991 (s, 1H), 6.614 (br, 1H), 5.309-5.256 (m, 4H), 4.708-4.681 (m, 1H), 4.031 (q, *J* = 7.2 Hz, 1H), 3.804 (br, 2H), 3.625-3.578 (m, 4H), 3.495-3.438 (m, 7H), 3.265 (q, *J* = 6.0 Hz, 1H), 3.141 (dd, *J* = 11.4 Hz, 5.4 Hz, 2H), 3.059-2.893 (m, 3H), 2.337 (dd, *J* = 12.6 Hz, 6.0 Hz, 4H), 1.992 (s, 1H), 1.177 (t, *J* = 6.6 Hz, 1H). ESI-HRMS m/z (M+H)⁺ calculated for C₆₀H₆₁ClFN₁₀O₁₃S 1215.3735, found 1215.3850.

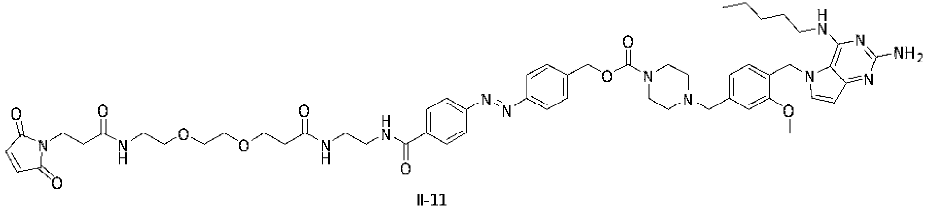

II-11: ¹H NMR (600 MHz, DMSO-*d6*): δ 12.428 (s, 1H), 9.137 (s, 1H), 8.719 (s, 1H), 8.072-7.921 (m, 9H), 7.719 (s, 2H), 7.586-7.575 (m, 2H), 7.421(s, 1H), 7.034-6.994 (m, 3H), 6.820 (d, *J* = 5.4 Hz, 1H), 6.692-6.681 (m, 1H), 6.156 (s, 1H), 5.217-5.193 (d, *J* = 14.4 Hz, 4H), 4.035-4.024 (m, 1H), 3.866 (s, 3H), 3.608-3.464 (m, 17H), 3.264-3.142 (m, 5H), 2.341 (br, 9H), 1.990 (s, 1H), 1.642 (br, 2H), 1.361-1.175 (m, 8H), 0.896 (br, 3H). ESI-HRMS m/z (M+H)⁺ calculated for C₅₅H₇₀N₁₃O₁₀ 1072.5290, found 1072.5399.

With reference to the preparation method of compound II-1 in Example 1, compound I-7 was substituted with compound I-11, and MMAE was substituted with SN-38 or exatecan, to prepare compounds II-12 and II-13.

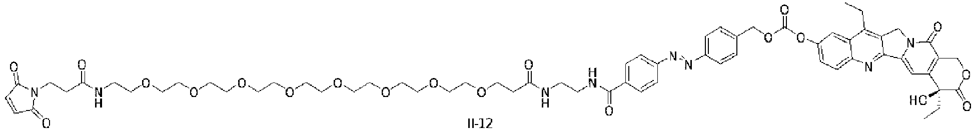

II-12:¹H NMR (600 MHz, DMSO-d6): δ 7.996 (d, J = 8.4 Hz, 2H), 7.925-7.828 (m, 6H), 7.610 (s, 1H), 7.530 (d, *J* = 8.4 Hz, 3H), 7.400 (br, 1H), 6.683-6.671 (m, 2H), 6.434-6.317 (m, 2H), 5.672 (d, *J* = 16.2 Hz, 1H), 5.352-5.143 (m, 6H), 3.796 (t, *J* = 7.2 Hz, 2H), 3.721 (t, *J* = 5.4 Hz, 3H), 3.616-3.485 (m, 42H), 3.378-3.370 (m, 2H), 3.126-3.095 (m, 2H), 2.508-2.476 (m, 5H), 2.346 (s, 3H), 1.924 (d, *J* = 6.6 Hz, 2H), 1.333-1.255 (m, 4H), 1.062 (t, *J* = 7.2 Hz, 3H). ESI-HRMS m/z (M+H)⁺ calculated for C₆₅H₇₉FN₈O₂₀ 1291.53, found 1291.54.

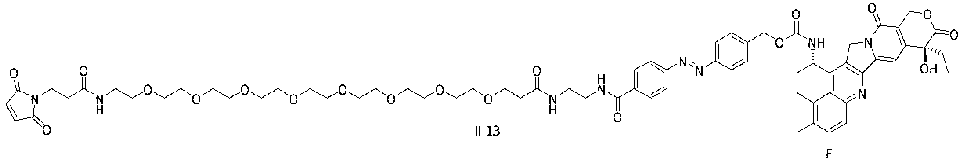

II-13:¹H NMR (600 MHz, DMSO-*d6*): δ 7.996 (d, *J* = 8.4 Hz, 2H), 7.925-7.828 (m, 6H), 7.610 (s, 1H), 7.530 (d, *J* = 8.4 Hz, 3H), 7.400 (br, 1H), 6.683-6.671 (m, 2H), 6.434-6.317 (m, 2H), 5.672 (d, *J* = 16.2 Hz, 2H), 5.352-5.143 (m, 6H), 3.796 (t, *J* = 7.2 Hz, 2H), 3.721 (t, *J* = 5.4 Hz, 3H), 3.616-3.545 (m, 42H), 3.378-3.370 (m, 2H), 3.126-3.095 (m, 2H), 2.508-2.476 (m, 5H), 2.346 (s, 3H), 1.924 (d, *J* = 6.6 Hz, 2H), 1.333-1.255 (m, 4H), 1.062 (t, *J* = 7.2 Hz, 3H). ESI-HRMS m/z (M+H)⁺ calculated for C₆₇H₈₁FN₉O₁₉ 1334.56, found 1334.56.

### 2) Preparation of compounds III-7, III-8, III-9, III-10, III-11, III-12, and III-13

With reference to the preparation method of compound III-1 in Example 1, compound II-1 was substituted with compound II-7, II-8, II-9, II-10, II-11, II-12, and II-13, respectively, to prepare compounds III-7, III-8, III-9, **III-10,** III-11, III-12, and III-13.

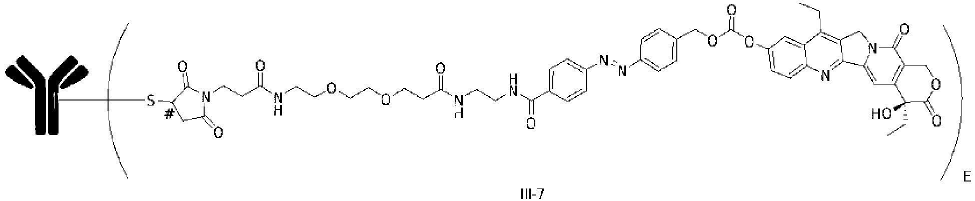

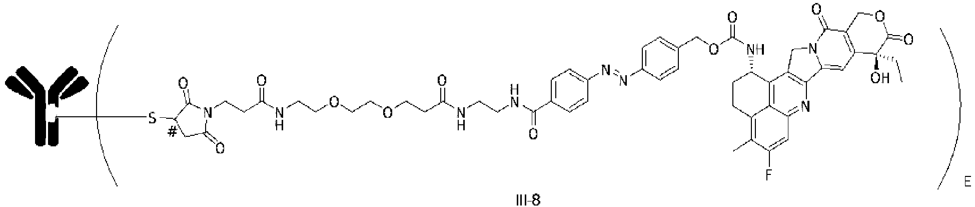

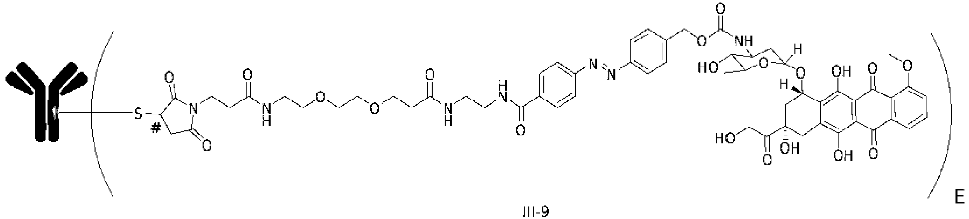

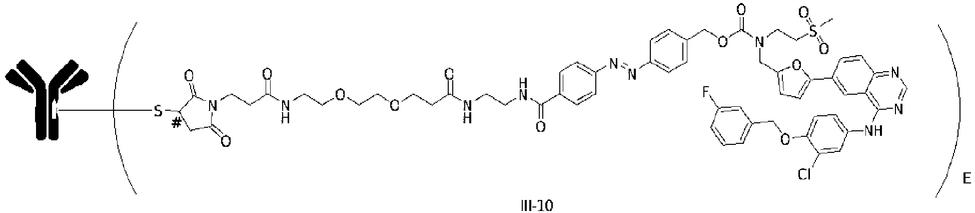

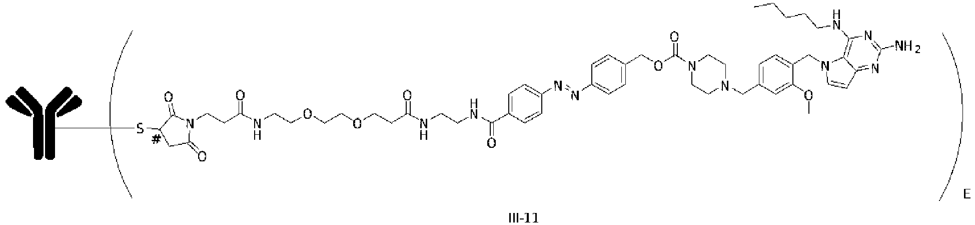

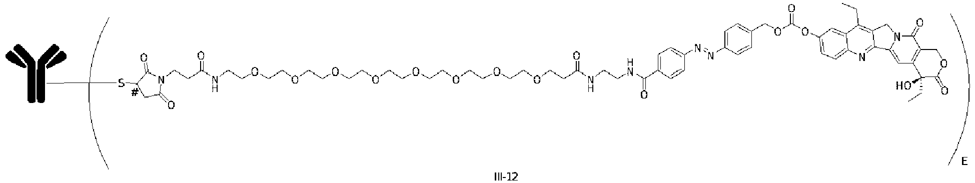

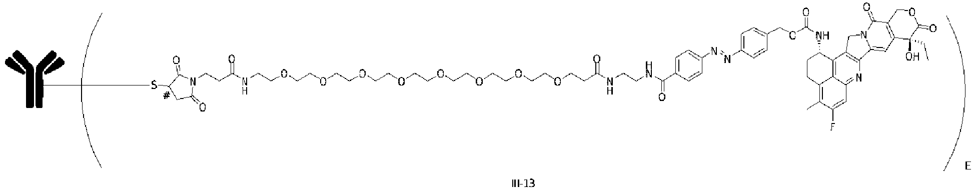

In compounds III-7, III-8, III-9, **III-10,** III-11, III-12, and III-13, the DAR values E each were about 4, and 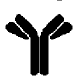 represents anti-HER2 antibody trastuzumab.

### Example 5: Preparation of compound III-14

### 1) Preparation of 4-nitroso benzoic acid

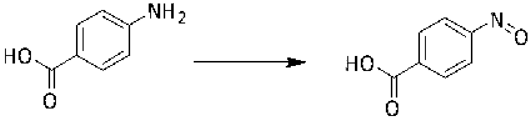

Potassium peroxymonosulfonate (17.93 g, 29.17 mmol) was dissolved in 150 mL of water, and a solution of p-aminobenzoic acid (2.0 g, 14.58 mmol) in DCM (20 mL) was slowly added under stirring. The resulting suspension was stirred and reacted under the condition of room temperature for 3 h, during which the reaction system gradually changed from white to yellow. After the reaction was completed, the reaction mixture was filtered, and the obtained yellow filer cake was washed with purified water several times and dried in vacuum under reduced pressure, to obtain the target product as a yellow powdery solid (2.13 g, 96.67% yield).¹H-NMR (400 MHz, DMSO-d6): δ 13.59 (s, 1H), 8.26 (dt, 2H), 8.04 (dt, 2H).

### 2) Preparation of (E)-4-((4-methyl-2-oxo-2H-chromen-7-yl)diazenyl)benzoic acid

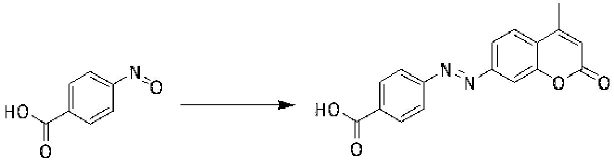

7-amino-4-methyl-2*H*-chromen-2-one (0.58 g, 3.31 mmol) and 4-nitrosobenzoic acid (0.5 g, 3.31 mmol) were dissolved in glacial acetic acid (50 mL). The mixture was stirred and reacted under the condition of room temperature overnight, and as the reaction went on, orange yellow precipitates were gradually formed, and the color of the reaction system changed from green to orange yellow. After the reaction was completed, precipitated insoluble substance was separated by suction filtration, and the filter cake was washed by AcOH and DCM, and further slurried and purified with DCM, to obtain the target product as an orange yellow powdery solid (465 mg, 45.6% yield).¹H-NMR (400 MHz, DMSO-*d6*): δ 13.34 (s, 1H), 8.17 (dt, 2H), 8.03 (m, 3H), 7.93 (dd, 1H), 7.87 (d, 1H), 6.55 (d, 1H), 2.51 (s, 3H). MS (ESI) m/z: 309.08 [M+H]⁺.

### 3) Preparation of tert-butyl (E)-(2-(4-((4-methyl-2-oxo-2H-chromen-7-yl)diazenyl)benzamido)ethyl)carbamate

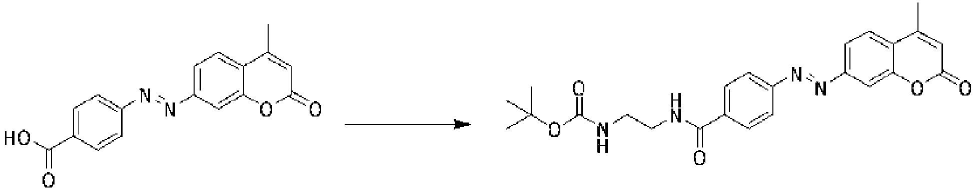

(*E*)-4-((4-methyl-2-oxo-2*H*-chromen-7-yl)diazenyl)benzoic acid (400 g, 6.62 mmol) was dissolved in 20 mL of DMF, and after complete dissolution, EDCI (373.8 mg, 1.95 mmol), HOBT (176 mg, 1.30 mmol) and DIPEA (226 µL, 1.3 mmol) were added in order. The resulting mixture was reacted at room temperature for 45 minutes, then mono-Boc-ethylenediamine (312 mg, 1.95 mmol) was added, and the resulting mixture was stirred and reacted under the condition of room temperature overnight. After the reaction was completed, the solvent was removed by concentration under reduced pressure, and the obtained residue was purified by silica gel column chromatography to obtain the target product as an orange yellow solid powder (400 mg, 68.3% yield).¹H-NMR (400 MHz, DMSO-*d6*): δ 8.69 (t, 1H), 8.05 (m, 5H), 7.93 (dd, 1H), 7.87 (d, 1H), 6.97 (t, 1H), 6.55 (d, 1H), 3.33 (m, 2H), 3.13 (q, 2H), 2.51 (s, 3H), 1.38 (s, 9H). MS (ESI) m/z: 451.19 [M+H]⁺; 473.17 [M+Na]⁺.

### 4) Preparation of (E)-N-(2-aminoethyl)-4-((4-methyl-2-oxo-2H-chromen-7-yl)diazenyl)benzamide

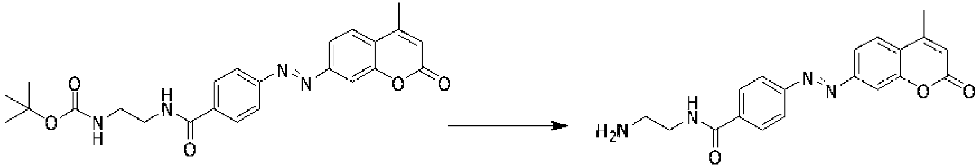

Tert-butyl (*E*)-(2-(4-((4-methyl-2-oxo-2*H*-chromen-7-yl)diazenyl)benzamido)ethyl)carbamate (150 mg, 0.33 mmol) was dissolved in 20 mL of DCM, then TFA (1 mL) was dropwise added under stirring. After the addition was completed, the mixture solution became clear, and the mixture was reacted at room temperature for 1 h. After the reaction was completed, the solvent was removed under reduced pressure, to obtain orange red solid residue, which was purified by silica gel column chromatography to obtain the target product as an orange solid powder (140 mg, 94.8% yield).¹H-NMR (400 MHz, DMSO-*d6*): δ 8.84 (t, 1H), 8.05 (m, 5H), 7.93 (dd, 1H), 7.87 (d, 1H), 6.96 (t, 1H), 6.56 (d, 1H), 3.51 (q, 2H), 3.16 (s, 2H), 2.98 (t, 2H), 2.51 (s, 3H). MS (ESI) m/z: 351.14 [M+H]⁺; 373.12 [M+Na]⁺.

### 5) Preparation of compound II-14

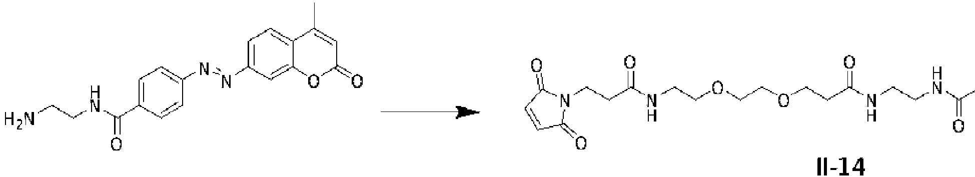

(*E*)-N-(2-aminoethyl)-4-((4-methyl-2-oxo-2H-chromen-7-yl)diazenyl)benzamide (115 mg, 0.257 mmol) was dissolved in 15 mL of DMF, 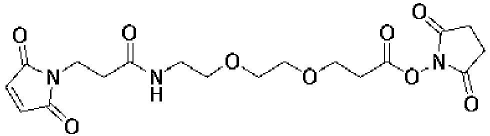 (131.21 mg, 0.308 mmol) and DIPEA (39.8 mg, 0.308 mmol) were added under stirring, and the resulting mixture was stirred and reacted at room temperature overnight. After the reaction was completed, the solvent was removed by concentration under reduced pressure to obtain an oily crude product, which is further purified by silica gel column chromatography to obtain the target product as an orange yellow solid powder (147 mg, 86.6% yield).¹H-NMR (400 MHz, DMSO-d6): δ 8.72 (t, 1H), 8.03 (m, 7H), 7.93 (dd, 1H), 7.86 (d, 1H), 7.00 (s, 2H), 6.55 (d, 1H), 3.71 (t, 2H), 3.58 (m, 2H), 3.48 (m, 2H), 3.36 (m, 2H), 3.25 (q, 2H), 3.14 (m, 4H), 2.92 (t, 2H), 2.81 (s, 3H), 2.33 (m, 4H). MS (ESI) m/z: 361.24 [M+H]⁺; 683.22 [M+Na] ⁺.

### 6) Preparation of compound III-14

With reference to the preparation method of compound III-1 in Example 1, compound II-1 was substituted with compound II-14, to prepare compound III-14.

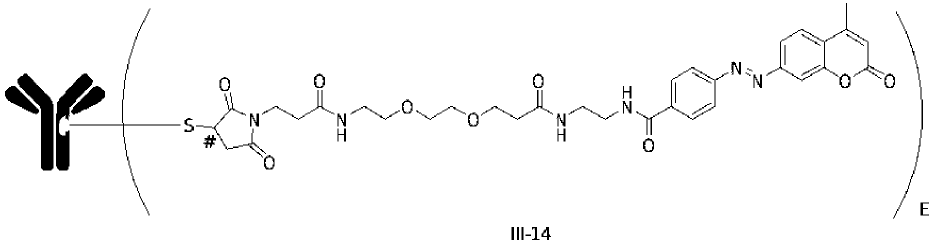

In compound III-14, the DAR value E of was about 4, and 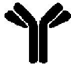 was anti-HER2 antibody trastuzumab.

### Example 6: Preparation of compound III-15

### 1) Preparation of tert-butyl 3-(2-(2-(2-((1,3-dioxoisoindolin-2-yl)oxy)ethoxy)ethoxy)ethoxy)propanoate

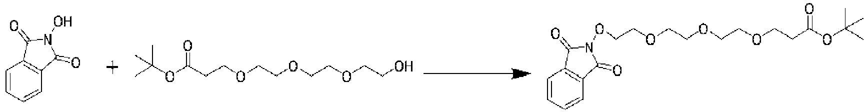

2-Hydroxy-isoindole-1,3-dione (1.27 g, 7.81 mmol) was added to a 100 mL eggplant-shaped flask, to which 30 mL of THF were added, and the resulting mixture was cooled in an ice bath to 0 °C. After 2-hydroxy-isoindole-1,3-dione was completely dissolved, tert-butyl 3-(2-(2-(2-hydroxyethoxy)ethoxy)ethoxy)propanoate (2 g, 7.1 mmol) and triphenylphosphine (2.04 g, 7.81 mmol) were added to the reaction solution in order. The reaction solution was placed at room temperature and continuously reacted for 1 h. The solvent was removed by concentration under reduced pressure. The residue was mixed with silica gel for sample loading, and purified by column chromatography to obtain the target product as a white solid (2.0 g, 61% yield).¹H-NMR (600 MHz, DMSO-*d6*): δ 7.864 (s, 4H), 4.275-4.261 (m, 2H), 3.731-3.716 (m, 2H), 3.560 (t, *J* = 6.6 Hz, 2H), 3.511-3.495 (m, 2H), 3.436-3.408 (m, 6H), 2.399 (t, *J* = 6.6 Hz, 2H), 1.387 (s, 9H). MS (ESI) m/z: 446.18 [M+Na]⁺.

### 2) Preparation of 3-(2-(2-(2-((1,3-dioxoisoindolin-2-yl)oxy)ethoxy)ethoxy)ethoxy)propanoic acid

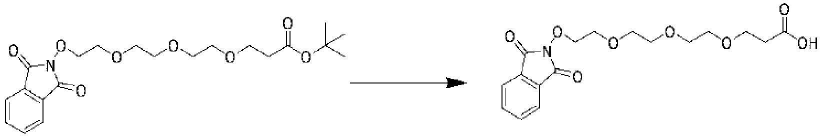

Tert-butyl 3-(2-(2-(2-((1,3-dioxoisoindolin-2-yl)oxy)ethoxy)ethoxy)ethoxy) propanoate (1.136 g, 2.68 mmol) was added to a 50 mL eggplant-shaped flask, to which 10 mL of a solution of 4N HCl in dioxane were added, and the resulting mixture was stirred and reacted at room temperature for 1 h, then the solvent was removed by concentration under reduced pressure. The residue was mixed with silica gel for sample loading and purified by column chromatography to obtain the target compound as a white solid (0.41 g, 42% yield).¹H-NMR (600 MHz, DMSO-*d6*): δ 12.152 (s, 1H), 7.872-7.871 (m, 4H), 4.282-4.268 (m, 2H), 3.738-3.722 (m, 2H), 3.580 (t, *J* = 6.6 Hz, 2H), 3.447-3.409 (m, 6H), 2.433 (t, *J* = 6.0 Hz, 2H). MS (ESI) m/z: 366.12 [M-H]⁻.

### 3) Preparation of (E)-N-(1-((1,3-dioxoisoindolin-2-yl)oxy)-12-oxo-3,6,9-trioxa-13-azapentadecan-15-yl)-4-((4-(hydroxymethyl)phenyl)diazenyl)benzamide (I-13)

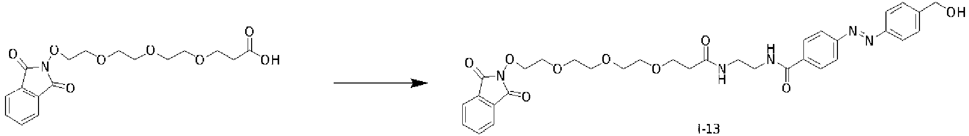

3-(2-(2-(2-((1,3-dioxoisoindolin-2-yl)oxy)ethoxy)ethoxy)ethoxy)propanoic acid (0.16 g, 0.44 mmol) was added to a 50 mL eggplant-shaped flask, to which 4 mL of anhydrous DMF were added. After the compound was completely dissolved, NHS (0.055 g, 0.48 mmol), and DIC (0.076 g, 0.6 mmol) were added to the reaction solution in order, and the resulting mixture was stirred at room temperature overnight. Then (*E*)-*N*-(2-aminoethyl)-4-((4-(hydroxymethyl)phenyl)diazenyl)benzamide and DIPEA were added to the reaction solution, and the resulting mixture was reacted at room temperature for 15 min. The solvent was removed by concentration under reduced pressure, and the residue was purified by column chromatography to obtain the target product as a yellow solid (0.071 g, 25%), which were directly used for the reaction in the next step.

### 4) Preparation of (E)-4-((4-((1-((1,3-dioxoisoindolin-2-yl)oxy)-12-oxo-3,6,9-trioxa-13-azapentadecan-15-yl)carbamoyl)phenyl)diazenyl)benzyl (4-nitrophenyl) carbonate (I-14)

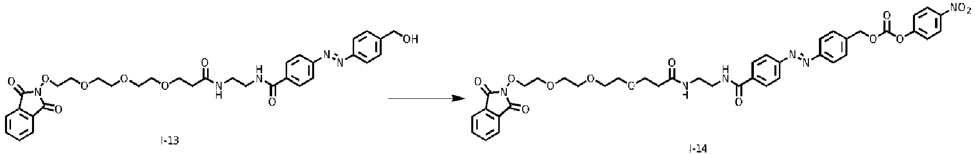

(*E*)-*N*-(1-((1,3-dioxoisoindolin-2-yl)oxy)-12-oxo-3,6,9-trioxa-13-azapentadecan-15-yl)-4-((4-(hydroxymethyl)phenyl)diazenyl)benzamide (I-13) (0.07 g, 0.11 mmol) was added to a 25 mL eggplant-shaped flask, to which 3 mL of anhydrous DMF were added. After the reaction raw materials were completely dissolved, bis(p-nitrophenyl)carbonate and DIPEA were added thereto in order, and the resulting mixture was reacted at room temperature for 4 h. The solvent was removed by concentration under vacuum, and the resulting crude product was mixed with silica gel for sample loading and purified by column chromatography to obtain the target product as a yellow solid (0.08 g, 90% yield), which were directly used for the reaction in the next step.

5) Preparation of 4-((*E*)-(4-((1-((1,3-dioxoisoindolin-2-yl)oxy)-12-oxo-3,6,9-trioxa-13-azapentadecan-15-yl)carbamoyl)phenyl)diazenyl)benzyl ((*S*)-1-(((*S*)-1-(((3*R*,4S,5*S*)-1-((*S*)-2-((1*R*,2*R*)-3-(((*S*)-1-((3-hydroxypropyl)amino)-1-oxo-3-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (I-15)

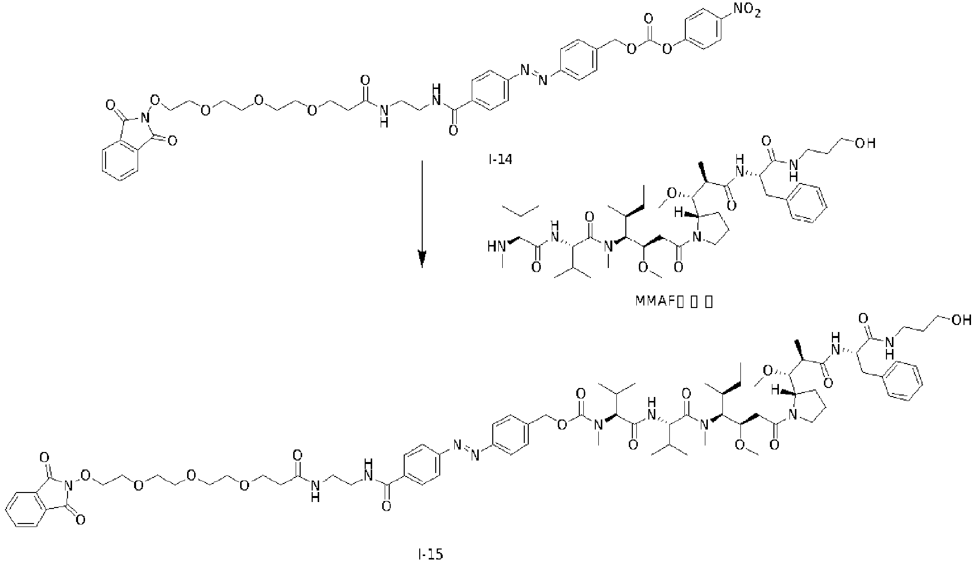

(*E*)-4-((4-((1-((1,3-dioxoisoindolin-2-yl)oxy)-12-oxo-3,6,9-trioxa-13-azapentadecan-15-yl)carbamoyl)phenyl)diazenyl)benzyl (4-nitrophenyl) carbonate (I-14) (0.034 g, 0.042 mmol) was added to a 25 mL eggplant-shaped flask, to which 2 mL of anhydrous DMF were added. After the compound raw materials were completely dissolved, an MMAF derivative, HOBT and DIPEA were added to the reaction solution in order, and the resulting mixture was stirred at room temperature overnight. The solvent was removed by concentration under reduced pressure. The residue was mixed with silica gel for sample loading, and purified by column chromatography to obtain the target compound as a yellow solid (0.01 g, 17% yield), which were directly used for the reaction in the next step.

### Synthesis steps of MMAF derivatives:

1) Preparation of ((2*R,*3*R*)-3-((*S*)-1-((3*R*,4*S*,5*S*)-4-((*S*)-2-((*S*)-2-((((9*H*-fluoren-9-yl)methoxy)carbonyl) (methyl)amino)-3-methylbutanamido)-N,3-dimethylbutanamido)-3-methoxy-5-methylheptanoyl)pyrrolidin-2-yl)-3-methoxy-2-methylpropanoyl)-L-phenylalanine

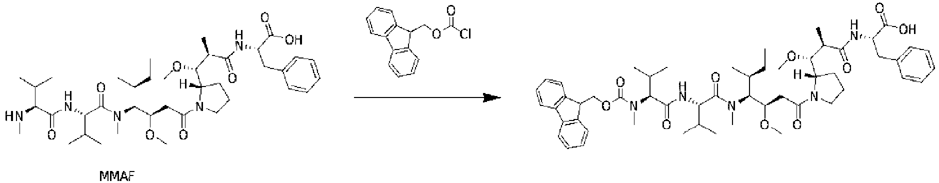

MMAF (300 mg, 0.39 mmol) was dissolved in 5 mL of DMF, and 9-fluorenylmethylchloroformate (50 mg, 0.195 mmol) and sodium bicarbonate (13 mg, 0.156 mmol) were added in order under stirring. The mixture was stirred and reacted at room temperature overnight. After the reaction was completed, the solvent was removed by concentration under reduced pressure. The residue was purified by column chromatography to obtain the target product as a white solid powder (200 mg, 57% yield). MS (ESI) m/z: 953.55 [M+H]⁺.

### 2) Preparation of (9H-fluoren-9-yl)methyl ((S)-1-(((S)-1-(((3R,4S,5S)-1-((S)-2-((1R,2R)-3-(((S)-1-((3-hydroxypropyl)amino)-1-oxo-3-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate

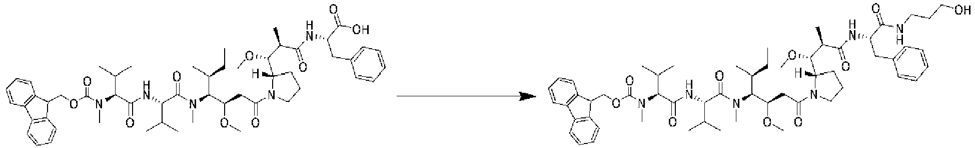

### ((2R,3R)-3-((S)-1-((3R,4S,5S)-4-((S)-2-((S)-2-((((9H-fluoren-9-yl)methoxy)carbonyl) (methyl)amino)-3-methylbutanamido)-N,3-dimethylbutanamido)-3-methoxy-5-methylheptanoyl)pyrrolidin-2-yl)-3-methoxy-2-methylpropanoyl)-L-phenylalanine (100 mg, 0.105 mmol), 3-aminopropan-1-ol (10 mg, 0.126 mmol) and HATU (60 mg, 0.158 mmol) were dissolved in 5 mL of ultra-dry DMF, then DIPEA (27 mg, 0.21 mmol) was added under stirring. The resulting mixture was stirred and reacted at room temperature for 2 h. After the reaction was completed, the solvent was removed by concentration under reduced pressure. The residue was purified by column chromatography to obtain the target product as a yellow solid powder (91 mg, 90% yield). MS (ESI) m/z :1010.60 [M+H]⁺.

### 3) Preparation of MMAF derivative

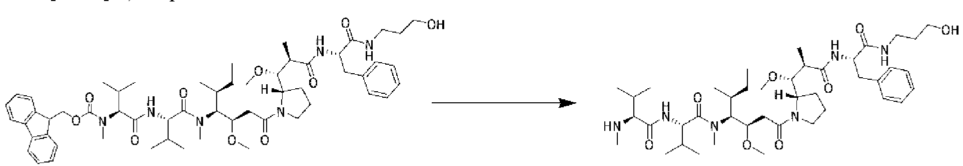

(9*H*-fluoren-9-yl)methyl ((*S*)-1-(((*S*)-1-(((3*R*,4*S*,5*S*)-1-((*S*)-2-((1*R*,2*R*)-3-(((*S*)-1-((3-hydroxypropyl)amino)-1-oxo-3-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (90 mg, 0.089 mmol) was dissolved in 2 mL of ultra-dry DMF, the resulting mixture was cooled to 0 °C in an ice-salt bath. 100 µL of hexahydropiperidine were added to the solution under stirring, and the resulting mixture was stirred and reacted for 20 min, then the reaction was completed. The solvent was removed by concentration under reduced pressure, and the residue was purified by column chromatography to obtain the target product as a white solid powder (39 mg, 56% yield). MS (ESI) m/z :788.54 [M+H]⁺.

### 6) Preparation of compound II-15

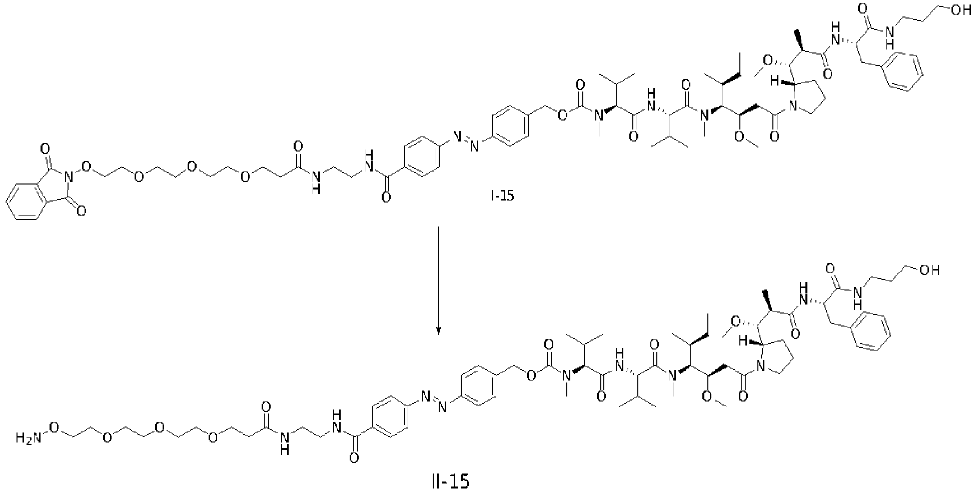

Compound I-15 (20 mg, 0.014 mmol) was added to a 10 mL eggplant-shaped flask, to which 1 mL of anhydrous DCM was added, then the reaction solution was cooled in an ice bath to 0 °C. Hydrazine hydrate (0.001 g, 0.021m) was diluted into 0.5 mL of anhydrous DCM, and the obtained dilution was slowly dropwise added to the reaction mixture for reacting overnight, to precipice insoluble solids. The reaction mixture was extracted with DCM several times, and washed with saturated brine, and the organic phases were combined and dried over anhydrous sodium sulfate overnight. The solvent was removed by concentration under reduced pressure, and the obtained crude product was purified by thin layer preparative chromatography to obtain the target product as a red brown solid (11mg, 59% yield).¹H-NMR (600 MHz, DMSO-*d6*): δ 8.679-8.670 (m, 1H), 8.083-8.016 (m, 3H), 7.975-7.870 (m, 4H), 7.641-7.551 (m, 1H), 7.245-7.126 (m, 5H), 5.274-5.160 (m, 1H), 4.657-4.624 (m, 1H), 4.491-4.395 (m, 2H), 3.970 (br, 1H), 3.803 (t, *J* = 8.4 Hz, 1H), 3.621-3.594 (m, 3H), 3.518-3.454 (m, 10H), 3.388-3.325 (m, 11H), 3.263-3.171 (m, 9H), 3.055-2.828 (m, 7H), 2.333 (t, *J* = 6.6 Hz, 2H), 2.180-2.119 (m, 4H), 1.531(t, *J* = 6.6 Hz, 3H), 1.346 (s, 4H), 1.234 (s, 10H), 1.042-0.749 (m, 26H). MS (ESI) m/z: ESI-MS m/z (M+H)⁺ calculated for C₆₈H₁₀₆N₁₁O₁₆ 1332.77, found 1332.78.

### 7) Synthesis of compound III-15

According to the method as described in the literature (PNAS. 111(5): 1766-71.), compound II-15 was conjugated with a site-directed mutatedanti-HER2 antibody to obtain the target antibody-drug conjugate III-15, wherein the site-directed mutated anti-HER2 antibody contains the unnatural amino acid *p*-acetylphenylalanine (pAF), and the carbonyl group on the pAF reacted with the hydroxylamino group on the compound II-15 to form an oxime bond, thereby achieving site-specific conjugation. In compound III-15, 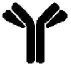 was a site-directed mutated anti-HER2 antibody, and the drug-antibody ratio (DAR) value E was about 2.

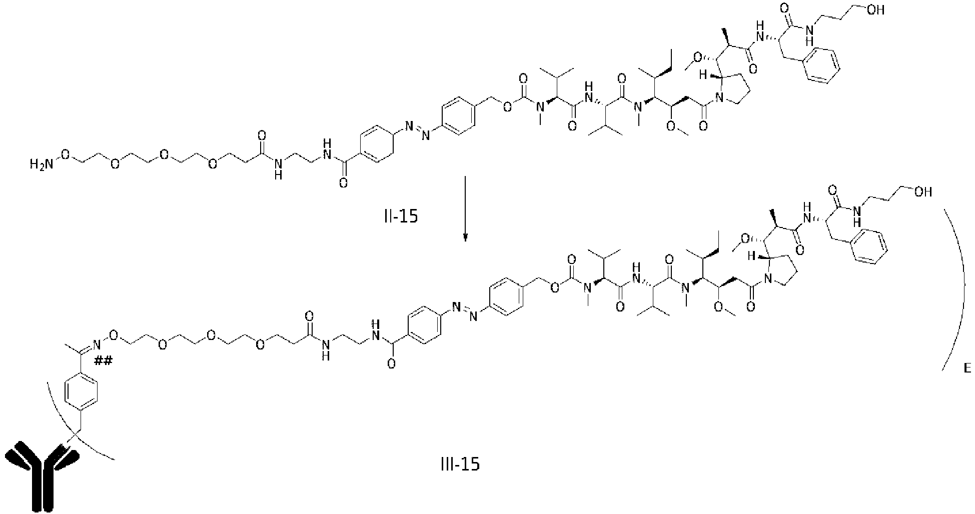

The site-directed mutated anti-HER2 antibody (Mol Cancer Ther (2020) 19 (9): 1833-1843.) had a sequence as shown below:

### Heavy chain:

EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSUSTK
GPSVFPLAPSSKSTSGGTAALGCLVKDYFPEPVTVSWNSGALTSGVHTFPAVLQSSGLYSLSSVVT
VPSSSLGTQTYICNVNHKPSNTKVDKKVEPKSCDKTHTCPPCPAPELLGGPSVFLFPPKPKDTLMIS
RTPEVTCVVVDVSHEDPEVKFNWYVDGVEVHNAKTKPREEQYNSTYRVVSVLTVLHQDWLNGK
EYKCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPG
(SEQ ID NO:1)

### Light chain:

DIQMTQSPSSLSASVGDRVTITCRASQDVNTAVAWYQQKPGKAPKLLIYSASFLYSGVPSRFS
GSRSGTDFTLTISSLQPEDFATYYCQQHYTTPPTFGQGTKVEIKRTVAAPSVFIFPPSDEQLKSGTAS
VVCLLNNFYPREAKVQWKVDNALQSGNSQESVTEQDSKDSTYSLSSTLTLSKADYEKHKVYACE
VTHQGLSSPVTKSFNRGEC (SEQ ID NO:2)

In the sequence, "U" represents acetylphenylalanine.

### Example 7: Preparation of III-16

### 1) Preparation of compound I-16

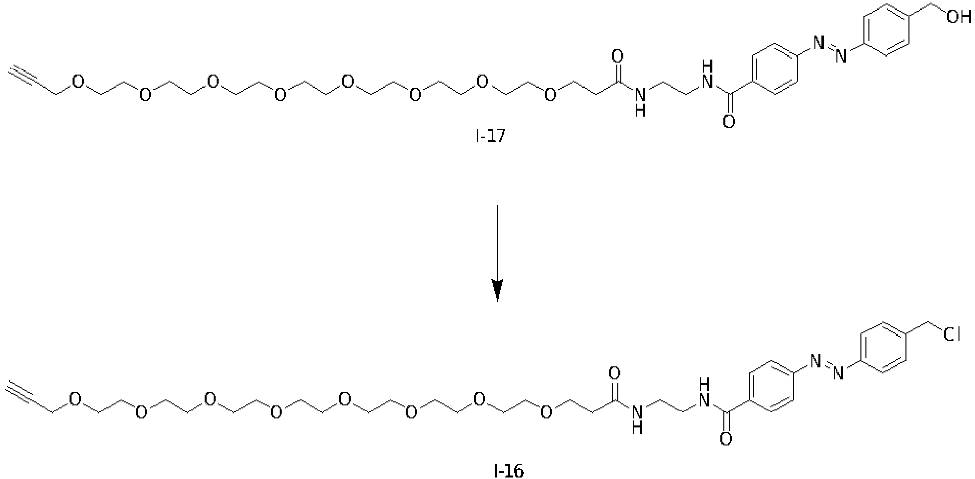

Cyanuric chloride was dissolved in 200 µL of anhydrous DMF, and the mixture was stirred at room temperature for 2 h, during which a large amount of white precipitates was formed. *(E*)-*N*-(2-(4-((4-(hydroxymethyl)phenyl)diazenyl)benzamido)ethyl)-4,7,10,13,16,19,22, 25-octaoxaoctacos-27-ynamide (100 mg, 0.14 mmol) was dissolved in 2 mL of anhydrous DCM, and subsequently was added to the reaction flask, the resulting mixture was stirred at room temperature for 2 days. After the reaction was completed, the solvent was removed by concentration under reduced pressure to obtain a crude product, which was purified by column chromatography to obtain the target product as a yellow solid (30 mg, 30% yield). The reaction product was directly used for the reaction in the next step.

### 2) Preparation of compound II-16

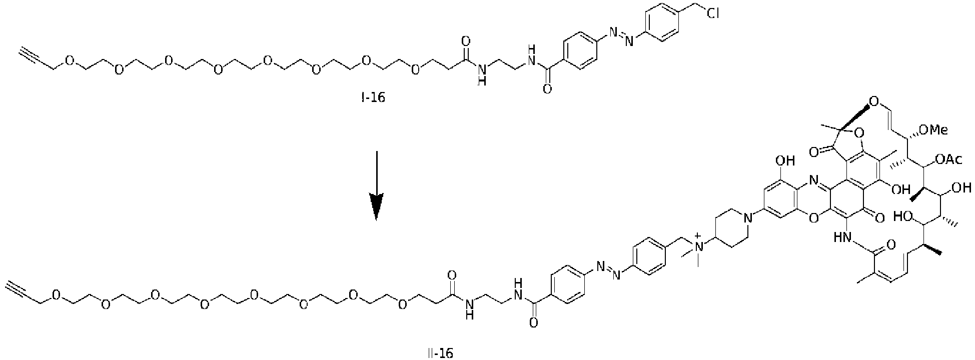

Compound I-16 (25 mg, 0.034 mmol) and an effector molecule rifalogue (28 mg, 0.03 mmol) were added to a 1.5 mL EP and dissolved by the addition of 400 µL of anhydrous DMF. Then DIPEA (19 mg, 0.15 mmol) was dropwise added to the reaction system, the mixture was shaken at room temperature for 5 days. After the reaction was completed, the solvent was removed by concentration under reduced pressure to obtain a crude product, which was purified by column chromatography to obtain the target product as a black solid (10 mg, 20% yield).HR-MS (ESI)m/z:1626.792 [M+H] ⁺.

### 3) Preparation of azido group-modified antibacterial antibody

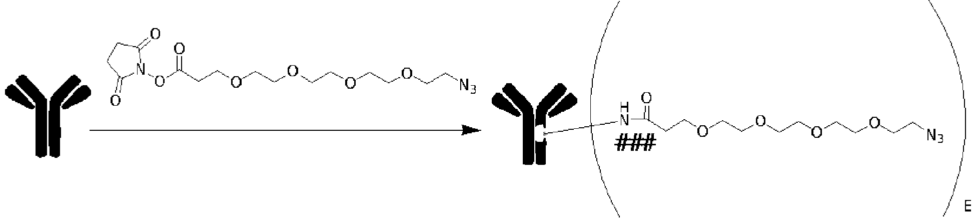

Antimicrobial antibody BD37 (26.25 mg, 1.75×10⁻⁴ mmol) was added to a 5 mL EP tube, to which a solution of compound 2,5-dioxopyrrolidin-1-yl 1-azido-3,6,9,12-tetraoxapentadecan-15-oate (0.54 mg, 1.4×10⁻³ mmol) in DMA was dropwise added. The mixture was reacted and stirred at room temperature overnight, the conjugated product was buffer-exchanged into PBS to obtain the azide group-modified antimicrobial antibody BD37, wherein the drug-antibody ratio (DAR) E was about 4, and 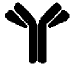 was an antibacterial antibody BD37.

The antibacterial antibody BD37 has the sequence:

### Heavy chain:

MKKNIAFLLASMFVFSIATNAYAEVQLVQSGAEVKKPGASVKVSCEASGYTLTSYDINWVR
QATGQGPEWMGWMNANSGNTGYAQKFQGRVTLTGDTSISTAYMELSSLRSEDTAVYYCARSSIL
VRGALGRYFDLWGRGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLTVDKSRWQQGN
VFSCSVMHEALHNHYTQKSLSLSPGK (SEQ ID NO:3)

### Light chain:

MKKNIAFLLASMFVFSIATNAYADIVMTQSPSILSASVGDRVTITCRASQTISGWLAWYQQKP
AEAPKLLIYKASTLESGVPSRFSGSGSGTEFTLTISSLQPDDFGIYYCQQYKSYSFNFGQGTKVEIKR
TVAAPSVFIFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC (SEQ ID NO:4)

### 4) Preparation of compound III-16

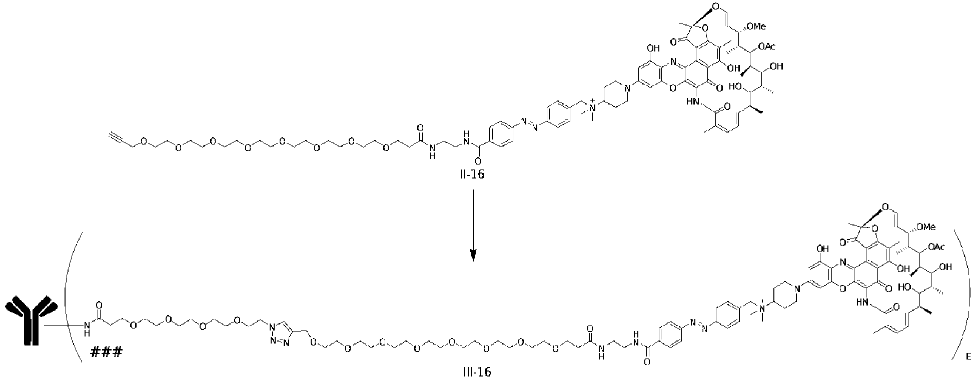

The azide group-modified antimicrobial antibody BD37 (2.5 mg, 1.67×10⁻⁵ mmol) was added to a 2 mL EP tube, to which a solution of compound II-16 (0.41 mg, 2.5×10⁻⁴ mmol) in DMA was added. Then, CuSO₄ (0.13 mg, 5.01×10⁻⁴ mmol), THPTA (0.87 mg, 2.0×10⁻³ mmol) and a sodium ascorbate (1.49 mg, 7.51×10⁻³ mmol) aqueous solution were added to the reaction system in order, and the mixture was reacted and stirred at room temperature for 5h, the conjugated product was buffer-exchanged into PBS to obtain the target antibody-drug conjugate III-16, wherein the drug-antibody ratio (DAR) E was about 2, and 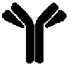 was the antibacterial antibody BD37.

### Example 8: Preparation of compound III-17

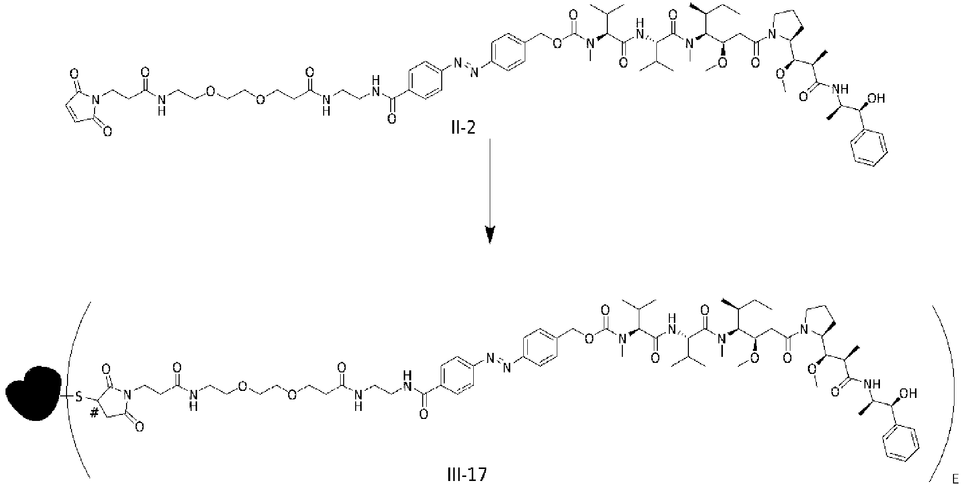

Albumin (1 equivalent, purchased from Zhejiang Hisun Pharmaceutical Co., LTD.) was added to a 10 mL reaction flask, to which a solution of compound II-2 (10 equivalents) in DMA was added. The mixture was stirred at room temperature overnight, the conjugated product was buffer-exchanged into PBS to obtain the target antibody-drug conjugate III-17, wherein the drug-antibody ratio (DAR) E was 1, and 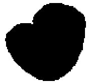 represented albumin.

### Example 9: Preparation of compound III-18

### 1) Preparation of (E)-1-(4-((4-(dimethylamino)phenyl)diazenyl)phenyl)but-3-yn-1-ol

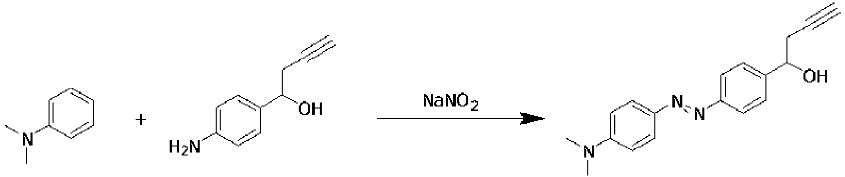

Compound 1-(4-aminophenyl)but-3-yn-1-ol (0.3 g, 1.86 mmol) was added to a 50 mL eggplant-shaped flask, to which 12 mL of concentrated hydrogen chloride and 6 mL of water were added, and the resulting mixture was cooled in an ice bath to 0 °C, then NaNO₂ (0.152 g, 2.2 mmol) was added thereto, the resulting mixture was stirred for 1h. *N*, *N*-dimethylaniline (0.27 g, 2.2 mmol) was dissolved in 2 mL of glacial acetic acid and added to the reaction solution, the mixture was reacted overnight. The solvent was removed by concentration under reduced pressure, and the residue was mixed with silica gel for sample loading, and purified by column chromatography to obtain a brown red solid (0.44 g, 80% yield). ESI-MS m/z (M+H)⁺ calculated for C₁₈H₁₉N₃O 294.15, found 294.16.

### 2) Preparation of (E)-1-(4-((4-(dimethylamino)phenyl)diazenyl)phenyl)but-3-yn-1-yl (4-nitrophenyl) carbonate

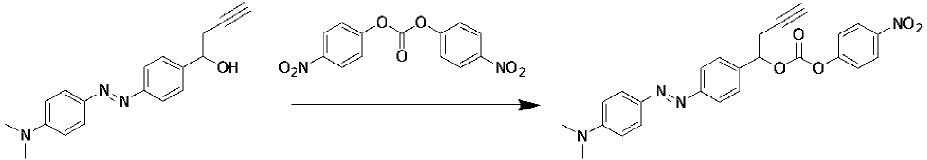

(*E*)-1-(4-((4-(dimethylamino)phenyl)diazenyl)phenyl)but-3-yn-1-ol (0.4 g, 1.36 mmol) was added to a 25 mL eggplant-shaped flask, to which 3 mL of anhydrous DMF were added. After the reaction raw materials were completely dissolved, bis(*p*-nitrophenyl)carbonate and DIPEA were added in order, and the resulting mixture was reacted at room temperature for 4 h. The solvent was removed by concentration under reduced pressure, and the obtained crude product was mixed with silica gel for sample loading, and purified by column chromatography to obtain the target product as a yellow solid (0.42 g, 67% yield). ESI-MS m/z (M+H)⁺ calculated for C₂₅H₂₂N₄O₅ 459.16, found 459.17.

### 3) Preparation of compound II-17

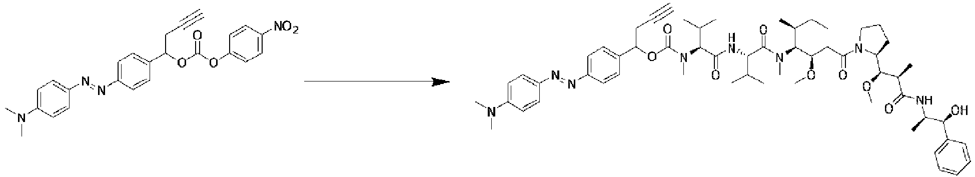

Compound (*E*)-1-(4-((4-(dimethylamino)phenyl)diazenyl)phenyl)but-3-yn-1-yl (4-nitrophenyl) carbonate (110 mg, 0.24 mmol), MMAE (172.0 mg, 0.24 mmol), HOBT (32.4 mg, 0.24 mmol) were added to a 50 mL eggplant-shaped flask, then 10 mL of anhydrous DMF were added, and the mixture was stirred at room temperature until completely dissolved. After the solids were completely dissolved, DIPEA (50 µL, 0.288 mmol) was dropwise added and the mixture was reacted at room temperature for 24 h. After the reaction was completed, the solvent was removed by concentration under reduced pressure to obtain a crude product, which was purified by column chromatography to obtain the target product as a white powdery solid (200 mg, 80% yield).HR-MS (ESI) m/z: 1037.64 [M+H]⁺.

### 4) Preparation of azido group-modified anti-HER2 antibody trastuzumab

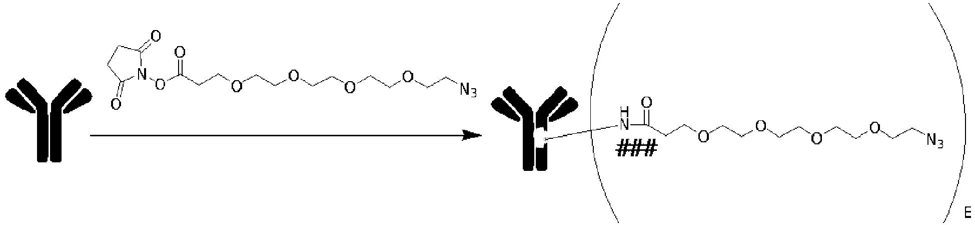

Anti-HER2 antibody trastuzumab (26.25 mg, 1.75×10⁻⁴ mmol) was added to a 5 mL EP tube, to which a solution of compound 2,5-dioxopyrrolidin-1-yl 1-azido-3,6,9,12-tetraoxapentadecan-15-oate (0.54 mg, 1.4×10⁻³ mmol) in DMA was dropwise added. The resulting mixture was reacted and stirred at room temperature overnight, the conjugated product was buffer-exchanged into PBS to obtain the azido group-modified anti-HER2 antibody trastuzumab, wherein the drug-antibody ratio (DAR) E was about 4, and 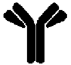 was anti-HER2 antibody trastuzumab.

### 5) Preparation of compound III-18

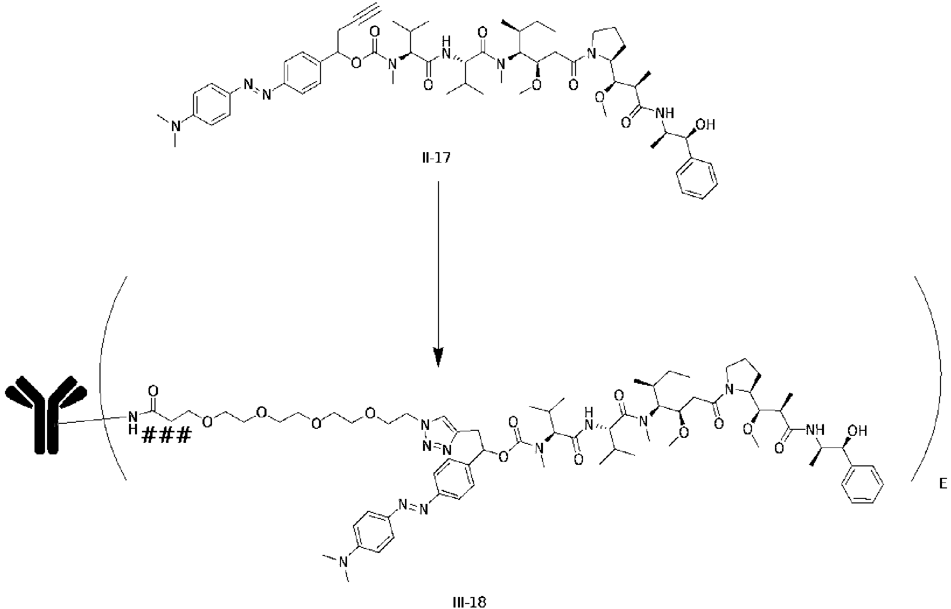

The azido group-modified anti-HER2 antibody trastuzumab (2.5 mg, 1.67×10⁻⁵ mmol) was added to a 2 mL EP tube, to which a solution of compound II-17 (0.26 mg, 2.5×10⁻⁴ mmol) in DMA was dropwise added. Then CuSO₄ (0.13 mg, 5.01×10⁻⁴ mmol), THPTA (0.87 mg, 2.0×10⁻³ mmol) and a sodium ascorbate (1.49 mg, 7.51×10⁻³ mmol) aqueous solution were added to the reaction system in order, and the mixture was reacted and stirred at room temperature for 5 h, the conjugated product was buffer-exchanged into PBS to obtain the target antibody-drug conjugate III-18, wherein the drug-antibody ratio (DAR) E was about 3, and 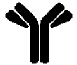 was anti-HER2 antibody trastuzumab.

### Example 10: Quality control study on the ADC represented by compound III-2

The present example evaluated the DAR and aggregation degree of the ADC represented by compound III-2 (FIG. 1). The main peaks observed in the hydrophobic interaction chromatography spectrum of compound III-2 corresponded to antibodies conjugated with 0, 2, 4 and 6 small molecule drugs, respectively, and the DAR of compound III-2 was about 4.0. The size exclusion chromatography results showed that compound III-2 existed almost completely in the monomeric form (with purity >95%). Results of the ultra-performance liquid chromatography coupled with quadrupole time-of-flight mass spectrometry (UPLC-Q-TOF-MSLC-MS) further verified that each antibody molecule of compound III-2 was conjugated with an average of 4.0 small molecule drugs.

### Example 11: Quality control study on compounds III-1, III-3, III-4, III-5 and VC-ADC

The present example evaluated the DARs and aggregation degrees of III-1, III-3, III-4, III-5 and Herceptin-valine-citruline-MMAE (VC-ADC) (FIG. 2). Results of the hydrophobic interaction chromatography showed that the DARs of compounds III-1, III-3, III-4, **III-5** and VC-ADC were about 4.0, respectively. The size exclusion chromatography results showed that compounds III-1, III-3, III-4, III-5 and VC-ADC existed almost completely in the monomeric form (with purity>95%).

### Example 12: Plasma stability study on compound III-2

The present example evaluated the stability of compound III-2 in 50% human plasma. Compound III-2 (25 µL, 0.0227 mM) was added to 2245 µL of 2-fold diluted human plasma to prepare a working solution. The working solution was incubated in a constant-temperature incubator at 37 °C. and 50 µL of aliquots were collected at predetermined time points (0 h, 3 h, 6 h, 24 h, 48 h, 72 h, 96 h, 120 h, 144 h, and 168 h). The collected samples were quenched with pre-cooled acetonitrile, then stored at - 80 °C. After the sampling was completed, all the samples were thawed at room temperature, and ultra-centrifuged to remove proteins; the MMAE content in the supernatant was quantified by LC-MS/MS. The results (FIG. 3) showed that compound III-2 showed excellent plasma stability, with the total release of MMAE less than 1% within 7 days in human plasma.

### Example 13: Study on the antigen binding affinity of compound III-2

The present example evaluated the binding affinity of compound III-2 to HER2 antigen. Each well of a 96-well plate was coated HER2 antigen (100 µL, 1µg/mL), and the plate was incubated overnight in a humidified chamber at 4 °C. Subsequently, 10% fetal bovine serum was added to block the protein binding sites, followed by incubation at 37 °C for 2 h. Serially diluted compound III-2 or Herceptin solutions were added to the wells and incubated at 37 °C for 1 h. After incubation, the plate was washed three times with PBS containing 0.2% Tween 20. Then, 100 µ of LHRP-labeled goat anti-human IgG-Fc secondary antibody (Thermo Fisher Scientific, USA) was added to each well, and the plate was incubated for 45 min at room temperature. After washes three times with PBS comprising 0.2% Tween 20, a TMB reagent was added to the wells for color development, and then 2M H₂SO₄ was added to terminate the color developing. The absorbance of each well at 450 nM was measured useing a microplate reader. The results showed (FIG. 4) that compound III-2 and Herceptin had HER2 antigen binding affinity comparable to HER2 antigen, with EC₅₀ values of 0.14 µg/mL and 0.042 µg/mL, respectively.

### Example 14: Study on the affinity binding affinity of compound III-2 on cell lines

The present example evaluated the antigen binding affinity of compound III-2 on HER2-positive SKOV3cell lines and HER2-negative MCF-7cell lines. The SKOV3 and MCF-7 cells were collected and counted with a cell counter, and the cells were transferred to 1.5 mL EP tubes (at a density of about 5×10⁵ cells/sample). The cells were washed once with FACS buffer (1 × PBS containing 2% fetal bovine serum) for subsequent use. Compound III-2 (5 µg/mL), Herceptin (5 µg/mL) or blank PBS control was added to the cells, respectively, followed by incubation at 4 °C for 30 min. After incubation, the cells were washed twice with FACS buffer and further incubated with PE dye-labeled goat anti-human IgG Fc secondary antibody at 4 °C for 30 minutes. After three washes with the FACS buffer, the sample was detected using a FACSCalibur. The results (FIG. 5) showed that both Herceptin and compound III-2 exhibited strong fluorescence signals in HER2-positive SKOV3 cells (the right panel in FIG. 5), while weak fluorescence was observed in HER2-negative MCF-7 cells (the left panel in FIG. 5), indicating that compound III-2 specifically targets the HER2 antigens on tumor cells.

### Example 15: Study on the cellular internalization of compound III-2 on cell lines

The present example evaluated the cellular internalization property of compound III-2 on HER2-positive SKOV3 cell line. The SKOV3 cells were collected in 1.5 mL EP tubes and incubated with compound III-2 or Herceptin at 4 °C for 1 hour. Then, the samples treated with compound III-2 and Herceptin were further divided into a control group and an experimental group. After the samples were washed twice with a pre-cooled FACS buffer, the experimental group was transferred to an incubator at 37 °C and incubated for 8 hours or 24 hours, while the control group was incubated at 4 °C for the corresponding durations. At the predetermined time points, all samples were collected and washed three times with FACS buffer, and then incubated with a PE dye-labeled goat anti-human IgG Fc secondary antibody for 30 minutes at 4 °C. After sampling, the relative mean fluorescence intensity (rMFI) of PE on the cell surface was measured by flow cytometry, and the internalization percentage was calculated with rMFI according to the following formula: Internalization percentage (%) = [100- (rMFI of experimental group / rMFI of control group)] ×100%. The results (FIG. 6) showed that compound III-2 exhibited a slightly higher internalization rate than Herceptin, with internalization efficiencies of 34.1% versus 22.1% at 8 h and 41.7% versus 31.1% at 24 h.

### Example 16: Study on the cellular binding and internalization imaging of compound III-2

The present example evaluated the cellular binding and internalization properties of compound III-2 in SKOV3cell line.

According to the manufacturer's instructions, Compound III-2 was fluorescently labeled by using FITC NHS ester (purchased from Innochem). Specifically, the compound III-2 was incubated and reacted with fluorescent dye (FITC NHS ester) in NaHCO₃ buffer (pH = 8.3) for 3 hours under stirring, the dye was labeled on the antibody, and the obtained FITC-III-2 was buffer-exchanged into a PBS buffer for subsequent use.

SKOV3 cells were inoculated into a chamber slide with 8 wells and incubated in a cell incubator overnight. After the culture medium was removed, FITC-III-2 was added to the cells and incubated at 4 °C and 37 °C, respectively. SKOV3 cells incubated at 4 °C were used for the measurement of the binding affinity of compound III-2, while cells incubated at 37 °C were used to study the internalization of compound III-2. At the predetermined time points, the cells were washed with PBS, the cell nuclei were stained with DAPI, and the lysosomes were stained with LysoTracker Red. After washes twice with PBS, fluorescence imaging was carried out using a laser scanning confocal microscope. The results (FIG. 7) showed that cells were incubated with Herceptin and compound III-2 at 4 °C, under such conditions, the antibody bound to the antigen on the cell membrane surface without intracellular internalization. After 24-hour incubation at 37 °C, intracellular protein fluorescence signals and lysosome fluorescence signals were co-localized (with pearson correlation coefficient = 0.87), indicating that compound III-2 was internalized and transported to the lysosomes.

### Example 17: Study on the in vitro cytotoxicity of compound III-2

The present example evaluated the in vitro cytotoxicity of test drugs such as compound III-2, trastuzumab (Herceptin), Herceptin-valine-citruline-MMAE (VC-ADC), and MMAE. The test cell lines included HER2-positive breast cancer cell line BT474, Herceptin-resistant BT474 cell line (BT 474-HDR), HER2-positive ovarian cancer cell line SKOV3, HER2-positive gastric cancer cell line NCI-N87, weakly HER2-positive breast cancer cell line MCF-7, HER2-negative breast cancer cell line MDA-MB-231, mouse embryonic fibroblast cell line NIH3T3, human renal epithelial cell line 293T, and human normal hepatocyte cell line LO₂ (all the above cell lines were purchased from ATCC).

Reagents, apparatus and consumables used in the experiment were listed in the following table.

| Reagents and consumables | Vendor | Cat# |
|---|---|---|
| FBS | Invitrogen | 10099 |
| Penicillin-Streptomycin | Invitrogen | 15140 |
| DMEM | Invitrogen | 10569010 |
| CellTiter Glo kit | Promega | G9243 |
| Instruments | Vendor | Cat# |
| Enspire | PE | 2300 |

The test procedure was shown as follows:
1. Thawing Cell
   Cryopreserved target cells was thawed by gentle agitation of the cryovial in a 37 °C water bath;
   after thawing, the cryovial was removed from the water bath and decontaminated by spraying or immersing in 70% ethanol;
   the cells in the cryovial were transferred into a centrifuge tube containing 9 mL of complete DMEM culture medium, followed by centrifugation at 200 g for 5 min;
   the cells were resuspended with culture medium, and precipitated and seeded into a 75 cm² culture flask;
   the cells were cultured in a Galaxy^{®} CO₂ incubator (48R, #CO48312044) under standard culture conditions of 37 °C, 20% oxygen and 5% carbon dioxide.
2. Spreading Cell
   Cells were subcultured three times per week at a ratio of 1:4 in a culture medium supplemented with 10% FBS (heat inactivated) and a 1% Penicillin-Streptomycin solution;
   for cell passaging, adherent cells were first rinsed with 3 mL of trypsin/EDTA solution (3 mL). Then, 3 mL of trypsin/EDTA solution was added to the T75 culture flask, and the flask was gently rotated to ensure uniform coverage of the cell. The cells were incubated at 37 °C until complete cell detachment was achieved. Cell detachment was verified under a microscope, then an equal volume of the cell culture medium was added to inactivate the trypsin, and the separated cells were collected, centrifuged at 200 g for 5 minutes, and then re-suspended in a fresh culture medium.
3. Inoculating cell
   The cells were collected and counted;
   100 µ L of a cell suspension with adjusted density were inoculated to a 96-well cell culture plate, and the final cell density was about from 5000 cells/well to 10000 cells/well;
   The culture plate was covered and incubated in a 5% CO₂ incubator with 20% oxygen at 37 °C for 24 h.
4. Preparing compounds
   A compound solution was serially diluted at a ratio of 1:3, wherein the maximum initial concentrations of compound III-2 and VC-ADC were about 400 µg/mL, and the initial concentration of MMAE was 400 nM;
5. Co-incubation of cells with test compounds
   The culture medium in the 96 wells plate inoculated with cells was discarded, 150 µL of fresh culture medium was added to each well, and then 50 µL of the prepared test compounds were added to the 96-well plate. After the cells were co-incubated with the compounds in an incubator under conditions of 37 °C, 5% CO₂, and 0.1% O₂ for 24 h. After incubation, the drug-containing medium was removed, and the cells were rinsed with a fresh culture medium. Then the cells were placed in an incubator at 37 °C, 5% CO₂, and 20% O₂ and continuously cultured for an additional 72 h or 120 h.
6. Reading plate
   The plate was taken out of the incubator and equilibrated for 15 minutes at room temperature;
   prior to the experiment, the CellTiter Glo reagent was incubated at 37 °C;
   The detection solution was prepared by diluting CellTiter-Glo reagent with PBS at a volume ratio of 3:7, and the diluted solution was added into the wells to be tested;
   the plate was then shaken at room temperature for 8 min, and allowed to stand for 2 min, and then signals were detected using an EnSpire reader.
7. Data analysis
   The cell viability percentage was calculated according to the following formula:$% Viability = 100 \times \left(Sample/HC\right)$
   wherein HC represents the control group of cells without test compound treatment.

The cytotoxicity (FIG. 8, Table 1) results showed that all the cell lines were highly sensitive to MMAE, with EC₅₀ values ranging from 0.01 nM to 0.56 nM. Under hypoxic conditions, compound III-2 showed potent cell proliferation inhibitory activity on NCI-N87, BT-474 and SKBR3 cell lines, with EC₅₀ values of 0.07 nM, 0.026 nM and 0.015 nM, respectively (FIG. 8), but compound III-2 showed weak inhibitory activity on HER2-negative cell lines (MCF-7, MDA-MB-231) (with EC₅₀ >100 nM). The above results confirmed that under hypoxic conditions, compound III-2 had proliferation inhibitory activity comparable to that of MMAE on HER2-positive cell lines, while had weak activity on HER2 negative cell lines.

**Table 1 Cell proliferation inhibitory activity of compound III-2 under hypoxic conditions (0.1% O₂)**

| Cell lines | | Test compounds (EC₅₀, nM) | | |
|---|---|---|---|---|
| | | Herceptin | MMAE | III-2 |
| HER2+ | NCI-N87 | 0.27 | 0.24 | 0.069 |
| | BT-474 | 0.22 | 0.032 | 0.027 |
| | SKBR3 | 0.13 | 0.058 | 0.016 |
| HER2- | MCF-7 | >66 | 0.015 | 115.4 |
| | MDA-MB-231 | >66 | 0.57 | >666 |

### Example 18: Study on the hypoxia-dependent cytotoxicity of compound III-2

The present example investigated the in vitro hypoxia-dependent cytotoxicity of hypoxia-activated compound III-2, and the culture procedures of Herceptin-resistant HER2-positive breast cancer cell line BT474-HDR and HER2-positive ovarian cancer cell line SKOV3 were consistent with those described in Example 14. The cell incubator was set at oxygen concentrations of 0.1%, 1.0%, 5.0% and 20.0%, respectively. The cytotoxicity (FIG. 9, Table 2) results showed that the cytotoxic activity of compound III-2 increased as the oxygen concentration decreased. Under the condition of the oxygen concentration at 20%, 5%, 1% and 0.1%, the EC₅₀ values of compound III-2 against SKOV3 cells were 66.8 nM, 3.74 nM, 0.079 nM and 0.063 nM, respectively, while the EC₅₀ values against BT474-HDR cells were 109.1 nM, 9.12 nM, 0.57 nM and 0.19 nM, respectively. The results showed that compound III-2 had potent proliferation inhibitory activity under hypoxic conditions (O₂ <1%), and weak activity under normoxic conditions. However, the conventional VC-ADC exhibited no oxygen concentration-dependent cytotoxicity, and maintained potent cell proliferation inhibitory activity no matter under normoxic conditions or under hypoxic conditions, indicating that the conventional ADCs have the risk of off-target toxicity (FIG. 9, Table 2).

**Table 2 Oxygen concentration-dependent cytotoxicity of compound III-2 and VC-ADC**

| O₂% | SKOV3 (EC₅₀, nM) | | BT474-HDR (EC₅₀, nM) | |
|---|---|---|---|---|
| | III-2 | VC-ADC | III-2 | VC-ADC |
| 20% O₂ | 66.8 | 0.062 | 109.1 | 0.26 |
| 5% O₂ | 3.74 | 0.070 | 9.12 | 0.10 |
| 1% O₂ | 0.079 | 0.057 | 0.57 | 0.10 |
| 0.1% O₂ | 0.063 | 0.051 | 0.19 | 0.052 |

### Example 19: Study on the bystander effect of compound III-2

The example investigated the bystander effects of hypoxia-activated compound III-2. HER2-positive ovarian cancer cell lines SKOV3 and weakly HER2-positive breast cancer cell lines MCF-7 were mixed at a cell density ratio of 1:1 and inoculated in well plates, and the culture procedures of the cells were consistent with those described in Example 14, wherein the oxygen concentrations were set to 0.1% and 20.0% in a cell incubator, respectively. The results (FIG. 10, Table 3) showed that under normoxic conditions, the cytotoxic activity of compound III-2 in the co-culture model decreased to the micromolar level (with EC₅₀ value of about 0.1 µM), and the result showed that compound III-2 had high safety in normal tissues (FIG. 3). Under hypoxic conditions, compound III-2 remained high cytotoxic activity in the co-culture model, with an EC₅₀ value of 0.053 nM. The results showed that compound III-2 has the bystander effect in tumor tissues and also has killing effects on tumor cells with low expression of HER2 (FIG. 10, Table 3).

**Table 3 Cell proliferation inhibitory activity of compoundIII-2 in a co-culture cell model**

| Compounds | SKOV3/MCF-7 Cocuture (EC₅₀, nM) | |
|---|---|---|
| | 20% O₂ | 0.1 O₂ |
| III-2 | 134.7 | 0.053 |

### Example 20: Study on the cytotoxicity of Compound III-2 on normal cells

The example evaluated the cytotoxicity of hypoxia-activated compound III-2 on normal cells. The culture procedures of the test cell lines including mouse embryonic fibroblast NIH3T3 cells, human renal epithelial cell 293T cells and human normal hepatocyte LO₂ cells were consistent with those described in Example 14. At the maximum tested concentration (666 nM), compound III-2 showed weak inhibitory activity against normal NIH3T3 and 293T cells, with a maximum inhibition rate <50% (FIG. 11, Table 4). However, conventional VC-ADCs still exhibited high off-target cytotoxicity (with EC₅₀ <10 nM, and a maximum inhibition > 90%, FIG. 11, Table 4).

**Table 4 Cell proliferation inhibitory activity of Compound III-2 on normal cell lines**

| Cell lines | Test compounds (EC₅₀, nM) | | |
|---|---|---|---|
| | Herceptin | VC-ADC | III-2 |
| NIH3T3 | >666 | 42.93 | >666 |
| 293T | >666 | 51.48 | >666 |
| LO2 | >666 | 53.97 | 266.93 |

### Example 21: Study on the MMAE release of compound II-2

The present example evaluated the release profile of SN-38 from compound II-2 under the action of azoreductase. Compound II-2 was reacted with 3-mercaptopropionic acid (MPA, 10 equivalents) to prepare a stock solution of MPA-II-2. The preparation MPA-II-2 was carried out according to the method reported in the reference literature (Y. Wang, S. Fan, W. Zhong, X. Zhou, S. Li, int. J. Mol Sci.2017, 18, e 1860.). Under 0.1% O₂ condition, MPA-II-2 (6 µL, 100 mM), NADPH (20 µL, 100 mL) and human NADPH-CYP reductase (5 µL, 10 µL or 40 µL, 18.8 mg/mL) were added to phosphate buffer saline containing 1% DMSO (v/v), and the mixture was incubated at 37 °C. The normoxic group (20% O₂) was treated with human NADPH-CYP reductase (10 µL, 18.8 mg/mL), with all other experimental parameters identical to the hypoxic group. Aliquots were collected at predetermined time points, and quenched with acetonitrile and stored at -80 °C. After the last aliquot was collected, all samples were centrifuged to remove proteins, followed by LC-MS/MS analysis.

The results (FIG. 12) showed that the release MMAE exhibited dual dependence on azoreductase concentration and oxygen concentration. Under hypoxic conditions (0.1% O₂), the release of MMAE increased as the azoreductase concentration increased, and after 24-hour incubation of the substrate with azoreductase (at 0.4 mg/mL, 0.1 mg/mL and 0.05 mg/mL), the release rates of MMAE were 100%, 74.3% and 27.49%, respectively. However, under normoxic conditions (20% O₂), after 24-hour incubation of the substrate with 0.1 mg/mL of azoreductase, only 14.99% of MMAE was released. The result indicated that the azoreductase-cleavable linker provided by the present disclosure can be cleaved to release cytotoxins under the combined action of hypoxic condition and azoreductase, whereas under the action of azoreductase alone, the release amount of the cytotoxins is very low (FIG. 12).

### Example 22: Effects of oxidants, reductants and salts on the stability of compound II-2

The present example evaluated the effects of oxidants, reductants and salts on the stability of compound II-2. Compound II-2 was reacted with N-acetylcysteine (NAC, 10 equivalents) to prepare a stock solution of NAC-II-2. The preparation of NAC-II-2 was carried out according to the method reported in the reference literature (Y. Wang, S. Fan, W. Zhong, X. Zhou, S. Li, int. J. Mol Sci.2017, 18, e 1860.). NAC-II-2 was incubated with Ary, Cys, NaCl, KCl, MgCl₂, CaCl₂, H₂O₂, NaClO, Arg, Glu, Ser, Vc, Glucose, and NADPH in a 20% O₂ environment for 24 h. Meanwhile, NAC-II-2 was incubated with azoreductase in a 0.1% O₂ environment for 24 h. All the samples, after the 24 h of incubation, were quenched with methanol, and then the release of MMAE in the samples were determined by LC-MS/MS. The results (FIG. 13) showed that NAC-II-2 ccould release MMAE only under hypoxic conditions via azoreductase, and exhibited excellent stability when co-incubation with other substances related to redox, such as Cys, NADPH, and NaClO.

### Example 23: Study on the intracellular metabolites of compoundIII-2

The present example evaluated the intracellular metabolite forms of compound III-2 under normoxic (20% O₂) and hypoxic (0.1% O₂) conditions. SKOV3 cells were inoculated in a 12-well plate (at about 5×10⁵ cells/well), then 100 nM of compound III-2 were added, and cells were co-incubated with compound III-2 for 6, 17 or 24 hours under an oxygen content of 20% or in 0.1% O₂. At predetermined time points, the cells were collected and washed three times with pre-cooled PBS. Methanol was added to the sample, and the mixtures were placed at -20 °C for 2 hours to achieve complete cell lysis, and then the samples were centrifuged at 13000 g for 20 minutes. 50 µL of the supernatant were collected from each sample, vacuum-dried, and re-dissolved in 100 µL of methanol, and the contents of MMAE and Cys-II-2 in the sample were quantified by LC-MS/MS. The results showed that SKOV3 cells were co-cultured with compound III-2 under hypoxic conditions, MMAE was detected in the SKOV3 cells, and the release of MMAE was time-dependent. The result directly showed that compound III-2 can be cleaved to release MMAE in tumor cells under hypoxic conditions (FIG. 14). More importantly, when SKOV3 cells were co-cultured with compound III-2 under normoxic conditions, the Cys-II-2 was the major metabolite and its release amount exhibited time dependency, while under hypoxic conditions, the amount of the Cys-II-2 in the cells was very low. The result showed that the Cys-II-2 released after the antibody degradation of compound III-2 could not be further cleaved by azoreductase under normoxic conditions (FIG. 14).

### Example 24: Study on the cytotoxicity of Cys-II-2

The present example evaluated the cytotoxicity of Cys-II-2 in BT474 and SKBR3 cells. Compound II-2 was reacted with L-cysteine (Cys, 10 equivalents) to prepare a stock solution of Cys-II-2. BT-474 and SKBR3 cells were inoculated in a 96-well plate and incubated in an incubator for 24 hours. Serially diluted MMAE and Cys-II-2 were separately added to the cell plates, and the cells were co-incubated with the drugs under an environment with an oxygen content 20% for 24 hours, then the drug-containing medium was replaced with fresh medium. SKBR3 cells were continuously cultured for 3 days, while BT474 cells were cultured for 5 days. The cell viability was detected using a CellTiter-Glo assay kit. The results (FIG. 15) showed that the cytotoxic activity of Cys-II-2 was more than ten folds to several hundred folds lower than that of MMAE.

### Example 25: Study on Tubulin Inhibitory Activity of Cys-II-2

The tubulin inhibition experiment was carried out by using a tubulin polymerization assay kit (Cat. No.BK 011P, Cytoskeleton, USA). 5 µL of serially diluted MMAE, Cys-II-2 or DMSO was added into each well of a 96-well plate, and incubated at 37 °C for 1 min, followed by the addition of 50 µL prepared tubulin reaction solution. The fluorescence signal at 420 nM was immediately monitored and recorded on a microplate reader. The results (FIG. 16) showed that the tubulin inhibitory activity of Cys-II-2 was reduced as compared to the MMAE in a concentration-dependent manner.

### Example 26: Study on the cell cycle arrest of compound III-2

The present example evaluated the cell cycle arrest effect of compound III-2 on SKOV3 cells. SKOV3 cells were inoculated into a 12-well plate (at a density of about 1×10⁵ cells/well) and incubated for 24 hours. Compound III-2 at different concentrations (of 0.3, 1 and 3 nM) was added to each well of the plate. For the hypoxic group, cells were cultured under 0.1% O₂ for 1 day, then transferred to a 20% O₂ environment for another 1 days of incubation. The normoxic group was continuously cultured under 20% O₂ for 2 days after drug administration. All cells in each well were collected into 1.5 mL EP tubes and fixed with pre-cooled 70% ethanol. Prior to detection, cells were washed once with cold PBS, stained with propidium iodide solution at 37 °C for 30 minutes in the dark, and then analyzed by using FACSCalibur. The cycle analysis results showed (FIG. 17) that compound III-2 induced G2/M phase arrest in tumor cells, with hypoxia selectivity. Under normoxic conditions, after treatment with compound III-2 at the concentrations of 0.3 nM, 1 nM and 3nM, the proportion of the cells in the G2/M phase was similar to that of the untreated control group (17.50%). In contrast, under hypoxic conditions, after the treatment with compound III-2 at the same concentrations, the proportion of the cells in the G2/M phase ranged from 71.12% to 87.22%.

### Example 27: Study on apoptosis Induced by compound III-2

The present example evaluated the effects of compound III-2 on apoptosis of SKOV3. SKOV3 cells were inoculated into a 12-well plate (at a density of about 1×10⁵ cells/well) and incubated for 24 hours. Compound III-2 at different concentrations (of 0.3 nM, 1 nM and 3 nM) was added to each well of the plate. For the hypoxic group, cells were cultured cultured under 0.1% O₂ for 1 day, then transferred to a 20% O₂ environment for another 2 days of incubation. The normoxic group was continuously cultured under 20% O₂ for 3 days after drug administration. All cells from each well were collected into 1.5 mL EP tubes, and washed once with pre-cooled PBS. The sample was stained with Annexin V-FITC and propidium iodide solution at room temperature for 20 minutes in the dark, and then analyzed by using FACSCalibur. The results (FIG. 18) showed that compound III-2 almost had no effect on cell apoptosis under normoxic conditions (20% O₂), and in contrast, compound III-2 induced apoptosis in more than a half of the cells under hypoxic conditions (0.1% O₂). The cell apoptosis results showed that compound III-2 has good hypoxic selectivity.

### Example 28: In vivo distribution of DyLight-680 dye-labeled compound III-2 in mice

The present example evaluated the in vivo distribution of compound III-2 in mice.

According to the manufacturer's instructions, DyLight 680 NHS ester dye (purchased from Thermo Fisher Scientific) was used to prepare DyLight 680-Herceptin and DyLight 680-III-2. Specifically, Herceptin or compound III-2 was mixed with a fluorescent dye (DyLight 680 NHS ester) and stirred and reacted for 3 hours in a NaHCO₃ buffer (pH = 8.3), to complete antibody labeling, and then the obtained DyLight 680-Herceptin and DyLight 680-III-2 were buffer-exchanged into a PBS buffer for subsequent use.

SKOV3 ovarian cancer tumor-bearing mice (purchased from Yicon (Beijing) BioMedical Technology Inc.) were administrated intravenously with 0.2 mL of PBS, DyLight 680-Herceptin, or DyLight 680-III-2 via tail vein injection. In vivo fluorescence imaging of the mice was performed at the predetermined time points (6 h, 24h, 30 h, 48 h, 72 h, 120 h, 216 h, 360 h) by using a Maestro in vivo fluorescence imaging system. The results (FIG. 19) showed that the DyLight 680-labeled DyLight 680-Herceptin (25 mg/kg) and the DyLight 680-III-2 (25 mg/kg) were were administered to the mice via tail vein injection, strong fluorescence signals were observed at a tumor site 6 hour after the administration, and the fluorescence signals at the tumor sites were clearly maintained for about 9 days, with the fluorescence signal completely disappearing on the 15^{th} day after the administration. From the variation curve of the fluorescence signal intensity, it was found that the curve of the DyLight 680-III-2 was almost consistent with that of the DyLight 680-Herceptin, indicating that compound III-2 had metabolic properties similar to Herceptin. In addition, the fluorescence intensity at the tumor sites was significantly higher than that at other normal tissues (FIG. 19). The above imaging results showed that compound III-2 has excellent tumor targeting property and a longer half-life in vivo.

### Example 29: Study on distribution and metabolism of Compound III-2

The present example evaluated distribution and metabolism of compound III-2. SKOV3 ovarian cancer tumor-bearing mice were randomly divided into 6 groups. After tail vein injection with a single dose of compound III-2 (25 mg/kg), the mice were euthanized at the predetermined time points of 6 h, 24h, 72 h, 96 h, 168 h, 192 h in order, and blood, heart, liver, spleen, lung, and tumor samples were collected. The blood and tissue samples were stored at -80 °C for further analysis. The results (FIG. 20, Table 5) showed that high concentrations of the prodrug Cys-II-2 were detected in blood and peripheral tissues 6 h after the administration of compound III-2. In addition, the results showed that Cys-II-2 was rapidly cleared in normal tissues, with a blood half-life of only 26 hours, which further reduced its toxic effects and side effects. At 96 h after the administration, Cys-II-2 was hardly detected in heart, liver, spleen, and lung. The peak concentration and area under the curve of the cytotoxin MMAE in the tumor were 293 times and 941 times those in peripheral blood, respectively, further proving that the excellent targeting capability of compound III-2. The area under the drug-time curve of MMAE in heart, liver, spleen and lung were 11%, 47%, 5% and 7% of area under the drug-time curve in tumors, respectively. The results showed that Cys-II-2 in peripheral tissues could not be metabolized to generate MMAE except in the liver, while Cys-II-2 in tumors could effectively release MMAE (Table 6).

**Table 5 Concentration of Cys-II-2 in each group**

| Time | Concentration of Cys-II-2 (ng/g or ng/mL) | | | | | |
|---|---|---|---|---|---|---|
| | Blood | Heart | Liver | Spleen | Lung | Tumour |
| 6 h | 821.71 | 312.52 | 4627.35 | 7641.37 | 1354.37 | 3810.89 |
| 96 h | 41.47 | BQL | BQL | BQL | BQL | 245.68 |

| | | | | | | |
|---|---|---|---|---|---|---|
| Note: BQL refers to "below quantification limit". | | | | | | |

**Table 6 Concentration of MMAE in each tissue**

| Time | Concentration of MMAE (ng/g or ng/mL) | | | | | |
|---|---|---|---|---|---|---|
| | Blood | Heart | Liver | Spleen | Lung | Tumour |
| 6 h | 1.54 | 112.72 | 348.31 | 21.29 | 22.96 | 54.24 |
| 24 h | 0.51 | 71.97 | 269.21 | 19.80 | 12.85 | 179.62 |
| 72 h | 0.23 | 32.46 | 115.44 | 20.24 | 19.79 | 292.45 |
| 96 h | 0.12 | 17.99 | 101.72 | 13.60 | 43.95 | 451.68 |
| 168 h | 0.10 | 2.70 | 26.86 | 3.82 | 1.93 | 198.90 |
| 192 h | BQL | 0.80 | 10.07 | 1.40 | 1.04 | 125.44 |

| | | | | | | |
|---|---|---|---|---|---|---|
| Note: BQL refers to "below quantification limit". | | | | | | |

### Example 30: Pharmacodynamic study of compound III-2 in NCI-N87 tumor-bearing mice

The present example evaluated the antitumor efficacy of compound III-2 in NCI-N87 tumor-bearing mice (purchased from Hangzhou Qingda Kerui Biotechnology Co. Ltd.). When the tumor volume reached about 100 mm³, the NCI-N87 tumor-bearing mice were randomly divided into 4 groups: PBS group, Herceptin treatment group, compound III-2 treatment group and VC-ADC treatment group, which were administered once a week by tail vein injection, with a total of 4 doses. The tumor size was measured with calipers twice weekly, and the tumor volume was calculated according to the formula 1/2 × length × width × width (mm). If the tumor volume of any individual mouse exceeded 2000 mm³, all mice were euthanized. The results (FIG. 21) showed that in the NCI-N87 tumor xenograft model, compound III-2 can significantly inhibit tumor growth at the administration dose of 5 mg/kg as compared with the control group, with a tumor inhibition rate of 90.97%. The result indicated that compound III-2 exhibits antitumor efficacy nearly equivalent to that of conventional VC-ADC. More importantly, complete tumor regression is observed in all mice in the administration group at a dose of 10 mg/kg, indicating the excellent antitumor therapeutic potential of compound III-2.

### Example 31: Pharmacodynamic study of compound III-2 in JIMT-1 tumor-bearing mice

The present example evaluated the antitumor efficacy of compound III-2 in JIMT-1 tumor-bearing mice (purchased from Hangzhou Qingda Kerui Biotechnology Co. Ltd.). In order to evaluate the antitumor activity of compound III-2 in JIMT-1 tumor-bearing mice with low expression of HER2 antigen, when the tumor volume reached about 150 mm³, the mice were randomly divided into two groups: PBS group and compound III-2 treatment group, which were administered once a week by tail vein injection, with a total of 4 doses. The tumor size was measured with calipers twice per week. The results (FIG. 22) showed that in the JIMT-1 tumor xenograft model, compound III-2 still exhibited antitumor effects at a dose of 12 mg/kg, and although complete tumor regression was not achieved, the tumor growth inhibition rate reached 62.36%.

### Example 32: Pharmacodynamic study on the combination administration of compound III-2 with Herceptin, Bevacizumab, Sunitinib or Docetaxel in NCI-N87 tumor-bearing mice

This example evaluates the antitumor efficacy of the combination administration of compound III-2 with Herceptin, bevacizumab, sunitinib or docetaxel in the NCI-N87 tumor-bearing mice. The mice were randomly divided into 7 groups (PBS group, compound III-2 monotherapy group (3 mg/kg), sunitinib monotherapy group (54 mg/kg), combination group of compound III-2 (3 mg/kg) and Herceptin (12 mg/kg), combination group of compound III-2 (3 mg/kg) and bevacizumab (5 mg/kg), combination group of compound III-2 (3 mg/kg) and sunitinib (54 mg/kg), combination group of compound III-2 (3 mg/kg) and docetaxel (8 mg/kg)). When the tumor volume reached about 100 mm³, PBS, Herceptin, compound III-2 and docetaxel was administered once per week by tail vein injection, in a total of 3 doses. Docetaxel was administered 6 hours in advance. Sunitinib was administered by intragastric administration once daily, for 3 consecutive weeks. The results (FIG. 23) showed that the combination group of compound III-2 and sunitinib exhibited superior antitumor efficacy (p <0.01), which was better than compound III-2 monotherapy and other combination group. In the combination groups, treatment with only 3mg/kg of compound III-2 could achieve nearly complete tumor regression, and the tumor growth inhibition rate was 98.93%. The above results showed that the excellent tumor growth inhibitory effect of the combination therapy may achieved due to the angiogenesis inhibitor sunitinib that can expand the hypoxic regions of the tumor tissues, and aggravate the hypoxic degree of the tumor tissues, thereby resulting in the further improvement of the therapeutic efficacy of compound of III-2.

### Example 33: Study on the maximum tolerated dose of compound III-2

The present example evaluated the tolerance and adverse reactions of healthy female CD-1 mice (purchased from Hangzhou Qingda Kerui Biotechnology Co. Ltd.) to compound III-2. Female CD-1 mice aged 7-9 weeks were acclimated to the experimental environment for at least 3 days prior to administration, with each dose group consisting of 3 mice. The healthy female CD-1 mice were administrated with 120, 160, 200, and 240 mg/kg of compound III-2 via single tail vein injection. Meanwhile, the marketed drug T-DM1 and the conventional ADC drug VC-ADC were used for a control study. After administration via tail vein injection, the mice were monitored daily for weight change and behaviors. The results (FIG. 24) showed that the maximum tolerated dose (MTD) of compound III-2 was higher than 200 mg/kg, while the mice in the VC-ADC group and the T-DM1 group experienced continuous weight loss (>20%) and severe adverse reactions after administration, and for example, all mice in the VC-ADC group died after receipt of treatment at a dose of 120 mg/kg.

### Example 34: Hematological and histopathological studies of compound III-2 in BALB/C mice

The present example evaluated the hematological and histopathological profiles of compoundIII-2 in BALB/C mice (purchased from Hangzhou Qingda Kerui Biotechnology Co. Ltd.). Female BALB/C mice (n = 3 /group) were administrated via tail vein injection with compound III-2 (60 mg/kg), VC-ADC (60 mg/kg) or PBS. After administration, the mice were monitored for weight change every 3 days. All mice were euthanized on day 7 for complete blood hematological and histopathological analyses. The results (FIG. 25) showed that there was no significant differences in serum biochemical indicators, routine blood parameters and histopathological markers between the compound III-2 treatment group and the control group, while the changes in serum biochemical indicators in the VC-ADC treatment group reflected liver side effects induced by VC-ADC, manifested as increased levels of serum alanine aminotransferase (ALT), aspartate aminotransferase (AST) and creatine kinase (CK). The side effects of VC-ADC were also reflected in the hematological changes, including increased counts in lymphocytes (LYMPH), monocytes (MONO), and neutrophils (NEUT).

### Example 35: Antibacterial activity study of compound III-16 against Staphylococcus aureus

The present example evaluated the antibacterial efficacy of compound III-16 against *Staphylococcus aureus* in macrophages. After 37.5 µg/mL of Compound III-16 and 150 µg/mL of antibacterial antibody (the heavy and light chain sequences of which are as set forth in SEQ ID NOS:3 and 4, respectively) were added to well plates, respectively, and co-incubated with *Staphylococcus aureus-*infected macrophages for 24 hours, and then the colonies were counted and imaged. The results (FIG. 26) showed that compound III-16 had excellent anti-*Staphylococcus aureus* activity.

### Example 36: Antibacterial activity study of compound III-16 against free Staphylococcus aureus

The present example evaluated the antibacterial efficacy of compound III-16 against free *Staphylococcus aureus*. 5 µL to 10 µL of a *Staphylococcus aureus* USA300 stock suspension stored at - 80 °C was taken and streaked on Brain Heart Infusion solid culture plates and incubated overnight at 37 °C in a biochemical incubator. A single USA300 colony was picked from the solid culture plates, inoculated into an appropriate volume of Brain Heart Infusion medium, and activated for 16 h to 18 h in a bacteria shaking incubator, then the bacterial suspension was centrifuged for 5 min at a rotating speed of 4000 rpm to collect the bacteria. The bacteria were resuspended in 1×PBS and the bacterial density was diluted to about 1×10⁻³/mL. The diluted bacterial suspension was added to 96-well plates at 100 µL per well. Serially diluted compound III-16 and rifalogue were added to the bacterial suspension in the 96-well plates, and the bacteria was cultured in a biochemical incubator at 37 °C for 16 h to 18h, then the OD₆₀₀ₙₘ was detected using a microplate reader. The results (FIG. 27) showed that the antibacterial activity of compound III-16 against free USA300 (with an MIC₉₀ of about 15.7 nM) was comparable to that of rifalogue (with MIC₉₀ of about 1.4 nM). Without being limited by theories, it is speculated that the activity of compound III-16 against free bacteria may be stemed from the cleavage of the linker in compound III-16 by azoreductase extracellularly secreted by the bacteria, which releases rifalogue to exert antibacterial effect.

### Example 37: Pharmacodynamic study of compoundIII-16 in a sepsis model

The present example evaluated the therapeutic efficacy of compound III-16 in a mouse sepsis model. 5 to 10 µ L of *Staphylococcus aureus* USA300 stock suspension stored at -80 °C was taken and streaked on Brain Heart Infusion solid culture plates and incubated overnight at 37 °C in a biochemical incubator. A single USA300 colony was picked from the solid culture plates, inoculated into an appropriate volume of Brain Heart Infusion medium, and activated at 37 °C for 16 h to 18 h in a bacteria shaking incubator, then the bacterial suspension was centrifuged for 5 min at a rotating speed of 4000 rpm to collect the bacteria. The bacteria were resuspended in 1×PBS and diluted to a density of OD₆₀₀ₙₘ = 5/mL. The Mice were injected with 100 µ L of the diluted USA300 via tail vein. At 24 hours after infection, the infected mice were randomly divided into groups and administrated with corresponding drugs. On 4 days after infection, all mice were euthanized, and major tissues including kidneys and hearts were isolated. After preparation of tissue homogenates were prepared, tissue homogenates of kidneys and hearts were plated for bacterial colony counting to evaluate the in vivo antibacterial efficacy of compound III-16 in mice. The results (FIG. 28) showed that the high-dose compound III-16 (60 mg/kg) exhibited excellent antibacterial activity in the kidney, and the colony count in the kidney decreased to the detection limit. The high-dose group of compound III-16, as compared to the control group, the the combination group of antibacterial antibody BD37 and rifalogue (60 mg/kg the BD37 combined with an equivalent rifalogue dose matching 60 mg/kg compound III-16), and the vancomycin group (110 mg/kg), exhibited statistical significances in renal colony counts (One-Way ANOVA: **** represents P < 0.0001, *** represents P <0.005, ** represents P <0.05).

The above experimental results showed that the ADCs (for example, the ADC as represented by III-2) provided by the present disclosure can be rapidly recognized and cleaved by azoreductase to efficiently release MMAE, thereby exhibiting antitumor effects both in vitro and in vivo. In addition, the ADCs (for example, the ADC as represented by III-16) provided by the present disclosure exhibited excellent antibacterial effects.

Finally, it should be illustrated that the above examples are intended only to illustrate the technical solution of the present disclosure but not to limit it; although the present disclosure has been described in detail with reference to preferred examples, one skill in the art should understand that modifications to the specific embodiments of the present disclosure or equivalent substitutions to a part of the technical features may be made, and each of them, without departing from the spirit of the technical solution of the present disclosure, should be covered in the scope of the technical solution as claimed in the present disclosure.

## Claims

1. A compound represented by Formula I, a geometric isomer, an optical isomer, a salt, a hydrate, a solvate, or a polymorph thereof, 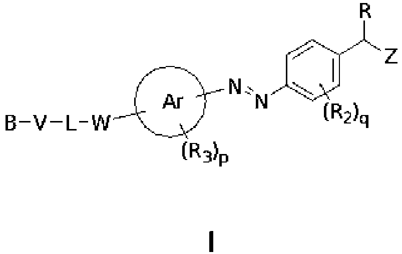
wherein 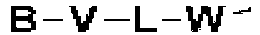 represents a linker, for conjugating an azobenzene group to a targeting compound;
Ar is a 5- or 6-membered aryl or heteroaryl;
R₂ and R₃ each independently are hydrogen, C₁₋₆ alkyl, fluoro, chloro, bromo, iodo, hydroxy, nitro, or cyano:
p is 0, 1, 2, or 3;
q is 0, 1, 2 or 3;
R is H, C₁₋₄ alkyl, 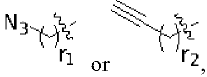 wherein r₁ and r₂ each independently are an integer from 1 to 12;
Z is hydroxy, fluoro, chloro, bromo, iodo or 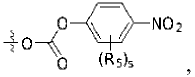 wherein s is 0, 1, 2, 3 or 4, and R₅ is hydrogen, fluoro, chloro, bromo, iodo, C₁₋₄ alkyl, nitro or C₁₋₄ alkoxy;
preferably, in 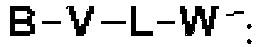
B is 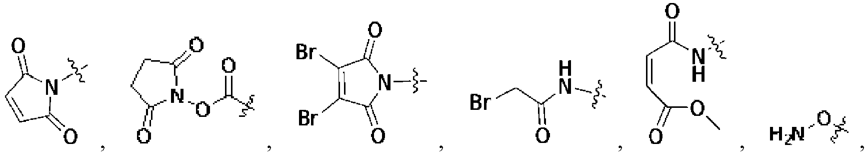 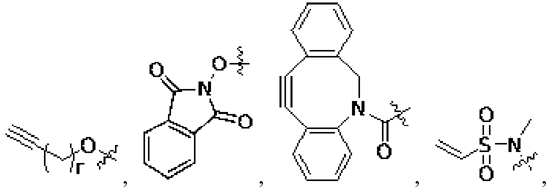 or H, wherein r is an integer from 1 to 4; or
B is selected from the group consisting of 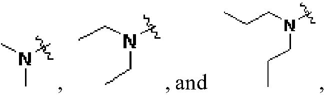 provided that V, L, W1, W2, and W3 are simultaneously absent;
V is 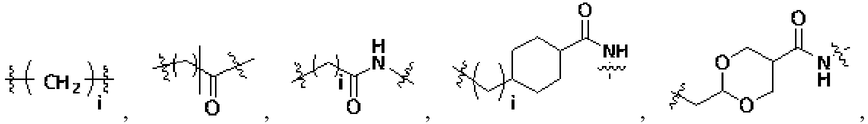 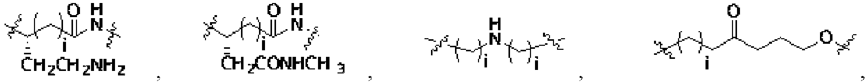 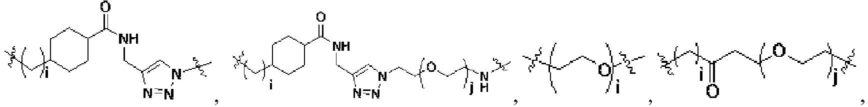 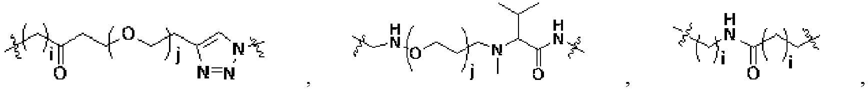 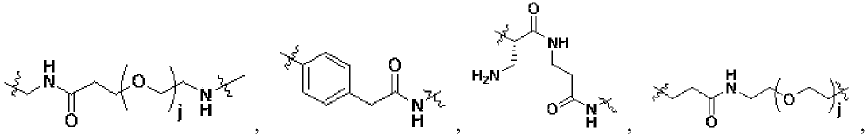 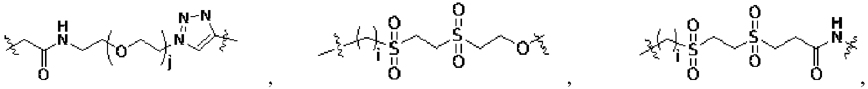 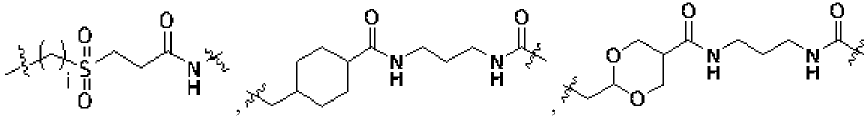 or absent, wherein each i independently is an integer from 0 to 6, and each j independently is an integer from 0 to 8;
L is -(CH₂CH₂O)ₖ-(CH₂)₁-, -CHR₁-, -(CH₂)ₘ -, 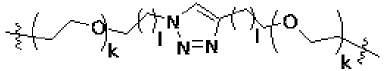 or absent, wherein R₁ is -(CH₂)ₙ-NHC(O)-(CH₂CH₂O)ₖ-CH₃, each k independently is an integer from 0 to 12, each 1 independently is an integer from 0 to 12, n is an integer from 0 to 12, and m is an integer from 0 to 30;
W is -W1-W2-W3-, wherein W1 is connected to L, W3 is connected to Ar, and W1 is selected from the group consisting of -O-, -S-, -NR₁₁-, -CONH-, -NHCO-, -S(O₂)NH-, and -NHS(O₂)-, or W1 is absent; W2 is selected from the group consisting of -(CH₂)ₒ-, and -[CH(R₁₁)CH(R₁₂)]ₒ-, or W2 is absent; W3 is selected from the group consisting of -O-, -S-, -NR₁₁-, -CONH-, -NHCO-, -S(O₂)NH-, and -NHS(O₂)-, or W3 is absent, wherein o is an integer from 0 to 6, each R₁₁ independently is selected from the group consisting of hydrogen and C₁₋₆ alkyl, and R₁₂ is selected from the group consisting of hydrogen and C₁₋₆ alkyl.

2. The compound, a geometric isomer, an optical isomer, a salt, a hydrate, a solvate, or a polymorph thereof according to claim 1, wherein
W is selected from the group consisting of -NH-CH₂-C(O)-NH-, -NH-CH₂-CH₂-C(O)-NH-, -C(O)-NH-, -NH-C(O)-, -C(O)-NH-CH₂-C(O)-NH-, -C(O)-NH-CH₂-CH₂-C(O)-NH-, -C(O)-NH-, -NH-C(O)-, -NH-CH(R₁₀)-C(O)-NH-, -C(O)-NH-(CH₂)ₒ-NH-C(O)-, -C(O)-NH-[CH(R₁₁)CH(R₁₂)]ₒ-NH-C(O)-, -NH-(CH₂)ₒ-NH-C(O)-, and -NH-[CH(R₁₁)CH(R₁₂)]ₒ-NH-C(O)-, wherein R₁₀ is H, methyl or ethyl, and o, R₁₁ and R₁₂ are as defined in claim 1.

3. The compound, a geometric isomer, an optical isomer, a salt, a hydrate, a solvate, or a polymorph thereof according to claim 1 or 2, wherein:
B is 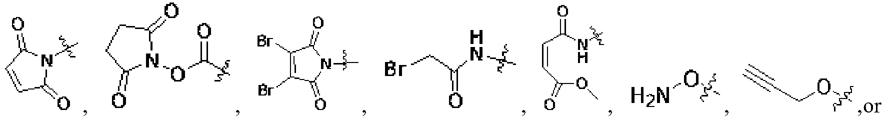 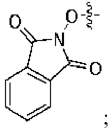
preferably, B is 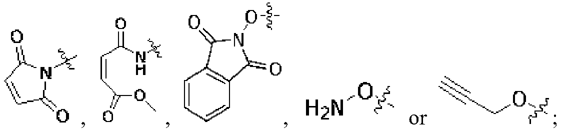 preferably, B is 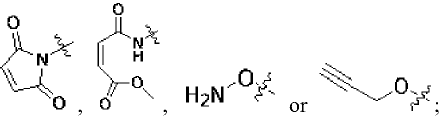
preferably, B is

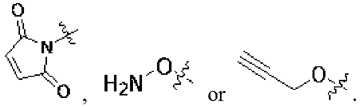

4. The compound, a geometric isomer, an optical isomer, a salt, a hydrate, a solvate, or a polymorph thereof according to any one of claims 1 to 3, **characterized in** any one or more of the following i) to iii):
i) V is 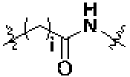 or absent, wherein i is as defined in claim 1;
ii) L is -(CH₂CH₂O)ₖ-(CH₂)ₗ-, -CHR₁- or -(CH₂)ₘ-, wherein k, l, R₁, and m are as defined in claim 1;
iii) W is -C(O)-NH-(CH₂)ₒ-NH-C(O)- or -C(O)-NH-[CH(R₁₁)CH(R₁₂)]ₒ-NH-C(O)-, and o, R₁₁ and R₁₂ are as defined in claim 1;
preferably, -V-L-W- is -(CH₂)ₘ-C(O)-NH-(CH₂)ₒ-NH-C(O)-, -(CH₂)ᵢ-C(O)-NH-(CH₂CH₂O)ₖ-(CH₂)ₗ-C(O)-NH-(CH₂)ₒ-NH-C(O)-, -(CH₂)ᵢ-C(O)-NH-CHR₁-C(O)-NH-(CH₂)ₒ-NH-C(O)-,or -(CH₂CH₂O)ₖ-(CH₂)ₗ-C(O)-NH-(CH₂)ₒ-NH-C(O)-, wherein R₁ is -(CH₂)ₙ-NHC(O)-(CH₂CH₂O)ₖ-CH₃, and m, o, i, k, l, and n are as defined in claim 1;
preferably, each i independently is 1, 2, 3 or 4; each k independently is 1, 2, 3, 4, 5, 6, 7, 8, 9, 10, 11 or 12; each m independently is 1, 2, 3 or 4; each o independently is 1, 2, 3 or 4; each l independently is 1, 2, 3 or 4; n is 1, 2, 3, 4, 5, 6, 7 or 8;
preferably, each i independently is 1, 2 or 3; each k independently is 1, 2, 3, 4, 5, 6, 7, 8, 9 or 10; each m independently is 1, 2 or 3; each o independently is 1, 2 or 3; each l independently is 1, 2 or 3; n is 1, 2, 3, 4 or 5;
preferably, -V-L-W- is -(CH₂)₂-C(O)-NH-(CH₂)₂-NH-C(O)-, -(CH₂)₂-C(O)NH-(CH₂CH₂O)₂-CH₂-CH₂-C(O)-NH-(CH₂)₂-NH-C(O)-, -(CH₂)₂-C(O)NH-(CH₂CH₂O)₄-CH₂-CH₂-C(O)-NH-(CH₂)₂-NH-C(O)-, -(CH₂)₂-C(O)NH-(CH₂CH₂O)₆-CH₂-CH₂-C(O)-NH-(CH₂)₂-NH-C(O)-, -(CH₂)₂-C(O)NH-(CH₂CH₂O)₈-CH₂-CH₂-C(O)-NH-(CH₂)₂-NH-C(O)-, -(CH₂CH₂O)₃-(CH₂)₂-C(O)-NH-(CH₂)₂-NH-C(O)-, -(CH₂)₂-C(O)NH-CH[(CH₂)₄ₛ-NHC(O)-(CH₂CH₂O)₃-CH₃]-C(O)-NH-(CH₂)₂-NH-C(O)-, -(CH₂CH₂O)₇-(CH₂)₂-C(O)-NH-(CH₂)₂-NH-C(O)-.

5. The compound, a geometric isomer, an optical isomer, a salt, a hydrate, a solvate, or a polymorph thereof according to any one of claims 1 to 4, **characterized in** any one or more of the following i) to v):
i) Ar is phenyl, pyridyl, pyrimidinyl, furanyl, imidazolyl, pyrrolyl, thiazolyl, pyrazolyl or thienyl;
preferably, Ar is phenyl, furanyl, imidazolyl or thienyl;
preferably, Ar is phenyl;
ii) R is H or C₁₋₄ alkyl;
preferably, R is H, methyl, ethyl, n-propyl or n-butyl, preferably is H, methyl, ethyl or n-propyl, further preferably is H, methyl or ethyl, and more further preferably is H;
iii) R₂ and R₃ each independently are hydrogen, C₁₋₄ alkyl, fluoro, chloro, bromo, iodo, hydroxyl, nitro or cyano;
preferably, R₂ and R₃ each independently are hydrogen, methyl, ethyl, n-propyl, iso-propyl, n-butyl, iso-butyl, sec-butyl, or tert-butyl; preferably, R₂ and R₃ each independently are fluoro, chloro, bromo, iodo, hydroxyl, nitro or cyano; preferably, R₂ and R₃ each independently are hydrogen;
iv) q is 0, 1 or 2, preferably is 0 or 1;
p is 0, 1 or 2, preferably is 0 or 1;
v) Z is hydroxyl, fluoro, chloro, bromo, iodo or 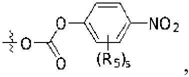 wherein s is 0, 1, 2, 3 or 4; R₅ is hydrogen, fluoro, chloro, bromo, iodo, methyl, ethyl, n-propyl, iso-propyl, n-butyl, iso-butyl, sec-butyl, tert-butyl, nitro, methoxy, ethoxy, n-propoxy, iso-propoxy, n-butoxy, iso-butoxy, sec-butoxy or tert-butoxy;
preferably, Z is chloro, OH or 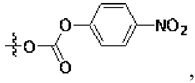 preferably is OH or 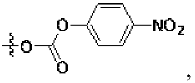 and further preferably is 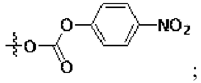
preferably, the compound has a structure represented by Formula I-1, 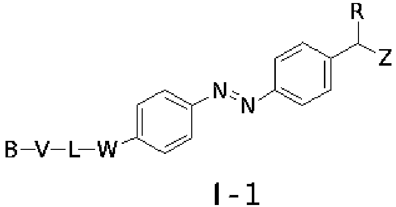
wherein B, V, L, W, R, and Z are as defined in claims 1 to 5.

6. The compound, a geometric isomer, an optical isomer, a salt, a hydrate, a solvate, or a polymorph thereof according to claim 1, wherein
B is selected from the group consisting of 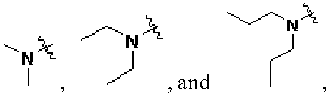 and V, L, W1, W2, and W3 each are absent;
R is 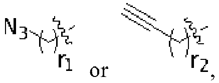 wherein r₁ and r₂ are as defined in claim 1;
preferably, r₁ and r₂ each independently are 1, 2, 3, 4, 5, 6, 7, 8, 9, 10, 11 or 12;
preferably, r₁ and r₂ each independently are 1, 2, 3, 4, 5, 6, 7, 8, 9 or 10;
preferably, r₁ and r₂ each independently are 1, 2, 3, 4, 5, 6, 7 or 8;
preferably, r₁ and r₂ each independently are 1, 2, 3, 4, 5 or 6;
preferably, r₁ and r₂ each independently are 1, 2, 3 or 4.

7. The compound, a geometric isomer, an optical isomer, a salt, a hydrate, a solvate, or a polymorph thereof according to claim 1, wherein the compound is selected from the group consisting of: 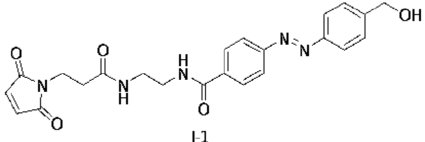 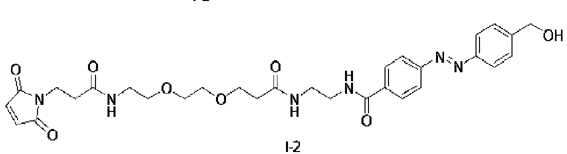 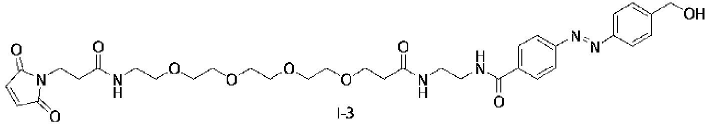 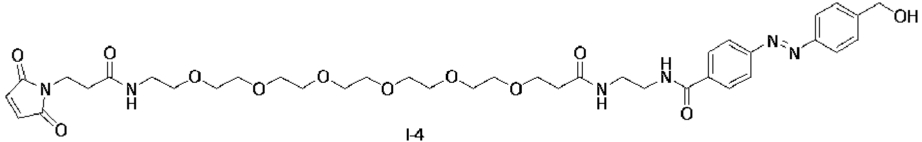 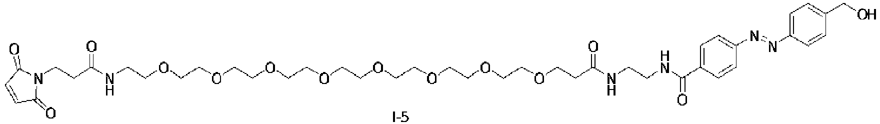 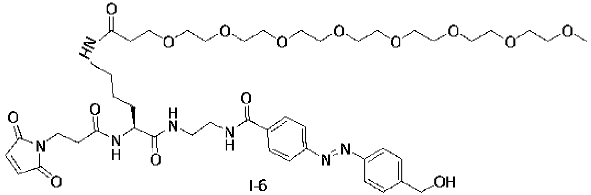 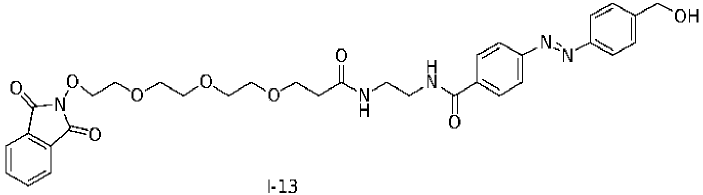 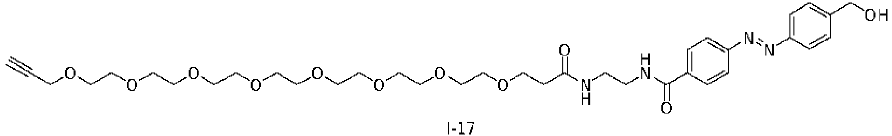 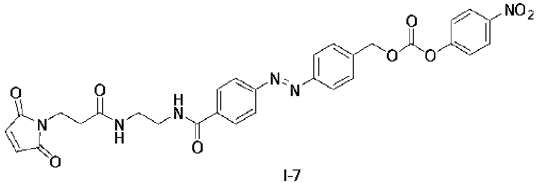 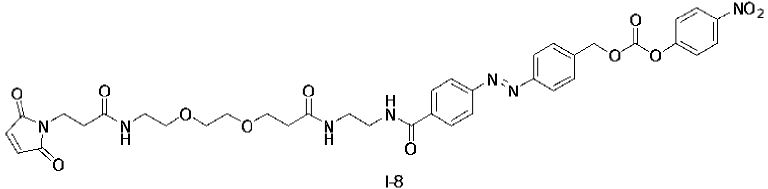 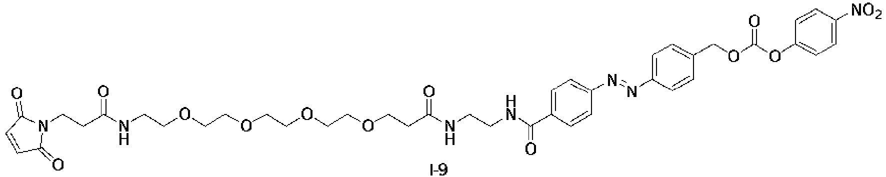 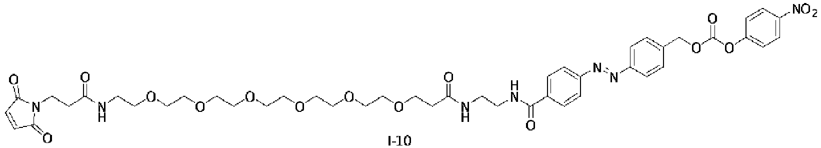 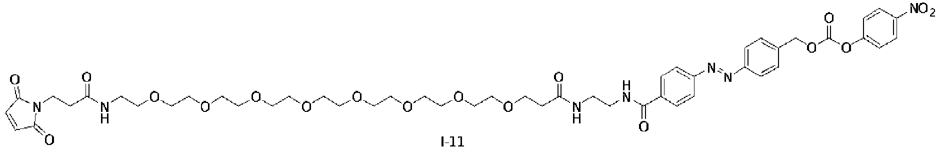 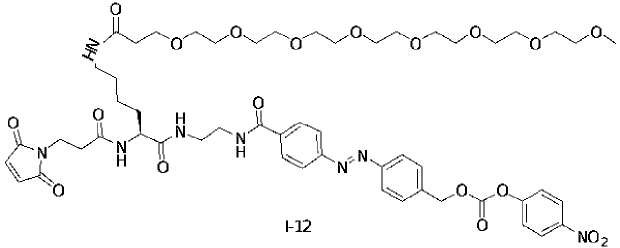 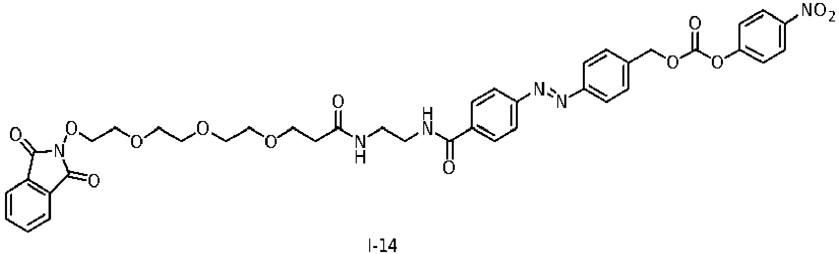 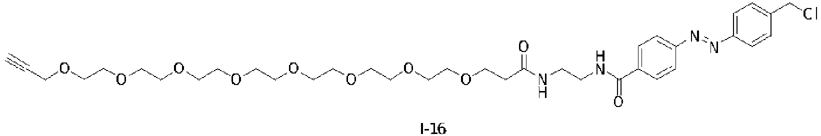 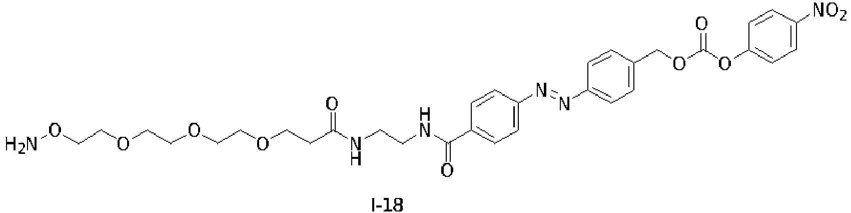 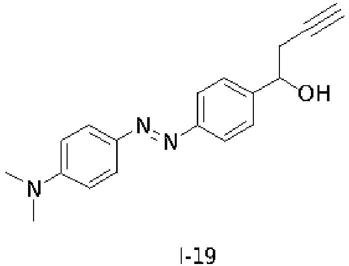 and

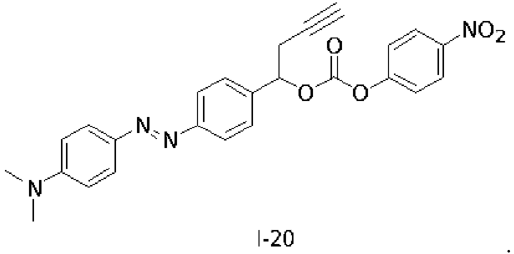

8. Use of the compound, a geometric isomer, an optical isomer, a salt, a hydrate, a solvate, or a polymorph thereof according to any one of claims 1 to 7 in the manufacture of an antibody-drug conjugate.

9. A compound represented by Formula II, a geometric isomer, an optical isomer, a salt, a hydrate, a solvate, or a polymorph thereof, 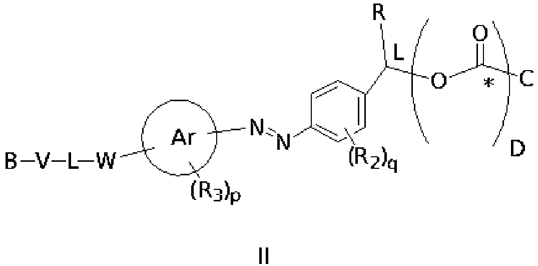
wherein: 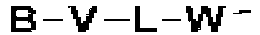 represents a linker, for conjugating an azobenzene group to a targeting compound,
Ar is a 5- or 6-membered aryl or heteroaryl;
R₂ and R₃ each independently are hydrogen, C₁₋₆ alkyl, fluoro, chloro, bromo, iodo, hydroxyl, nitro or cyano;
p is 0, 1, 2, or 3;
q is 0, 1, 2 or 3;
R is H, C₁₋₄ alkyl, 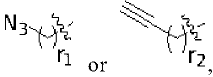 wherein r₁ and r₂ each independently are an integer from 1 to 12;
D is 0 or 1;
C is an active compound, selected from the group consisting of a drug, a cytotoxin, a detection reagent, a diagnostic reagent, and a targeting carrier; preferably, C is an antitumor drug, an antiviral drug, an antibiotic, an antibacterial drug or an immunomodulatory drug; further preferably, C is an antitumor drug, such as a small molecule, a peptide or a nucleotide including a cytotoxin, a tubulin inhibitor, a DNA alkylating agent, a DNA intercalating agent, an RNA polymerase inhibitor, a kinase inhibitor, a topoisomerase inhibitor, a kinesin spindle protein inhibitor, and an antimetabolite drug, an immunomodulatory drug such as a Toll-like receptor agonist (e.g., a TLR7 agonist) or a STING agonist, or an antibacterial drug such as rifampicin and an analog thereof (e.g., rifalogue, KRM-1657);
C is conjugated to the carbonyl group (i.e. the position *) or the C atom at the position L via the N atom or the O atom in the active compound molecule,
preferably, in 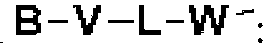
B is 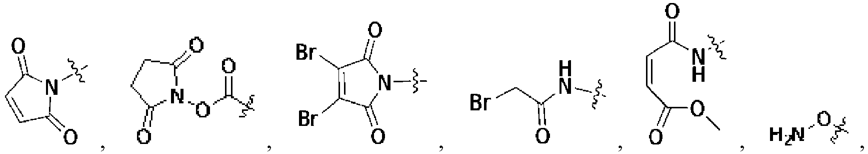 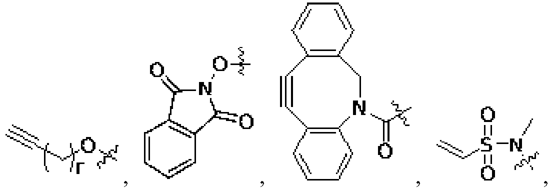 or H, wherein r is 1-4; or
B is selected from the group consisting of 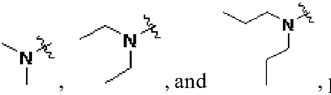 provided that V, L, W1, W2, and W3 are simultaneously absent;
V is 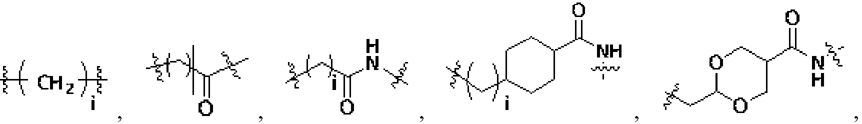 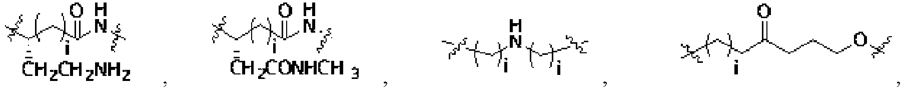 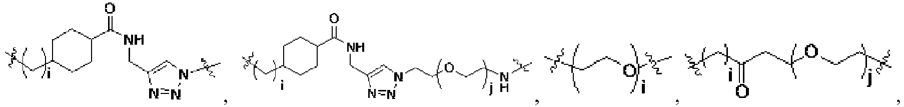 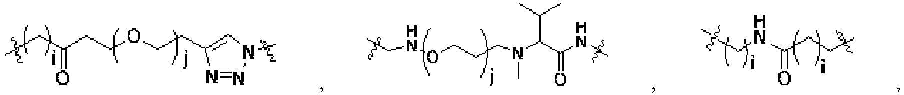 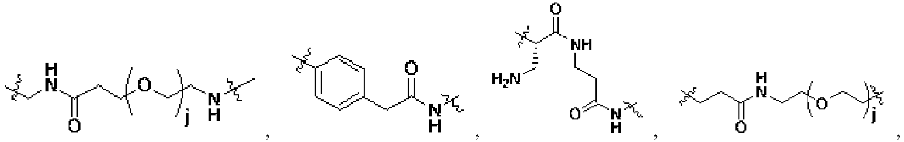 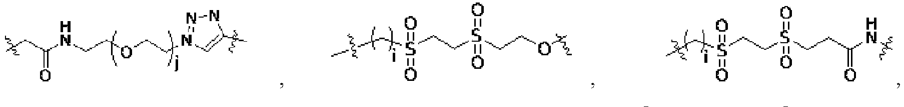 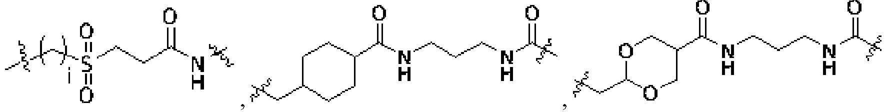 or absent, wherein each i independently is an integer from 0 to 6, and each j independently is an integer from 0 to 8;
L is -(CH₂CH₂O)ₖ-(CH₂)ₗ-, -CHRₗ-, -(CH₂)ₘ -, 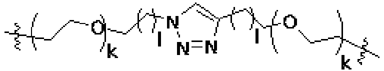 or absent, wherein R₁ is -(CH₂)ₙ-NHC(O)-(CH₂CH₂O)ₖ-CH₃, each k independently is an integer from 0 to 12, each l independently is an integer from 0 to 12, n is an integer from 0 to 12, and m is an integer from 0 to 30;
W is -W1-W2-W3-, wherein W1 is connected to L, W3 is connected to Ar, and W1 is selected from the group consisting of -O-, -S-, -NR₁₁-, -CONH-, -NHCO-, -S(O₂)NH-, and -NHS(O₂)-, or W1 is absent; W2 is selected from the group consisting of -(CH₂)ₒ-, and -[CH(R₁₁)CH(R₁₂)]ₒ-, or W2 is absent; W3 is selected from the group consisting of -O-, -S-, -NR₁₁-, -CONH-, -NHCO-, -S(O₂)NH-, and -NHS(O₂)-, or W3 is absent; wherein o is an integer from 0 to 6, each R₁₁ independently is selected from the group consisting of hydrogen and C₁₋₆ alkyl, and R₁₂ is selected from the group consisting of hydrogen and C₁₋₆ alkyl.

10. The compound, a geometric isomer, an optical isomer, a salt, a hydrate, a solvate, or a polymorph thereof according to claim 9, wherein
W is selected from the group consisting of -NH-CH₂-C(O)-NH-, -NH-CH₂-CH₂-C(O)-NH-, -C(O)-NH-, -NH-C(O)-, -C(O)-NH-CH₂-C(O)-NH-, -C(O)-NH-CH₂-CH₂-C(O)-NH-, -C(O)-NH-, -NH-C(O)-, -NH-CH(R₁₀)-C(O)-NH-, -C(O)-NH-(CH₂)ₒ-NH-C(O)-, -C(O)-NH-[CH(R₁₁)CH(R₁₂)]ₒ-NH-C(O)-, -NH-(CH₂)ₒ-NH-C(O)-, and -NH-[CH(R₁₁)CH(R₁₂)]ₒ-NH-C(O)-, wherein R₁₀ is H, methyl or ethyl, and o, R₁₁ and R₁₂ are as defined in claim 9.

11. The compound, a geometric isomer, an optical isomer, a salt, a hydrate, a solvate, or a polymorph thereof according to claim 9 or 10, wherein
B is 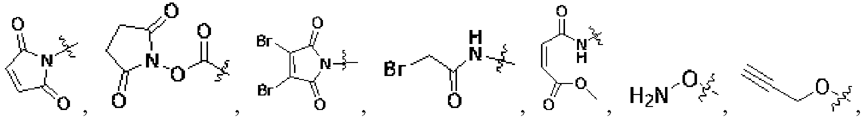 or 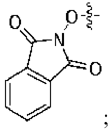
preferably, B is 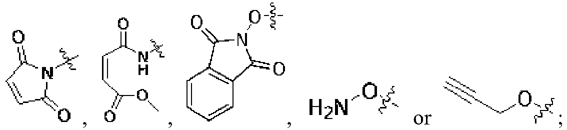 preferably, B is 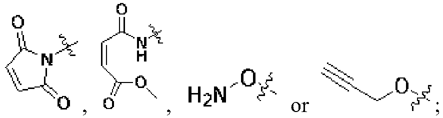
preferably, B is

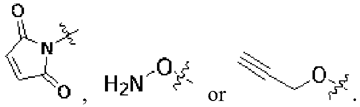

12. The compound, a geometric isomer, an optical isomer, a salt, a hydrate, a solvate, or a polymorph thereof according to any one of claims 9 to 11, **characterized in** any one or more of the following i) to iii):
i) V is 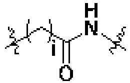 or absent, wherein i is as defined in claim 9;
ii) L is -(CH₂CH₂O)ₖ-(CH₂)ₗ-, -CHR₁- or -(CH₂)ₘ-, wherein k, l, R₁, and m are as defined in claim 9;
iii) W is -C(O)-NH-(CH₂)ₒ-NH-C(O)- or -C(O)-NH-[CH(R₁₁)CH(R₁₂)]ₒ-NH-C(O)-, and o, R₁₁ and R₁₂ are as defined in claim 9;
preferably, -V-L-W- is -(CH₂)ₘ-C(O)-NH-(CH₂)ₒ-NH-C(O)-, -(CH₂)ᵢ-C(O)-NH-(CH₂CH₂O)ₖ-(CH₂)ₗ-C(O)-NH-(CH₂)ₒ-NH-C(O)-, -(CH₂)ᵢ-C(O)-NH-CHR₁-C(O)-NH-(CH₂)ₒ-NH-C(O)-, or -(CH₂CH₂O)ₖ-(CH₂)ₗ-C(O)-NH-(CH₂)ₒ-NH-C(O)-, wherein R₁ is -(CH₂)ₙ-NHC(O)-(CH₂CH₂O)ₖ-CH₃, and m, o, i, k, l, and n are as defined in claim 9;
preferably, each i independently is 1, 2, 3 or 4; each k independently is 1, 2, 3, 4, 5, 6, 7, 8, 9, 10, 11 or 12; each m independently is 1, 2, 3 or 4; each o independently is 1, 2, 3 or 4; each l independently is 1, 2, 3 or 4; n is 1, 2, 3, 4, 5, 6, 7 or 8;
preferably, each i independently is 1, 2 or 3; each k independently is 1, 2, 3, 4, 5, 6, 7, 8, 9 or 10; each m independently is 1, 2 or 3; each o independently is 1, 2 or 3; l each independently is 1, 2 or 3; n is 1, 2, 3, 4 or 5;
preferably, -V-L-W- is -(CH₂)₂-C(O)-NH-(CH₂)₂-NH-C(O)-, -(CH₂)₂-C(O)NH-(CH₂CH₂O)₂-CH₂-CH₂-C(O)-NH-(CH₂)₂-NH-C(O)-, -(CH₂)₂-C(O)NH-(CH₂CH₂O)₄-CH₂-CH₂-C(O)-NH-(CH₂)₂-NH-C(O)-, -(CH₂)₂-C(O)NH-(CH₂CH₂O)₆-CH₂-CH₂-C(O)-NH-(CH₂)₂-NH-C(O)-, -(CH₂)₂-C(O)NH-(CH₂CH₂O)₈-CH₂-CH₂-C(O)-NH-(CH₂)₂-NH-C(O)-, -(CH₂CH₂O)₃-(CH₂)₂-C(O)-NH-(CH₂)₂-NH-C(O)-, -(CH₂)₂-C(O)NH-CH[(CH₂)₄-NHC(O)-(CH₂CH₂O)₈-CH₃]-C(O)-NH-(CH₂)₂-NH-C(O)-, or -(CH₂CH₂O)₇-(CH₂)₂-C(O)-NH-(CH₂)₂-NH-C(O)-.

13. The compound, a geometric isomer, an optical isomer, a salt, a hydrate, a solvate, or a polymorph thereof according to any one of claims 9 to 12, **characterized in** any one or more of the following i) to v):
i) Ar is phenyl, pyridyl, pyrimidinyl, furanyl, imidazolyl, pyrrolyl, thiazolyl, pyrazolyl or thienyl;
preferably, Ar is phenyl, furanyl, imidazolyl or thienyl;
preferably, Ar is phenyl;
ii) R is H or C₁₋₄ alkyl;
preferably, R is H, methyl, ethyl, n-propyl or n-butyl, preferably is H, methyl, ethyl or n-propyl, further preferably is H, methyl or ethyl, and more further preferably is H;
iii) R₂ andR₃ each independently are hydrogen, C₁₋₄ alkyl, fluoro, chloro, bromo, iodo, hydroxyl, nitro or cyano;
preferably, R₂ andR₃ each independently are hydrogen, methyl, ethyl, n-propyl, iso-propyl, n-butyl, isobutyl, sec-butyl, or tert-butyl; preferably, R₂ and R₃ each independently are fluoro, chloro, bromo, iodo, hydroxyl, nitro or cyano; preferably, R₂ and R₃ each independently are hydrogen;
iv) q is 0, 1 or 2, preferably is 0 or 1;
p is 0, 1 or 2, preferably is 0 or 1;
v) C is selected from the group consisting of: auristatin, monomethyl auristatin E (MMAE), monomethyl auristatin F (MMAF) and a derivative thereof 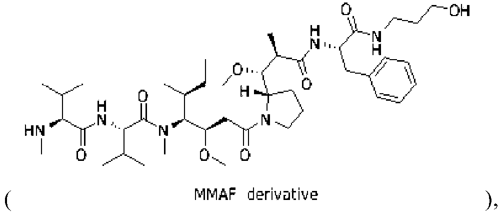 maytansine or a derivative thereof (such as maytansinoids, DM1, DM3, DM4), paclitaxel, calicheamicin, duocarmycin, doxorubicin, camptothecin, PBD (pyrrolobenzodiazepines) cytotoxin and a derivative thereof, SN-38, exatecan, doxorubicin, a TLR7 agonist with CAS No. 1821304-87-3, lapatinib, rifampicin and an analog thereof (for example, rifalogue, KRM-1657), preferably is monomethyl auristatin E (MMAE), SN-38, exatecan, doxorubicin, a TLR7 agonist with CAS No. 1821304-87-3, lapatinib, an MMAF derivative or rifalogue, further preferably is monomethyl auristatin E (MMAE), rifampicin or rifalogue, and more further preferably is monomethyl auristatin E (MMAE) or rifalogue;
preferably, the compound has a structure represented by Formula II-1, 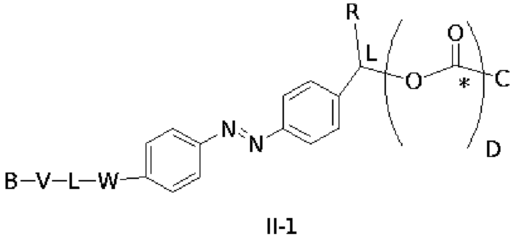
wherein B, V, L, W, R, C, and D are as defined in claims 9 to 13.

14. The compound, a geometric isomer, an optical isomer, a salt, a hydrate, a solvate, or a polymorph thereof according to claim 9, wherein
B is selected from the group consisting of 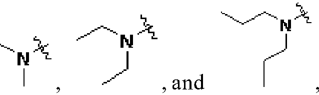 and V, L, W1, W2, and W3 each are absent;
R is 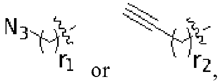 wherein r₁ and r₂ are as defined in claim 9;
preferably, r₁ and r₂ each independently are 1, 2, 3, 4, 5, 6, 7, 8, 9, 10, 11 or 12;
preferably, r₁ and r₂ each independently are 1, 2, 3, 4, 5, 6, 7, 8, 9 or 10;
preferably, r₁ and r₂ each independently are 1, 2, 3, 4, 5, 6, 7 or 8;
preferably, r₁ and r₂ each independently are 1, 2, 3, 4, 5 or 6;
preferably, r₁ and r₂ each independently are 1, 2, 3 or 4.

15. The compound, a geometric isomer, an optical isomer, a salt, a hydrate, a solvate, or a polymorph thereof according to claim 9, wherein the compound is selected from the group consisting of: 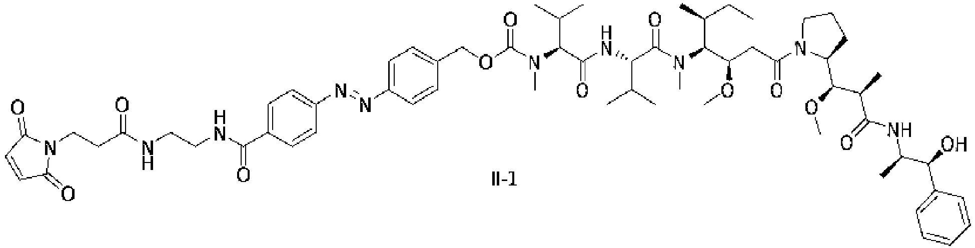 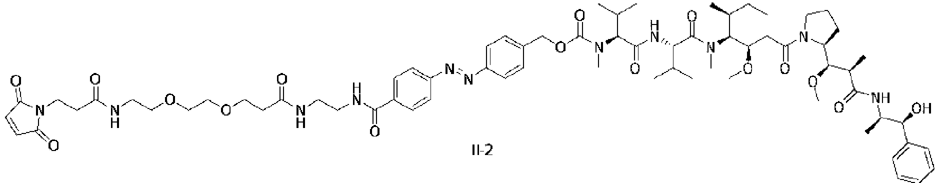 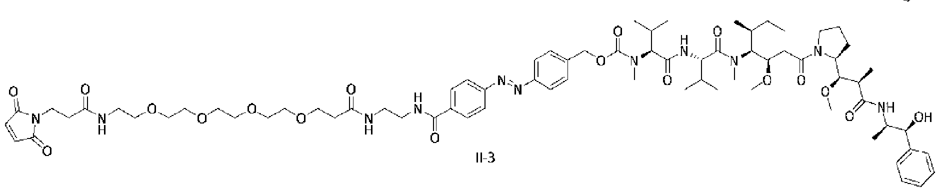 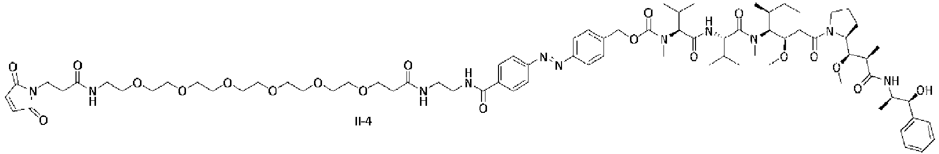 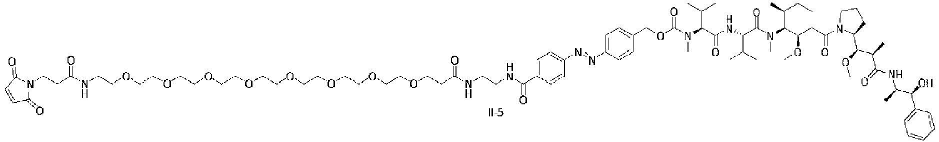 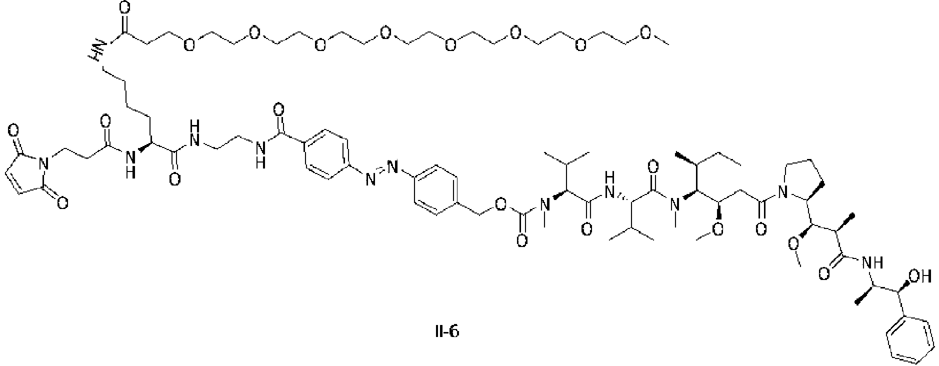 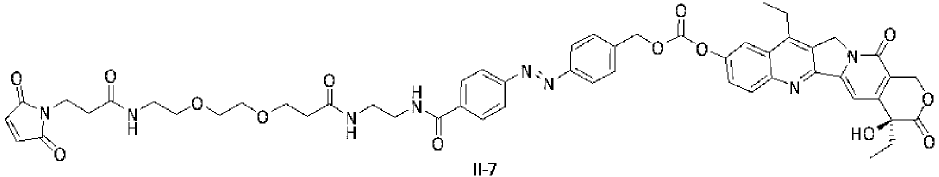 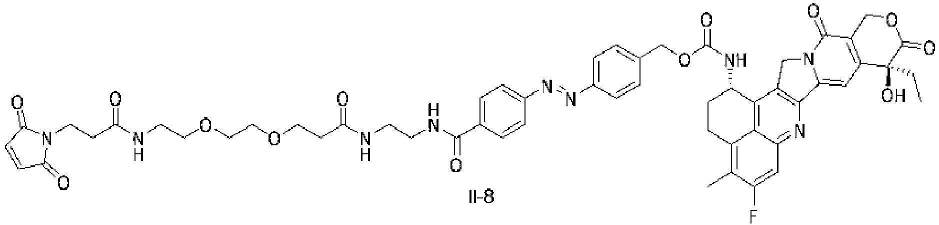 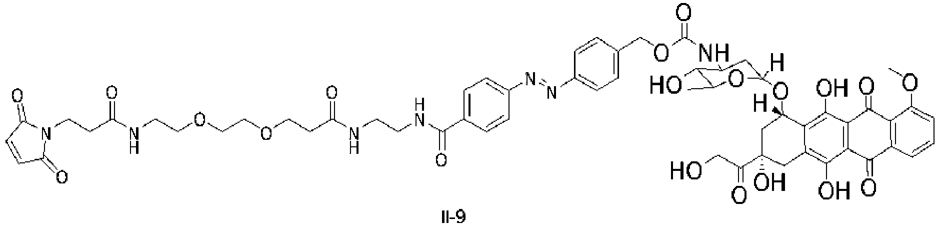 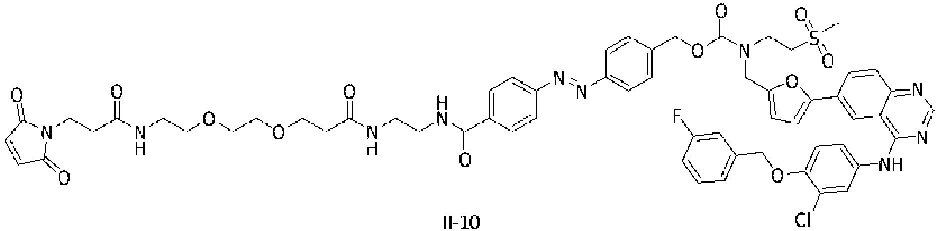 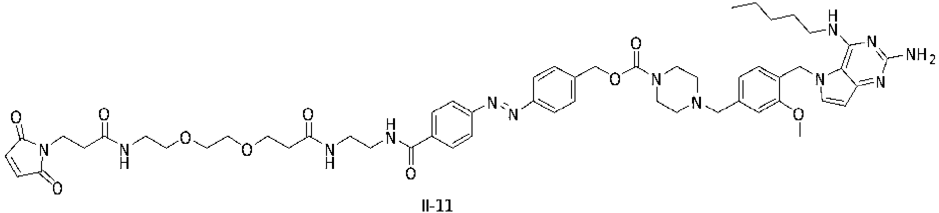 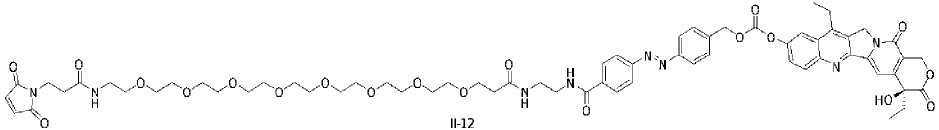 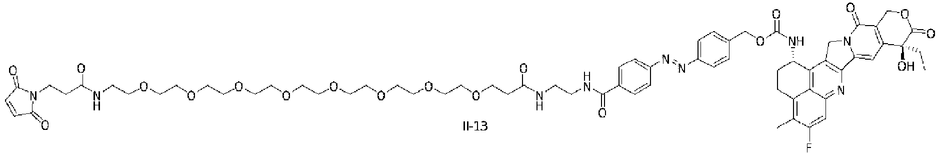 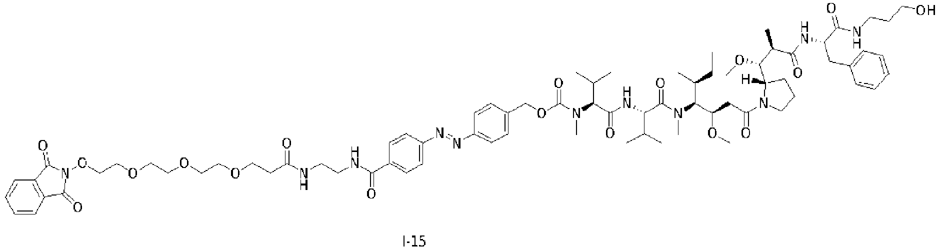 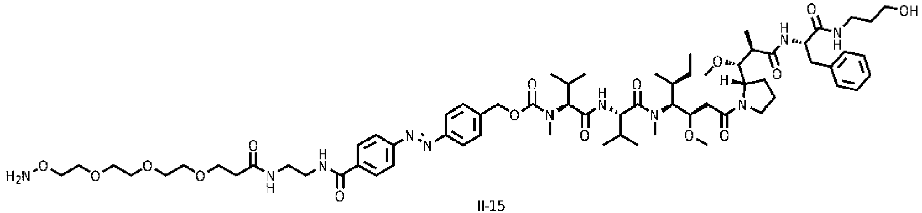 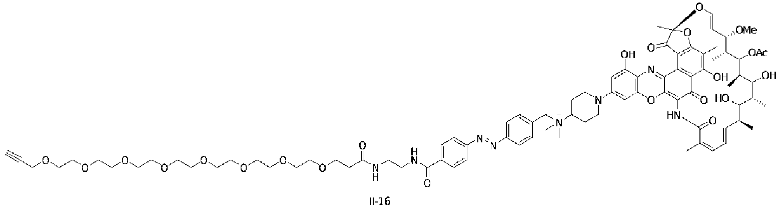 and

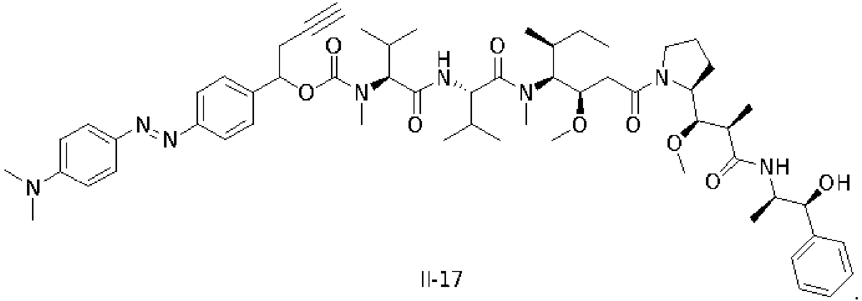

16. Use of the compound, a geometric isomer, an optical isomer, a salt, a hydrate, a solvate, or a polymorph thereof according to any one of claims 9 to 15 in the manufacture of an antibody-drug conjugate.

17. A compound represented by Formula III or Formula IV, a geometric isomer, an optical isomer, a pharmaceutically acceptable salt, a hydrate, a solvate or a polymorph thereof, 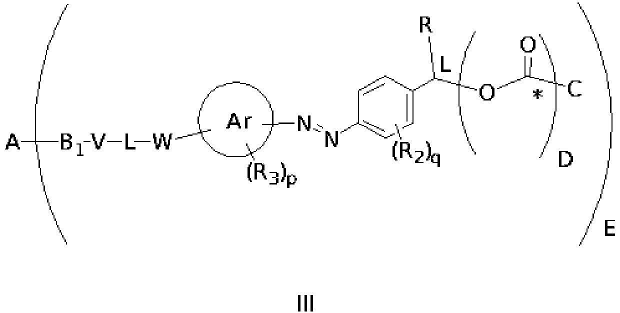 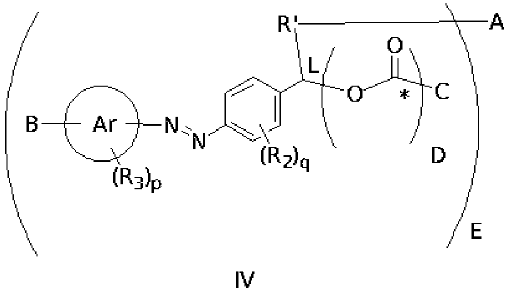
wherein, A represents a targeting compound; 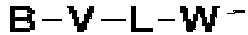 represents a linker, for conjugating an azobenzene group to a targeting compound,
Ar is a 5- or 6-membered aryl or heteroaryl;
R₂ and R₃ each independently are hydrogen, C₁₋₆ alkyl, fluoro, chloro, bromo, iodo, hydroxyl, nitro or cyano;
p is 0, 1, 2, or 3;
q is 0, 1, 2 or 3;
R is H, C₁₋₄ alkyl, 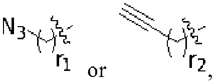 wherein r₁ and r₂ each independently are an integer from 1 to 12;
R' is 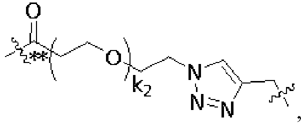 wherein k₂ is 1, 2, 3, 4, 5, 6, 7, 8, 9, 10, 11 or 12;
B is selected from the group consisting of 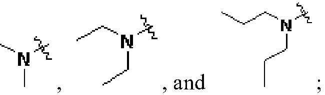
D is 0 or 1;
C is an active compound, selected from the group consisting of a drug, a cytotoxin, a detection reagent, a diagnostic reagent, and a targeting carrier; preferably, C is an antitumor drug, an antiviral drug, an antibiotic, an antibacterial drug or an immunomodulatory drug; further preferably, C is an antitumor drug, such as a a small molecule, a peptide or a nucleotide including a cytotoxin, a tubulin inhibitor, a DNA alkylating agent, a DNA intercalating agent, an RNA polymerase inhibitor, a kinase inhibitor, a topoisomerase inhibitor, a kinesin spindle protein inhibitor, and an antimetabolite drug, an immunomodulatory drug such as a Toll-like receptor agonist (e.g., a TLR7 agonist) or a STING agonist, antibacterial drugs such as rifampicin and analogs thereof (e.g., rifalogue, KRM-1657); C is conjugated to the carbonyl group (i.e. the position *) or the C atom at the position L via the N atom or the O atom in the active compound molecule;
E is a number from 1 to 20;
preferably, in 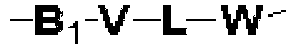
B₁ is 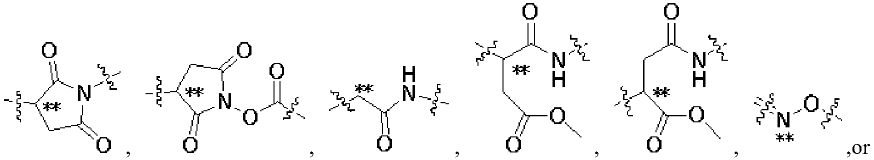 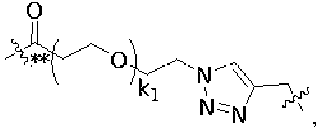 wherein k₁ is 1, 2, 3, 4, 5, 6, 7, 8, 9, 10, 11 or 12;
V is 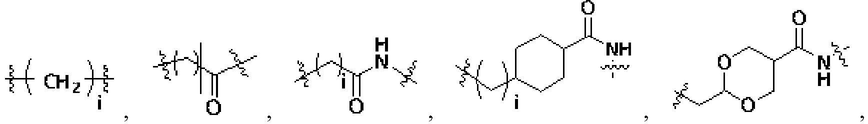 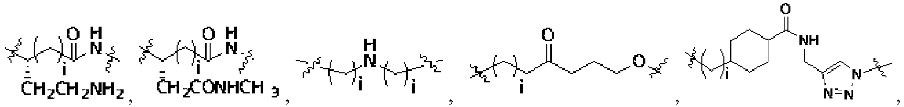 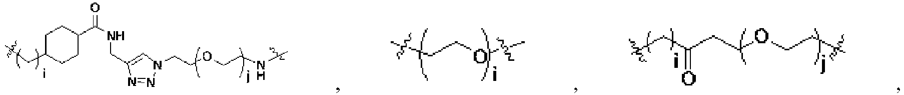 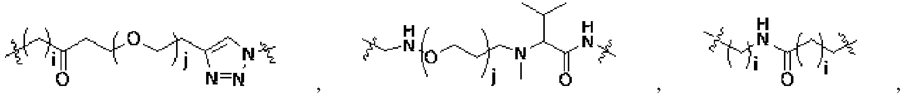 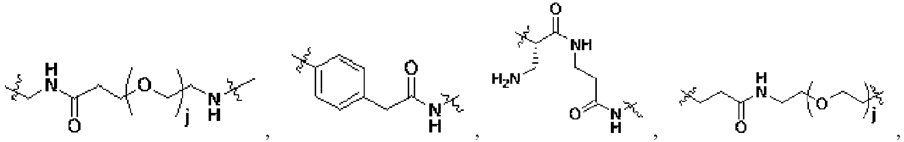 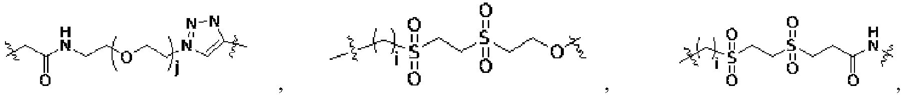 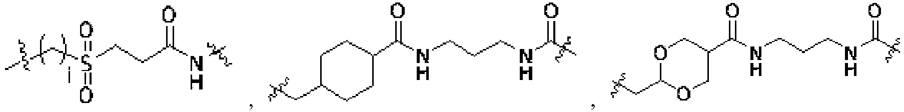 or absent, wherein each i independently is an integer from 0 to 6, and each j independently is an integer from 0 to 8;
L is -(CH₂CH₂O)ₖ-(CH₂)ₗ-, -CHR₁-, -(CH₂)ₘ -, 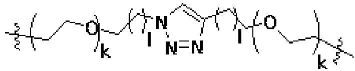 or absent, wherein R₁ is -(CH₂)ₙ-NHC(O)-(CH₂CH₂O)ₖ-CH₃, each k independently is an integer from 0 to 12, each l independently is an integer from 0 to 12, n is an integer from 0 to 12, and m is an integer from 0 to 30;
W is -W1-W2-W3-, wherein W1 is connected to L, W3 is connected to Ar, and W1 is selected from the group consisting of -O-, -S-, -NR₁₁-, -CONH-, -NHCO-, -S(O₂)NH-, and -NHS(O₂)-, or W1 is absent; W2 is selected from the group consisting of -(CH₂)ₒ-, and -[CH(R₁₁)CH(R₁₂)]ₒ-, or W2 is absent; W3 is selected from the group consisting of -O-, -S-, -NR₁₁-, -CONH-, -NHCO-, -S(O₂)NH-, and -NHS(O₂)-, or W3 is absent; wherein o is an integer from 0 to 6, each R₁₁ independently is selected from the group consisting of hydrogen and C₁₋₆ alkyl, and R₁₂ is selected from the group consisting of hydrogen and C₁₋₆ alkyl;
preferably, A is a targeting compound, selected from the group consisting of proteins, antibodies, polypeptides, enzymes and small molecules;
preferably, A is conjugated to the position ** of the group B₁ via the S atom or the N atom in the targeting compound molecules, or A is conjugated to the position ** of the group B₁ via oxime bond which formed by reacting a carbonyl group in the targeting compound molecule with the hydroxylamno group in the group B₁, or A is conjugated to the position** of the group R' via the N atom in the targeting compound molecule.

18. The compound, a geometric isomer, an optical isomer, a pharmaceutically acceptable salt, a hydrate, a solvate or a polymorph thereof according to claim17, wherein the compound is the compound represented by Formula III, wherein W is selected from the group consisting of -NH-CH₂-C(O)-NH-, -NH-CH₂-CH₂-C(O)-NH-, -C(O)-NH-, -NH-C(O)-, -C(O)-NH-CH₂-C(O)-NH-, -C(O)-NH-CH₂-CH₂-C(O)-NH-, -C(O)-NH-, -NH-C(O)-, -NH-CH(R₁₀)-C(O)-NH-, -C(O)-NH-(CH₂)ₒ-NH-C(O)-, - C(O)-NH-[CH(R₁₁)CH(R₁₂)]ₒ-NH-C(O)-, -NH-(CH₂)ₒ-NH-C(O)-, and -NH-[CH(R₁₁)CH(R₁₂)]ₒ-NH-C(O)-, wherein R₁₀ is H, methyl or ethyl, and o, R₁₁ and R₁₂ are as defined in claim 17.

19. The compound, a geometric isomer, an optical isomer, a pharmaceutically acceptable salt, a hydrate, a solvate or a polymorph thereof according to claim17 or 18, wherein the compound is a compound represented by Formula III, wherein
B₁ is 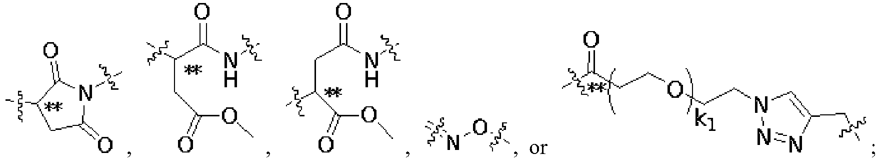 preferably, B₁ is 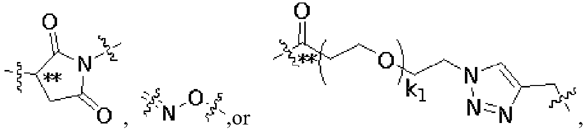 wherein k₁ is as defined in claim17;
preferably, k₁ is 1, 2, 3, 4, 5, 6, 7, 8, 9 or 10; further preferably, k₁ is 1, 2, 3, 4, 5, 6, 7 or 8; more further preferably, k₁ is 2, 3, 4, 5, 6, 7 or 8.

20. The compound, a geometric isomer, an optical isomer, a pharmaceutically acceptable salt, a hydrate, a solvate or a polymorph thereof according to any one of claims 17 to 19, wherein the compound is a compound represented by Formula III, and **characterized in** any one or more of the following i) to iii):
i) V is 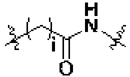 or absent, wherein i is as defined in claim 17;
ii) L is -(CH₂CH₂O)ₖ-(CH₂)ₗ-, -CHR₁- or -(CH₂)ₘ-, wherein k, l, R₁, and m are as defined in claim 17;
iii) W is -C(O)-NH-(CH₂)ₒ-NH-C(O)- or -C(O)-NH-[CH(R₁₁)CH(R₁₂)]ₒ-NH-C(O)-, and o, R₁₁ and R₁₂ are as defined in claim 17;
preferably, -V-L-W- is -(CH₂)ₘ-C(O)-NH-(CH₂)ₒ-NH-C(O)-, -(CH₂)ᵢ-C(O)-NH-(CH₂CH₂O)ₖ-(CH₂)ₗ-C(O)-NH-(CH₂)ₒ-NH-C(O)-, -(CH₂)ᵢ-C(O)-NH-CHR₁-C(O)-NH-(CH₂)ₒ-NH-C(O)-, or -(CH₂CH₂O)ₖ-(CH₂)ₗ-C(O)-NH-(CH₂)ₒ-NH-C(O)-, wherein R₁ is -(CH₂)ₙ-NHC(O)-(CH₂CH₂O)ₖ-CH₃, and m, o, i, k, l, and n are as defined in claim 17;
preferably, each i independently is 1, 2, 3 or 4; each k independently is 1, 2, 3, 4, 5, 6, 7, 8, 9, 10, 11 or 12; each m independently is 1, 2, 3 or 4; each o independently is 1, 2, 3 or 4; each l independently is 1, 2, 3 or 4; n is 1, 2, 3, 4, 5, 6, 7 or 8;
preferably, each i independently is 1, 2 or 3; each k independently is 1, 2, 3, 4, 5, 6, 7, 8, 9 or 10; each m independently is 1, 2 or 3; each o independently is 1, 2 or 3; each l independently is 1, 2 or 3; n is 1, 2, 3, 4 or 5;
preferably, -V-L-W- is -(CH₂)₂-C(O)-NH-(CH₂)₂-NH-C(O)-, -(CH₂)₂-C(O)NH-(CH₂CH₂O)₂-CH₂-CH₂-C(O)-NH-(CH₂)₂-NH-C(O)-, -(CH₂)₂-C(O)NH-(CH₂CH₂O)₄-CH₂-CH₂-C(O)-NH-(CH₂)₂-NH-C(O)-, -(CH₂)₂-C(O)NH-(CH₂CH₂O)₆-CH₂-CH₂-C(O)-NH-(CH₂)₂-NH-C(O)-, -(CH₂)₂-C(O)NH-(CH₂CH₂O)₈-CH₂-CH₂-C(O)-NH-(CH₂)₂-NH-C(O)-, -(CH₂CH₂O)₃-(CH₂)₂-C(O)-NH-(CH₂)₂-NH-C(O)-, -(CH₂)₂-C(O)NH-CH[(CH₂)₄-NHC(O)-(CH₂CH₂O)₈-CH₃]-C(O)-NH-(CH₂)₂-NH-C(O)-, or -(CH₂CH₂O)₇-(CH₂)₂-C(O)-NH-(CH₂)₂-NH-C(O)-.

21. The compound, a geometric isomer, an optical isomer, a pharmaceutically acceptable salt, a hydrate, a solvate or a polymorph thereof according to any one of claims 17 to 20, wherein the compound is a compound represented by Formula III, and **characterized in** any one or more of the following i) to vii):
i) Ar is phenyl, pyridyl, pyrimidinyl, furanyl, imidazolyl, pyrrolyl, thiazolyl, pyrazolyl or thienyl;
preferably, Ar is phenyl, furanyl, imidazolyl or thienyl;
preferably, Ar is phenyl;
ii) R is H or C₁₋₄ alkyl;
preferably, R is H, methyl, ethyl, n-propyl or n-butyl, preferably is H, methyl, ethyl or n-propyl, further preferably is H, methyl or ethyl, and more further preferably is H;
iii) R₂ and R₃ each independently are hydrogen, C₁₋₄ alkyl, fluoro, chloro, bromo, iodo, hydroxyl, nitro or cyano;
preferably, R₂ and R₃ each independently are hydrogen, methyl, ethyl, n-propyl, iso-propyl, n-butyl, isobutyl, sec-butyl, or tert-butyl; preferably, R₂ and R₃ each independently are fluoro, chloro, bromo, iodo, hydroxyl, nitro or cyano; preferably, R₂ and R₃ each independently are hydrogen;
iv) q is 0, 1 or 2, preferably is 0 or 1;
p is 0, 1 or 2, preferably is 0 or 1;
v) C is selected from the group consisting of: auristatin, monomethyl auristatin E (MMAE), monomethyl auristatin F (MMAF) and a derivative thereof 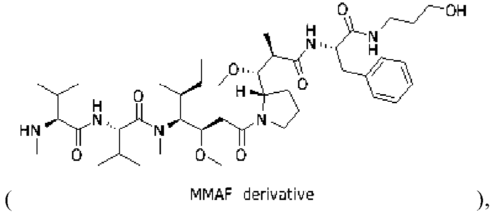 maytansine or and a derivative thereof (such as maytansinoids, DM1, DM3, DM4), paclitaxel, calicheamicin, duocarmycin, doxorubicin, camptothecin, PBD (pyrrolobenzodiazepines) cytotoxin and a derivative thereof, SN-38, exatecan), doxorubicin, a TLR7 agonist with CAS No. 1821304-87-3, lapatinib, rifampicin and an analog thereof (for example, rifalogue, KRM-1657), preferably is SN-38, exatecan, doxorubicin, a TLR7 agonist with CAS No. 1821304-87-3, lapatinib, monomethyl auristatin E (MMAE), an MMAF derivative or rifalogue, further preferably is monomethyl auristatin E (MMAE), rifampicin or rifalogue, and more further preferably is monomethyl auristatin E (MMAE) or rifalogue;
vi) A is an antibody, preferably is a monoclonal antibody (MAB), a bispecific antibody or a multispecific antibody, or
A comprises an antibody fragment or a substitute thereof or a variant thereof, a protein ligand or a protein scaffold;
preferably, A is a monoclonal antibody, bispecific antibody or multispecific antibody with a thiol or amino group as a conjugating site, or a site-directed mutated or modified monoclonal antibody, bispecific antibody or multispecific antibody with a thiol or amino group as a conjugating site, or a site-directed mutated monoclonal antibody, bispecific antibody or multispecific antibody with a carbonyl group in *p-*acetylphenylalanine (pAF) as a conjugating site,
preferably, A is selected from the group consisting of: humanized anti-HER2 monoclonal antibody mil40, trastuzumab (HERCEPTIN), pertuzumab (PERJETA), cetuximab (ERBITUX), panitumumab (VECTIBIX), rituximab (RITUXAN), alemtuzumab (CAMPATH), ibritumomab tiuxetan (ZEVALIN), tositumomab (BEXXAR), ofatumumab (ARZERRA), bevacizumab (AVASTIN), ipilimumab (YERVOY), denosumab (XGEVA), pembrolizumab (KEYTRUDA), nivolumab (Opdivo), avelumab (Bavencio), atezolizumab (Tecentriq),durvalumab (Imfinzi), sacituzumab, rovalpituzumab, and biological analogs thereof, and antibacterial antibody,
more preferably, Ais trastuzumab, or
A is albumin, preferably human serum albumin, or
A is an antibody, comprising:
(a) the following three heavy chain variable region (VH) complementarity determining regions (CDRs):
(i) VH CDR1, having a CDR1 sequence contained in the heavy chain as set forth in SEQ ID NO:1, or having a sequence with substitution, deletion or addition of one or several amino acids (for example, substitution, deletion or addition of one or two amino acids) as compared with the CDR1 sequence contained in the VH;
(ii) VH CDR2, having a CDR2 sequence contained in the heavy chain as set forth in SEQ ID NO:1, or having a sequence with substitution, deletion or addition of one or more amino acids (for example, substitution, deletion or addition of one or two amino acids) as compared with the CDR2 sequence contained in the VH; and
(iii) VH CDR3, having a CDR3 sequence contained in the heavy chain as set forth in SEQ ID NO:1, or having a sequence with substitution, deletion or addition of one or more amino acids (for example, substitution, deletion or addition of one or two amino acids) as compared with the CDR3 sequence contained in the VH;
and/or
(b) the following three light chain variable region (VL)CDRs:
(iv) VL CDR1, having a CDR1 sequence contained in the light chain as set forth in SEQ ID NO:2, or having a sequence having one or more amino acid substitutions, deletions or additions (for example, substitution, deletion or addition of one or two amino acids) as compared with the CDR1 sequence contained in the VL;
(v) VL CDR2, having a CDR2 sequence contained in the light chain as set forth in SEQ ID NO:2, or having a sequence with substitution, deletion or addition of one or several amino acids (for example, substitution, deletion or addition of one or two amino acids) as compared with the CDR2 sequence contained in the VL; and
(vi) VL CDR3, having a CDR3 sequence contained in the light chain as set forth in SEQ ID NO:2, or having a sequence with substitution, deletion or addition of one or several amino acids (for example, substitution, deletion or addition of one or two amino acids) as compared with the CDR3 sequence contained in the VL;
preferably, the substitution described in any one of (i) to (vi) is a conservative substitution;
preferably, the CDR1, CDR2 and CDR3 contained in the heavy chain variable region (VH), and/or the CDR1, CDR2 and CDR3 contained in the light chain variable region (VL) are defined by the Kabat, Chothia or IMGT numbering system;
preferably, the amino acid sequence of the heavy chain of the antibody is as set forth in SEQ ID NO:1, and the amino acid sequence of the light chain of the antibody is as set forth in SEQ ID NO:2; or
A is an antibody, comprising:
(a) the following three heavy chain variable region (VH) complementarity determining regions (CDRs):
(i) VH CDR1, having a CDR1 sequence contained in the heavy chain as set forth in SEQ ID NO:3, or having a sequence with substitution, deletion or addition of one or several amino acids (for example, substitution, deletion or addition of one or two amino acids) as compared with the CDR1 sequence contained in the VH;
(ii) VH CDR2, having a CDR2 sequence contained in the heavy chain as set forth in SEQ ID NO:3, or having a sequence with substitution, deletion or addition of one or more amino acids (for example, substitution, deletion or addition of one or two amino acids) as compared with the CDR2 sequence contained in the VH; and
(iii) VH CDR3, having a CDR3 sequence contained in the heavy chain as set forth in SEQ ID NO:3, or having a sequence with substitution, deletion or addition of one or more amino acids (for example, substitution, deletion or addition of one or two amino acids) as compared with the CDR3 sequence contained in the VH;
and/or
(b) the following three light chain variable region (VL)CDRs:
(iv) VL CDR1, having a CDR1 sequence contained in the light chain as set forth in SEQ ID NO:4, or having a sequence having one or more amino acid substitutions, deletions or additions (for example, substitution, deletion or addition of one or two amino acids) as compared with the CDR1 sequence contained in the VL;
(v) VL CDR2, having a CDR2 sequence contained in the light chain as set forth in SEQ ID NO:4, or having a sequence with substitution, deletion or addition of one or several amino acids (for example, substitution, deletion or addition of one or two amino acids) as compared with the CDR2 sequence contained in the VL; and
(vi) VL CDR3, having a CDR3 sequence contained in the light chain as set forth in SEQ ID NO:4 or having a sequence with substitution, deletion or addition of one or several amino acids (for example, substitution, deletion or addition of one or two amino acids) as compared with the CDR3 sequence contained in the VL.
preferably, the substitution described in any one of (i) to (vi) is a conservative substitution;
preferably, the CDR1, CDR2 and CDR3 contained in the heavy chain variable region (VH), and/or the CDR1, CDR2 and CDR3 contained in the light chain variable region (VL) are defined by the Kabat, Chothia or IMGT numbering system;
preferably, the amino acid sequence of the heavy chain of the antibody is as set forth in SEQ ID NO:3, and the amino acid sequence of the light chain of the antibody is as set forth in SEQ ID NO:4;
vii) E is a number from 1 to 15, preferably is a number from 1 to 10, further preferably is a number from 1 to 8, such as about 1, about 2, about 3, about 4, about 5, about 6, about 7, about 8.

22. The compound, a geometric isomer, an optical isomer, a pharmaceutically acceptable salt, a hydrate, a solvate or a polymorph thereof according to any one of claims 17 to 21, wherein the compound has a structure represented by FormulaIII-1, Formula III-2 or Formula III-3, 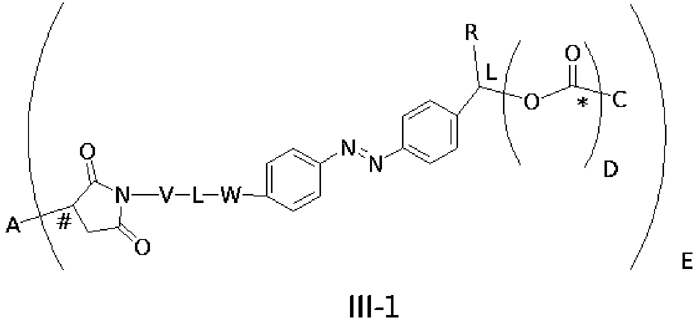 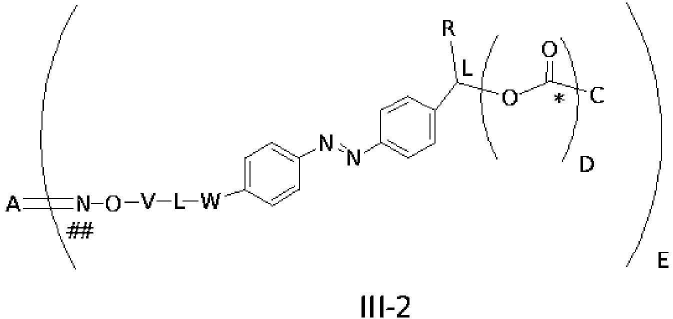 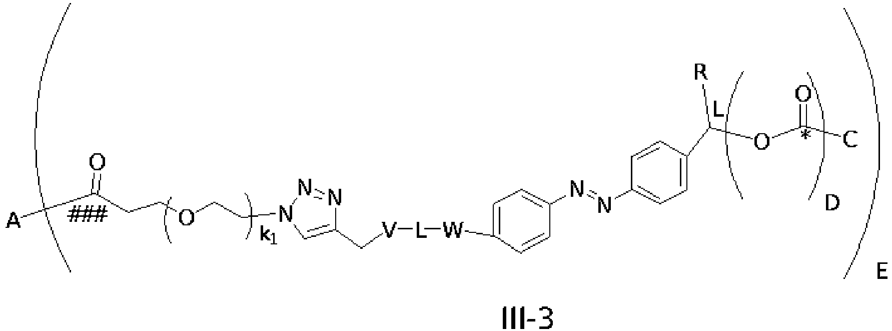
wherein A, k₁, V, L, W, R, C, D, and E are as defined in any one of claims 17 to 21,
preferably, A is conjugated to the position # via the S atom in the targeting compound molecule, or A is conjugated to the position ## via the N atom in the targeting compound molecule, or A is conjugated to the position ### via the carbonyl group in the targeting compound molecule.

23. The compound, a geometric isomer, an optical isomer, a pharmaceutically acceptable salt, a hydrate, a solvate or a polymorph thereof according to claim 17, wherein the compound is a compound represented by Formula IV, and **characterized in** any one or more of the following i) to vi):
i) Ar is phenyl, pyridyl, pyrimidinyl, furanyl, imidazolyl, pyrrolyl, thiazolyl, pyrazolyl or thienyl;
preferably, Ar is phenyl, furanyl, imidazolyl or thienyl;
preferably, Ar is phenyl;
ii) R₂ and R₃ each independently are hydrogen, C₁₋₄ alkyl, fluoro, chloro, bromo, iodo, hydroxyl, nitro or cyano;
preferably, R₂ and R₃ each independently are hydrogen, methyl, ethyl, n-propyl, iso-propyl, n-butyl, isobutyl, sec-butyl, or tert-butyl; preferably, R₂ and R₃ each independently are fluoro, chloro, bromo, iodo, hydroxyl, nitro or cyano; preferably, R₂ and R₃ each independently are hydrogen;
iii) q is 0, 1 or 2, preferably is 0 or 1;
p is 0, 1 or 2, preferably is 0 or 1;
iv) C is selected from the group consisting of: auristatin, monomethyl auristatin E (MMAE), monomethyl auristatin F (MMAF) and a derivative thereof 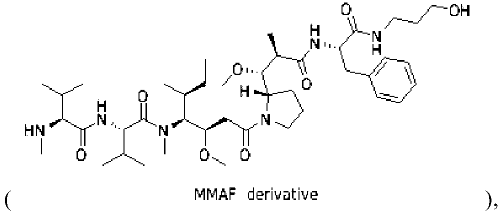 maytansine or a derivative thereof (such as maytansinoids, DM1, DM3, DM4), paclitaxel, calicheamicin, duocarmycin, doxorubicin, camptothecin, PBD (pyrrolobenzodiazepines) cytotoxin and a derivative thereof, SN-38, exatecan, doxorubicin, a TLR7 agonist with CAS No. 1821304-87-3, lapatinib, rifampicin and an analog thereof (for example, rifalogue, KRM-1657), preferably is SN-38, exatecan, doxorubicin, a TLR7 agonist with CAS No. 1821304-87-3, lapatinib, monomethyl auristatin E (MMAE), an MMAF derivative or rifalogue, further preferably is monomethyl auristatin E (MMAE), rifampicin or rifalogue; more further preferably is monomethyl auristatin E (MMAE) or rifalogue;
v) A is an antibody, preferably a monoclonal antibody (MAB), a bispecific antibody or a multispecific antibody, or
A contains an antibody fragment or a substitute thereof or a variant thereof, a protein ligand or a protein scaffold;
preferably, A is a monoclonal antibody, bi-specific antibody or multispecific antibody with an amino or thiol group as a conjugating site, or a site-directed mutated or modified monoclonal antibody, bispecific antibody or multispecific antibody with an amino or thiol group as a conjugating site,
preferably, A is selected from the group consisting of: humanized anti-HER2 monoclonal antibody mil40, trastuzumab (HERCEPTIN), pertuzumab (PERJETA), cetuximab (ERBITUX), panitumumab (VECTIBIX), rituximab (RITUXAN), alemtuzumab (CAMPATH), ibritumomab tiuxetan (ZEVALIN), tositumomab (BEXXAR), ofatumumab (ARZERRA), bevacizumab (AVASTIN), ipilimumab (YERVOY), denosumab (XGEVA), pembrolizumab (KEYTRUDA), nivolumab (Opdivo), avelumab (Bavencio), atezolizumab (Tecentriq), durvalumab (Imfinzi), sacituzumab, rovalpituzumab, and biological analogs thereof, and antibacterial antibody,
more preferably, Ais trastuzumab, or
A is albumin, preferably is human serum albumin, or
A is an antibacterial antibody, or
A is an antibody, comprising:
(a) the following three heavy chain variable region (VH) complementarity determining regions (CDRs):
(i) VH CDR1, having a CDR1 sequence contained in the heavy chain as set forth in SEQ ID NO:1, or having a sequence with substitution, deletion or addition of one or several amino acids (for example, substitution, deletion or addition of one or two amino acids) as compared with the CDR1 sequence contained in the VH;
(ii) VH CDR2, having a CDR2 sequence contained in the heavy chain as set forth in SEQ ID NO:1, or having a sequence with substitution, deletion or addition of one or more amino acids (for example, substitution, deletion or addition of one or two amino acids) as compared with the CDR2 sequence contained in the VH; and
(iii) VH CDR3, having a CDR3 sequence contained in the heavy chain as set forth in SEQ ID NO:1, or having a sequence with substitution, deletion or addition of one or more amino acids (for example, substitution, deletion or addition of one or two amino acids) as compared with the CDR3 sequence contained in the VH;
and/or
(b) the following three light chain variable region (VL)CDRs:
(iv) VL CDR1, having a CDR1 sequence contained in the light chain as set forth in SEQ ID NO:2, or having a sequence having one or more amino acid substitutions, deletions or additions (for example, substitution, deletion or addition of one or two amino acids) as compared with the CDR1 sequence contained in the VL;
(v) VL CDR2, having a CDR2 sequence contained in the light chain as set forth in SEQ ID NO:2, or having a sequence with substitution, deletion or addition of one or several amino acids (for example, substitution, deletion or addition of one or two amino acids) as compared with the CDR2 sequence contained in the VL; and
(vi) VL CDR3, having a CDR3 sequence contained in the light chain as set forth in SEQ ID NO:2, or having a sequence with substitution, deletion or addition of one or several amino acids (for example, substitution, deletion or addition of one or two amino acids) as compared with the CDR3 sequence contained in the VL;
preferably, the substitution described in any one of (i) to (vi) is a conservative substitution;
preferably, the CDR1, CDR2 and CDR3 contained in the heavy chain variable region (VH), and/or the CDR1, CDR2 and CDR3 contained in the light chain variable region (VL) are defined by the Kabat, Chothia or IMGT numbering system;
preferably, the amino acid sequence of the heavy chain of the antibody is as set forth in SEQ ID NO:1, and the amino acid sequence of the light chain of the antibody is as set forth in SEQ ID NO:2; or
A is an antibody, comprising:
(a) the following three heavy chain variable region (VH) complementarity determining regions (CDRs):
(i) VH CDR1, having a CDR1 sequence contained in the heavy chain as set forth in SEQ ID NO:3, or having a sequence with substitution, deletion or addition of one or several amino acids (for example, substitution, deletion or addition of one or two amino acids) as compared with the CDR1 sequence contained in the VH;
(ii) VH CDR2, having a CDR2 sequence contained in the heavy chain as set forth in SEQ ID NO:3, or having a sequence with substitution, deletion or addition of one or more amino acids (for example, substitution, deletion or addition of one or two amino acids) as compared with the CDR2 sequence contained in the VH; and
(iii) VH CDR3, having a CDR3 sequence contained in the heavy chain as set forth in SEQ ID NO:3, or having a sequence with substitution, deletion or addition of one or more amino acids (for example, substitution, deletion or addition of one or two amino acids) as compared with the CDR3 sequence contained in the VH;
and/or
(b) the following three light chain variable region (VL)CDRs:
(iv) VL CDR1, having a CDR1 sequence contained in the light chain as set forth in SEQ ID NO:4, or having a sequence having one or more amino acid substitutions, deletions or additions (for example, substitution, deletion or addition of one or two amino acids) as compared with the CDR1 sequence contained in the VL;
(v) VL CDR2, having a CDR2 sequence contained in the light chain as set forth in SEQ ID NO:4, or having a sequence with substitution, deletion or addition of one or several amino acids (for example, substitution, deletion or addition of one or two amino acids) as compared with the CDR2 sequence contained in the VL; and
(vi) VL CDR3, having a CDR3 sequence contained in the light chain as set forth in SEQ ID NO:4 or having a sequence with substitution, deletion or addition of one or several amino acids (for example, substitution, deletion or addition of one or two amino acids) as compared with the CDR3 sequence contained in the VL;
preferably, the substitution described in any one of (i) to (vi) is a conservative substitution;
preferably, the CDR1, CDR2 and CDR3 contained in the heavy chain variable region (VH), and/or the CDR1, CDR2 and CDR3 contained in the light chain variable region (VL) are defined by the Kabat, Chothia or IMGT numbering system;
preferably, the amino acid sequence of the heavy chain of the antibody is as set forth in SEQ ID NO:3, and the amino acid sequence of the light chain of the antibody is as set forth in SEQ ID NO:4;
vi)E is a number from 1 to 15, preferably is a number from 1 to 10, and further preferably is a number from 1 to 8, such as about 1, about 2, about 3, about 4, about 5, about 6, about 7, about 8,
preferably, wherein the compound has a structure represented by Formula IV-1, 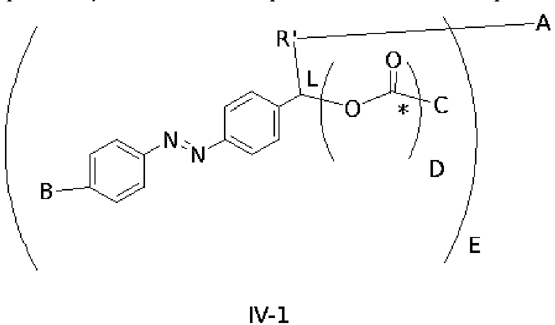
wherein, B, R', C, D, E, and A are as above defined,
preferably, k₂ is 1, 2, 3, 4, 5, 6, 7, 8, 9 or 10; preferably, k₂ is 1, 2, 3, 4, 5, 6, 7 or 8.

24. The compound, a geometric isomer, an optical isomer, a pharmaceutically acceptable salt, a hydrate, a solvate or a polymorph thereof according to claim 17, wherein the compound is selected from the group consisting of: 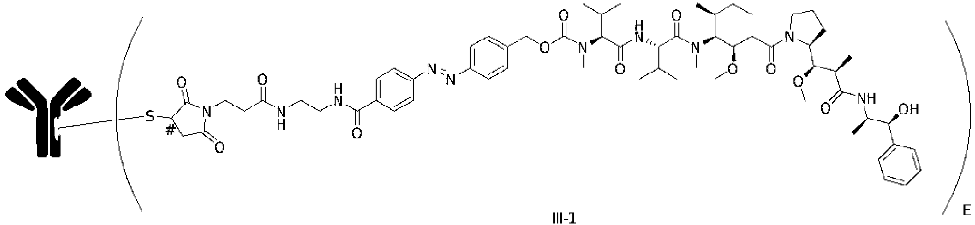 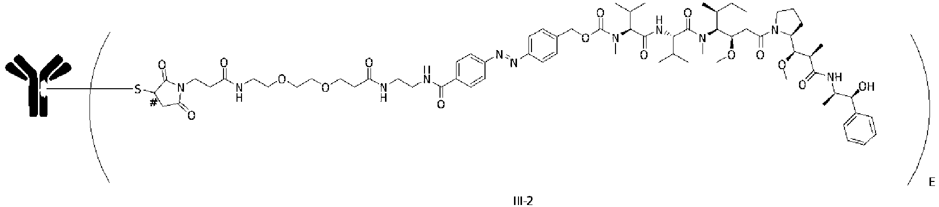 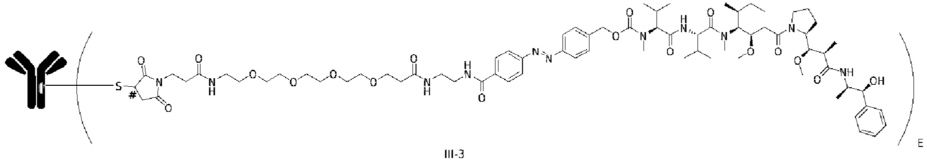 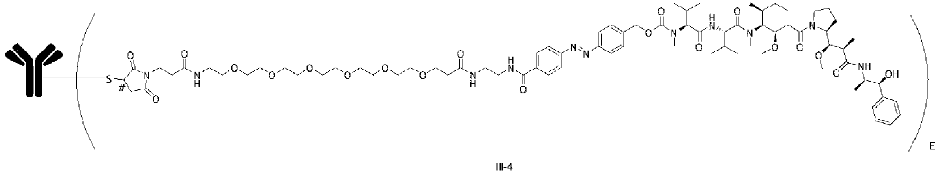 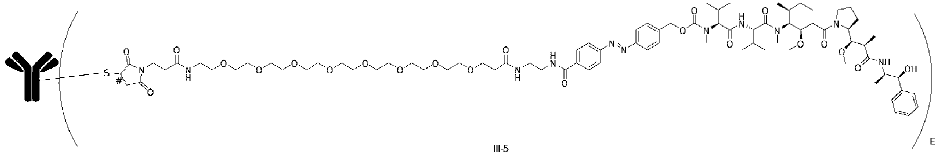 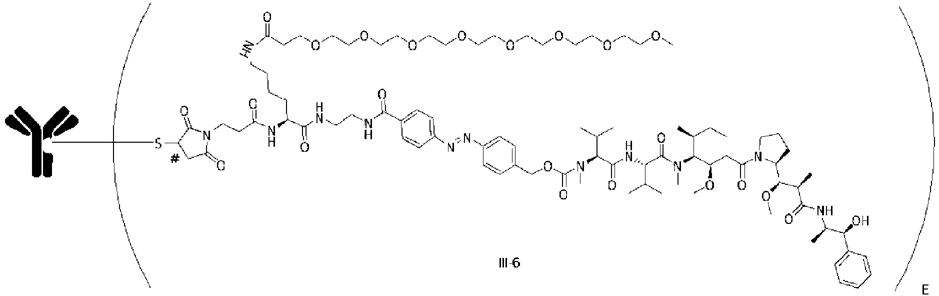 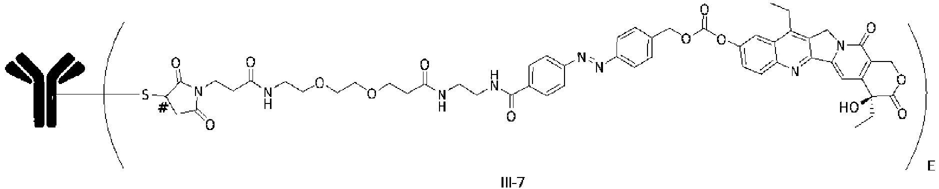 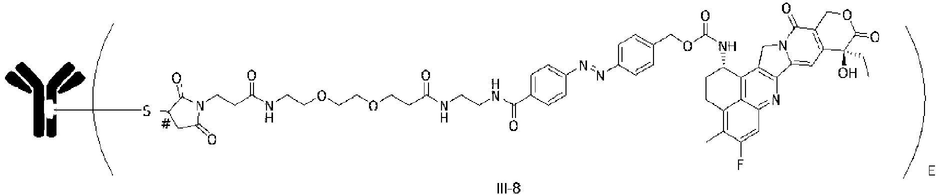 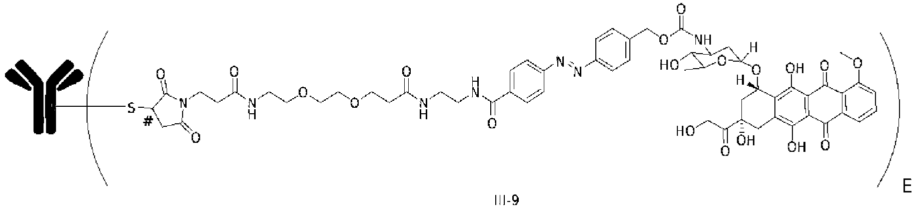 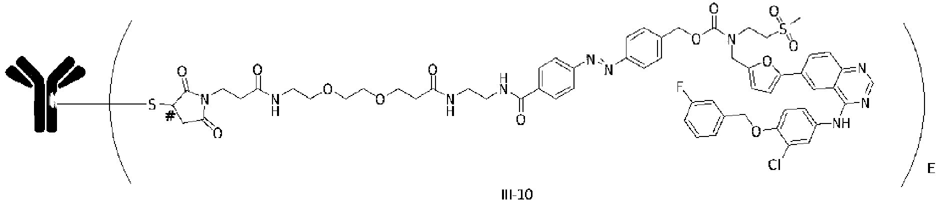 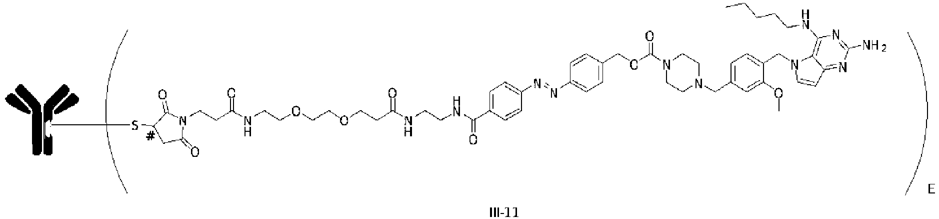 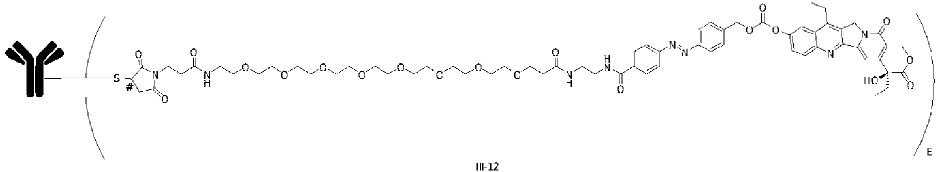 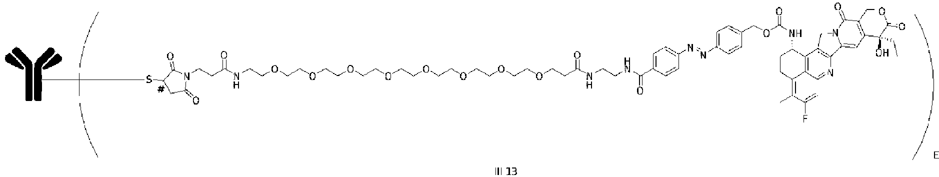 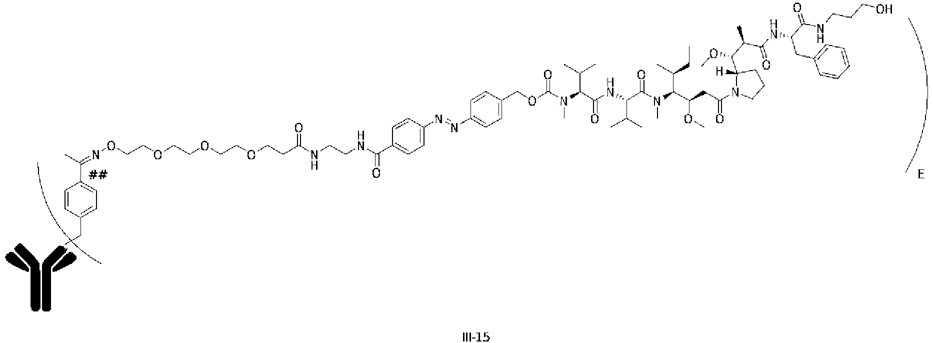 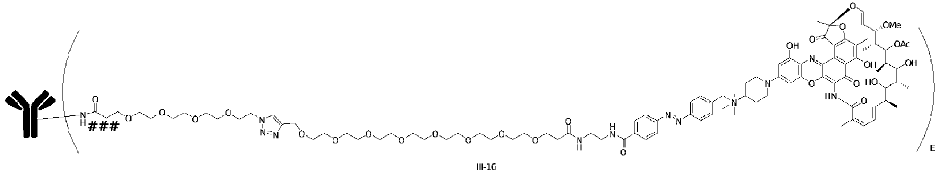 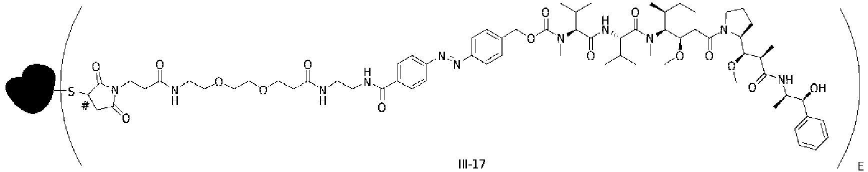 and 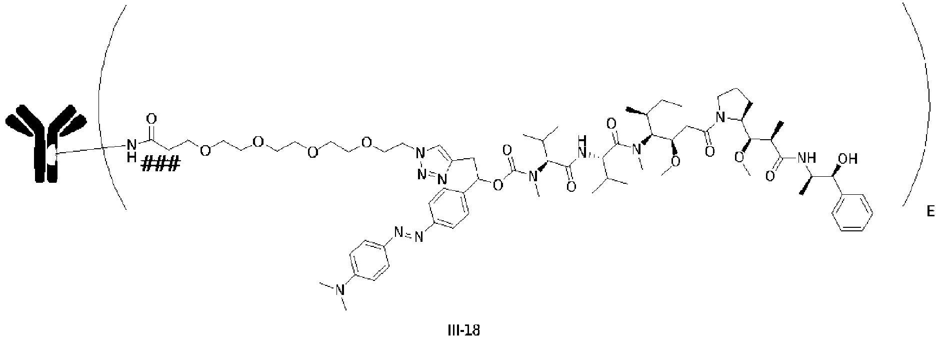
wherein, 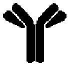 represents A, and A and E are as defined in claim 17,
preferably, A is an antibody, preferably a monoclonal antibody (MAB), a bispecific antibody or a multispecific antibody, and further preferably is trastuzumab, an antibody site-specifically mutated with an unnatural amino acid or an antibacterial antibody,
preferably, A is an antibody, which is connected to the position # via the S atom in the antibody molecule or connected to the position ## via the carbonyl group in the antibody molecule or connected to the position ### via the N atom in the antibody molecule, 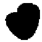 is a protein, preferably human serum albumin, the albumin being connected to the position # via the S atom in the albumin molecule.

25. A pharmaceutical composition comprising the compound, a geometric isomer, an optical isomer, a pharmaceutically acceptable salt, a hydrate, a solvate or a polymorph thereof according to any one of claims 17 to 24, and optionally one or more pharmaceutically acceptable carriers or excipients,
preferably, the pharmaceutical composition further comprises an additional drug,
preferably, the additional drug comprises one or more other antibiotics (for example, antibiotics useful for MRSA infections) such as vancomycin, co-trimoxazole, tetracycline, doxycycline/minocycline, clindamycin, cephalosporins (for example, cephalexin), nafcillin (naficillin), fidaxomicin, linezolid, and/or any other suitable antibiotic.

26. Use of the compound, a geometric isomer, an optical isomer, a pharmaceutically acceptable salt, a hydrate, a solvate or a polymorph thereof according to any one of claims 17 to 24 in the manufacture of a medicament for treating a disease or condition or alleviating the severity of the disease or condition, wherein the disease or condition is selected from the group consisting of a tumor, an infectious disease, a hematological disease, a metabolic disease, and an inflammation,
preferably, the tumor is selected from the group consisting of cancer, lymphoma, lymphoid neoplasm, blastoma, sarcoma, and leukemia,
preferably, the cancer is selected from the group consisting of: breast cancer (e.g., HER2 positive breast cancer); squamous cell carcinoma (e.g., epithelial squamous cell carcinoma); lung cancer, including small cell lung cancer, non-small cell lung cancer, lung adenocarcinoma and lung squamous cell carcinoma; peritoneal cancer; liver cancer; gastric cancer; gastrointestinal cancer; pancreatic adenocarcinoma; glioblastoma; cervical cancer; ovarian cancer; bladder cancer; urethral carcinoma; hepatocellular carcinoma; intestinal cancer; colon cancer; colorectal cancer; endometrial cancer; uterine cancer; salivary gland cancer; renal cancer; prostate cancer; vulvar cancer; thyroid cancer; anal cancer; penile cancer; melanoma; multiple myeloma and B-cell lymphoma; brain cancer; gallbladder cancer; esophageal cancer; cholangiocarcinoma; head and neck cancer and associated metastatic tumors,
preferably, the infectious disease includes bacterial infections such as infections caused by *Staphylococcus aureus*, *Mycobacterium tuberculosis, Enterococcus faecium, Acinetobacter baumannii, Clostridium difficile, Streptococcus pneumoniae, and Pseudomonas aeruginosa*, infection-caused sepsis, tuberculosis or bacterial ocular infections, infections of heart, brain or skin, gastrointestinal tract infections, bacterial meningitis, and abscesses in any organ (such as muscle, liver, meninges, or lung),
preferably, the infectious disease is cellulitis, bacteremia, skin necrosis, eyelid infection, ocular infection, neonatal conjunctivitis, osteomyelitis, impetigo, ecthyma, scalded skin syndrome, food poisoning, pneumonia, surgical infection, urinary tract infection, burn infection, meningitis, endocarditis, sepsis, toxic shock syndrome, septic arthritis, tuberculosis, prosthetic joint-associated infections, catheter-associated infection, or implant-associated infection,
preferably, the infectious disease is periprosthetic joint infection caused by *Staphylococcus aureus* ,
preferably, the infectious disease is *Staphylococcus aureus* infection of catheter and/or tissue surrounding the catheter,
preferably, the infectious disease is *Staphylococcus aureus* infection of foreign body and/or tissue surrounding the foreign body,
preferably, the infectious disease is tuberculosis.
